# EUROPÄISCHE PATENTSCHRIFT

(11) **EP 2 731 933 B1**
(45) Veröffentlichungstag und Bekanntmachung des Hinweises auf die Patenterteilung: **30.09.2015**
(21) Anmeldenummer: 12740097.6
(22) Anmeldetag: 11.07.2012
(51) Int. Cl.: C07D 231/12, C07D 231/16, C07D 231/20, C07D 261/08, C07D 319/06, C07C 255/62, C07C 255/66, C07C 325/02

(54) **2,3-DIPHENYL-VALERONITRILDERIVATE, VERFAHREN ZU DEREN HERSTELLUNG UND DEREN VERWENDUNG ALS HERBIZIDE UND PFLANZENWACHSTUMSREGULATOREN**
2,3-DIPHENYL-VALERONITRILE DERIVATIVES, METHOD FOR THE PRODUCTION THEREOF AND USE THEREOF AS HERBICIDES AND PLANT GROWTH REGULATORS
DÉRIVÉS DE 2,3-DIPHÉNYL-VALÉRONITRILE, LEURS PROCÉDÉS DE PRÉPARATION ET LEUR UTILISATION COMME HERBICIDES ET RÉGULATEURS DE CROISSANCE DE PLANTES

(30) Priorität: 15.07.2011 DE 102011079241; 15.07.2011 US 201161508306 P
(43) Veröffentlichungstag der Anmeldung: 21.05.2014
(73) Patentinhaber: Bayer Intellectual Property GmbH, 40789 Monheim (DE)
(72) Erfinder: MOSRIN, Marc, 65933 Frankfurt am Main (DE); JAKOBI, Harald, 60598 Frankfurt (DE); ANGERMANN, Alfred, 65830 Kriftel (DE); GATZWEILER, Elmar, 61231 Bad Nauheim (DE); HÄUSER-HAHN, Isolde, 51375 Leverkusen (DE); HEINEMANN, Ines, 65719 Hofheim (DE); ROSINGER, Christopher Hugh, 65719 Hofheim (DE); LEHR, Stefan, 69006 Lyon (FR); SCHNATTERER, Stefan, 65795 Hattersheim (DE)
(74) Vertreter: BIP Patents
(86) Internationale Anmeldenummer: PCT/EP2012/063613
(87) Internationale Veröffentlichungsnummer: WO 2013/010882

(56) Entgegenhaltungen:
- WO-A1-2011/042378
- WO-A1-2011/073143
- WO-A2-2011/003776

## Beschreibung

Die Erfindung betrifft das technische Gebiet der Herbizide und Pflanzenwachstumsregulatoren, beispielsweise der Herbizide zur Bekämpfung von Unkräutern und Ungräsern, gegebenfalls in Nutzpflanzenkulturen, oder der Pflanzenwachstumsregulatoren, welche zur Beeinflussung des Wachstums von Kulturpflanzen eingesetzt werden können.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Aus den veröffentlichten Patentanmeldungen EP-A-5341, EP-A-266725 (US 4,224,052), EP-A-270830, JP-04/297454, JP-04/297455, JP-05/058979, WO 2011/003775, WO 2011/003776, WO 2011/042378 und WO 2011/073143 sind herbizide Cyanobutyrate bekannt.

EP-A-5341 beschreibt herbizide Ester und Amide von 4-Cyano-3,4-diphenyl-butansäuren, die an den Phenylresten gegebenfalls substitutiert sind. Gemäß EP-A-5341 eignen sich die threo-Isomere allgemein zur nicht-selektiven Bekämpfung von Schadpflanzen, während die erythro-threo-Isomerengemische für die selektive Bekämpfung von Schadpflanzen in einigen Nutzpflanzenkulturen in Betracht kommen. In EP-A-5341 wird außerdem erwähnt, dass die 2 Enantiomere, die zur threo-Form gehören, unterschiedlich wirksam sind, was am Beispiel der unterschiedlichen Wirkungen der Enantiomeren des Enantiomerenpaares der in den Phenylresten unsubstituierten 4-Cyano-3,4-diphenyl-butansäure untersucht worden ist.

Aus EP-A-266725 sind einige erythro-threo-Isomerengemische bekannt, die selektiv zur Unkrautkontrolle in Reiskulturen eingesetzt werden können.

EP-A-270830 beschreibt, dass threo-Isomere und erythro-threo-Isomerengemische als Pflanzenregulatoren verwendet werden können und die Ausbildung eines Fruchtstands bei verschiedenen Schadgräsern verhindern können.

Aus WO 2011/003775 sind spezielle Ester von 4-Cyano-3,4-diphenyl-butansäuren bekannt, die als wirksame Herbizide, vorzugsweise auch in Nutzpflanzenkulturen eingesetzt werden können.

Aus WO 2011/003776, WO 2011/042378 und WO 2011/073143 sind 4-Cyano-3,4-diphenyl-butansäuren und -säureester bekannt, die an den Phenylresten spezifisch substituiert sind und als wirksame Herbizide, vorzugsweise auch in Nutzpflanzenkulturen eingesetzt werden können.

Die herbizide Wirkung der bekannten Verbindungen der genannten Stoffklasse, insbesondere bei niedrigen Aufwandmengen, bzw. deren Verträglichkeit gegenüber Kulturpflanzen bleiben verbesserungswürdig.

Aus den genannten Gründen besteht weiterhin ein Bedarf nach alternativen wirkungsstarken Herbiziden für die selektive Anwendung in Pflanzenkulturen oder die Anwendung auf Nichtkulturland. Auch ist es wünschenswert, alternative chemische Wirkstoffe bereitzustellen, die gegebenenfalls mit Vorteilen als Herbizide öder Pflanzenwachstumsregulatoren eingesetzt werden können.

Eine Aufgabe der vorliegenden Erfindung ist daher die Bereitstellung von Verbindungen mit herbizider Wirkung, die bereits bei relativ niedrigen Aufwandmengen gegen wirtschaftlich wichtige Schadpflanzen hochwirksam sind und vorzugsweise bei guter Wirksamkeit gegen Schadpflanzen selektiv in Kulturpflanzen eingesetzt werden können.

Überraschenderweise wurde nun gefunden, dass gegenüber den Wirkstoffen der genannten Cyanobutyrate an der Säure- bzw. Estergruppe strukturell weiter modifizierte Verbindungen, welche zur Gruppe der 2,3-Diphenyl-valeronitrilderivate gehören, besondere herbizide Wirkungen aufweisen und vorzugsweise in einigen Kulturen selektiv zur Schadpflanzenkontrolle eingesetzt werden können.

Gegenstand der vorliegenden Erfindung sind Verbindungen der Formel (I) oder deren Salze, 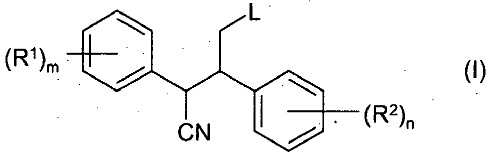 in welcher
- L: einen Rest der Formel 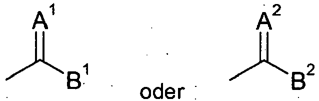 bedeutet,
- A¹: Sauerstoff, Schwefel oder =N-R^{A} bedeutet,
- B¹: einen Rest der Formeln (B1) bis (B14) bedeutet, 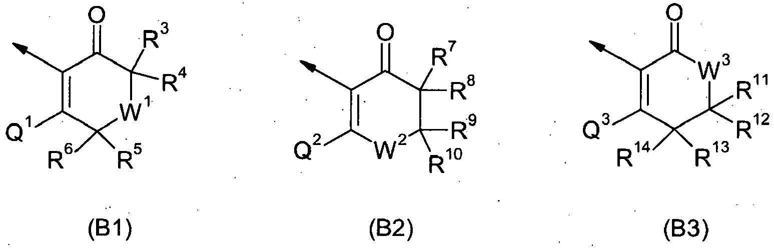 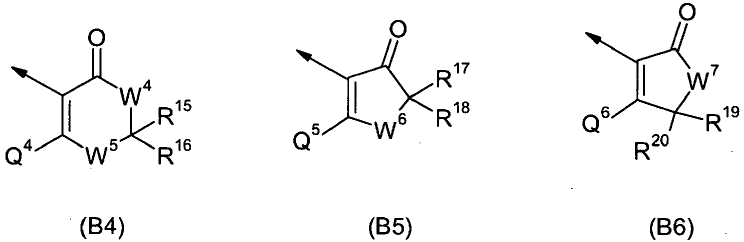 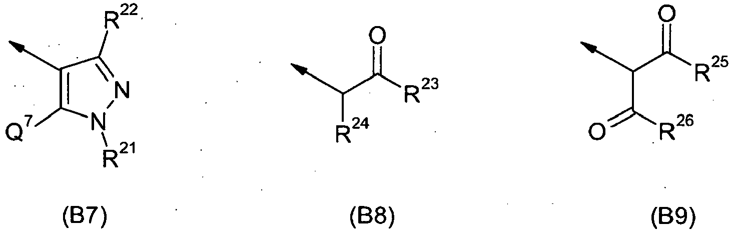 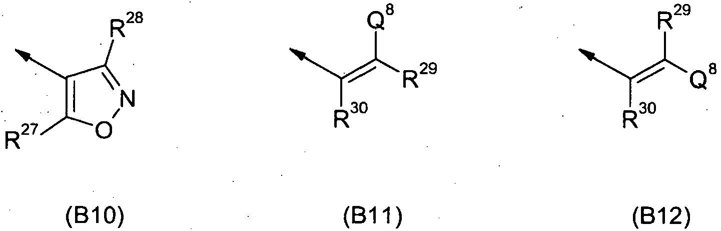 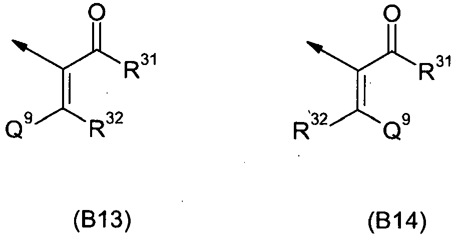
- A²: einen Rest der Formeln (A1) bis (A12) bedeutet, 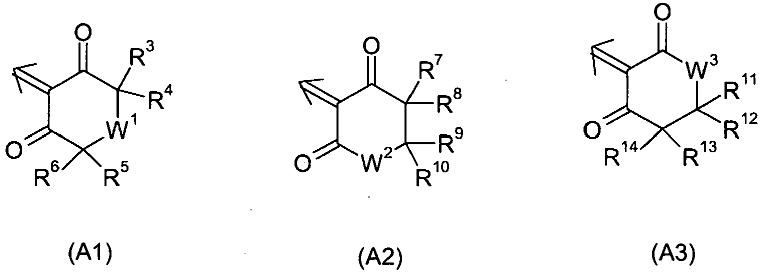 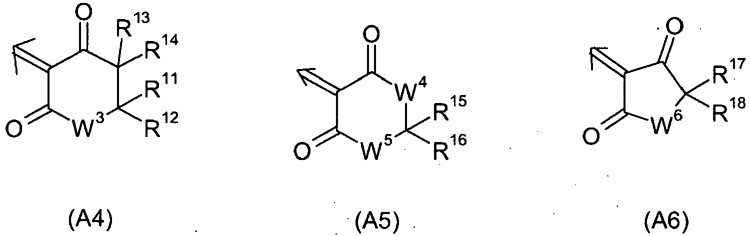 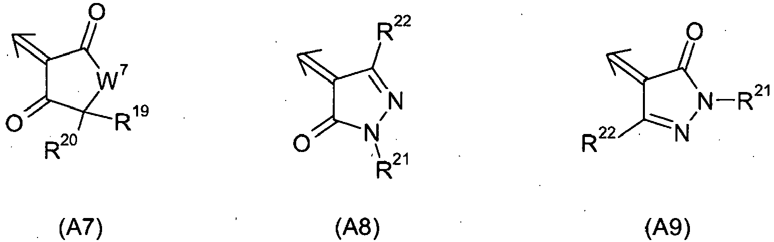 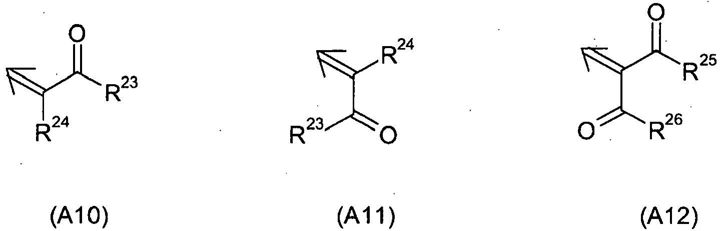
- B²: Hydroxy, Thio, Halogen oder eine Gruppe der Formel OR³³, OM, SR³⁴, SM oder -NR^{B}R^{C} bedeutet,
- W¹ und W⁶: jeweils unabhängig voneinander die divalente Gruppe Sauerstoff, Schwefel oder eine Gruppe der Formel NH, N-[(C₁-C₄)Alkyl], C=O (Carbonyl), S=O (Sulfinyl), SO₂ (Sulfonyl) oder CR³⁵R³⁶ bedeuten,
- W², W³, W⁴, W⁵ und W⁷: jeweils unabhängig voneinander die divalente Gruppe Sauerstoff, Schwefel oder eine Gruppe der Formel NH, N-[(C₁-C₄)Alkyl], C=O (Carbonyl), S=O (Sulfinyl) oder SO₂ (Sulfonyl) bedeuten,
- Q¹, Q², Q³, Q⁴, Q⁵, Q⁶, Q⁷, Q⁸ und Q⁹: jeweils unabhängig voneinander Hydroxy, Thio, Halogen oder eine Gruppe der Formel OR³⁷, SR³⁸, SM oder OM bedeuten,
- M: ein Äquivalent eines Kations, vorzugsweise ein Metallionenäquivalent, ein Ammoniumion, dass gegebenenfalls durch 1 bis 4 gleiche oder verschiedene Reste aus der Gruppe (C₁-C₆)Alkyl, (C₂-C₆)Alkenyl, (C₂-C₆)Alkinyl, (C₃-C₆)Cycloalkyl, (C₅-C₆)Cycloalkenyl, (C₅-C₆)Cycloalkinyl, Phenyl, (C₃-C₆)Cycloalkyl-(C₁-C₄)alkyl, (C₅-C₆)Cycloalkenyl-(C₁-C₄)alkyl, Phenyl(C₁-C₄)alkyl substituiert ist, oder ein tertiäres Sulfoniumion, das vorzugsweise durch 3 gleiche oder verschiedene Reste aus der Gruppe (C₁-C₆)Alkyl, (C₂-C₆)Alkenyl, (C₂-C₆)Alkinyl, (C₃-C₆)Cycloalokyl, (C₅-C₆)Cycloalkenyl, (C₅-C₆)Cycloalkinyl, Phenyl, (C₃-C₆)Cycloalkyl-(C₁-C₄)alkyl, (C₅-C₆)Cycloalkenyl-(C₁-C₄)alkyl, Phenyl(C₁-C₄)alkyl, insbesondere (C₁-C₄)Alkyl substituiert ist, bedeutet,
- RA, R^{B} und R^{C}: jeweils unabhängig voneinander Wasserstoff, Hydroxy, (C₁-C₄)Alkoxy, (C₁-C₄)Alkyl, (C₁-C₄)Haloalkyl, Phenyl oder Benzyl, wobei jeder der letztgenannten beiden Reste gegebenenfalls ein- oder mehrfach, gleich oder verschieden substituiert ist und vorzugsweise unabhängig voneinander unsubstitutiert oder durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
oder -NR*R**, wobei R*, R** jeweils unabhängig voneinander H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy oder im Fall Cycloalkyl auch Oxo substituiert ist, bedeuten oder R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
- (R¹)ₘ: m Substituenten R¹ bedeutet, wobei R¹, wenn m = 1, oder jeder der Substituenten R¹, wenn m größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR³⁹, C(O)NR⁴⁰R⁴¹, C(O)-Het¹, NR⁴²R⁴³ oder Het² bedeutet
oder wobei jeweils zwei am Ring ortho-ständige Gruppen R¹ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht, wobei die Gruppe -Z¹-A*-Z² zusammen mit den an die Gruppe gebundenen C-Atomen des Phenylrings einen ankondensierten 5 oder 6 Ring bilden,
- (R²)ₙ: n Substituenten R² bedeutet, wobei R², wenn n = 1, oder jeder der Substituenten R², wenn n größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR⁴⁴, C(O)NR⁴⁵R⁴⁶, C(O)-Het³, NR⁴⁷R⁴⁸ oder Het⁴ bedeutet
oder
wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht, wobei die Gruppe -Z³-A**-Z⁴ zusammen mit den an die Gruppe gebundenen C-Atomen des Phenylrings einen ankondensierten 5 oder 6 Ring bilden,
- R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹ und R²⁰: jeweils unabhängig voneinander Wasserstoff, Halogen, Cyano, Nitro, Hydroxy, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkyl, (C₁-C₄)Haloalkoxy-(C₁-C₄)alkyl, (C₃-C₆)Cycloalkyl, (C₃-C₆)Halocycloalkyl, (C₃-C₆)Cycloalkoxy, (C₂-C₆)Alkenyl, (C₂-C₆)Haloalkenyl, (C₂-C₆)Alkinyl, (C₂-C₆)Haloalkinyl, (C₁-C₄)Alkylthio, (C₁-C₄)Alkylsulfonyl, (C₁-C₄)Alkylsulfinyl, Phenyl, das gegebenenfalls substituiert ist, vorzugsweise unsubstituiert oder durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist, oder eine Gruppe C(O)OR⁴⁹, C(O)NR⁵⁰R⁵¹, C(O)Het⁵, NR⁵²R⁵³ oder Het⁶ bedeuten oder R³ und R⁵ in der Gruppe der Formel (B1) oder (A1) gemeinsam eine divalente Brücke aus einer geradkettigen Alkylen- oder Alkenylen-gruppe mit 2 oder 3 C-Atomen, wobei eine CH₂-Gruppe in der Kette noch durch ein Sauerstoffatom ersetzt sein kann und wobei eine CH₂-Gruppe oder CH-Gruppe in der Kette gegebenenfalls durch ein oder mehrere Reste aus der Gruppe (C₁-C₄)Alkyl, (C₁-C₄)Haloalkyl, (C₁-C₄)Alkoxy und (C₁-C₄)Haloalkoxy substituiert ist, vorzugsweise eine divalente aliphatische Brücke der Formel -CH₂CH₂-, -CH₂CH₂CH₂-, -CH(CH₃)CH₂-, -C(CH₃)₂CH₂-, -CH₂CH(CH₃)-, -CH₂C(CH₃)₂-, -C(CH₃)CH(CH₃)-, -CH₂CH₂CH₂-, -CH=CH-, -CH=CHCH₂-, -CH₂CH=CH-, -OCH₂-, -CH₂O-, -OCH₂CH₂-, -CH₂OCH₂-, -CH₂CH₂O- oder -OCH₂O-, insbesondere der Formel -CH₂CH₂- oder -CH=CH- bedeuten,
- R²¹: Wasserstoff, (C₁-C₄)Alkyl, (C₁-C₄)Halogenalkyl, (C₃-C₆)Cycloalkyl, (C₃-C₆)Halogencycloalkyl oder eine Gruppe C(O)OR⁵⁴, C(O)NR⁵⁵R⁵⁶, C(O)Het⁷, NR⁵⁷R⁵⁸ oder Het⁸ bedeutet,
- R²²: Wasserstoff, (C₁-C₄)Alkyl, (C₁-C₄)Halogenalkyl, (C₃-C₆)Cycloalkyl, (C₃-C₆)Halogencycloalkyl, (C₁-C₄)Alkylthio, (C₁-C₄)Alkylsulfonyl, (C₁-C₄)Alkylsulfinyl oder eine Gruppe der Formel C(O)OR⁵⁹, C(O)NR⁶⁰R⁶¹, C(O)Het⁹, NR⁶²R⁶³ oder Het¹⁰ bedeutet,
- R²³ und R²⁹: jeweils unabhängig voneinander Wasserstoff, (C₁-C₄)Alkyl, (C₁-C₄)Halogenalkyl, (C₃-C₆)Cycloalkyl, (C₃-C₆)Halogencycloalkyl, (C₁-C₄)Alkoxy oder (C₁-C₄)Halogenalkoxy bedeuten,
- R²⁴ und R³⁰: jeweils unabhängig voneinander Wasserstoff, Halogen, Cyano, (C₁-C₄)Alkyl, (C₁-C₄)Halogenalkyl, (C₃-C₆)Cycloalkyl, (C₃-C₆)Halogencycloalkyl, (C₁-C₄)Alkoxy oder (C₁-C₄)Halogenalkoxy bedeuten,
- R²⁵, R²⁶, R³¹ und R³²: jeweils unabhängig voneinander Wasserstoff, (C₁-C₄)Alkyl, (C₁-C₄)Halogenalkyl, (C₁-C₄)Alkoxy, (C₁-C₄)Halogenalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Halogencycloalkyl, (C₃-C₆)Cycloalkoxy, (C₂-C₆)Alkenyl, (C₂-C₆)Halogenalkenyl, (C₂-C₆)Alkinyl, (C₂-C₆)Halogenalkinyl, (C₁-C₄)Alkylthio, (C₁-C₄)Alkylsulfonyl, (C₁-C₄)Alkylsulfinyl, NR⁶⁴R⁶⁵ oder Het¹¹ bedeuten,
- R²⁷: Wasserstoff, (C₁-C₆)Alkyl, (C₁-C₄)Halogenalkyl, (C₃-C₆)Cycloalkyl oder (C₃-C₆)Halogencycloalkyl bedeutet,
- R²⁸: Wasserstoff, (C₁-C₆)Alkyl, (C₁-C₄)Halogenalkyl oder einen Rest der Formel C(O)OR⁶⁶ bedeutet,
- R³³, R³⁴, R³⁷ und R³⁸: jeweils unabhängig voneinander (C₁-C₈)Alkyl, (C₃-C₆)Cycloalkyl, Phenyl, wobei jeder der letztgenannten drei Reste jeweils unabhängig voneinander unsubstituiert oder durch einen oder mehrere Reste aus der Gruppe Halogen, Cyano, Nitro, Hydroxy, (C₁-C₄)Alkoxy, (C₁-C₄)Alkylthio und im Falle eines Cycloalkyl- oder Phenylrestes als Basisrest auch (C₁-C₄)Alkyl und (C₁-C₄)Haloalkyl substituiert ist, oder eine Gruppe der Formel -C(O)R⁶⁷, -C(O)OR⁶⁸, -C(O)NR⁶⁹R⁷⁰, -C(O)Het¹² oder -SO₂R¹¹ bedeuten,
- R³⁵: und R³⁶ jeweils unabhängig voneinander wie R³ definiert sind oder vorzugweise Wasserstoff, (C₁-C₄)Alkyl oder (C₁-C₄)Halogenalkyl bedeuten,
- R³⁹, R⁴⁴, R⁴⁹, R⁵⁴, R⁵⁹ und R⁶⁶: jeweils unabhängig voneinander Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M, vorzugsweise (C₁-C₄)Alkyl bedeuten,
- R⁴⁰, R⁴¹, R⁴², R⁴³, R⁴⁵, R⁴⁶, R⁴⁷, R⁴⁸, R⁵⁰, R⁵¹, R⁵², R⁵³, R⁵⁵, R⁵⁶, R⁵⁷, R⁵⁸, R⁶⁰, R⁶¹,R⁶², R⁶³, R⁶⁴, R⁶⁵, R⁶⁹ und R⁷⁰: jeweils unabhängig voneinander Wasserstoff, (C₁-C₆)Alkyl, (C₂-C₆)Alkenyl oder (C₂-C₆)Alkinyl, wobei jeder der letztgenannten 3 Reste jeweils unabhängig voneinander unsubstituiert oder durch einen oder mehrere Reste aus der Gruppe Halogen, Nitro, Cyano und Phenyl, das gegebenenfalls substituiert ist, vorzugweise unsubstitutiert oder durch einen oder mehrere Reste aus der Gruppe Halogen, Nitro, Cyano, (C₁-C₄)Alkyl und (C₁-C₄)Haloalkyl substituiert ist, substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, Phenyl und Benzyl, wobei jeder der letztgenannten 2 Reste gegebenenfalls substituiert ist, vorzugsweise unsubstituiert oder durch einen oder mehrere Reste aus der Gruppe Halogen, Nitro, Cyano, (C₁-C₄)Alkyl und (C₁-C₄)Haloalkyl substituiert ist, substituiert ist, bedeuten,
- R⁶⁷: Wasserstoff, (C₁-C₈)Alkyl oder (C₁-C₈)Halogenalkyl, wobei jeder der letztgenannten 2 Reste unabhängig voneinander gegebenenfalls im Alkylteil durch Sauerstoff oder Schwefel unterbrochen ist, oder Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl, (C₂-C₄)Alkinyl, (C₃-C₆)Cycloalkyl, (C₁-C₄)Halogenalkyl und (C₃-C₆)Halogencycloalkyl substituiert ist, bedeutet,
- R⁶⁸: (C₁-C₈)Alkyl, (C₁-C₈)Halogenalkyl, (C₃-C₆)Cycloalkyl, (C₃-C₆)Halogencycloalkyl, (C₂-C₈)Alkenyl, (C₂-C₈)Haloalkenyl oder (C₂-C₄)Alkinyl bedeutet,
- R⁷¹: (C₁-C₆)Alkyl, (C₁-C₆)Halogenalkyl oder Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl, (C₂-C₄)Alkinyl, (C₃-C₆)Cycloalkyl, (C₁-C₄)Halogenalkyl und (C₃-C₆)Halogencycloalkyl substituiert ist, bedeutet,
- Het¹, Het², Het³, Het⁴, Het⁵, Het⁶, Het⁷, Het⁸, Het⁹, Het¹⁰, Het¹¹ und Het¹²: jeweils unabhängig voneinander einen gesättigten oder teilungesättigten Rest eines Heterocyclus mit 3 bis 9 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, vorzugsweise den Rest eines gesättigten Heterocyclus der genannten Art, insbesondere eine Morpholino- Piperidino- oder Pyrrolidinogruppe bedeuten und
- m, n: jeweils unabhängig voneinander 0, 1, 2, 3, 4 oder 5, vorzugsweise 0, 1, 2, oder 3 bedeuten.

In der Formel (I) bedeutet die Formel "(R¹)ₘ" m Reste R¹, die als Substituenten am betreffenden Phenylring gebunden sind, wobei die Reste im Falle m größer 1 gleich oder verschieden sein können und die jeweils näher genannte Bedeutung haben. Im Fall m = 0 ist der betreffende Phenylring nicht durch Substituenten R¹ substituiert, d. h. alle Ring-C-Atome des Phenylrings in den Positionen 2 bis 6 sind mit einem Wasserstoffatom verbunden. Entsprechendes gilt für die Substitution des anderen Phenylrings gemäß der Formel (R²)ₙ.

In den Formeln (B1) bis (B14) für Reste der Gruppe B¹ ist die freie Bindung, mit der die Gruppe B¹ in der Gruppe der Formel -C(=A¹)-B¹ am in der Formel gezeigten C-Atom gebunden ist, mit einem Pfeil (←) gekennzeichnet, um eine Verwechselung mit der Kurzschreibweise für den Rest "Methyl" zu vermeiden.

In den Formeln (A1) bis (A12) für Reste der Gruppe A² ist die freie Doppelbindung, mit der die Gruppe A² in der Gruppe der Formel -C(=A²)-B² an dem in der Formel gezeigten C-Atom gebunden ist, mit einem Pfeil (⇐) gekennzeichnet, um eine Verwechselung mit der Kurzschreibweise in Formeln für den Rest "Methylen" ("H₂C=") zu vermeiden.

Die erfindungsgemäßen Verbindungen der Formel (I) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen. Die Erfindung umfasst auch alle Tautomeren, wie Keto- und Enol-Tautomeren, und deren Mischungen und Salze, wenn entsprechende funktionelle Gruppen vorhanden sind.

Die Verbindungen der Formel (I) enthalten in Position 2 und 3 des substituierten Valeronitrilgerüstes zwei Chiralitätszentren und treten deshalb in mindestens vier Stereoisomeren auf und deren Gemischen, d. h. 2 enantiomere Erythro-Isomere und 2 enantiomere Threo-Isomere. Je nach Substituenten (R¹)ₘ und (R²)ₙ können ein oder mehrere weitere Chiralitätszentren enthalten sein.

Gegenstand Erfindung sind daher auch Erythro-Threo-Gemische (Diastereomerengemische) der Verbindungen der Formel (I).

Gegenstand der Erfindung sind auch die die racemischen Erythro-Isomere oder die racemischen Threo-Isomere der Verbindungen der Formel (I).

Gegenstand der Erfindung sind auch die optisch aktiven (2R, 3S)- und (2S, 3R)-Erythro-Isomere und deren Gemische mit einem Überschuss an einem Enantiomeren (siehe nachfolgende Formel (I) mit Bezifferung der Positionen 1 bis 5 im Valeronitrilgerüst).

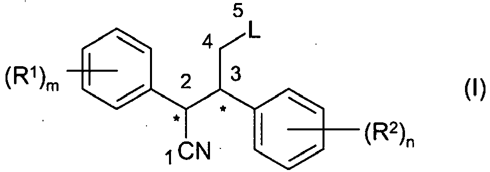

Gegenstand der Erfindung sind auch die optisch aktiven (2R, 3R)- und (2S, 3S)-Threo-Isomere und deren Gemische mit einem Überschuss an einem Enantiomeren. Aufgrund der zwei Chiralitätszentren in Position 2 und 3 existieren Verbindungen derselben chemischen Konstitution als 4 stereoisomere Konfigurationen, und zwar zwei Erythro-Enantiomere mit den Konfigurationen (2R,3S) [= Erythro-1] bzw. (2S,3R) [= Erythro-2] und zwei Threo-Enantiomere mit den Konfigurationen (2S,3S) [= Threo-1] bzw. (2R,3R) [= Threo-2]; siehe nachfolgendes Schema:

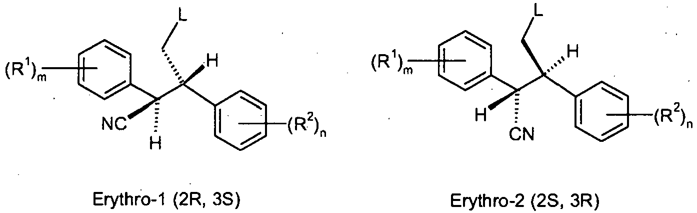

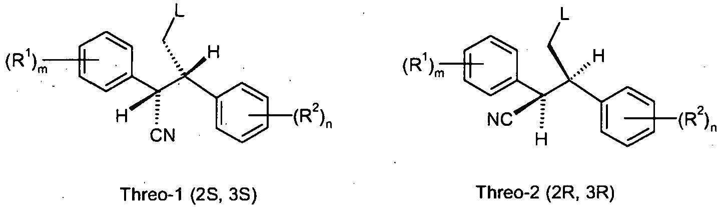

Die erfindungsgemäßen Verbindungen (I) stellen Diastereomerengemische der 4 Stereoisomeren dar, umfassen aber auch die getrennten diastereomeren Erythro-oder Threo-Formen, jeweils als racemisches Gemisch der Erythro-Enantiomere oder Threo-Enantiomere oder als reine oder stereochemisch angereicherte Enantiomere Erythro-1, Erythro-2, Threo-1 oder Threo-2.

Bevorzugt sind die Diastereomerengemische der Formel (I) (Erythro-Threo-Gemische).

Weiterhin bevorzugt sind die racemischen Threo-Gemische der Formel (I) aus den genannten Enantiomeren Threo-1 und Threo-2 im Verhältnis 50:50.

Weiter bevorzugt sind die (2R, 3R)-Enantiomere Threo-2 der Formel (la) oder deren Salze, 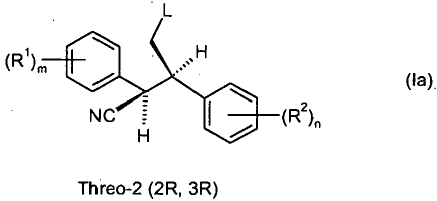 worin (R¹)ₘ und (R²)ₙ wie in Formel (I) definiert sind,
wobei die stereochemische Konfiguration am C-Atom in Position 2 des Valeronitrilderivats eine stereochemische Reinheit von 60 bis 100 % (R), vorzugsweise 70 bis 100 % (R), mehr bevorzugt 80 bis 100 % (*R*), insbesondere 90 bis 100 % (R), bezogen auf das vorliegende Gemisch der threo-Enantiomeren aufweist und
die stereochemische Konfiguration am C-Atom in Position 3 des Valeronitrilderivats eine stereochemische Reinheit von 60 bis 100 % (R), vorzugsweise 70 bis 100 % (R), mehr bevorzugt 80 bis 100 % (R), insbesondere 90 bis 100 % (R), bezogen auf das vorliegende Gemisch der threo-Enantiomeren aufweist.

Die Verbindungen der Formel (I) können im Falle geeigneter saurer Substituenten durch Umsetzung mit Basen Salze bilden, wobei der saure Wasserstoff durch ein für die Landwirtschaft geeignetes Kation ersetzt wird.

Die Verbindungen der Formel (I) können durch Anlagerung einer geeigneten anorganischen oder organischen Säure, an eine basische Gruppe, wie z.B. Amino oder Alkylamino, Salze bilden. Geeignete vorhandene saure Gruppen, wie z.B. Carbonsäuregruppen, können innere Salze mit ihrerseits protonierbaren Gruppen, wie Aminogruppen bilden.

Die Verbindungen der Formel (I) können vorzugsweise in Form landwirtschaftlich einsetzbarer Salze vorliegen, wobei es ansonsten auf die Art des Salzes in der Regel nicht ankommt. Im Allgemeinen kommen dabei die Salze derjenigen Kationen oder die Säureadditionssalze derjenigen Säuren in Betracht, deren Kationen, beziehungsweise Anionen, die herbizide Wirkung der Verbindungen (I) nicht negativ beeinträchtigen.

Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium oder substituiertes Ammonium verwendet werden, wobei hier ein bis vier Wasserstoffatome durch (C₁-C₄)Alkyl, Hydroxy-(C₁-C₄)alkyl, (C₁-C₄)Alkoxy-(C₁-C₄)alkyl, Hydroxy-(C₁-C₄)alkoxy-(C₁-C₄)alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri(C₁-C₄)methylsulfonium oder Sulfoxoniumionen, vorzugsweise Tri(C₁-C₄)methylsulfoxonium, in Betracht. Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von (C₁-C₄)Alkansäuren, vorzugsweise Formiat, Acetat, Propionat, Butyrat oder Trifluoracetat.

In der Formel (I) und allen nachfolgenden Formeln werden Bezeichnungen für chemische Reste verwendet, die Sammelbegriffe für die Aufzählung individueller Gruppenmitglieder darstellen oder individuelle chemische Reste spezifisch bezeichnen. In der Regel werden Bezeichnungen verwendet, die dem Fachmann geläufig sind und/oder insbesondere die nachstehend erläuterten Bedeutungen haben.

Ein Kohlenwasserstoffrest ist ein aliphatischer, cycloaliphatischer oder aromatischer monocyclischer oder, im Falle eines gegebenenfalls substituierten Kohlenwasserstoffrestes, auch ein bicyclischer oder polycyclischer organischer Rest auf Basis der Elemente Kohlenstoff und Wasserstoff, beispielsweise umfassend die Reste Alkyl, Alkenyl, Alkinyl, Cycloalkyl, Cycloalkenyl, Aryl, Phenyl, Naphthyl, Indanyl, Indenyl, etc.; Entsprechendes gilt für Kohlenwasserstoffreste in zusammengesetzte Bedeutungen wie Kohlenwasserstoffoxyresten oder anderen über Heteroatomgruppen gebundene Kohlenwasserstoffresten.

Wenn nicht näher definiert, weisen die Kohlenwasserstoffreste vorzugsweise 1 bis 20 C-Atome, weiter bevorzugt 1 bis 16 C-Atome, insbesondere 1 bis 12 C-Atome auf. Die Kohlenwasserstoffreste, auch in den speziellen Resten Alkyl, Alkoxy, Haloalkyl, Haloalkoxy, Alkylamino und Alkylthio, sowie die entsprechenden ungesättigten und/oder substituierten Reste können im Kohlenstoffgerüst jeweils geradkettig oder verzweigt sein.

Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl. Alkenyl schließt insbesondere auch geradkettige oder verweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl;

Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Ein 3- bis 9-gliedriger carbocyclischer Ring bedeutet (C₃-C₉)Cycloalkyl oder (C₅-C₉)Cycloalkenyl.

(C₃-C₉)Cycloalkyl bedeutet ein carbocyclisches, gesättigtes Ringsystem mit vorzugsweise 3-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfasst, wobei die Substituenten auch mit einer Doppelbindung am Cycloalkylrest, z. B. eine Alkylidengruppe wie Methyliden, gebunden sein können.

(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Halogen bedeutet beispielsweise Fluor, Chlor, Brom oder Iod. Haloalkyl, -alkenyl und -alkinyl, gegebenenfalls auch als Halogenalkyl, Halogenalkenyl bzw. Halogenalkinyl bezeichnet, bedeuten durch gleiche oder verschiedene Halogenatome, vorzugsweise aus der Gruppe Fluor, Chlor, Brom und Iod, insbesondere aus der Gruppe Fluor, Chlor und Brom, ganz besonders aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH2F, CF₃CF₂O, OCH₂CF₃ und OCH₂CH₂Cl; Entsprechendes gilt für Haloalkenyl und andere durch Halogen substituierte Reste wie beispielsweise Halocycloalkyl (= Halogencycloalkyl).

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Im Falle gegebenenfalls substituiertes Aryl sind auch mehrcyclische Systeme, wie Tetrahydronaphthyl, Indenyl, Indanyl, Fluorenyl, Biphenylyl, umfasst, wobei die Bindungsstelle am aromatischen System ist.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P, B, Si, Se) der gesättigt, ungesättigt oder heteroaromatisch ist und dabei unsubstituiert oder substituiert sein kann, wobei die Bindungsstelle an einem Ringatom lokalisiert ist. Wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält.

Ist der Heterocyclylrest oder der heterocyclische Ring gegebenenfalls substituiert, kann er mit anderen carbocyclischen oder heterocyclischen Ringen annelliert sein. Bevorzugt sind benzokondensierte (benzoannellierte) heterocyclische bzw. heteroaromatische Ringe.

Im Falle von gegebenenfalls substituiertem Heterocyclyl werden auch mehrcyclische Systeme umfasst, wie beispielsweise 8-Aza-bicyclo[3.2.1]octanyl oder 1-Azabicyclo[2.2.1]heptyl.

Im Falle von gegebenenfalls substituiertem Heterocyclyl werden auch spirocyclische Systeme umfasst, wie beispielsweise 1-Oxa-5-aza-spiro[2.3]hexyl.

Vorzugsweise ist er ein Rest eines heteroaromatischen Rings mit einem Heteroatom aus der Gruppe N, O und S, beispielsweise der Rest eines Fünf- oder Sechsrings, wie Pyridyl, Pyrrolyl, Thienyl oder Furyl;
weiterhin bevorzugt ist er ein Rest eines entsprechenden heteroaromatischen Rings mit 2, 3 oder 4 Heteroatomen, z. B. Pyrimidinyl, Pyridazinyl, Pyrazinyl, Triazinyl, Tetrazinyl, Thiazolyl, Thiadiazolyl, Oxazolyl, Isoxazolyl, Pyrazolyl, Imidazolyl oder Triazolyl oder Tetrazolyl.

Bevorzugt ist er dabei ein Rest eines heteroaromatischen Fünf- oder Sechsrings mit 1 bis 4 Heteroatomen, wie z. B. 1,2,3-Triazolyl, 1,2,4-Triazolyl, Tetrazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Tetrazolyl, 1,2,3-Triazinyl, 1,2,4-Triazinyl, 1,3,5-Triazinyl, 1,2,3,4-Tetrazinyl, 1,2,3,5-Tetrazinyl, 1,2,4,5-Tetrazinyl, Thiazolyl, Isothiazolyl, Oxazolyl, Isoxazolyl, Pyrazolyl, Imidazolyl.

Weiter bevorzugt sind dabei heteroaromatische Reste von Fünfring-Heterocyclen mit 3 N-Atomen, wie 1,2,3-Triazol-1-yl, 1,2,3-Triazol-4-yl, 1,2,3-Triazol-5-yl, 1,2,5-Triazol-1-yl, 1,2,5-Triazol-3-yl, 1,3,4-Triazol-1-yl, 1,3,4-Triazol-2-yl, 1,2,4-Triazol-3-yl, 1,2,4-Triazol-5-yl;
weiter bevorzugt sind auch heteroaromatische Reste von Sechsring-Heterocyclen mit 3 N-Atomen, wie 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl;
weiter bevorzugt sind auch heteroaromatische Reste von Fünfring-Heterocyclen mit zwei N-Atomen und einem O-Atom, wie 1,2,4-Oxadiazol-3-yl; 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl,
weiter bevorzugt sind auch heteroaromatische Reste von Fünfring-Heterocyclen mit zwei N-Atomen und einem S-Atom, wie 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl;
weiter bevorzugt sind auch heteroaromatische Reste von Fünfring-Heterocyclen mit vier N-Atomen, wie 1,2,3,4-Tetrazol-1-yl, 1,2,3,4-Tetrazol-5-yl, 1,2,3,5-Tetrazol-1-yl, 1,2,3,5-Tetrazol-4-yl, 2*H*-1,2,3,4-Tetrazol-5-yl, 1*H*-1,2,3,4-Tetrazol-5-yl,
weiter bevorzugt sind auch heteroaromatische Reste von Sechsring-Heterocyclen, wie 1,2,4,5-Tetrazin-3-yl;
weiter bevorzugt sind auch heteroaromatische Reste von Fünfring-Heterocyclen mit drei N-Atomen und einem O- oder S-Atom, wie 1,2,3,4-Oxatriazol-5-yl; 1,2,3,5-Oxatriazol-4-yl; 1,2,3,4-Thiatriazol-5-yl;1,2,3,5-Thiatriazol-4-yl;
weiter bevorzugt sind auch heteroaromatische Reste von Sechsring-Heterocyclen, wie beispielsweise 1,2,4,6-Thiatriazin-1-yl; 1,2,4,6-Thiatriazin-3-yl; 1,2,4,6-Thiatriazi n-5-yl.

Weiterhin bevorzugt ist der heterocyclische Rest oder Ring ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,

Weiterhin bevorzugt ist er auch ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten. Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl. Bevorzugte heterocyclische Reste sind auch benzokondensierte oder benzoannellierte heteroaromatische Ringe, beispielsweise Benzofuryl, Benzisofuryl, Benzothiophenyl, Benzisothiophenyl, Isobenzothiophenyl, Indolyl, Isoindolyl, Indazolyl, Benzimidazolyl, Benztriazolyl, Benzoxazolyl, 1,2-Benzisoxazolyl, 2,1-Benzisoxazolyl, Benzothiazolyl, 1,2-Benzisothiazolyl, 2,1-Benzoisothiazolyl, 1,2,3-Benzoxadiazolyl, 2,1,3-Benzoxadiazolyl, 1,2,3-Benzothiadiazolyl, 2,1,3-Benzothiadiazolyl, Chinolyl (Chinolinyl), Isochinolyl (Isochinolinyl), Chinnolinyl, Phtalazinyl, Chinazolinyl, Chinoxalinyl, Naphthyridinyl, Benzotriazinyl, Purinyl, Pteridinyl, Indolizinyl, Benzo-1,3-dioxylyl, 4H-Benzo-1,3-dioxinyl, und 4H-Benzo-1,4-dioxinyl, und wo es möglich ist, N-Oxide und Salze davon.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamine, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfasst, die mit einer Doppelbindung am Ring gebunden sind, z. B. mit einer Alkylidengruppe wie Methyliden oder Ethyliden substituiert sind.

Der Ausdruck "Reste aus der Gruppe (gefolgt von der Gruppe = Liste der Substituenten)", wo immer verwendet, soll gleichbedeutend sein mit "Reste ausgewählt aus der Gruppe (...)" oder "Reste ausgewählt aus der Gruppe bestehend aus (...)". Der Ausdruck "ein oder mehrere Reste aus der Gruppe (gefolgt von der Gruppe = Liste der Substituenten)", wo immer verwendet, soll gleichbedeutend sein mit "ein oder mehrere gleiche oder verschiedene Reste ausgewählt aus der Gruppe (...)" oder "Reste ausgewählt aus der Gruppe bestehend aus (...)".

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst. Mit "Basisrest" wird der jeweilige Grundkörper eines Restes bezeichnet, an dem Substituenten einer Substituentenebene gebunden sind.

Bevorzugte Substituenten für die Substituentenebenen sind beispielsweise Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkyl-aminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.

Zwei Substituenten können auch gemeinsam eine gesättigte oder ungesättigte Kohlenwasserstoff-Brücke oder eine entsprechende Brücke, in denen C-Atome, CH-Gruppen oder CH₂-Gruppen durch Heteroatome ersetzt sind, bilden und damit einen ankondensierten oder annellierten Cyclus bilden. Bevorzugt werden dabei benzokondensierte Systeme auf Basis der Grundkörper gebildet.

Gegebenenfalls substituiertes Phenyl bedeutet vorzugsweise Phenyl oder Phenyl, das unsubstituiert oder durch einen oder mehrere Reste aus der Gruppe Halogen, Cyano, (C₁-C₄)Alkyl, (C₁-C₄)Haloalkyl, (C₁-C₄)Alkoxy-(C₁-C₄)alkyl, (C₁-C₄)Alkoxy, (C₁-C₄)Haloalkoxy, (C₁-C₄)Alkoxy-(C₁-C₄)alkoxy, (C₁-C₄)Alkylthio und Nitro substituiert ist, insbesondere Phenyl, das gegebenenfalls durch einen oder mehrere Reste aus der Gruppe Halogen, (C₁-C₄)Alkyl, (C₁-C₄)Haloalkyl und (C₁-C₄)Alkoxy substituiert ist.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren.

Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.

Weiter bevorzugt bedeutet Acyl einen Alkanoylrest mit 1 bis 6 C-Atomen, insbesondere 1 bis 4 C-Atomen. (C₁-C₄)Alkanoyl bedeutet dabei den Rest einer Alkansäure mit 1 bis 4 C-Atomen nach Abtrennen der OH-Gruppe der Säuregruppe, d.h. Formyl, Acetyl, n-Propionyl, i-Propionyl oder n-, i-, sec. oder tert.-Butanoyl. Die "yl-Position" eines Restes bezeichnet das C-Atom mit der freien Bindung. Erfindungsgemäße bzw. erfindungsgemäß verwendete Verbindungen der Formel (I) und/oder deren Salze werden abgekürzt auch als "Verbindungen (I)" bezeichnet. Gegenstand der Erfindung sind auch alle Stereoisomeren, die von Formel (I) umfasst sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfasst und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Gegenstand der Erfindung sind auch alle Tautomeren der Verbindungen der Formel (I), die durch Verschiebung eines Wasserstoffatoms entstehen können (z. B. Keto-Enol-Tautomere). Die Tautomeren sind ebenfalls von der Verbindung der Formel (I) umfasst, auch wenn die Formel (I) nur eines der jeweiligen im Gleichgewicht stehenden bzw. ineinander umwandelbaren Tautomeren formal richtig beschreibt. Beispielsweise sind Verbindungen der Formel (I), in denen L einen Rest der Formel (L') bedeutet, entsprechend L einen Rest der Formel -C(=A¹)-B¹, A¹ ein Sauerstoffatom und B¹ einen Rest der Formel (B1), worin Q¹ für OH steht, bedeuten, tautomer zu Verbindungen der Formel (I), worin L einen Rest der Formel (L") bedeutet, entsprechend L einen Rest der Formel -C(=A²)-B², A² einen Rest der Formel (A1) und B² eine Gruppe OH bedeuten (siehe Schema):

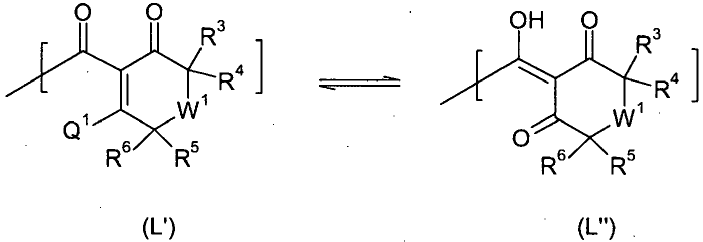

Die Tautomeren der Verbindungen (I) mit (L') und (L") werden somit sowohl vom Umfang der Formel (I), in denen L einen Rest der Formel -C(=A¹)-B¹ bedeutet, als auch vom Umfang der Formel (I), in denen L einen Rest der Formel -C(=A²)-B² bedeutet, umfasst.

Die Verbindungen der Formel (I) umfassen auch alle physikalischen Formen, in denen diese in Reinsubstanz oder gegebenenfalls in Mischung mit anderen Stoffen auftreten können, insbesondere auch polymorphe Kristallformen der Verbindungen der Formel (I) oder deren Salze oder Lösungsmitteladditionsverbindungen (z. B. Hydrate).

Vor allem aus den Gründen der höheren herbiziden Wirkung, besseren Selektivität, besseren Herstellbarkeit, besseren Formulierbarkeit und/oder anderer relevanter Eigenschaften, wie sind erfindungsgemäße Verbindungen der genannten Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Unabhängig von den jeweils anderen Resten gemäß den Symbolen (R¹)ₘ und (R²)ₙ, L und den Definitionen für m und n in Formel (I) sowie den Definitionen für die Reste (oder chemische Gruppen) gemäß den Symbolen A¹, A², B¹, B², R^{A}, R^{B}, W¹ bis W⁷, Q¹ bis Q⁹, M, R³ bis R⁷¹, R* und R** in den entsprechenden Unterbedeutungen zu Resten in der Formel (I) sind erfindungsgemäße Verbindungen der Formel (I) bzw. deren erfindungsgemäßen Verwendungen mit nachfolgend aufgeführten bevorzugten Bedeutungen der betreffenden Symbole bzw. chemischen Reste oder chemischen Gruppen von besonderem Interesse.

Bevorzugt sind Verbindungen (I), worin
- (R¹)ₘ: m Substituenten R¹ bedeutet, wobei R¹, wenn m = 1, oder jeder der Substituenten R¹, wenn m größer als 1, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁₋C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR³⁹, C(O)NR⁴⁰R⁴¹, C(O)-Het¹, NR⁴²R⁴³ oder Het² bedeutet
oder wobei jeweils zwei am Ring ortho-ständige Gruppen R¹ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht, wobei die Gruppe -Z¹-A*-Z² zusammen mit den an die Gruppe gebundenen C-Atomen des Phenylrings einen ankondensierten 5 oder 6 Ring bilden,
- R³⁹: Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeutet, vorzugsweise Wasserstoff, (C₁-C₄)Alkyl oder die genannte Gruppe M bedeutet,
- R⁴⁰, R⁴¹, R⁴², R⁴³, Het¹ und Het²,: die genannten Bedeutungen haben, vorzugsweise
- R⁴⁰, R⁴¹, R⁴² und R⁴³: jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, insbesondere Wasserstoff, (C₁-C₄)Alkyl oder (C₁-C₄)Haloalkyl bedeuten,
- Het¹ und Het²: jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, vorzugsweise den Rest eines gesättigten Heterocyclus der genannten Art, insbesondere eine Morpholino- Piperidino- oder Pyrrolidinogruppe bedeuten und
- m: 0, 1, 2, 3, 4 oder 5, vorzugsweise 0, 1, 2, oder 3 bedeutet.

Weiter bevorzugt sind Verbindungen (I), worin
- (R¹)ₘ: m Substituenten R¹ bedeutet, wobei R¹, wenn m = 1, oder jeder der Substituenten R¹, wenn m größer als 1, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl, oder einen Rest der Formel C(O)OR³⁹, C(O)NR⁴⁰R⁴¹, C(O)-Het¹, NR⁴²R⁴³ oder Het² bedeutet, oder wobei jeweils zwei am Ring ortho-ständige Gruppen R¹ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht, wobei die Gruppe -Z¹-A*-Z² zusammen mit den an die Gruppe gebundenen C-Atomen des Phenylrings einen ankondensierten 5 oder 6 Ring bilden,
- R³⁹: Wasserstoff, (C₁-C₄)Alkyl oder die genannte Gruppe M bedeutet,
- R⁴⁰, R⁴¹, R⁴² und R⁴³: jeweils unabhängig voneinander Wasserstoff, (C₁-C₄)Alkyl, (C₁-C₄)Haloalkyl, Benzyl, (C₃-C₆)Cycloalkyl oder Phenyl bedeuten, insbesondere Wasserstoff, Methyl oder Ethyl bedeuten,
- Het¹ und Het²: jeweils unabhängig voneinandereine Morpholino- Piperidino- oder Pyrrolidinogruppe bedeuten und
- m: 0, 1, 2, 3, 4 oder 5, vorzugsweise 0, 1, 2, oder 3 bedeutet.

Weiter bevorzugt sind dabei Verbindungen (I), worin
- (R¹)ₘ: m Substituenten R¹ bedeutet, wobei R¹, wenn m = 1, oder jeder der Substituenten R¹, wenn m größer als 1 ist, unabhängig voneinander Halogen, Cyano, Nitro, Methyl, Ethyl, Methoxy, Ethoxy, Methylthio, Ethylthio, (C₁-C₂)Alkylsulfinyl, (C₁-C₂)Alkylsulfonyl, (C₁-C₂)Haloalkyl, (C₁-C₂)Haloalkoxy, (C₁-C₂)Haloalkylthio, (C₁-C₂)Haloalkylsulfinyl, (C₁-C₂)Haloalkylsulfonyl oder (C₁-C₂)Alkoxy-(C₁-C₂)alkyl bedeutet,
insbesondere jeder der Substituenten R¹ unabhängig voneinander Halogen, wie Fluor, Chlor, Brom oder Iod, oder Cyano, Nitro, Methyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, insbesondere Halogen wie Fluor, Chlor oder Brom bedeutet, und
- m: 0, 1, 2, 3, 4 oder 5, vorzugsweise 0, 1, 2, 3 oder 4, insbesondere 0, 1, 2 oder 3 bedeutet.

Weiter bevorzugt sind Verbindungen der Formel (I) oder deren Salze, worin
- m: 0 (= die Zahl Null, d. h. keine Substituenten R¹ vorhanden, d. h. alle freien Bindungen am Ring sind mit Wasserstoff besetzt) bedeutet oder vorzugsweise
- (R¹)ₘ: 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulfinyl, 4-Methylsulfonyl, 2-Methylsulfonyl, 3-Methylsulfonyl, 4-Methylsulfonyl, 2-Nitro, 3-Nitro, 4-Nitro, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-CI-3-F), (2-CI-4-F), (2-CI-5-F), (2-CI-6-F), (3-Cl-2-F), (3-CI-4-F), (3-Cl-5-F), (3-Cl-6-F), (4-CI-2-F), (4-CI-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor, 3,4,5-Trichlor oder auch (4-Br-3-F), (3-Br-4-F), (3-Br-5-F), (4-Br-3-Cl), (3-Br-4-Cl), (4-CN-3-F), (3-CN-4-F), (3-CN-5-F), (4-CN-3-Cl) oder (3-CN-4-Cl) bedeutet, wobei die Bezifferung der Reste sich auf die Position des Restes am Phenyl-1-yl-Rest bezieht, in dem das C-Atom, das in 2-Position am Valeronitril-Grundkörper gebunden ist, die 1-Position im Ring hat.

Weiter bevorzugt sind Verbindungen der Formel (I) oder deren Salze, worin
- (R¹)ₘ: 3-Brom, 4-Brom, 3-Chlor, 4-Chlor, 3-Fluor, 4-Fluor, 2-Cyano, 3-Cyano, 4-Cyano, 2,5-Difluor, 3,4-Difluor, 3,5-Difluor, 3,4-Dichlor, 3,5-Dichlor, (3-Cl-4-F), (3-CI-5-F), (4-CI-3-F), (3-Br-4-F), (4-Br-3-F), 3,4,5-Trifluor, 3,4,5-Trichlor oder oder auch (4-Br-3-F), (3-Br-4-F), (3-Br-5-F), (4-Br-3-Cl), (3-Br-4-Cl), (4-CN-3-F), (3-CN-4-F), (3-CN-5-F), (4-CN-3-Cl) oder (3-CN-4-Cl) bedeutet.

Dabei sind insbesondere bevorzugt:
Verbindungen der Formel (I) oder deren Salze, worin (R¹)ₘ 3-Chlor bedeutet.
Verbindungen der Formel (I) oder deren Salze, worin (R¹)ₘ 4-Chlor bedeutet.
Verbindungen der Formel (I) oder deren Salze, worin (R¹)ₘ 3-Fluor bedeutet.
Verbindungen der Formel (I) oder deren Salze, worin (R¹)ₘ 4-Fluor bedeutet.
Verbindungen der Formel (I) oder deren Salze, worin (R¹)ₘ 3-Cyano bedeutet.
Verbindungen der Formel (I) oder deren Salze, worin (R¹)ₘ (3-CN-4-F) bedeutet.
Verbindungen der Formel (I) oder deren Salze, worin (R¹)ₘ (3-Br-4-F) bedeutet.
Verbindungen der Formel (I) oder deren Salze, worin (R¹)ₘ 3,4-Difluor bedeutet.
Verbindungen der Formel (I) oder deren Salze, worin (R¹)ₘ 3,4-Dichlor bedeutet.
Verbindungen der Formel (I) oder deren Salze, worin (R¹)ₘ 3,5-Difluor bedeutet.
Verbindungen der Formel (I) oder deren Salze, worin (R¹)ₘ 3,5-Dichlor bedeutet.
Verbindungen der Formel (I) oder deren Salze, worin (R¹)ₘ (3-Cl-4-F) bedeutet.
Verbindungen der Formel (I) oder deren Salze, worin (R¹)ₘ (3-Cl-5-F) bedeutet.
Verbindungen der Formel (I) oder deren Salze, worin (R¹)ₘ (4-Cl-3-F) bedeutet.

Bevorzugt sind auch Verbindungen (I), worin
- (R²)ₙ: n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(Q)OR⁴⁴, C(0)NR⁴⁵R⁴⁶, C(O)-Het³, NR⁴⁷R⁴⁸ oder Het⁴ bedeutet
oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht, wobei die Gruppe -Z³-A**-Z⁴ zusammen mit den an die Gruppe gebundenen C-Atomen des Phenylrings einen ankondensierten 5 oder 6 Ring bilden,
- R⁴⁴: Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeutet,
- R⁴⁵, R⁴⁷, R⁴⁷, R⁴⁸, Het³ und Het⁴,: die genannten Bedeutungen haben, vorzugsweise
- R⁴⁵, R⁴⁶, R⁴⁷ und R⁴⁸: jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, bedeuten,
- Het³ und Het⁴: jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, vorzugsweise den Rest eines gesättigten Heterocyclus der genannten Art, insbesondere eine Morpholino- Piperidino- oder Pyrrolidinogruppe bedeuten und
- n: 0, 1, 2, 3, 4 oder 5, vorzugsweise 0, 1, 2, oder 3 bedeutet.

Weiter bevorzugt sind dabei Verbindungen (I), worin
- (R²)ₙ: n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl oder einen Rest der Formel C(O)OR⁴⁴, C(O)NR⁴⁵R⁴⁶, C(O)-Het³, NR⁴⁷R⁴⁸ oder Het⁴ bedeutet oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht, wobei die Gruppe -Z³-A**-Z⁴ zusammen mit den an die Gruppe gebundenen C-Atomen des Phenylrings einen ankondensierten 5 oder 6 Ring bilden,
- R⁴⁴: Wasserstoff, (C₁-C₄)Alkyl oder die genannte Gruppe M bedeutet,
- R⁴⁵, R⁴⁶, R⁴⁷, R⁴⁸, Het³ und Het⁴,: die genannten Bedeutungen haben, vorzugsweise R⁴⁵, R⁴⁶, R⁴⁷ und R⁴⁸ jeweils unabhängig voneinander Wasserstoff oder (C₁-C₄)Alkyl, (C₁-C₄)Haloalkyl, Benzyl, (C₃-C₆)Cycloalkyl oder Phenyl bedeuten,
- Het³ und Het⁴: jeweils unabhängig voneinander eine Morpholino- Piperidino- oder Pyrrolidinogruppe bedeuten und
- n: 0, 1, 2, 3, 4 oder 5, vorzugsweise 0, 1, 2, 3 oder 4, insbesondere 0, 1, 2 oder 3 bedeutet.

Weiter bevorzugt sind dabei Verbindungen (I), worin
- (R²)ₙ: n Substituenten R² bedeutet,
wobei R², wenn n = 1, oder jeder der Substituenten R², wenn n größer als 1 ist, unabhängig voneinander Halogen, Cyano, Nitro, Methyl, Ethyl, Methoxy, Ethoxy, Methylthio, Ethylthio, (C₁-C₂)Alkylsulfinyl, (C₁-C₂)Alkylsulfonyl, (C₁-C₂)Haloalkyl, (C₁-C₂)Haloalkoxy, (C₁-C₂)Haloalkylthio, (C₁-C₂)Haloalkylsulfinyl, (C-₁-C₂)Haloalkylsulfonyl oder (C₁-C₂)Alkoxy-(C₁-C₂)alkyl bedeutet,
insbesondere jeder der Substituenten R² unabhängig voneinander Halogen, wie Fluor, Chlor, Brom oder Iod, oder Cyano, Nitro, Methyl, Methoxy, Methylthio, Methylsulfinyl, Methylsulfonyl, Trifluormethyl, Trifluormethoxy, Trifluoralkylthio, Trifluormethylsulfinyl oder Trifluormethylsulfonyl bedeutet, insbesondere Halogen wie Fluor, Chlor oder Brom bedeutet, und
- n: 0, 1, 2, 3, 4 oder 5, vorzugsweise 0, 1, 2, 3 oder 4, insbesondere 0, 1, 2 oder 3 bedeutet.

Weiter bevorzugt sind Verbindungen der Formel (1) oder deren Salze, worin
- n: 0 (= die Zahl Null, d. h. keine Substituenten R² vorhanden, d. h. alle freien Bindungen am Ring sind mit Wasserstoff besetzt) bedeutet oder vorzugsweise
- (R²)ₙ: 2-Brom, 3-Brom, 4-Brom, 2-Chlor, 3-Chlor, 4-Chlor, 2-Fluor, 3-Fluor, 4-Fluor, 2-Cyano, 3-Cyano, 4-Cyano, 2-Methyl, 3-Methyl, 4-Methyl, 2-Ethyl, 3-Ethyl, 4-Ethyl, 2-CF₃, 3-CF₃, 4-CF₃, 2-Methoxy, 3-Methoxy, 4-Methoxy, 2-Ethoxy, 3-Ethoxy, 4-Ethoxy, 2-Methylthio, 3-Methylthio, 4-Methylthio, 2-Methylsulfinyl, 3-Methylsulfinyl, 4-Methylsulfonyl, 2-Methylsulfonyl, 3-Methylsulfonyl, 4-Methylsulfonyl, 2-Nitro, 3-Nitro, 4-Nitro, 2,3-Dimethyl, 2,4-Dimethyl, 2,5-Dimethyl, 2,6-Dimethyl, 3,4-Dimethyl, 3,5-Dimethyl, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-CI-3-F), (2-CI-4-F), (2-CI-5-F), (2-CI-6-F), (3-Cl-2-F), (3-Cl-4-F), (3-Cl-5-F), (3-Cl**-**6-F), (4-CI-2-F), (4-Cl-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor, 3,4,5-Trichlor oder auch 2-Nitro, 3-Nitro, 4-Nitro, 2,5-Dicyano, 2,6-Dicyano, (4-Br-2-F), (4-Br-3-F), (4-CN-3-F), (4-Nitro-3-F), (4-Methoxy-3-F), (3-Cyano-4-F), (3-Nitro-4-F), (3-Cyano-4-Cl), (3-Nitro-4-Cl) oder (5-Cyano-2-F) bedeutet, wobei die Bezifferung der Reste sich auf die Position des Restes am Phenyl-1-yl-Rest bezieht, in dem das C-Atom, das in 3-Position am Valeronitril-Grundkörper gebunden ist, die 1-Position im Ring hat.

Weiter bevorzugt sind Verbindungen der Formel (I) oder deren Salze, worin
- (R²)ₙ: 2-Brom, 3-Brom, 4-Brom, 2-Chlor, 3-Chlor, 4-Chlor, 2-Fluor, 3-Fluor, 4-Fluor, 2-Nitro, 3-Nitro, 4-Nitro, 2-Methoxy, 3-Methoxy, 4-Methoxy, 2,3-Difluor, 2,4-Difluor, 2,5-Difluor, 2,6-Difluor, 3,4-Difluor, 3,5-Difluor, 2,3-Dichlor, 2,4-Dichlor, 2,5-Dichlor, 2,6-Dichlor, 3,4-Dichlor, 3,5-Dichlor, (2-Cl-3-F), (2-Cl-4-F), (2-Cl-5-F), (2-CI-6-F), (3-Cl-2-F), (3-CI-4-F), (3-CI-5-F), (3-Cl-6-F), (4-Cl-2-F), (4-Cl-3-F), 2,3,4-Trifluor, 2,3,5-Trifluor, 2,3,6-Trifluor, 2,4,6-Trifluor, 3,4,5-Trifluor, 2,3,4-Trichlor, 2,3,5-Trichlor, 2,3,6-Trichlor, 2,4,6-Trichlor, 3,4,5-Trichlor oder auch 2-Nitro, 3-Nitro, 4-Nitro, 2,5-Dicyano, 2,6-Dicyano, (4-Br-2-F), (4-Br-3-F), (4-CN-3-F), (4-NO₂-3-F), (4-OMe-3-F), (3-CN-4-F), (3-NO₂-4-F), (3-CN-4-Cl), (3-NO₂-4-Cl) oder (5-CN-2-F) bedeutet.

Weiter bevorzugt sind dabei auch Verbindungen der Formel (I) oder deren Salze, worin
- (R²)ₙ: 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, 3,4-Dichlor, 3,5-Dichlor, (3-Cl-2-F), (3-CI-4-F), (3-Cl-5-F), (3-Cl-6-F), (4-Cl-2-F), (4-CI-3-F) bedeutet.

Dabei sind insbesondere bevorzugt:
Verbindungen der Formel (I) oder deren Salze, worin (R²)ₙ 3-Chlor bedeutet.
Verbindungen der Formel (I) oder deren Salze, worin (R²)ₙ 4-Chlor bedeutet.
Verbindungen der Formel (I) oder deren Salze, worin (R²)ₙ 2-Fluor bedeutet.
Verbindungen der Formel (I) oder deren Salze, worin (R²)ₙ 3-Fluor bedeutet.
Verbindungen der Formel (I) oder deren Salze, worin (R²)ₙ 4-Fluor bedeutet.
Verbindungen der Formel (I) oder deren Salze, worin (R²)ₙ 2,3-Difluor bedeutet.
Verbindungen der Formel (I) oder deren Salze, worin (R²)ₙ 2,4-Difluor bedeutet.
Verbindungen der Formel (I) oder deren Salze, worin (R²)ₙ 2,5-Difluor bedeutet.
Verbindungen der Formel (I) oder deren Salze, worin (R²)ₙ 2,6-Difluor bedeutet.
Verbindungen der Formel (I) oder deren Salze, worin (R²)ₙ 3,4-Difluor bedeutet.
Verbindungen der Formel (I) oder deren Salze, worin (R²)ₙ 3,5-Difluor bedeutet.
Verbindungen der Formel (I) oder deren Salze, worin (R²)ₙ (3-Cl-2-F) bedeutet.
Verbindungen der Formel (I) oder deren Salze, worin (R²)ₙ (3-Cl-4-F) bedeutet.
Verbindungen der Formel (I) oder deren Salze, worin (R²)ₙ (3-Cl-5-F) bedeutet.
Verbindungen der Formel (I) oder deren Salze, worin (R²)ₙ (3-Cl-6-F) bedeutet.
Verbindungen der Formel (I) oder deren Salze, worin (R²)ₙ (4-Cl-2-F) bedeutet.
Verbindungen der Formel (I) oder deren Salze, worin (R²)ₙ (4-Cl-3-F) bedeutet.

Weiter bevorzugt sind:
Verbindungen der Formel (I) oder deren Salze, worin m = 0 und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten.
Verbindungen der Formel (I) oder deren Salze, worin (R¹)ₘ 3-Chlor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-CI-2-F), (3-CI-4-F), (3-CI-5-F), (3-CI-6-F), (4-CI-2-F) oder (4-CI-3-F) bedeuten.
Verbindungen der Formel (I) oder deren Salze, worin(R¹)ₘ 4-Chlor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl**-**2-F), (3-Cl-4-F), (3-Cl-5-F), (3-Cl-6-F), (4-Cl-2-F) oder (4-Cl-3-F) bedeuten.
Verbindungen der Formel (I) oder deren Salze, worin (R¹)ₘ 3-Fluor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-CI-5-F), (3-CI-6-F), (4-Cl-2-F) oder (4-Cl-3-F) bedeuten.
Verbindungen der Formel (I) oder deren Salze, worin (R¹)ₘ (3-CN-4-F) und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-CI-4-F), (3-Cl-5-F), (3-CI-6-F), (4-CI-2-F) oder (4-Cl-3-F) bedeuten.
Verbindungen der Formel (I) oder deren Salze, worin (R¹)ₘ (3-Br-4-F) und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-CI-2-F), (3-CI-4-F), (3-CI-5-F), (3-Cl-6-F), (4-CI-2-F) oder (4-Cl-3-F) bedeuten.
Verbindungen der Formel (I) oder deren Salze, worin (R¹)ₘ 4-Fluor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-CI-4-F), (3-CI-5-F), (3-Cl-6-F), (4-Cl-2-F) oder (4-Cl-3-F) bedeuten.
Verbindungen der Formel (I) oder deren Salze, worin (R¹)ₘ 3-Cyano und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-CI-6-F), (4-CI-2-F) oder (4-CI-3-F) bedeuten.
Verbindungen der Formel (I) oder deren Salze, worin (R¹)ₘ 3,4-Difluor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-CI-2-F), (3-CI-4-F), (3-CI-5-F), (3-CI-6-F), (4-Cl-2-F) oder (4-Cl-3-F) bedeuten.
Verbindungen der Formel (I) oder deren Salze, worin (R¹)ₘ 3,4-Dichlor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-CI-4-F), (3-Cl-5-F), (3-CI-6-F), (4-Cl-2-F) oder (4-CI-3-F) bedeuten.
Verbindungen der Formel (I) oder deren Salze, worin (R¹)ₘ 3,5-Difluor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten.
Verbindungen der Formel (I) oder deren Salze, worin (R¹)ₘ 3,5-Dichlor und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-CI-4-F), (3-Cl-5-F), (3-CI-6-F), (4-Cl-2-F) oder (4-Cl-3-F) bedeuten.
Verbindungen der Formel (I) oder deren Salze, worin (R¹)ₘ (3-Cl-4-F) und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-CI-5-F), (3-Cl-6-F), (4-Cl-2-F) oder (4-Cl-3-F) bedeuten.
Verbindungen der Formel (I) oder deren Salze, worin (R¹)ₘ (4-Cl-3-F) und (R²)ₙ 3-Chlor, 4-Chlor, 2-Fluor, 3-Fluor, 4-Fluor, 2,3-Difluor, 2,4-Difluor, 2,5-Difluor, 2,6-Difluor, 3,4-Difluor, 3,5-Difluor, (3-Cl-2-F), (3-Cl-4-F), (3-Cl-5-F), (3-Cl-6-F), (4-Cl-2-F) oder (4-Cl-3-F) bedeuten.

Auch weiter bevorzugt sind:
Verbindungen der Formel (I) oder deren Salze, worin n = 0 und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-Cl-3-F) oder auch (3-CN-4-F), (3-Br-4-F), bedeuten.
Verbindungen der Formel (I) oder deren Salze, worin (R²)ₙ 3-Chlor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-CI-4-F), (3-CI-5-F) oder (4-Cl-3-F) oder auch (3-CN-4-F), (3-Br-4-F) bedeuten.
Verbindungen der Formel (I) oder deren Salze, worin (R²)ₙ 4-Chlor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-CI-3-F) oder auch (3-CN-4-F), (3-Br-4-F) bedeuten.
Verbindungen der Formel (I) oder deren Salze, worin (R²)ₙ 3-Fluor und (R¹)ₘ 3-Chlor, 4-Chlor, -3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-Cl-3-F) oder auch (3-CN-4-F), (3-Br-4-F) bedeuten.
Verbindungen der Formel (I) oder deren Salze, worin (R²)ₙ 4-Fluor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-CI-4-F), (3-CI-5-F) oder (4-Cl-3-F) oder auch (3-CN-4-F), (3-Br-4-F) bedeuten.
Verbindungen der Formel (I) oder deren Salze, worin (R²)ₙ 2,3-Difluor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-CI-5-F) oder (4-Cl-3-F) oder auch (3-CN-4-F), (3-Br-4-F) bedeuten.
Verbindungen der Formel (I) oder deren Salze, worin (R²)ₙ 2,4-Difluor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-CI-4-F), (3-Cl-5-F) oder (4-CI-3-F) oder auch (3-CN-4-F), (3-Br-4-F) bedeuten.
Verbindungen der Formel (I) oder deren Salze, worin (R²)ₙ 2,5-Difluor und (R¹)ₘ 3-Chlor, 4-Chlor, 3-Fluor, 4-Fluor, 3,4-Diflüor, 3,5-Difluor, 3,4-Dichlor, 3,5-Dichlor, (3-CI-4-F), (3-Cl-5-F) oder (4-CI-3-F) oder auch (3-CN-4-F), (3-Br-4-F) bedeuten.
Verbindungen der Formel (I) oder deren Salze, worin (R²)ₙ 2,6-Difluor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-CI-5-F) oder (4-Cl-3-F) oder auch (3-CN-4-F), (3-Br-4-F) bedeuten.
Verbindungen der Formel (I) oder deren Salze, worin (R²)ₙ 3,4-Difluor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-CI-3-F) oder auch (3-CN-4-F), (3-Br-4-F) bedeuten.
Verbindungen der Formel (I) oder deren Salze, worin (R²)ₙ 3,5-Difluor und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-Cl-3-F) oder auch (3-CN-4-F), (3-Br-4-F) bedeuten.
Verbindungen der Formel (I) oder deren Salze, worin (R²)ₙ (3-Cl-2-F) und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-CI-3-F) oder auch (3-CN-4-F), (3-Br-4-F) bedeuten.
Verbindungen der Formel (I) oder deren Salze, worin (R²)ₙ (3-CI-4-F) und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-Cl-3-F) oder auch (3-CN-4-F), (3-Br-4-F) bedeuten.
Verbindungen der Formel (I) oder deren Salze, worin (R²)ₙ (3-CI-5-F) und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-Cl-3-F) oder auch (3-CN-4-F), (3-Br-4-F) bedeuten.
Verbindungen der Formel (I) oder deren Salze, worin (R²)ₙ (3-Cl-6-F) und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-Cl-3-F) oder auch (3-CN-4-F), (3-Br-4-F) bedeuten.
Verbindungen der Formel (I) oder deren Salze, worin (R²)ₙ (4-CI-2-F) und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-CI-3-F) oder auch (3-CN-4-F), (3-Br-4-F) bedeuten.
Verbindungen der Formel (I) oder deren Salze, worin (R²)ₙ (4-Cl-3-F) und (R¹)ₘ 3-Chlor, 4-Chlor, 3-Fluor, 4-Fluor, 3-Cyano, 3,4-Difluor, 3,5-Difluor, 3,4-Dichlor, 3,5-Dichlor, (3-Cl-4-F), (3-Cl-5-F) oder (4-Cl-3-F) oder auch (3-CN-4-F), (3-Br-4-F) bedeuten.

Weiter bevorzugt sind Verbindungen der Formel (I) oder deren Salze, worin m = 1 bedeutet.

Weiter bevorzugt sind Verbindungen der Formel (I) oder deren Salze, worin m = 1 und R² Halogen, wie Fluor oder Chlor, bedeuten,

Weiter bevorzugt sind Verbindungen der Formel (I) oder deren Salze, worin m = 2 oder 3, insbesondere 2 bedeutet.

Weiter bevorzugt sind Verbindungen der Formel (I) oder deren Salze, worin m = 2 und jedes R² aus der Gruppe Halogen ausgewählt ist und vorzugsweise Fluor oder Chlor, insbesondere Fluor bedeutet.

Generell sind unter den Verbindungen mit den obengenannten Bedeutungen für einzelne Gruppen oder Kombinationen von Gruppen (R¹)ₘ beziehungsweise (R²)ₙ solche bevorzugt, in denen die übrigen Gruppen oder Kombinationen von Gruppen in den Verbindungen gemäß bevorzugt genannten Bedeutungen definiert sind.

Bevorzugt sind auch Verbindungen (I), worin
- L: einen Rest der Formel 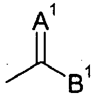 bedeutet,
- A¹: Sauerstoff, Schwefel oder =N-R^{A}, vorzugsweise Sauerstoff bedeutet,
- B¹: einen Rest der genannten Formeln (B1) bis (B14) bedeutet,
- R^{A}: die obengenannte Bedeutung hat, vorzugsweise Wasserstoff, Hydroxy, (C₁-C₄)Alkoxy, (C₁-C₄)Alkyl, (C₁-C₄)Haloalkyl, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder Benzyl, das im Phenylteil unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder -NR*R**, wobei R*, R** jeweils unabhängig voneinander H, (C₁-C₄)Alkyl, (C₂-C₄)Alkenyl, (C₂-C₄)Alkinyl, (C₁-C₄)Alkoxy-(C₁-C₄)alkyl, (C₁-C₄)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy oder im Fall Cycloalkyl auch Oxo substituiert ist, bedeuten oder
R* und R** zusammen mit dem N-Atom einen 3- bis 6-gliedrigen und vorzugsweise gesättigten Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten, bedeutet,
insbesondere R^{A} Wasserstoff, (C₁-C₄)Alkyl, Phenyl, Benzyl, oder -NR*R**, wobei R*, R** jeweils unabhängig voneinander H oder (C₁-C₄)Alkyl bedeuten, bedeutet,
- (R¹)ₘ, (R²)ₙ, R³, R⁴, R⁵, R⁶, R⁷, R⁸, R⁹, R¹⁰, R¹¹, R¹², R¹³, R¹⁴, R¹⁵, R¹⁶ R¹⁷, R¹⁸, R¹⁹,R²⁰, R²¹, R²², R²³, R²⁴, R²⁵, R²⁵, R²⁷, R²⁸, R²⁹, R³⁰, R³¹ und R³²: die obengenannten Bedeutungen bzw. bevorzugten Bedeutungen haben,
- W¹ und W⁶: jeweils unabhängig voneinander die divalente Gruppe Sauerstoff, Schwefel oder eine Gruppe der Formel NH, N-[(C₁-C₄)Alkyl], C=O, S=O, SO₂ oder CR³⁵R³⁶ bedeuten,
- W², W³, W⁴, W⁵ und W⁷: jeweils unabhängig voneinander die divalente Gruppe der Formel O, S, NH, N-[(C₁-C₄)Alkyl], C=O, S=O oder SO₂ bedeuten,
- Q¹, Q², Q³, Q⁴, Q⁵, Q⁶, Q⁷, Q⁸ und Q⁹: jeweils unabhängig voneinander Hydroxy, Thio, Halogen oder eine Gruppe der Formel OR³⁷, SR³⁸, SM oder OM bedeuten,
- M: ein Äquivalent eines Kations,
- R³⁵ und R³⁶: jeweils unabhängig voneinander wie R³ definiert sind oder vorzugsweise unabhängig voneinander H, (C₁-C₄)Alkyl oder (C₁-C₄)Halogenalkyl, vorzugsweise H oder (C₁-C₂)Alkyl, insbesondere H, Methyl oder Ethyl bedeuten,
- R³⁷, R³⁸, R⁶⁶, P⁶⁷, R⁶⁸, R⁶⁹, R⁷⁰, R⁷¹ Her¹² und M: die obengenannten Bedeutungen haben, vorzugsweise
- R³⁷ und R³⁸: jeweils unabhängig voneinander (C₁-C₆)Alkyl, (C₁-C₆)Haloalkyl, (C₁-C₄)Alkoxy-(C₁-C₄)alkyl, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, Phenyl, das unsubstituiert oder durch einen oder mehrere Reste aus der Gruppe Halogen, Cyano, Hydroxy, Nitro, (C₁-C₄)Alkyl, (C₁-C₄)Haloalkyl und (C₁-C₄)Alkoxy substituiert ist, oder eine Gruppe der Formel -C(O)R⁶⁷, -C(O)OR⁶⁸, -C(O)NR⁶⁹R⁷⁰, -C(O)Het¹² oder-SO₂R⁷¹ bedeuten,
insbesondere R³⁷ und R³⁸ jeweils unabhängig voneinander (C₁-C₆)Alkyl, (C₁-C₄)Halogenalkyl, (C₃-C₆)Cycloalkyl, (C₃-C₆)Halogencycloalkyl, Phenyl, das unsubstituiert oder durch einen oder mehrere Reste aus der Gruppe Halogen und (C₁-C₄)Alkyl substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
- R⁶⁷: Wasserstoff, (C₁-C₆)Alkyl, (C₁-C₄)Haloalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl und (C₁-C₄)Haloalkyl substituiert ist, insbesondere H oder (C₁-C₄)Alkyl bedeutet,
- R⁶⁸: (C₁-C₆)Alkyl oder (C₁-C₄)Haloalkyl, insbesondere (C₁-C₄)Alkyl bedeutet,
- R⁶⁹ und R⁷⁰: jeweils unabhängig voneinander Wasserstoff, (C₁-C₄)Alkyl, Benzyl, (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, substituiert ist, bedeuten oder insbesondere jeweils unabhängig voneinander H oder (C₁-C₃)Alkyl bedeuten,
- R⁷¹: (C₁-C₄)Alkyl, (C₁-C₄)Halogenalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl und (C₁-C₄)Haloalkyl substituiert ist, bedeutet, insbesondere (C₁-C₄)Alkyl, (C₁-C₄)Halogenalkyl, Benzyl oder Phenyl, das unsubstituiert oder durch einen oder mehrere Reste aus der Gruppe Halogen, (C₁-C₄)Alkyl und (C₁-C₄)Haloalkyl substituiert ist, bedeutet,
- Het¹²: einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, insbesondere unsubstituiert oder durch einen oder mehrere Reste aus der Gruppe (C₁-C₄)Alkyl und (C₁-C₄)Halogenalky) substituiert ist, insbesondere eine Morpholino- Piperidino- oder Pyrrolidinogruppe bedeutet und
- M: ein Äquivalent eines Kations, vorzugsweise ein Metallionenäquivalent, ein Ammoniumion, dass gegebenenfalls durch 1 bis 4 gleiche oder verschiedene Reste aus der Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, oder ein tertiäres Sulfoniumion, das vorzugsweise durch 3 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, bedeutet.

Bevorzugt sind auch Verbindungen (I), worin
- B¹: einen Rest der genannten Formeln (B1) bis (B14) bedeutet,
- R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹ und R²⁰: jeweils unabhängig voneinander H, Halogen, Cyano, Nitro, Hydroxy, (C₁-C₄)Alkyl, (C₁-C₄)Haloalkyl, (C₃-C₆)Cycloalkyl, (C₁-C₄)Alkoxy oder (C₁-C₄)Haloalkoxy, insbesondere H oder (C₁-C₄)Alkyl bedeuten oder R³ und R⁵ in der Gruppe der Formel (B1) gemeinsam eine wie oben definierte divalente Brücke bedeuten,
- R²¹: H, (C₁-C₄)Alkyl, (C₁-C₄)Haloälkyl, (C₃-C₆)Cycloalkyl, (C₃-C₆)Halogencycloalkyl oder eine Gruppe CCOOR⁵⁴, C(O)NR⁵⁵R⁵⁶, C(O)Het⁷, NR⁵⁷R⁵⁸ oder Het⁸, vorzugsweise (C₁-C₄)Alkyl, (C₁-C₄)Haloalkyl, (C₃-C₆)Cycloalkyl oder eine Gruppe C(O)OR⁵⁴ bedeutet,
- R²²: H, (C₁-C₄)Alkyl, (C₁-C₄)Halogenalkyl, (C₃-C₆)Cycloalkyl, (C₃-C₆)Halogencycloalkyl, (C₁-C₄)Alkylthio, (C₁-C₄)Alkylsulfonyl oder eine Gruppe der Formel C(O)OR⁵⁹, C(O)NR⁶⁰R⁶¹, C(O)Het⁹, NR⁶²R⁶³ oder Het¹⁰ , vorzugsweise (C₁-C₄)Alkyl, (C₁-C₄)Haloalkyl, (C₃-C₆)Cycloalkyl, (C₁-C₄)Alkylsülfonyl oder eine Gruppe C(O)OR⁵⁹ bedeutet,
- R²³ und R²⁹: jeweils unabhängig voneinander H, (C₁-C₄)Alkyl, (C₁-C₄)Halogenalkyl, (C₃-C₆)Cycloalkyl, (C₃-C₆)Halogencycloalkyl, (C₁-C₄)Alkoxy oder (C₁-C₄)Halogenalkoxy, vorzugsweise H, (C₁-C₄)Alkyl, (C₁-C₄)Halogenalkyl, (C₃-C₆)Cycloalkyl, beispielsweise Cyclopropyl, (C₁-C₄)Alkoxy oder (C₁-C₂)Haloalkoxy bedeuten,
- R²⁴ und R³⁰: jeweils unabhängig voneinander H, Halogen, Cyano, (C₁-C₄)Alkyl, (C₁-C₄)Halogenalkyl, (C₃-C₆)Cycloalkyl, (C₁-C₄)Alkoxy oder (C₁-C₄)Halogenalkoxy, vorzugsweise H, Halogen, Cyano, (C₁-C₄)Alkyl, (C₁-C₄)Halogenalkyl, (C₃-C₆)Cycloalkyl, beispielsweise Cyclopropyl, (C₁-C₄)Alkoxy oder (C₁-C₂)Haloalkoxy bedeuten,
- R²⁵, R²⁶, R³¹ und R³²: jeweils unabhängig voneinander H, (C₁-C₄)Alkyl, (C₁-C₄)Halogenalkyl, (C₁-C₄)Alkoxy, (C₁-C₄)Haloalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Cycloalkoxy, (C₂-C₆)Alkenyl, (C₂-C₆)Alkinyl, (C₁-C₄)Alkylthio, (C₁-C₄)Alkyisulfonyl, NR⁶⁴R⁶⁵ oder Het¹¹, vorzugsweise H, (C₁-C₄)Alkyl, (C₁-C₄)Halogenalkyl, (C₁-C₄)Alkoxy, (C₁-C₄)Haloalkoxy, (C₁-C₂)Alkoxy-(C₁-C₂)alkyl, (C₁-C₂)Halogenalkoxy-(C₁-C₂)alkyl oder (C₃-C₆)Cycloalkyl, beispielsweise Cyclopropyl, bedeuten,
- R²⁷: H, (C₁-C₄)Alkyl, (C₁-C₄)Halogenalkyl oder (C₃-C₆)Cycloalkyl, beispielsweise Cyclopropyl, vorzugsweise H oder (C₁-C₃)Alkyl bedeutet,
- R²⁸: H, (C₁-C₄)Alkyl, (C₁-C₄)Halogenalkyl oder einen Rest der Formel C(O)OR⁶⁶, vorzugsweise H oder (C₁-C₃)Alkyl bedeutet,
- R⁵⁴, R⁵⁹: und R⁶⁶ jeweils unabhängig voneinander H, (C₁-C₄)Alkyl, (C₁-C₄)Halogenalkyl, (C₃-C₆)Cycloalkyl, (C₂-C₄)Alkenyl, (C₂-C₄)Alkinyl oder die genannte Gruppe M bedeuten, vorzugsweise H oder (C₁-C₃)Alkyl,
- R⁵⁵, R⁵⁶, R⁵⁷, R⁵⁸, R⁶⁰, R⁶¹, R⁶², R⁶³, R⁶⁴ und R⁶⁵: jeweils unabhängig voneinander H, (C₁-C₄)Alkyl, das unsubstituiert oder durch einen oder mehrere Reste aus der Gruppe Halogen, Nitro, Cyano und Phenyl, das unsubstitutiert oder durch einen oder mehrere Reste aus der Gruppe Halogen, Nitro, Cyano, (C₁-C₄)Alkyl und (C₁-C₄)Haloalkyl substituiert ist, substituiert ist, oder (C₃-C₆)Cycloalkyl, das unsubstituiert oder durch einen oder mehrere Reste aus der Gruppe Halogen und (C₁-C₄)Alkyl und (C₁-C₄)Haloalkyl substituiert ist, oder Phenyl, das unsubstituiert oder durch einen oder mehrere Reste aus der Gruppe Halogen, Nitro, Cyano, (C₁-C₄)Alkyl und (C₁-C₄)Haloalkyl substituiert ist, bedeuten
- Het⁷, Het⁸, Het⁹, Het¹⁰ und Het¹¹: jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, insbesondere unsubstituiert oder durch einen oder mehrere Reste aus der Gruppe (C₁-C₄)Alkyl und (C₁-C₄)Halogenalkyl substituiert ist, insbesondere eine Morpholino- Piperidino- oder Pyrrolidinogruppe bedeuten.

Bevorzugt sind dabei Verbindungen (I), worin B¹ einen Rest (B1) bedeutet, 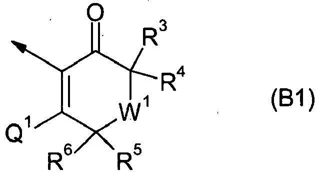 in welcher
W¹, Q¹, R³, R⁴, R⁵ und R⁶, die obengenannten Bedeutungen bzw. bevorzugten Bedeutungen haben, vorzugsweise
- W¹: die divalente Gruppe der Formel O, S, NH, N-[(C₁-C₄)Alkyl], C=O, S=O, SO₂ oder CR³⁵R³⁶, insbesondere die divalente Gruppe der Formel O, S, C=O oder CR³⁵R³⁶ bedeutet,
wobei R³⁵ und R³⁶ jeweils unabhängig voneinander Wasserstoff, (C₁-C₄)Alkyl oder (C₁-C₄)Halogenalkyl bedeuten,
insbesondere W¹ die divalente Gruppe C(O), CH₂, CH(CH₃) oder C(CH₃)₂ bedeutet,
- Q¹: Hydroxy, Thio, Halogen oder eine Gruppe der Formel OR³⁷, SR³⁸, SM oder OM bedeutet, insbesondere Hydroxy bedeutet,
- R³, R⁴, R⁵ und R⁶: jeweils unabhängig voneinander H, Halogen, Cyano, Nitro, Hydroxy, (C₁-C₄)Alkyl, (C₁-C₄)Haloalkyl, (C₁-C₄)Alkoxy oder (C₁-C₄)Haloalkoxy, insbesondere Wasserstoff oder (C₁-G₄)Alkyl bedeuten oder
R³ und R⁵ gemeinsam eine divalente Brücke der Formel -CH₂CH₂-, -CH₂CH₂CH₂-, -CH(CH₃)CH₂-, -C(CH₃)₂CH₂-, -CH₂CH(CH₃)-, -CH₂C(CH₃)₂-, -C(CH₃)CH(CH₃)-, -CH₂CH₂CH₂-, -CH=CH-, -CH=CHCH₂-, -CH₂CH=CH-, -OCH₂-, -CH₂O-, -OCH₂CH₂-, -CH₂OCH₂-, -CH₂CH₂O- oder -OCH₂O-, insbesondere der Formel -CH₂CH₂- oder -CH=CH- bedeuten,
- R³⁷, R³⁸, R⁶⁷ R⁶⁸, R⁶⁹, R⁷⁰, R⁷¹, Het¹² und M: die obengenannten Bedeutungen haben, vorzugsweise
- R³⁷ und R³⁸: jeweils unabhängig voneinander (C₁-C₆)Alkyl, (C₁-C₆)Haloalkyl, (C₁-C₄)Alkoxy-(C₁-C₄)alkyl, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, Phenyl, das unsubstituiert oder durch einen oder mehrere Reste aus der Gruppe Halogen, Cyano, Hydroxy, Nitro, (C₁-C₄)Alkyl, (C₁-C₄)Haloalkyl und (C₁-C₄)Alkoxy substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
insbesondere R³⁷ und R³⁸ jeweils unabhängig voneinander (C₁-C₆)Alkyl, (C₁-C₄)Halogenalkyl, (C₃-C₆)Cycloalkyl, (C₃-C₆)Halogencycloalkyl, Phenyl, das unsubstituiert oder durch einen oder mehrere Reste aus der Gruppe Halogen und (C₁-C₄)Alkyl substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten, weiter bevorzugt R³⁷ und R³⁸ jeweils unabhängig voneinander (C₁-C₄)Alkyl, Phenyl, das unsubstituiert öder durch einen oder mehrere Reste aus der Gruppe Halogen und (C₁-C₄)Alkyl substituiert ist, oder eine Gruppe der Formel -C(O)R⁶⁷, -C(O)OR⁶⁸ oder -SO₂R⁷¹ bedeuten,
- R⁶¹: Wasserstoff, (C₁-C₆)Alkyl, (C₁-C₄)Haloalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl und (C₁-C₄)Haloalkyl substituiert ist, insbesondere H oder (C₁-C₄)Alkyl, , beispielsweise H, Methyl, Ethyl, n-Propyl, i-Propyl, n-Butyl, sec-Butyl, iso-Butyl oder t-Butyl bedeutet,
- R⁶⁸: (C₁-C₆)Alkyl oder (C₁-C₄)Haloalkyl, insbesondere (C₁-C₄)Alkyl bedeutet,
- R⁶⁹ und R⁷⁰: jeweils unabhängig voneinander Wasserstoff, (C₁-C₄)Alkyl, Benzyl, (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, substituiert ist, bedeuten oder insbesondere jeweils unabhängig voneinander H oder (C₁-C₃)Alkyl bedeuten,
- R⁷¹: (C₁-C₄)Alkyl, (C₁-C₄)Halogenalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl und (C₁-C₄)Haloalkyl substituiert ist, bedeutet, insbesondere (C₁-C₄)Alkyl, (C₁-C₄)Halogenalkyl, Benzyl oder Phenyl, das unsubstituiert oder durch einen oder mehrere Reste aus der Gruppe Halogen, (C₁-C₄)Alkyl und (C₁-C₄)Haloalkyl substituiert ist, bedeutet,
- Het¹²: eine Morpholino- Piperidino- oder Pyrrolidinogruppe bedeutet und
- M: ein Äquivalent eines Kations, vorzugsweise ein Metallionenäquivalent, ein Ammoniumion, dass gegebenenfalls durch 1 bis 4 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, oder ein tertiäres Sulfoniumion, das vorzugsweise durch 3 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, bedeutet.

Beispiele für bevorzugte Reste B¹ der Formel (B1) sind die Reste der Formeln (B1-1) bis (B1-11).

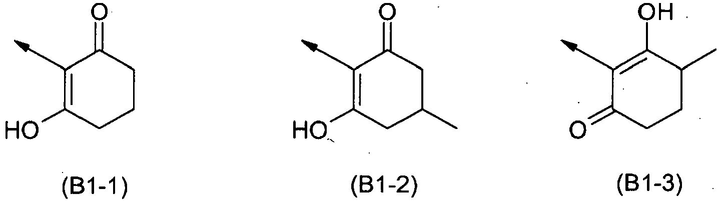

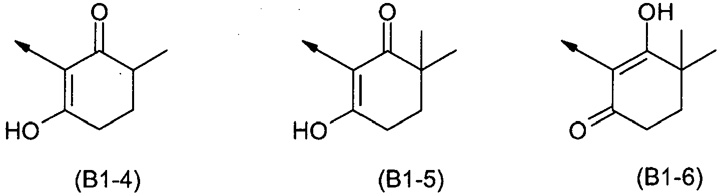

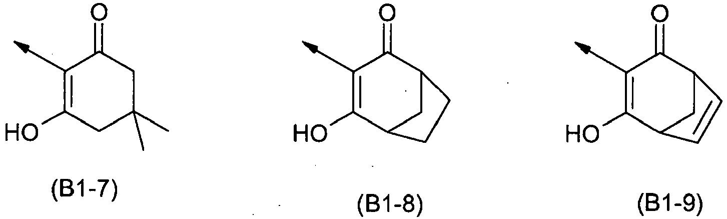

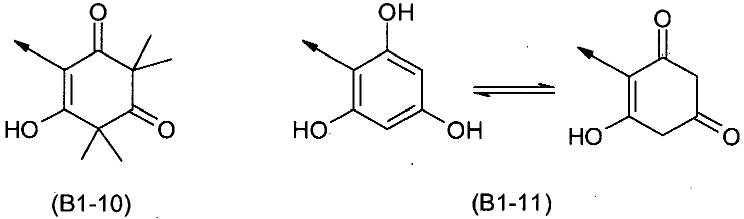

In den Formeln (B1-1) bis (B1-11) sind die Substituenten "Methyl" am Ringsystem in der üblichen chemischen Kurzschreibweise als Strich angeben, der von einem Ring-C-atom ausgeht. Diese Schreibweise wird auch in nachfolgenden Formeln zu Resten B¹ verwendet.

Die Formel (B1-11) umfasst auch Tautomeren, wobei das links gezeichnete das thermodynamisch stabilere darstellt, während das rechts gezeichnete das formal der allgemeinen Formel (B1) genau entsprechende darstellt, wenn Q¹ = OH und W¹ = C(O) bedeuten.

Bevorzugt sind dabei Verbindungen (I), worin B¹ einen Rest (B4) bedeutet, 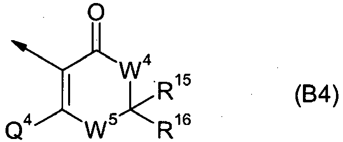 in welcher
W⁴, W⁵, Q⁴, R¹⁵ und R¹⁶, die obengenannten Bedeutungen bzw. bevorzugten Bedeutungen haben, vorzugsweise
- W⁴ und W⁵: jeweils unabhängig voneinander die divalente Gruppe der Formel O, S, NH, N-[(C₁-C₄)Alkyl], C=O, S=O oder SO₂, insbesondere die divalente Gruppe der Formel O oder S, insbesondere O bedeutet,
- Q⁴: Hydroxy, Thio, Halogen oder eine Gruppe der Formel OR³⁷, SR³⁸, SM oder OM bedeutet, insbesondere Hydroxy bedeutet,
- R¹⁵ und R¹⁶: jeweils unabhängig voneinander H, Halogen, Cyano, Nitro, Hydroxy, (C₁-C₄)Alkyl, (C₁-C₄)Haloalkyl, (C₁-C₄)Alkoxy oder (C₁-C₄)Haloalkoxy, insbesondere Wasserstoff oder (C₁-C₄)Alkyl bedeuten,
- R³⁷, R³⁸ und M: die obengenannten Bedeutungen haben, vorzugsweise
- R³⁷ und R³⁸: jeweils unabhängig voneinander (C₁-C₄)Alkyl, (C₁-C₄)Haloalkyl, (C₁-C₄)Alkoxy-(C₁-C₄)alkyl, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, Phenyl, das unsubstituiert oder durch einen oder mehrere Reste aus der Gruppe Halogen, Cyano, Hydroxy, Nitro, (C₁-C₄)Alkyl, (C₁-C₄)Haloalkyl und (C₁-C₄)Alkoxy substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
insbesondere R³⁷ und R³⁸ jeweils unabhängig voneinander (C₁-C₆)Alkyl, (C₁-C₄)Halogenalkyl, (C₃-C₆)Cycloalkyl, (C₃-C₆)Halogencycloalkyl, Phenyl, das unsubstituiert oder durch einen oder mehrere Reste aus der Gruppe Halogen und (C₁-C₄)Alkyl substituiert ist, oder eine Gruppe der Formel -C(O)R⁶⁷, -C(O)OR⁶⁸, -C(O)NR⁶⁹R⁷⁰, -C(O)Het¹² oder-SO₂R⁷¹ bedeuten, weiter bevorzugt R³⁷ und R³⁸ jeweils unabhängig voneinander (C₁-C₄)Alkyl, Phenyl, das unsubstituiert oder durch einen oder mehrere Reste aus der Gruppe Halogen und (C₁-C₄)Alkyl substituiert ist, oder eine Gruppe der Formel -C(O)R⁶⁷, -C(O)OR⁶⁸ oder -SO₂R⁷¹ bedeuten,
- R⁶⁷, R⁶⁸, R⁶⁹, R⁷⁰, R⁷¹, Het¹² und M: die obengenannten Bedeutungen haben, vorzugsweise
- R⁶⁷: Wasserstoff, (C₁-C₆)Alkyl, (C₁-C₄)Haloalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl und (C₁-C₄)Haloalkyl substituiert ist, insbesondere H oder (C₁-C₄)Alkyl bedeutet,
- R⁶⁸: (C₁-C₆)Alkyl oder (C₁-C₄)Haloalkyl, insbesondere (C₁-C₄)Alkyl bedeutet,
- R⁶⁹ und R⁷⁰: jeweils unabhängig voneinander Wasserstoff, (C₁-C₄)Alkyl, Benzyl, (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, substituiert ist, bedeuten oder insbesondere jeweils unabhängig voneinander H oder (C₁-C₃)Alkyl bedeuten,
- R⁷¹: (C₁-C₄)Alkyl, (C₁-C₄)Halogenalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl und (C₁-C₄)Haloalkyl substituiert ist, bedeutet, insbesondere (C₁-C₄)Alkyl, (C₁-C₄)Halogenalkyl, Benzyl oder Phenyl, das unsubstituiert oder durch einen oder mehrere Reste aus der Gruppe Halogen, (C₁-C₄)Alkyl und (C₁-C₄)Haloalkyl substituiert ist, bedeutet,
- Het¹²: eine Morpholino- Piperidino- oder Pyrrolidinogruppe bedeutet und
- M: ein Äquivalent eines Kations, vorzugsweise ein Metallionenäquivalent, ein Ammoniumion, dass gegebenenfalls durch 1 bis 4 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, oder ein tertiäres Sulfoniumion, das vorzugsweise durch 3 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, bedeutet.

Beispiele für einen bevorzugte Reste B¹ der Formel (B4) ist der Reste der Formel (B4-1).

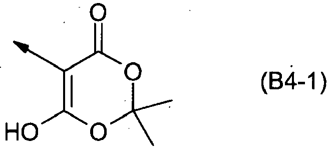

Bevorzugt sind auch Verbindungen (I), worin B¹ einen Rest (B5) oder (B6) bedeutet, 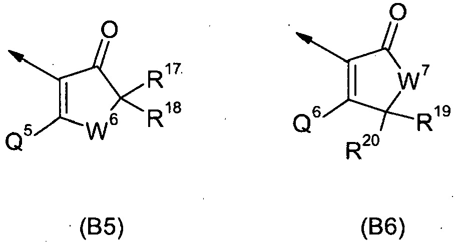 worin
W⁶, W⁷, Q⁵, Q⁶, R¹⁷, R¹⁸, R¹⁹ und R²⁰, die obengenannten Bedeutungen bzw.
bevorzugten Bedeutungen haben, vorzugsweise
- W⁶: die divalente Gruppe der Formel Ö, S, NH, N-[(C₁-C₄)Alkyl], C=O, S=O, SO₂ oder eine Gruppe der Formel CR³⁵R³⁶, insbesondere die divalente Gruppe der Formel O, S, NH, N-[(C₁-C₄)Alkyl] oder eine Gruppe der Formel CR³⁵R³⁶, weiter bevorzugt O, NH, N-CH₃, N-C₂H₅ oder eine Gruppe der Formel CH₂, CH(CH₃), CH(C₂H₅) oder C(CH₃)₂, ganz besonders O oder CH₂ bedeutet,
- W⁷: die divalente Gruppe der Formel O, S, NH, N-[(C₁-C₄)Alkyl], C=O, S=O oder SO₂, insbesondere die divalente Gruppe der Formel O, S, NH oder N-[(C₁-C₄)Alkyl], insbesondere O bedeutet,
- Q⁵ und Q⁶: jeweils unabhängig voneinander Hydroxy, Thio, Halogen oder eine Gruppe der Formel OR³⁷, SR³⁸, SM oder OM bedeutet, insbesondere Hydroxy bedeutet,
- R¹⁷, R¹⁸, R¹⁹ und R²⁰: jeweils unabhängig voneinander H, Halogen, Cyano, Nitro, Hydroxy, (C₁-C₄)Alkyl, (C₁-C₄)Haloalkyl, (C₁-C₄)Alkoxy oder (C₁-C₄)Haloalkoxy, insbesondere Wasserstoff oder (C₁-C₄)Alkyl bedeuten,
- R³⁵ und R³⁶: jeweils unabhängig voneinander Wasserstoff, (C₁-C₄)Alkyl oder (C₁-C₄)Halogenalkyl, insbesondere H, Methyl oder Ethyl bedeuten,
- R³⁷, R³⁸ und M: die obengenannten Bedeutungen haben, vorzugsweise wie bei Verbindungen der Formel (B1) bzw. (B4) oben definiert sind.

Beispiele für bevorzugte Reste B¹ der Formeln (B5) und (B6) sind die Reste der Formeln (B5-1), (B5-2), (B5-3), (B5-4), (B5-5), (B5-6), (B5-7), (B5-8), (B5-9), (B6-1), (B6-2), (B6-3):

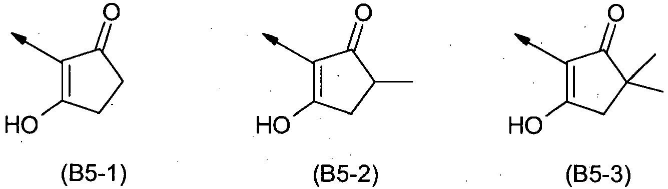

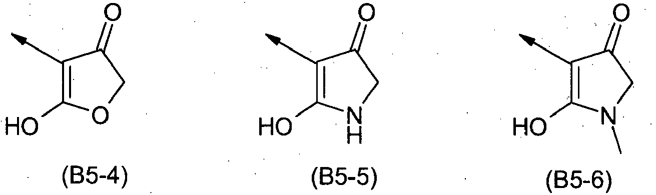

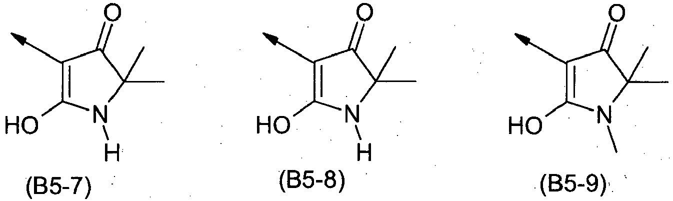

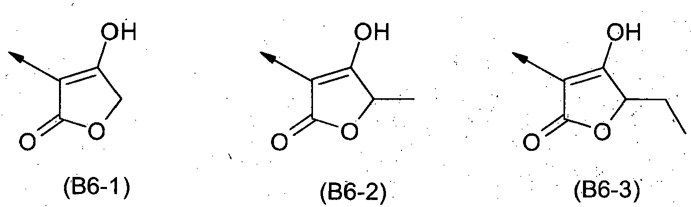

Bevorzugt sind auch Verbindungen (I), worin B¹ einen Rest (B7) bedeutet, 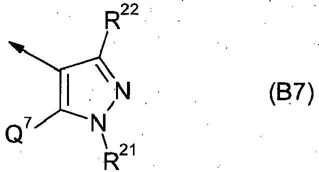 worin
Q⁷, R²¹ und R²², die obengenannten Bedeutungen bzw. bevorzugten Bedeutungen haben, vorzugsweise
- Q⁷: Hydroxy, Thio, Halogen oder eine Gruppe der Formel OR³⁷, SR³⁸, SM oder OM bedeutet, insbesondere Hydroxy bedeutet,
- R²¹: Wasserstoff, (C₁-C₄)Alkyl, (C₁-C₄)Halogenalkyl, (C₃-C₆)Cycloalkyl, (C₃-C₆)Halogencycloalkyl oder eine Gruppe C(O)OR⁵⁴, C(O)NR⁵⁵R⁵⁶, G(O)Het⁷, NR⁵⁷R⁵⁸ oder Het⁸ bedeutet, insbesondere H, (C₁-C₄)Alkyl, (C₁-C₄)Halogenalkyl oder (C₃-C₆)Cycloalkyl, beispielsweise Cyclopropyl bedeutet,
- R²²: Wasserstoff, (C₁-C₄)Alkyl, (C₁-C₄)Halogenalkyl, (C₃-C₆)Cycloalkyl, (C₃-C₆)Halogencycloalkyl, (C₁-C₄)Alkylthio, (C₁-C₄)Alkylsulfonyl, (C₁-C₄)Alkylsulfinyl oder eine Gruppe der Formel C(O)OR⁵⁹, C(O)NR⁶⁰R⁶¹, C(O)Het⁹, NR⁶²R⁶³ oder Het¹⁰ bedeutet, insbesondere H, (C₁-C₄)Alkyl, (C₁-C₄)Halogenalkyl, (C₃-C₆)Cycloalkyl, (C₃-C₆)Halogencycloalkyl, (C₁-C₄)Alkylthio, (C₁-C₄)Alkylsulfonyl oder eine Gruppe der Formel C(O)OR⁵⁹ bedeutet und
- R⁵⁹: Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeuten, vorzugsweise Wasserstoff, (C₁-C₃)Alkyl oder die genannte Gruppe M bedeuten,

Beispiele für bevorzugte Reste B¹ der Formel (B7) sind die Reste der Formeln (B7-1), (B7-2), (B7-3), (B7-4) und (B7-5):

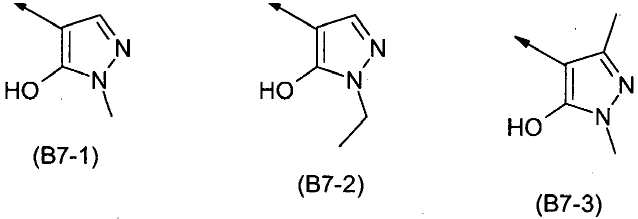

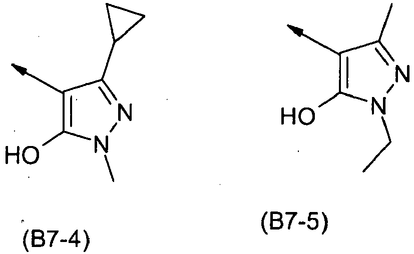

Bevorzugt sind auch Verbindungen (I), worin B¹ einen Rest (B8) bedeutet, 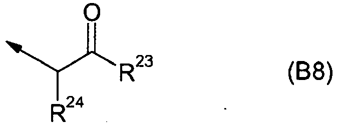 worin
R²³ und R²⁴, die obengenannten Bedeutungen bzw. bevorzugten Bedeutungen haben, vorzugsweise
- R²³: Wasserstoff, (C₁-C₄)Alkyl, (C₁-C₄)Halogenalkyl, (C₃-C₆)Cycloalkyl, (C₃-C₆)Halogencycloalkyl, (C₁-C₄)Alkoxy oder (C₁-C₄)Halogenalkoxy, insbesondere Wasserstoff, (C₁-C₄)Alkyl, (C₁-C₄)Halogenalkyl, (C₃-C₆)Cycloalkyl, (C₃-C₄)Halogencycloalkyl, (C₁-C₄)Alkoxy oder (C₁-C₄)Halogenalkoxy, weiter bevorzugt Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy oder t-Butoxy bedeutet,
- R²⁴: Wasserstoff, Halogen, Cyano, (C₁-C₄)Alkyl, (C₁-C₄)Halogenalkyl, (C₃-C₆)Cycloalkyl, beispielsweise Cyclopropyl, (C₁-C₄)Alkoxy oder (C₁-C₄)Halogenalkoxy, insbesondere Wasserstoff, (C₁-C₃)Alkyl, Cyano oder Cyclopropyl, weiter bevorzugt Cyano bedeutet.

Beispiele für bevorzugte Reste B¹ der Formel (B8) sind die Reste der Formeln (B8-1), (B8-2), (B8-3), (B8-4), (B8-5) und (B8-6):

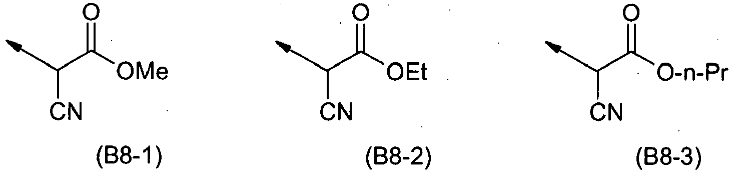

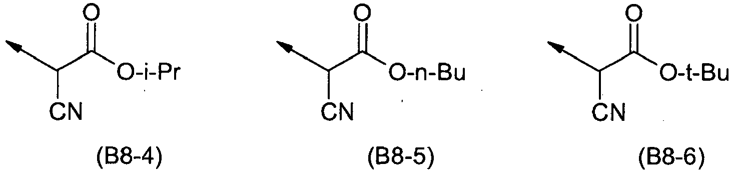

In den Formeln zu (B8-1) bis (B8-6) und entsprechend in anderen Beispielformeln bedeuten OMe = Methoxy, OEt = Ethoxy, O-n-Pr = n-Propoxy, O-i-Pr = Isopropoxy, O-n-Bu = n-Butoxy, O-i-Bu = i-Butoxy, O-s-Bu = sec-Butoxy und O-t-Bu = tert.-Butoxy.

Die Verbindungen der Formel (B8) sind tautomer zu den Verbindungen der Formel (B11) und (B12), wenn Q⁸ = OH oder OM und R²⁴ = R³⁰ und R²³ =R²⁹bedeuten. Vor allem wenn R²⁴ und R³⁰ jeweils CN bedeuten ist ein substantieller Anteil im Gleichgewicht durch das Tautomer der Formel (B11) beschrieben.

Bevorzugt sind auch Verbindungen (I), worin B¹ einen Rest (B9) bedeutet, 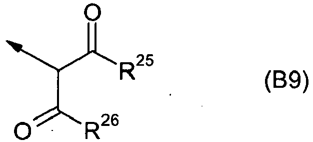 worin
- R²⁵ und R²⁶: jeweils unabhängig voneinander die obengenannten Bedeutungen oder bevorzugten Bedeutungen haben, weiter bevorzugt jeweils unabhängig voneinander Wasserstoff, (C₁-C₄)Alkyl, (C₁-C₄)Halogenalkyl, (C₁-C₄)Alkoxy, (C₁-C₄)Halogenalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Halogencycloalkyl, (C₃-C₆)Cycloalkoxy, (C₂-C₆)Alkenyl, (C₂-C₆)Halogenalkenyl, (C₂-C₆)Alkinyl, (C₂-C₆)Halogenalkinyl, (C₁-C₄)Alkylthio, (C₁-C₄)Alkylsulfonyl, (C₁-C₄)Alkylsulfinyl, NR⁶⁴R⁶⁵ oder Het¹¹ bedeuten oder weiter bevorzugt
- R²⁵: Wasserstoff, (C₁-C₄)Alkyl, (C₁-C₄)Alkoxy, (C₁-C₄)Halogenalkoxy oder (C₃-C₆)Cycloalkyl, beispielsweise Cyclopropyl, insbesondere (C₁-C₄)Alkoxy bedeutet und
- R²⁶: Wasserstoff, (C₁-C₄)Alkyl, (C₁-C₄)Alkoxy, (C₁-C₄)Halogenalkoxy oder (C₃-C₆)Cycloalkyl, beispielsweise Cyclopropyl, insbesondere (C₁-C₄)Alkoxy bedeutet.

Beispiele für bevorzugte Reste B¹ der Formel (B9) sind die Reste der Formeln (B9-1), (B9-2), (B9-3), (B9-4), (B9-5), (B9-6), (B9-7), (B9-8) und (B9-9):

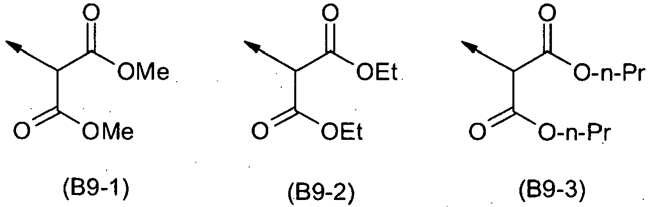

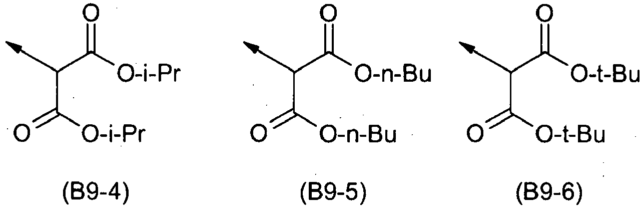

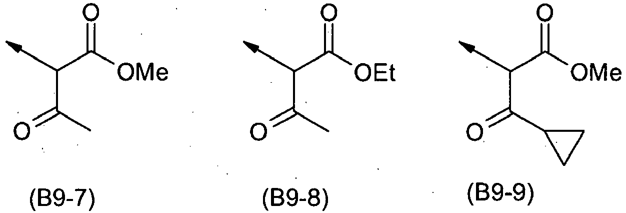

Die Verbindungen der Formel (B9) sind tautomer zu den Verbindungen der Formel (B13) und (B14), wenn Q⁹ = OH oder OM und R²⁵ = R³¹ und R²⁶ = R³² bedeuten.

Bevorzugt sind auch Verbindungen (I), worin B¹ einen Rest (B10) bedeutet, 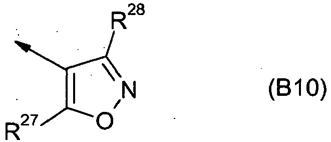 worin
R²⁷ und R²⁸, die obengenannten Bedeutungen bzw. bevorzugten Bedeutungen haben, vorzugsweise
- R²⁷: H, (C₁-C₄)Alkyl, (C₁-C₄)Halogenalkyl oder (C₃-C₆)Cycloalkyl, beispielsweise Cyclopropyl, bedeutet,
- R²⁸: H, (C₁-C₄)Alkyl, (C₁-C₄)Halogenalkyl oder einen Rest der Formel C(O)OR⁶⁶ bedeutet und
- R⁶⁶: Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeuten, vorzugsweise Wasserstoff, (C₁-C₃)Alkyl oder die genannte Gruppe M bedeuten.

Beispiele für bevorzugte Reste B¹ der Formel (B10) sind die Reste der Formeln (B10-1), (B10-2), (B10-3) und (B10-4):

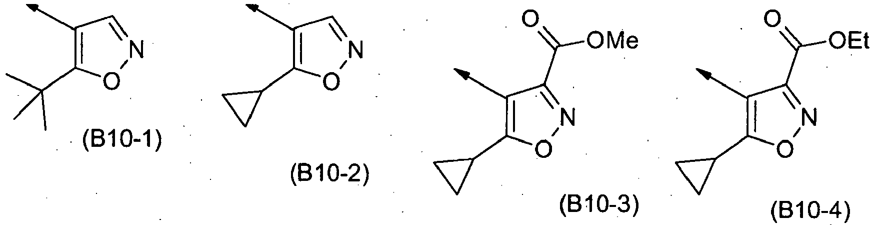

Bevorzugt sind auch Verbindungen (I), worin B¹ einen Rest (B11) bedeutet, 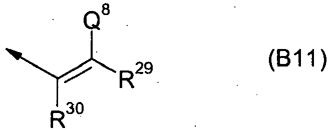 worin
Q⁸, R²⁹ und R³⁰, die obengenannten Bedeutungen bzw. bevorzugten Bedeutungen haben, vorzugsweise
- Q⁸: Hydroxy, Halogen oder eine Gruppe der Formel OR³⁷, SR³⁸ oder OM bedeutet, insbesondere Hydroxy bedeutet,
- R²⁹: H, (C₁-C₄)Alkyl, (C₁-C₄)Halogenalkyl, (C₃-C₆)Cycloalkyl, beispielweise Cyclopropyl, (C₁-C₄)Alkoxy oder (C₁-C₄)Halogenalkoxy bedeutet,
- R³⁰: H, Halogen, Cyano, (C₁-C₄)Alkyl, (C₁-C₄)Halogenalkyl, (C₃-C₆)Cycloalkyl, beispielsweise Cyclopropyl, (C₁-C₄)Alkoxy oder (C₁-C₄)Halogenalkoxy bedeutet.

Beispiele für bevorzugte Reste B¹ der Formel (B11) sind die Reste der Formeln (B11-1) und (B11-2):

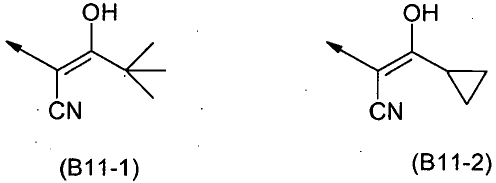

Bevorzugt sind auch Verbindungen (I), worin B¹ einen Rest (B13) bedeutet, 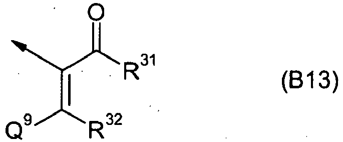 worin
Q⁹, R³¹ und R³², die obengenannten Bedeutungen bzw. bevorzugten Bedeutungen haben, vorzugsweise
- Q⁹: Hydroxy, Halogen oder eine Gruppe der Formel OR³⁷, SR³⁸ oder OM bedeutet, insbesondere Hydroxy bedeutet,
- R³¹ und R³²: jeweils unabhängig voneinander H, (C₁-C₄)Alkyl, (C₁-C₄)Halogenalkyl, (C₁-C₄)Alkoxy, (C₁-C₄)Halogenalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Halogencycloalkyl, (C₃-C₆)Cycloalkoxy, (C₁-C₄)Alkylthio, (C₁-C₄)Alkylsulfonyl, (C₁-C₄)Alkylsulfinyl, NR⁶⁴R⁶⁵ oder Het¹¹ bedeuten, insbesondere H, (C₁-C₄)Alkyl, (C₁-C₄)Halogenalkyl, (C₁-C₄)Alkoxy, (C₁-C₄)Halogenalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Halogencycloalkyl, (C₃-C₆)Cycloalkoxy, (C₁-C₄)Alkylthio bedeuten.

Beispiele für bevorzugte Reste B¹ der Formel (B13) sind die Reste der Formeln (B13-1), (B13-2), (B13-3), (B13-4), (B13-5), (B13-6), (B13-7), (B13-8) und (B13-9):

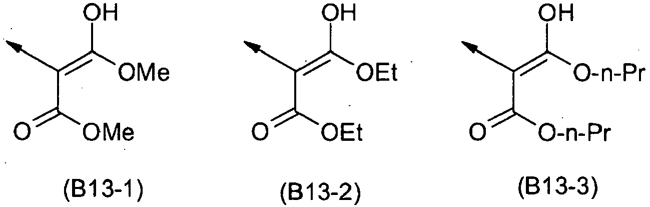

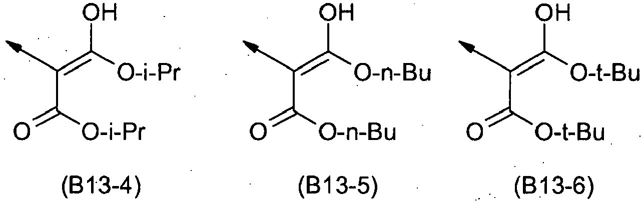

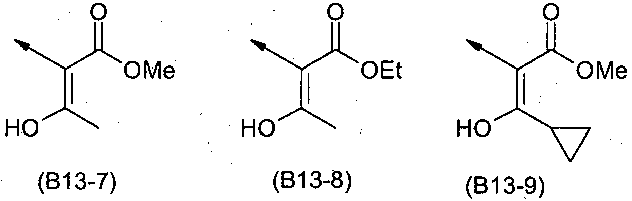

Bevorzugt sind auch Verbindungen (I), worin
- L: einen Rest der Formel 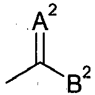
- A²: einen Rest der genannten Formeln (A1) bis (A12) bedeutet,
- B²: Hydroxy, Thio, Halogen oder eine Gruppe der Formel OR³³, OM, SR³⁴, SM oder -NR^{B}R^{C}, vorzugsweise Hydroxy, Halogen oder eine Gruppe der Formel OR³³, OM, SR³⁴ oder -NR^{B}R^{C} bedeutet,
- R^{B} und R^{C}: unabhängig voneinander die obengenannte Bedeutung haben, vorzugsweise unabhängig voneinander Wasserstoff, Hydroxy, (C₁-C₄)Alkoxy, (C₁-C₄)Alkyl, (C₁-C₄)Haloalkyl, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder Benzyl, das im Phenylteil unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder -NR*R**, wobei R*, R** jeweils unabhängig voneinander H, (C₁-C₄)Alkyl, (C₂-C₄)Alkenyl, (C₂-C₄)Alkinyl, (C₁-C₄)Alkoxy-(C₁-C₄)alkyl, (C₁-C₄)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy oder im Fall Cycloalkyl auch Oxo substituiert ist, bedeuten oder
R* und R** zusammen mit dem N-Atom einen 3- bis 6-gliedrigen und vorzugsweise gesättigten Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten oder insbesondere
R^{B} Wasserstoff, Hydroxy, (C₁-C₄)Alkoxy, (C₁-C₄)Alkyl, Phenyl, Benzyl oder -NR*R**, wobei R*, R** jeweils unabhängig voneinander H oder (C₁-C₄)Alkyl bedeuten, bedeutet und
R^{C} Wasserstoff, (C₁-C₄)Alkyl, Phenyl oder Benzyl bedeutet oder
die Gruppe -NR^{B}R^{C} einen Rest der Formel -NH₂, -NHOH, -NHOCH₃, -NHCH₃, -N(CH₃)₂, -NH-NH₂ oder -NH-N(CH₃)₂ bedeutet,
- (R¹)m, (R²)n, R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹,R²⁰, R²¹, R²², R²³, R²⁴, R²⁵ und R²⁶: die obengenannten Bedeutungen bzw. bevorzugten Bedeutungen haben,
- W¹ und W⁶: jeweils unabhängig voneinander die divalente Gruppe Sauerstoff, Schwefel oder eine Gruppe der Formel NH, N-[(C₁-C₄)Alkyl], C=O, S=O, SO₂ oder CR³⁵R³⁶ bedeuten,
- W², W³, W⁴, W⁵ und W⁷: jeweils unabhängig voneinander die divalente Gruppe der Formel O, S, NH, N-[(C₁-C₄)Alkyl], C=O, S=O oder SO₂ bedeuten,
- R³³, R³⁴ und M: die obengenannten Bedeutungen bzw. bevorzugten Bedeutungen haben, vorzugsweise
- R³³ und R³⁴: jeweils unabhängig voneinander (C₁-C₄)Alkyl, (C₁-C₄)Haloalkyl, (C₁-C₄)Alkoxy-(C₁-C₄)alkyl, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, Phenyl, das unsubstituiert oder durch einen oder mehrere Reste aus der Gruppe Halogen, Cyano, Hydroxy, Nitro, (C₁-C₄)Alkyl, (C₁-C₄)Haloalkyl und (C₁-C₄)Alkoxy substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
insbesondere R³³ und R³⁴ jeweils unabhängig voneinander (C₁-C₆)Alkyl, (C₁-C₄)Halogenalkyl, (C₃-C₆)Cycloalkyl, (C₃-C₆)Halogencycloalkyl, Phenyl, das unsubstituiert oder durch einen oder mehrere Reste aus der Gruppe Halogen und (C₁-C₄)Alkyl substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
- R³⁵ und R³⁶: jeweils unabhängig voneinander wie R³ definiert sind oder vorzugsweise unabhängig voneinander H, (C₁-C₄)Alkyl oder (C₁-C₄)Halogenalkyl, insbesondere H oder (C₁-C₂)Alkyl, insbesondere H, Methyl oder Ethyl bedeuten,
- R⁶⁷: Wasserstoff, (C₁-C₆)Alkyl, (C₁-C₄)Haloalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl und (C₁-C₄)Haloalkyl substituiert ist, insbesondere H oder (C₁-C₄)Alkyl bedeutet,
- R⁶⁸: (C₁-C₆)Alkyl oder (C₁-C₄)Haloalkyl, insbesondere (C₁-C₄)Alkyl bedeutet,
- R⁶⁹ und R⁷⁰: jeweils unabhängig voneinander Wasserstoff, (C₁-C₄)Alkyl, Benzyl, (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, substituiert ist, bedeuten oder insbesondere jeweils unabhängig voneinander H oder (C₁-C₃)Alkyl bedeuten,
- R⁷¹: (C₁-C₄)Alkyl, (C₁-C₄)Halogenalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl und (C₁-C₄)Haloalkyl substituiert ist, bedeutet, insbesondere (C₁-C₄)Alkyl, (C₁-C₄)Halogenalkyl, Benzyl oder Phenyl, das unsubstituiert oder durch einen oder mehrere Reste aus der Gruppe Halogen, (C₁-C₄)Alkyl und (C₁-C₄)Haloalkyl substituiert ist, bedeutet,
- Het¹²: einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, insbesondere unsubstituiert oder durch einen oder mehrere Reste aus der Gruppe (C₁-C₄)Alkyl und (C₁-C₄)Halogenalkyl substituiert ist, insbesondere eine Morpholino- Piperidino- oder Pyrrolidinogruppe bedeutet.

Bevorzugt sind auch Verbindungen (I), worin
- A²: einen Rest der genannten Formeln (A1) bis (A12) bedeutet,
- R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹ und R²⁰: jeweils unabhängig voneinander H, Halogen, Cyano, Nitro, Hydroxy, (C₁-C₄)Alkyl, (C₁-C₄)Haloalkyl, (C₃-C₆)Cycloalkyl, (C₁-C₄)Alkoxy oder (C₁-C₄)Haloalkoxy, insbesondere H oder (C₁-C₄)Alkyl bedeuten oder R³ und R⁵ in der Gruppe der Formel (A1) gemeinsam eine wie oben definierte divalente Brücke bedeuten,
- R²¹: H, (C₁-C₄)Alkyl, (C₁-C₄)Haloalkyl, (C₃-C₆)Cycloalkyl, (C₃-C₆)Halogencycloalkyl oder eine Gruppe C(O)OR⁵⁴, C(O)NR⁵⁵R⁵⁶, C(O)Het⁷, NR⁵⁷R⁵⁸ oder Het⁸, vorzugsweise (C₁-C₄)Alkyl, (C₁-C₄)Haloalkyl, (C₃-C₆)Cycloalkyl oder eine Gruppe C(O)OR⁵⁴ bedeutet,
- R²²: H, (C₁-C₄)Alkyl, (C₁-C₄)Halogenalkyl, (C₃-C₆)Cycloalkyl, (C₃-C₆)Halogencycloalkyl, (C₁-C₄)Alkylthio, (C₁-C₄)Alkylsulfonyl oder eine Gruppe der Formel C(O)OR⁵⁹, C(O)NR⁶⁰R⁶¹, C(O)Het⁹, NR⁶²R⁶³ oder Het¹⁰ , vorzugsweise (C₁-C₄)Alkyl, (C₁-C₄)Haloalkyl, (C₃-C₆)Cycloalkyl, (C₁-C₄)Alkylsulfonyl oder eine Gruppe C(O)OR⁵⁹ bedeutet,
- R²³: H, (C₁-C₄)Alkyl, (C₁-C₄)Halogenalkyl, (C₃-C₆)Cycloalkyl, (C₃-C₆)Halogencycloalkyl, (C₁-C₄)Alkoxy oder (C₁-C₄)Halogenalkoxy, vorzugsweise H, (C₁-C₄)Alkyl, (C₁-C₄)Halogenalkyl, (C₃-C₆)Cycloalkyl, beispielsweise Cyclopropyl, (C₁-C₄)Alkoxy oder (C₁-C₂)Haloalkoxy bedeutet,
- R²⁴: H, Halogen, Cyano, (C₁-C₄)Alkyl, (C₁-C₄)Halogenalkyl, (C₃-C₆)Cycloalkyl, (C₁-C₄)Alkoxy oder (C₁-C₄)Halogenalkoxy, vorzugsweise H, Halogen, Cyano, (C₁-C₄)Alkyl, (C₁-C₄)Halogenalkyl, (C₃-C₆)Cycloalkyl, beispielsweise Cyclopropyl, (C₁-C₄)Alkoxy oder (C₁-C₂)Haloalkoxy bedeutet,
- R²⁵ und R²⁶: jeweils unabhängig voneinander H, (C₁-C₄)Alkyl, (C₁-C₄)Halogenalkyl, (C₁-C₄)Alkoxy, (C₁-C₄)Haloalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Cycloalkoxy, (C₂-C₆)Alkenyl, (C₂-C₆)Alkinyl, (C₁-C₄)Alkylthio, (C₁-C₄)Alkylsulfonyl, NR⁶⁴R⁶⁵ oder Het¹¹, vorzugsweise H, (C₁-C₄)Alkyl, (C₁-C₄)Halogenalkyl, (C₁-C₄)Alkoxy, (C₁-C₄)Haloalkoxy, (C₁-C₂)Alkoxy-(C₁-C₂)alkyl, (C₁-C₂)Halogenalkoxy-(C₁-C₂)alkyl oder (C₃-C₆)Cycloalkyl, beispielsweise Cyclopropyl, bedeuten,
- R⁵⁴ und R⁵⁹: jeweils unabhängig voneinander H, (C₁-C₄)Alkyl, (C₁-C₄)Halogenalkyl, (C₃-C₆)Cycloalkyl, (C₂-C₄)Alkenyl, (C₂-C₄)Alkinyl oder die genannte Gruppe M bedeuten,
- R⁵⁵, R⁵⁶, R⁵⁷, R⁵⁸, R⁶⁰, R⁶¹, R⁶², R⁶³, R⁶⁴ und R⁶⁵: jeweils unabhängig voneinander H, (C₁-C₄)Alkyl, das unsubstituiert oder durch einen oder mehrere Reste aus der Gruppe Halogen, Nitro, Cyano und Phenyl, das unsubstitutiert oder durch einen oder mehrere Reste aus der Gruppe Halogen, Nitro, Cyano, (C₁-C₄)Alkyl und (C₁-C₄)Haloalkyl substituiert ist, substituiert ist, oder (C₃-C₆)Cycloalkyl, das unsubstituiert oder durch einen oder mehrere Reste aus der Gruppe Halogen und (C₁-C₄)Alkyl und (C₁-C₄)Haloalkyl substituiert ist, oder Phenyl, das unsubstituiert oder durch einen oder mehrere Reste aus der Gruppe Halogen, Nitro, Cyano, (C₁-C₄)Alkyl und (C₁-C₄)Haloalkyl substituiert ist, bedeuten
- Het⁷, Het⁸, Het⁹, Het¹⁰ und Het¹¹: jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, insbesondere unsubstituiert oder durch einen oder mehrere Reste aus der Gruppe (C₁-C₄)Alkyl und (C₁-C₄)Halogenalkyl substituiert ist, insbesondere eine Morpholino- Piperidino- oder Pyrrolidinogruppe bedeuten.

Bevorzugt sind dabei die Verbindungen (I), worin A² einen der Reste (A1) bis (A12) bedeutet, und
- B²: Hydroxy, Thio, Halogen oder eine Gruppe der Formel OR³³, SR³⁴, SM oder OM bedeutet, insbesondere Hydroxy, Halogen oder eine Gruppe der Formel OR³³, SR³⁴ oder OM bedeutet, weiter bevorzugt Hydroxy bedeutet,
- R³³, R³⁴ und M: die obengenannten Bedeutungen oder bevorzugten Bedeutungen haben, vorzugsweise
- R³³ und R³⁴: jeweils unabhängig voneinander (C₁-C₆)Alkyl, (C₁-C₆)Haloalkyl, (C₁-C₄)Alkoxy-(C₁-C₄)alkyl, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, Phenyl, das unsubstituiert oder durch einen oder mehrere Reste aus der Gruppe Halogen, Cyano, Hydroxy, Nitro, (C₁-C₄)Alkyl, (C₁-C₄)Haloalkyl und (C₁-C₄)Alkoxy substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
insbesondere R³⁷ und R³⁸ jeweils unabhängig voneinander (C₁-C₆)Alkyl, (C₁-C₄)Halogenalkyl, (C₃-C₆)Cycloalkyl, (C₃-C₆)Halogencycloalkyl, Phenyl, das unsubstituiert oder durch einen oder mehrere Reste aus der Gruppe Halogen und (C₁-C₄)Alkyl substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten, weiter bevorzugt R³⁷ und R³⁸ jeweils unabhängig voneinander (C₁-C₄)Alkyl, Phenyl, das unsubstituiert oder durch einen oder mehrere Reste aus der Gruppe Halogen und (C₁-C₄)Alkyl substituiert ist, oder eine Gruppe der Formel -C(O)RS⁷, -C(O)OR⁶⁸ oder -SO₂R⁷¹ bedeuten,
- R⁶⁷: Wasserstoff, (C₁-C₆)Alkyl, (C₁-C₄)Haloalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl und (C₁-C₄)Haloalkyl substituiert ist, insbesondere H oder (C₁-C₄)Alkyl, beispielsweise H, Methyl, Ethyl, n-Propyl, i-Propyl, n-Butyl, sec-Butyl, iso-Butyl oder t-Butyl bedeutet,
- R⁶⁸: (C₁-C₆)Alkyl oder (C₁-C₄)Haloalkyl, insbesondere (C₁-C₄)Alkyl bedeutet,
- R⁶⁹ und R⁷⁰: jeweils unabhängig voneinander Wasserstoff, (C₁-C₄)Alkyl, Benzyl, (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, substituiert ist, bedeuten oder insbesondere jeweils unabhängig voneinander H oder (C₁-C₃)Alkyl bedeuten,
- R⁷¹: (C₁-C₄)Alkyl, (C₁-C₄)Halogenalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl und (C₁-C₄)Haloalkyl substituiert ist, bedeutet, insbesondere (C₁-C₄)Alkyl, (C₁-C₄)Halogenalkyl, Benzyl oder Phenyl, das unsubstituiert oder durch einen oder mehrere Reste aus der Gruppe Halogen, (C₁-C₄)Alkyl und (C₁-C₄)Haloalkyl substituiert ist, bedeutet,
- Het¹²: eine Morpholino- Piperidino- oder Pyrrolidinogruppe bedeutet und
- M: ein Äquivalent eines Kations, vorzugsweise ein Metallionenäquivalent, ein Ammoniumion, dass gegebenenfalls durch 1 bis 4 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, oder ein tertiäres Sulfoniumion, das vorzugsweise durch 3 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl, insbesondere (C₁-C₄)Alkyl substituiert ist, bedeutet.

Bevorzugt sind dabei Verbindungen (I), worin A² einen Rest (A1) bedeutet, 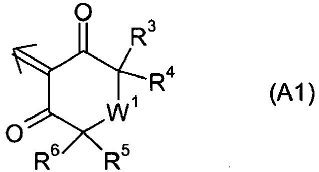 in welcher
W¹, R³, R⁴, R⁵ und R⁶, die obengenannten Bedeutungen bzw. bevorzugten Bedeutungen haben, vorzugsweise
- W¹: die divalente Gruppe der Formel O, S, NH, N-[(C₁-C₄)Alkyl], C=O, S=O, SO₂ oder CR³⁵R³⁶, insbesondere die divalente Gruppe der Formel O, S, C=O oder CR³⁵R³⁶ bedeutet,
wobei R³⁵ und R³⁶ jeweils unabhängig voneinander Wasserstoff, (C₁-C₄)Alkyl oder (C₁-C₄)Halogenalkyl bedeuten,
insbesondere W¹ die divalente Gruppe C(O), CH₂, CH(CH₃) oder C(CH₃)₂ bedeutet,
- R³, R⁴, R⁵ und R⁶: jeweils unabhängig voneinander H, Halogen, Cyano, Nitro, Hydroxy, (C₁-C₄)Alkyl, (C₁-C₄)Haloalkyl, (C₁-C₄)Alkoxy oder (C₁-C₄)Haloalkoxy, insbesondere Wasserstoff oder (C₁-C₄)Alkyl bedeuten oder
R³ und R⁵ gemeinsam eine divalente Brücke der Formel -CH₂CH₂-, -CH₂CH₂CH₂-, -CH(CH₃)CH₂-, -C(CH₃)₂CH₂-, -CH₂CH(CH₃)-, -CH₂C(CH₃)₂-, -C(CH₃)CH(CH₃)-, -CH₂CH₂CH₂-, -CH=CH-, -CH=CHCH₂-, -CH₂CH=CH-, -OCH₂-, -CH₂O-, -OCH₂CH₂-, -CH₂OCH₂-, -CH₂CH₂O- oder -OCH₂O-, insbesondere der Formel -CH₂CH₂- oder -CH=CH- bedeuten.

Beispiele für bevorzugte Reste A² der Formel (A1) sind die Reste der Formeln (A1-1) bis (A1-9):

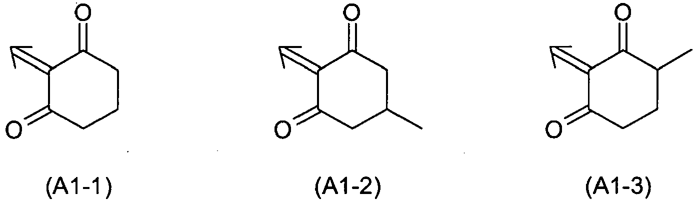

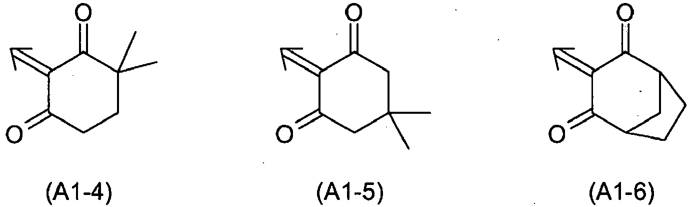

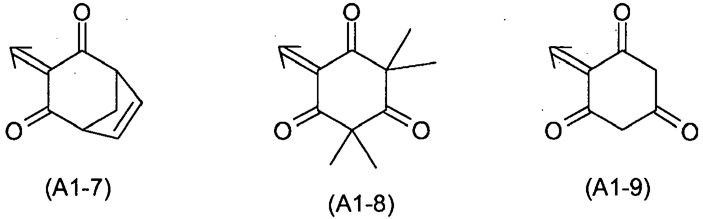

Im Falle der unsymmetrisch zur C-C-Doppelbindung substituierten Reste (A1), wie (A1-3) und (A1-4) sind entsprechende Doppelbindungsisomere, die von der allgemeinen Formel (A1) umfasst sind, ebenfalls bevorzugt.

Bevorzugt sind auch Verbindungen (I), worin A² einen Rest (A5) bedeutet, 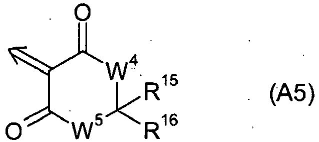 in welcher
W⁴, W⁵, R¹⁵ und R¹⁶, die obengenannten Bedeutungen bzw. bevorzugten Bedeutungen haben, vorzugsweise
- W⁴ und W⁵: jeweils unabhängig voneinander die divalente Gruppe der Formel O, S, NH, N-[(C₁-C₄)Alkyl], C=O, S=O oder SO₂, insbesondere die divalente Gruppe der Formel O oder S, insbesondere O bedeutet und
- R¹⁵ und R¹⁶: jeweils unabhängig voneinander H, Halogen, Cyano, Nitro, Hydroxy, (C₁-C₄)Alkyl, (C₁-C₄)Haloalkyl, (C₁-C₄)Alkoxy oder (C₁-C₄)Haloalkoxy, insbesondere Wasserstoff oder (C₁-C₄)Alkyl bedeuten.

Beispiele für bevorzugte Reste A² der Formel (A5) ist der Rest der Formel (A5-1):

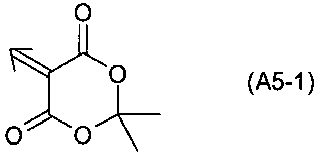

Bevorzugt sind auch Verbindungen (I), worin A² einen Rest (A6) oder (A7) bedeutet, 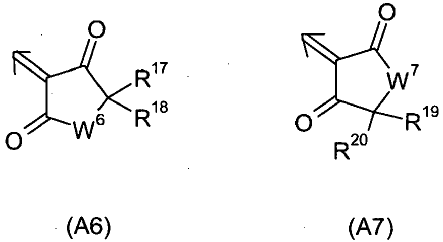 in welchen
W⁶, W⁷ , R¹⁷, R¹⁸, R¹⁹ und R²⁰, die obengenannten Bedeutungen oder bevorzugten Bedeutungen haben, vorzugsweise
- W⁶: die divalente Gruppe der Formel O, S, NH, N-[(C₁-C₄)Alkyl], C=O, S=O, SO₂ oder CR³⁵R³⁶, insbesondere die divalente Gruppe der Formel O, S, NH, N-[(C₁-C₄)Alkyl] oder eine Gruppe der Formel CR³⁵R³⁶, weiter bevorzugt O, NH, N-CH₃, N-C₂H₅ oder eine Gruppe der Formel CH₂, CH(CH₃), CH(C₂H₅) oder C(CH₃)₂, ganz besonders O oder CH₂ bedeutet, wobei R³⁵ und R³⁶ jeweils unabhängig voneinander Wasserstoff, (C₁-C₄)Alkyl oder (C₁-C₄)Halogenalkyl bedeuten, insbesondere W⁶ die divalente Gruppe CH₂, CH(CH₃) oder C(CH₃)₂ bedeutet,
- W⁷: die divalente Gruppe der Formel O, S, NH, N-[(C₁-C₄)Alkyl], C=O, S=O oder SO₂, insbesondere die divalente Gruppe der Formel O, S, NH oder N-[(C₁-C₄)Alkyl], insbesondere O bedeutet und
- R¹⁷, R¹⁸, R¹⁹ und R²⁰: jeweils unabhängig voneinander H, Halogen, Cyano, Nitro, Hydroxy, (C₁-C₄)Alkyl, (C₁-C₄)Haloalkyl, (C₁-C₄)Alkoxy oder (C₁₋C₄)Haloalkoxy, insbesondere H oder (C₁-C₄)Alkyl bedeuten.

Beispiele für bevorzugte Reste A² der Formel (A6) sind Reste der Formeln (A6-1) bis (A6-11):

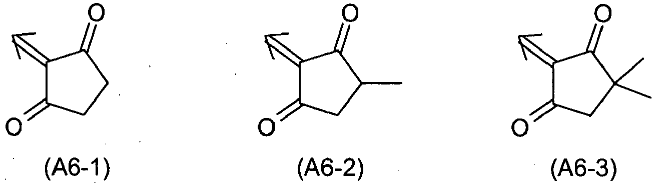

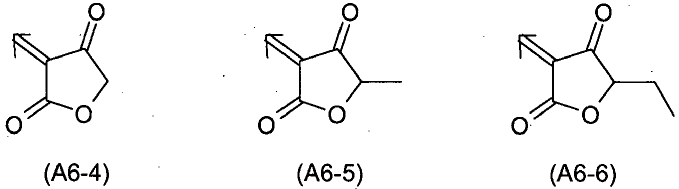

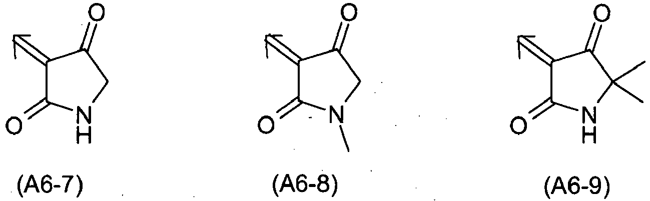

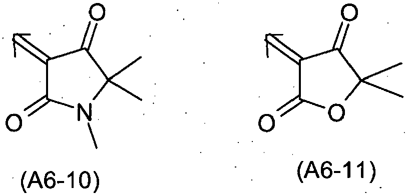

Bevorzugte Reste A² der Formel (A7) sind entsprechende Reste der Formeln (A7-4) bis (A7-11) (ohne Formelbilder), welche Doppelbindungs-Isomere bezüglich der mit Pfeil 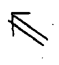 gezeichneten Doppelbindung der Reste der Formeln (A6-4) bis (A6-11) darstellen.

Im Falle der unsymmetrisch zur C-C-Doppelbindung substituierten Reste (A6), wie (A6-2) und (A6-3) sind entsprechende Doppelbindungsisomere, die von der allgemeinen Formel (A6) umfasst sind, ebenfalls bevorzugt.

Bevorzugt sind auch Verbindungen (I), worin A² einen Rest (A8) oder (A9) bedeutet, 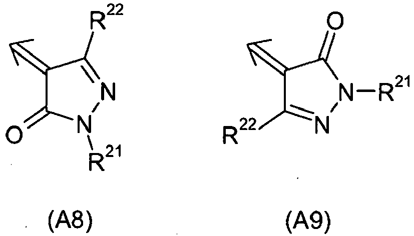 worin
R²¹ und R²² die obengenannten Bedeutungen oder bevorzugten Bedeutungen haben, vorzugsweise
- R²¹: Wasserstoff, (C₁-C₄)Alkyl, (C₁-C₄)Halogenalkyl, (C₃-C₆)Cycloalkyl, (C₃-C₆)Halogencycloalkyl oder eine Gruppe C(O)OR⁵⁴, C(O)NR⁵⁵R⁵⁶, C(O)Het⁷, NR⁵⁷R⁵⁸ oder Het⁸ bedeutet, insbesondere H, (C₁-C₄)Alkyl, (C₁-C₄)Halogenalkyl oder (C₃-C₆)Cycloalkyl, beispielsweise Cyclopropyl bedeutet und
- R²²: Wasserstoff, (C₁-C₄)Alkyl, (C₁-C₄)Halogenalkyl, (C₃-C₆)Cycloalkyl, (C₃-C₆)Halogencycloalkyl, (C₁-C₄)Alkylthio, (C₁-C₄)Alkylsulfonyl, (C₁-C₄)Alkylsulfinyl oder eine Gruppe der Formel C(O)OR⁵⁹, C(O)NR⁶⁰R⁶¹, C(O)Het⁹, NR⁶²R⁶³ oder Het¹⁰ bedeutet, insbesondere H, (C₁-C₄)Alkyl, (C₁-C₄)Halogenalkyl, (C₃-C₆)Cycloalkyl, (C₃-C₆)Halogencycloalkyl, (C₁-C₄)Alkylthio oder (C₁-C₄)Alkylsulfonyl bedeutet.

Beispiele für bevorzugte Reste A² der Formel (A8) sind Reste der Formeln (A8-1) bis (A8-5):

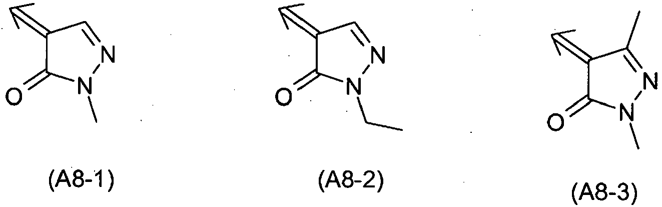

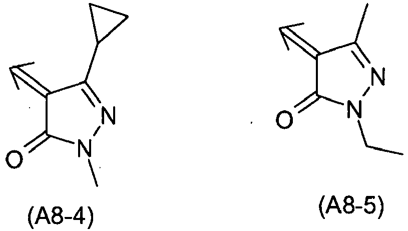

Bevorzugte Reste A² der Formel (A9) sind entsprechende Reste der Formeln (A9-1) bis (A9-5) (ohne Formelbilder), welche Doppelbindungs-Isomere bezüglich der mit Pfeil 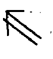 gezeichneten Doppelbindung der Reste der Formeln (A8-1) bis (A8-5) darstellen.

Bevorzugt sind auch Verbindungen (I), worin A² einen Rest (A10) oder (A11) bedeutet, 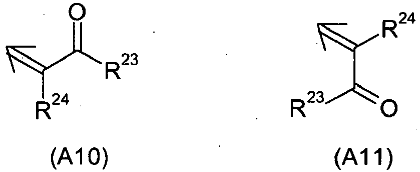 in welchen
- R²³: H, (C₁-C₄)Alkyl, (C₁-C₄)Halogenalkyl, (C₃-C₆)Cycloalkyl, beispielweise Cyclopropyl, (C₁-C₄)Alkoxy oder (C₁-C₄)Halogenalkoxy, weiter bevorzugt Methyl, Ethyl, n-Propyl, i-Propyl, n-Butyl, i-Butyl, s-Butyl, t-Butyl, Methoxy, Ethoxy, n-Propoxy, i-Propoxy, n-Butoxy, i-Butoxy, s-Butoxy oder t-Butoxy bedeutet, und
- R²⁴: H, Halogen, Cyano, (C₁-C₄)Alkyl, (C₁-C₄)Halogenalkyl, (C₃-C₆)Cycloalkyl, beispielsweise Cyclopropyl, (C₁-C₄)Alkoxy oder (C₁-C₄)Halogenalkoxy, insbesondere Wasserstoff, (C₁-C₃)Alkyl, Cyano oder Cyclopropyl, weiter bevorzugt Cyano bedeutet.

Beispiele für bevorzugte Reste A² der Formel (A10) sind die Reste der Formeln (A10-1), (A10-2), (A10-3), (A10-4), (A10-5), (A10-6), (A10-7), (A10-8) und (A10-9):

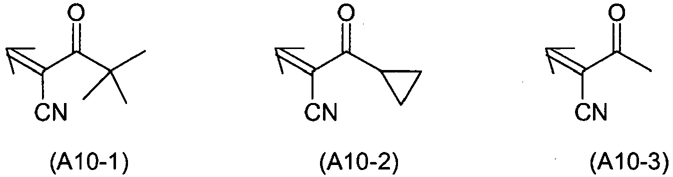

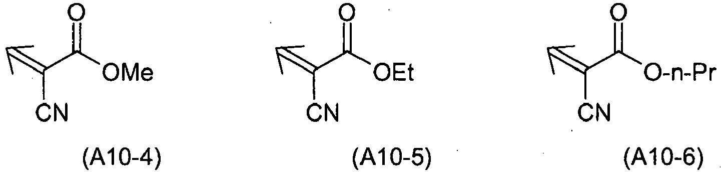

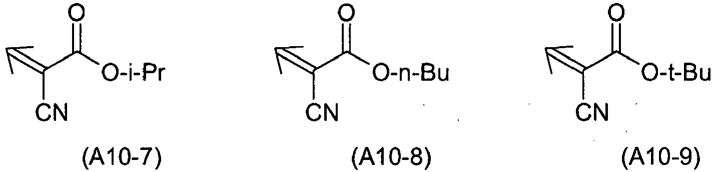

Bevorzugte Reste A² der Formel (A11) sind entsprechende Reste der Formeln (A11-1) bis (A11-9) (ohne Formelbilder), welche Doppelbindungs-Isomere bezüglich der mit Pfeil 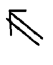 gezeichneten Doppelbindung der Reste der Formeln (A10-1) bis (A10-9) darstellen.

Bevorzugt sind auch Verbindungen (I), worin A² einen Rest (A12) bedeutet, 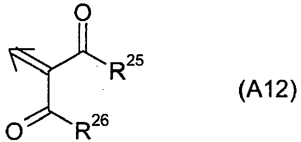 worin
- R²⁵ und R²⁶: jeweils unabhängig voneinander H, (C₁-C₄)Alkyl, (C₁-C₄)Halogenalkyl; (C₁-C₄)Alkoxy, (C₁-C₄)Halogenalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Halogencycloalkyl, (C₃-C₆)Cycloalkoxy, (C₁-C₄)Alkylthio, (C₁-C₄)Alkylsulfonyl, (C₁-C₄)Alkylsulfinyl, NR⁶⁴R⁶⁵ oder Het¹¹ bedeuten, insbesondere H, (C₁-C₄)Alkyl, (C₁-C₄)Halogenalkyl, (C₁-C₄)Alkoxy, (C₁-C₄)Halogenalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Halogencycloalkyl, (C₃-C₆)Cycloalkoxy, (C₁-C₄)Alkylthio bedeuten.

Beispiele für bevorzugte Reste A² der Formel (A12) sind Reste der Formeln (A12-1) bis (A12-9):

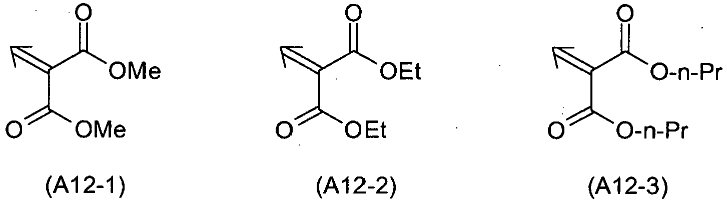

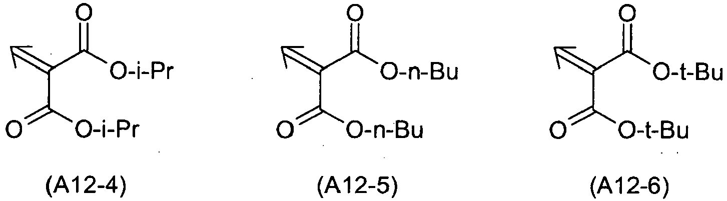

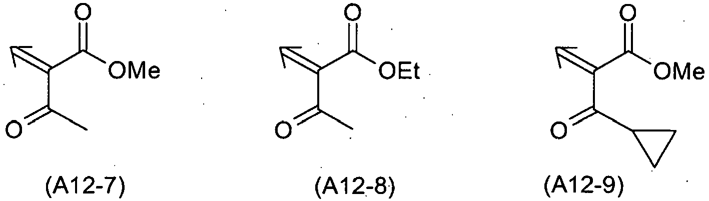

Im Falle der unsymmetrisch zur C-C-Doppelbindung substituierten Reste (A12), wie (A12-7), (A12-8) und (A12-9) sind entsprechende Doppelbindungsisomere, die von der allgemeinen Formel (A12) umfasst sind, ebenfalls bevorzugt.

Die erfindungsgemäßen Verbindungen der Formel (I) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen.

Die Erfindung umfasst auch alle Tautomeren, wie Keto- und Enol-Tautomeren, und deren Mischungen und Salze, wenn entsprechende funktionelle Gruppen vorhanden sind.

Gegenstand der vorliegenden Erfindung sind auch Verfahren zur Herstellung der Verbindungen der allgemeinen Formel (I) und/oder deren Salze. Dazu gehören Verfahren, die analog bekannten Methoden durchgeführt werden können.
(a) Beispielsweise werden Verbindungen der Formel (I) und deren Salze, worin L einen Rest der Formel C(=A¹)-B¹ bedeutet, B¹ einen Rest der Formel (B1) bis (B9) und (B11) bis (B14) bedeutet und Q¹ bis Q⁹ jeweils OH, SH, SM oder OM bedeuten und die übrigen Reste wie in der jeweils herzustellenden Verbindung der Formel (I) definiert sind, nach Verfahren [Variante (a)] erhalten, dadurch gekennzeichnet, dass man eine Verbindung der Formel (II), 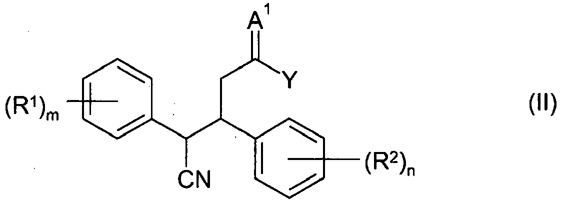 worin A¹, (R¹)ₘ und (R²)ₙ wie in Formel (I) definiert sind und Y eine Abgangsgruppe, vorzugsweise ein Halogenatom, insbesondere ein Chloratom bedeutet,
   mit einer Verbindung der Formel H-B¹, worin B¹ wie im Rest L definiert ist und Q¹ bis Q⁹ jeweils wie in der herzustellenden Verbindung der Formel (I) definiert ist, direkt zur Verbindung (I) umsetzt oder zum O- bzw. S-Alkylierungsprodukt als Zwischenprodukt umsetzt und das entstandene Zwischenprodukt der O- bzw. S-Alkylierung in die Verbindung der Formel (I) als C-Alkylierungsprodukt umlagert [siehe Schema weiter unten am Beispiel des Reaktanten der Formel (H-B1*) als Verbindung H-B¹].
   Nach Variante (a) werden vorzugsweise Verbindungen der Formel H-B¹ als Reaktanten verwendet und Verbindungen der Formel (I) hergestellt, worin jeweils Q¹ bis Q⁹ in der Gruppe B¹ den Rest OH oder OM bedeuten.
   Die C-C-Acylierungen können analog bekannten Verfahren ein- oder mehrstufig als basenkatalysierte Reaktionen durchgeführt werden.
   Analoge einstufige Verfahren unter Verwendung von Basen wie Calciumhydroxid sind beispielsweise aus B. S. Jensen, Acta Chem. Scand. 1959, Bd. 13, S. 1668 bekannt (siehe nachfolgendes Schema zur Herstellung der Verbindung (I-A). Analoge einstufige Verfahren unter Verwendung von Basen wie Natriumhydrid sind beispielsweise von Mulrooney et al J. Org. Chem. 75, 2010, 16, und Yadav et al Chem. Lett. 39, 2010, 280 beschrieben. Die Umsetzung kann beispielsweise in einem geeigneten polaren aprotischen organischen Lösungsmittel, wie Dimethylformamid oder Tetrahydrofuran, bei einer Temperatur von 0 bis 120 °C erfolgen.
   Analoge einstufige Verfahren unter Verwendung von organischen Basen wie Triethylamin in Gegenwart von Lewis Säuren wie Magnesium Chlorid sind beispielweise von Little et al J. Org. Chem. 65, 2000, 8096 beschrieben. Die Umsetzung kann dabei beispielsweise in einem geeigneten organischen aprotischen Lösungsmittel, wie Dichlormethan, bei einer Temperatur von 0 bis 120 °C erfolgen.
   Analoge zweistufige Verfahren, bei denen zunächst eine basenkatalysierte O-Acylierung und anschließend eine Umlagerung durchgeführt werden, sind beispielsweise aus EP-A-0186117, WO 2008/125213, WO 2011/012248; EP 0625 505 und dort zitierter Literatur bekannt; siehe nachfolgendes Schema am Beispiel der Herstellung der Verbindung (I-A) über das O-Alkylierungsprodukt (I-Zw), wobei die Verbindung (I-A) der Verbindung (I) entspricht, in der B¹ einen Rest der Formel (B1) bedeutet und Q¹ einen Rest OH bedeutet. 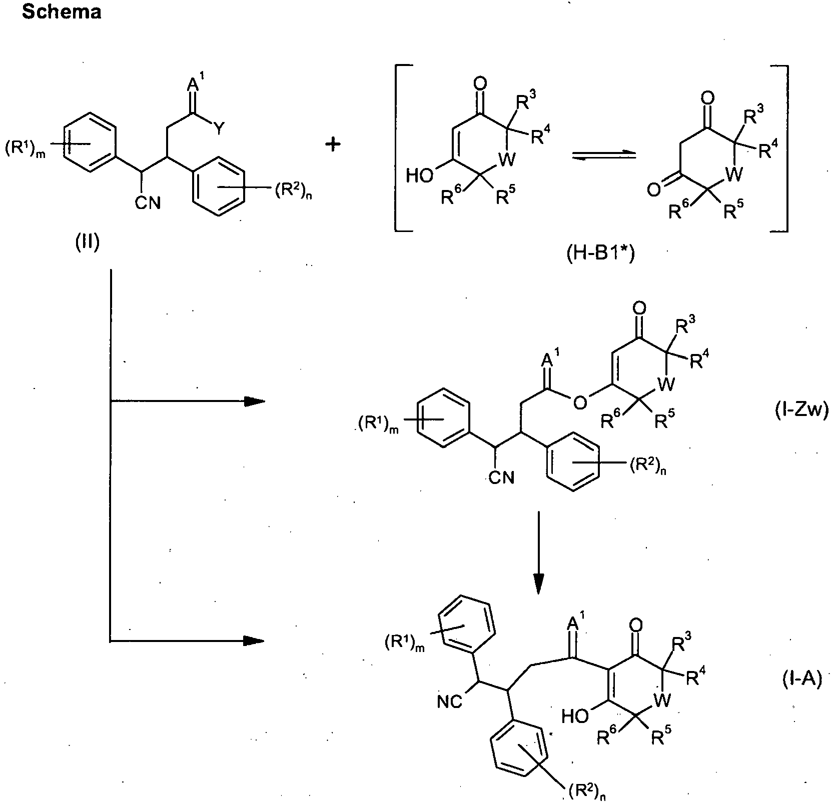
   Das Verfahren mit Umlagerung der Verbindung (I-Zw) oder analoger Zwischenverbindungen wird gegebenenfalls unter Verwendung eines Umlagerungsreagenzes durchgeführt. Es kommen hierbei zur Umlagerung geeignete Chemikalien in Betracht, beispielsweise die aus analogen Umsetzungen bekannten Reagenzien Trimethylsilylcyanid, Kaliumcyanid und Acetoncyanhydrin.
   Das Umlagerungsverfahren zur Herstellung der Verbindungen der allgemeinen Formel (I) wird vorzugsweise unter Verwendung eines oder mehrerer Verdünnungsmittel durchgeführt. Als Verdünnungsmittel kommen vor allem unter den Reaktionsbedingungen inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlor-methan, Chloroform, Tetrachlorkohlenstoff; Ether, wie Diethylether, Diisopropyl-ether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethyl-acetamid, N-Methylformanilid, N-Methyl-pyrrolidon oder Hexamethylphosphor-säuretriamid; Ester wie Essigsäuremethylester oder Essigsäureethylester, Sulfoxide, wie Dimethylsulfoxid. Die Reaktionstemperaturen können bei der Durchführung des Umlagerungsverfahrens in einem größeren Bereich variiert werden. Im Allgemeinen arbeitet man bei Temperaturen zwischen 0°C und 150°C, vorzugsweise zwischen 10°C und 120°C. Die Umlagerung wird im Allgemeinen unter Normaldruck durchgeführt. Es ist jedoch auch möglich, das Verfahren unter erhöhtem oder vermindertem Druck - im Allgemeinen zwischen 0,1 bar und 10 bar - durchzuführen.
   Im obengenannten und den nachfolgenden Verfahren werden partiell Lösemittel (gleichbedeutend mit "Lösungsmittel") verwendet. In diesem Zusammenhang bezeichnen "inerte Lösemittel" jeweils Lösemittel, die unter den jeweiligen Reaktionsbedingungen inert sind, jedoch nicht unter beliebigen Reaktionsbedingungen inert sein müssen.
   Die jeweils beschriebenen Verfahren können in Apparaturen, wie sie im Labor, Technikum und in Anlagen für die Herstellung kommerzieller Mengen und großtechnischer Verfahren üblich sind oder alternativ auch in einem Mikrowellenofen durchgeführt werden.
(b) Verbindungen der Formel (I) und deren Salze, worin L einen Rest der Formel -C(=A¹)-B¹ bedeutet, B¹ einen Rest der Formel (B1) bis (B7) und (B11) bis (B14) bedeutet und Q¹ bis Q⁹ in der Gruppe B¹ jeweils eine Gruppe der Formel SH oder SM bedeuten und die übrigen Reste wie in der jeweils herzustellenden Verbindung der Formel (I) definiert sind, werden beispielsweise nach Verfahren [Variante (b)] erhalten, dadurch gekennzeichnet, dass man eine Verbindung der Formel (I), in der L einen Rest der Formel -C(=A¹)-B¹ und dabei A¹ wie in Formel (I) definiert ist und B¹ analog zum Rest B¹ in Formel (I) definiert ist, mit dem Unterschied, dass Q¹ bis Q⁹ in der Gruppe B¹ jeweils die Gruppe OH oder OM bedeuten, mit einem Halogenierungsmittel, vorzugsweise einem Carbonsäurehalogenid, beispielsweise mit Oxalylchlorid (Cl-CO-CO-Cl) zur Verbindung (I), worin Q¹ bis Q⁹ in der Gruppe B¹ jeweils ein Halogenatom, vorzugsweise Chloratom, bedeuten, mit einem Schwefelreagens, vorzugsweise einem Sulfid, beispielsweise Dinatriumsulfid, umsetzt und die gewünschte Verbindung der Formel (I) gegebenenfalls aus dem Reaktionsgemisch abtrennt oder isoliert. Solche Methoden sind dem Fachmann grundsätzlich bekannt und beispielsweise in Journal of Heterocyclic Chemistry, 25(3), 901-6; 1988 für die nukleophile Substitution mit Na₂S beschrieben. Die Reaktion kann beispielweise auch in einem aprotischen organischen Lösungsmittel wie Tetrahedrofuran, gegebenenfalls unter Erhitzen im Rückfluss, durchgeführt werden; vgl. EP 249150.
(c) Verbindungen der Formel (I) und deren Salze, worin L einen Rest der Formel -C(=A¹)-B¹ bedeutet, B¹ einen Rest der Formel (B1) bis (B7) und (B11) bis (B14) bedeutet und Q¹ bis Q⁹ in der Gruppe B¹ jeweils eine Gruppe der Formel OR³⁷ bedeuten und die übrigen Reste wie in der jeweils herzustellenden Verbindung der Formel (I) definiert sind, werden beispielsweise nach Verfahren [Variante (c)] erhalten, dadurch gekennzeichnet, dass man eine Verbindung der Formel (I), in der L einen Rest der Formel -C(=A¹)-B¹ und dabei A¹ wie in Formel (I) definiert ist und B¹ analog zum Rest B¹ in Formel (I) definiert ist, mit dem Unterschied, dass Q¹ bis Q⁹ in der Gruppe B¹ jeweils die Gruppe OH oder OM bedeuten, mit einer Verbindung (Alkylierungs-, Acylierungs- oder Arylierungsmittel) der Formel Y'-R³⁷, worin Y' eine Abgangsgruppe darstellt, umsetzt und die gewünschte Verbindung der Formel (I) gegebenenfalls aus dem Reaktionsgemisch abtrennt oder isoliert. Solche Methoden sind dem Fachmann grundsätzlich bekannt und beispielsweise in DOS 2513750 (= DE 2513750) beschrieben.
   Die Umsetzung nach Variante (c) kann dabei unter Verwendung von Basen, vorzugsweise Aminbasen wie Triethylamin, beispielweise in einem geeigneten organischen aprotischen Lösungsmittel, wie Dichlormethan, vorzugsweise bei einer Temperatur von 0 °C bis 25 °C erfolgen; vgl. WO 2009115788.
(d) Verbindungen der Formel (I) und deren Salze, worin L einen Rest der Formel -C(=A¹)-B¹ bedeutet, B¹ einen Rest der Formel (B1) bis (B7) und (B11) bis (B14) bedeutet und Q¹ bis Q⁹ in der Gruppe B¹ jeweils eine Gruppe der Formel SR³⁸ bedeuten und die übrigen Reste wie in der jeweils herzustellenden Verbindung der Formel (I) definiert sind, werden beispielsweise nach Verfahren [Variante (d)] erhalten, dadurch gekennzeichnet, dass man eine Verbindung der Formel (I), in der L einen Rest der Formel -C(=A¹)-B¹ und dabei A¹ wie in Formel (I) definiert ist und B¹ analog zum Rest B¹ in Formel (I) definiert ist, mit dem Unterschied, dass und Q¹ bis Q⁹ in der Gruppe B¹ jeweils die Gruppe SH oder SM bedeuten, mit einer Verbindung (Alkylierungs-, Acylierungs- oder Arylierungsmittel) der Formel Y'R³⁸, worin Y' eine Abgangsgruppe darstellt, umsetzt und die gewünschte Verbindung der Formel (I) gegebenenfalls aus dem Reaktionsgemisch abtrennt oder isoliert. Solche Methoden sind dem Fachmann grundsätzlich bekannt. Bevorzugt sind Umsetzungen in Gegenwart von geeigneten anorganischen oder organischen Basen wie Kaliumkarbonat oder Kalium-tert.-Butylat in Lösungsmitteln wie Tetrahydrofuran bei Temperaturen von 0 bis 80 °C; vgl. WO 2008113089.
(e) Verbindungen der Formel (I) und deren Salze, worin L einen Rest der Formel -C(=A¹)-B¹ bedeutet, B¹ einen Rest der Formel (B1) bis (B7) und (B11) bis (B14) bedeutet und Q¹ bis Q⁹ in der Gruppe B¹ jeweils eine Gruppe der Formel SR³⁸ bedeuten und die übrigen Reste wie in der jeweils herzustellenden Verbindung der Formel (I) definiert sind, werden beispielsweise auch nach Verfahren [Variante (e)] erhalten, dadurch gekennzeichnet, dass man eine Verbindung der Formel (I), in der L einen Rest der Formel -C(=A¹)-B¹ und dabei A¹ wie in Formel (I) definiert ist und B¹ analog zum Rest B¹ in Formel (I) definiert ist, mit dem Unterschied, dass Q¹ bis Q⁹ in der Gruppe B¹ jeweils ein Halogenatom, vorzugsweise Chloratom bedeuten, mit einer Thioverbindung der Formel H-S-R³⁸ umsetzt und die gewünschte Verbindung der Formel (I) gegebenenfalls aus dem Reaktionsgemisch abtrennt oder isoliert. Solche Methoden sind dem Fachmann grundsätzlich bekannt und beispielsweise in WO 2009/018925 (z. B. dort Seite 21 und Details zu Verfahren j) und dort zitierter Literatur beschrieben.
(f) Verbindungen der Formel (I) und deren Salze, worin L einen Rest der Formel -C(=A¹)-B¹ bedeutet und B¹ eine Gruppe der Formel (B10) bedeutet, [= Verbindung (I-B10)] werden beispielsweise auch nach Verfahren [Variante (f)] erhalten, dadurch gekennzeichnet, dass man eine Verbindung der Formel (I), in der L einen Rest der Formel -C(=A¹)-B* bedeutet, A¹ wie in Formel (I) definiert ist und B* einen Rest der Formel -CH₂-CO-R²⁷, worin R²⁷ wie in Formel (B10) definiert ist, bedeutet [= Verbindung (I-B)], 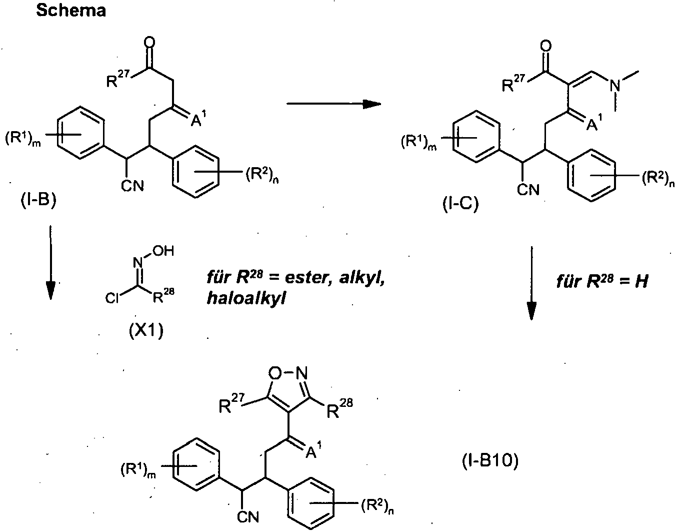 mit N,N-Dimethylformamiddimethylacetal zur Verbindung der Formel (I-C) umsetzt (siehe Schema) und die Verbindung (I-C) unter Ringschluss mit Hydroxylamin oder dessen Salzen zur Verbindung der Formel (I-B10), worin R²⁸ = H ist, umsetzt oder die Verbindung (I-B) mit einer Verbindung der Formel (X1),

   HO-N=C(Cl)-R²⁸ (X1)

   worin R²⁸ wie in Formel (B10) definiert ist, unter Ringschluss zur Verbindung der Formel (I-B10) umsetzt, wobei R²⁸ in Formel (X1) wie in der herzustellenden Verbindung der Formel (I-B10), ausgenommen R²⁸ = H, definiert ist.
   Die Umsetzung mit N,N-Dimethylformamiddimethylacetal ist von analogen Beispielen aus EP 636622 bekannt. Der Ringschluss mit Hydroxylamin oder dessen Salzen kann beispielsweise in einem geeigneten organischen polaren Lösungsmittel, wie Ethanol oder Acetonitril, gegebenenfalls unter Katalyse einer Base, vorzugsweise einer organischen Base wie Triethylamin, bei einer Temperatur im Bereich von 0°C bis 150°C, vorzugsweise im Bereich von Raumtemperatur bis zum Siedepunkts des betreffenden Lösungsmittels hergestellt werden; vgl. EP 636622.
(g) Verbindungen der Formel (I) und deren Salze, worin L einen Rest der Formel -C(=A¹)-B¹ bedeutet und B¹ eine Gruppe der Formel (B10) bedeutet, [= Verbindung (I-B10)] werden beispielsweise auch nach Verfahren [Variante (g)] erhalten, dadurch gekennzeichnet, dass man eine Verbindung der Formel (II), 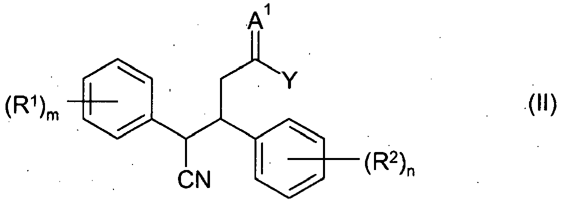 worin A¹, (R¹)ₘ und (R²)ₙ wie in Formel (I) definiert sind und Y eine Abgangsgruppe, vorzugsweise ein Halogenatom, insbesondere ein Chloratom bedeutet,
   mit einer Verbindung der Formel X-(B10), worin (B10) wie in der Gruppe B¹ definiert ist und X ein Abgangsgruppe, vorzugsweise Halogen, insbesondere Iod bedeutet, gegebenenfalls in Gegenwart eines Kupplungsreagenzes zur Verbindung der formel (I) umsetzt (Kreuzkupplungsreaktion)
   oder
   mit einer aktivierten Verbindung der Formel X'-(B10), worin X' ein Metall oder eine Melall-derivatgruppe, vorzugsweise eine Cl-Zn-Gruppe bedeutet und (B10) wie in der Gruppe B¹ definiert ist, zur Verbindung der Formel (I) umsetzt.
   Die aktivierte Verbindung X'-(B10) kann dabei beispielsweise zunächst aus der genannten Verbindung der Formel X-(B10) durch Reaktion mit Metall oder Metallsalzen, beispielsweise mit Mg und Zn-Salzen hergestellt werden (vergleiche nachfolgendes Schema). 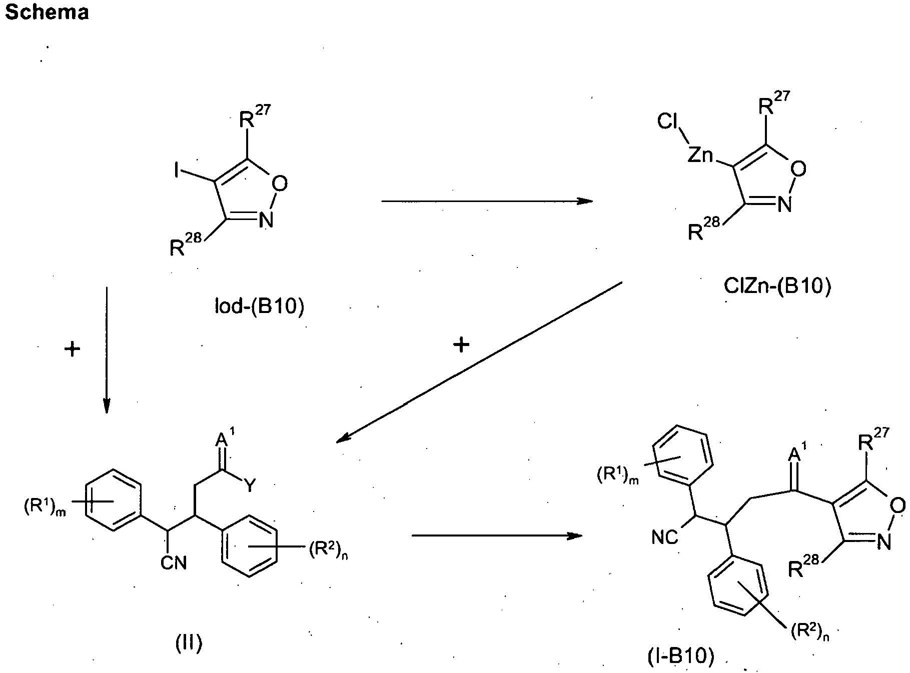
   Das Verfahren für die Kreuzkupplung zwischen (II) und Iod-(B10) oder analoger Zwischenverbindungen wird gegebenenfalls unter Verwendung eines Kupplungsreagenzes durchgeführt. Es kommen hierbei zur Kupplung geeignete Chemikalien in Betracht, beispielsweise die aus analogen Umsetzungen bekannten Palladium-Reagenzien wie beispielsweise Tetrakis(triphenylphosphine)palladium(0) (z.B. analog Negishi et al., Tetrahedron Lett. 24, 1983, 5181).
   Das Verfahren für die Herstellung von ClZn-(B10) oder analogen Verbindungen kann unter Verwendung von geeigneten Metallen oder Metallderivaten durchgeführt werden. Im Falle von ClZn-(B10) eignet sich beispielweise die Reaktion von Iod-(B10) mit Magnesium-Spänen und Zink Chlorid (siehe analog Knochel et al. Chem. A Eur. J. 15, 2009, 7192).
   Das Kupplungsverfahren zur Herstellung der Verbindungen der allgemeinen Formel (I-B10) wird vorzugsweise unter Verwendung eines oder mehrerer Verdünnungsmittel durchgeführt. Als Verdünnungsmittel kommen vor allem unter den Reaktionsbedingungen inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlor-methan, Chloroform, Tetrachlorkohlenstoff; Ether, wie Diethylether, Diisopropyl-ether, Dioxan, Tetrahydrofuran oder Ethylenglykoldimethyl- oder -diethylether; Amide, wie N,N-Dimethylformamid, N-Methyl-pyrrolidon oder Hexamethylphosphor-säuretriamid.
   Die Reaktionstemperaturen können bei der Durchführung des Kupplungsverfahrens in einem größeren Bereich variiert werden. Im Allgemeinen arbeitet man bei Temperaturen zwischen 0°C und 150°C, vorzugsweise zwischen 10°C und 120°C. Die Kupplung wird im Allgemeinen unter Normaldruck durchgeführt. Es ist jedoch auch möglich, das Verfahren unter erhöhtem oder vermindertem Druck - im Allgemeinen zwischen 0,1 bar und 10 bar - durchzuführen.
(h) Verbindungen der Formel (I) und deren Salze, worin L einen Rest der Formel -C(=A²)-B² bedeutet und B² einen Rest der Formel OR³³ oder SR³⁴ bedeutet, werden beispielsweise nach Verfahren [Variante (h)] erhalten, dadurch gekennzeichnet, dass man eine Verbindung der Formel (I'), 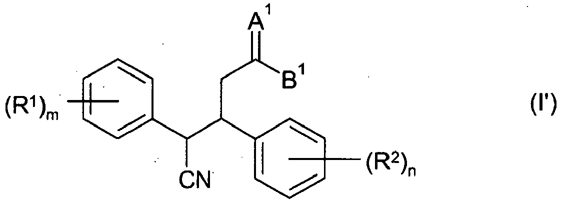 worin (R¹)ₘ und (R²)ₙ wie in Formel (I) definiert sind, A¹ einen Rest O oder S und B¹ einen Rest der Formel (B1), (B2), (B3), (B4), (B5), (B6), (B7), (B8), (B9), (B11), (B12), (B13) oder (B14) wie oben für Verbindungen (I), in denen L einen Rest -C(=A¹)-B¹ bedeutet, definiert ist, wobei Q¹ bis Q⁹ in den Resten B¹ jeweils OH oder OM bedeuten,
   mit einer Verbindung der Formel Y'-B², worin B² wie im Rest L definiert ist und Y' eine Abgangsgruppe, beispielsweise Chlor, bedeutet, zur Verbindung (I), in der B² den Rest OR³³ (wenn A¹ = O) bzw. SR³⁴ (wenn A¹ = S) bedeutet, umsetzt, wobei die Gruppe -C(=A²)- in der Verbindung (I) im Vergleich zur Gruppe -C-B¹ in Verbindung (I') das stabilere Tautomer oder vorzugsweise ein strukturell fixiertes Tautomer darstellt, in dem der Rest -C(=A²)- dem Rest =C(-B¹)- tautomer entspricht.
   Mit "strukturell fixiertes Tautomer" ist hier gemeint, dass im Rest -C(=A²)-B² die Gruppe B² einen Rest der Formel OR³³ oder SR³⁴ (d. h. nicht OH, OM, SH oder SM) bedeutet.
   Die Verbindung der Formel Y'-B² ist in Abhängigkeit der Bedeutung des Restes R³³ bzw. R³⁴ entweder ein Alkylierungsmittel, Acylierungsmittel oder Arylierungsmittel. Die Umsetzung kann dementsprechend unter den Bedingungen für analoge Alkylierungen, Acylierungen oder Arylierungen, gegebenenfalls in Gegenwart geeigneter Katalysatoren (Säuren und Basen) oder speziell Arylierungskatalysatoren durchgeführt werden.
(i) Verbindungen der Formel (I) und deren Salze, worin L einen Rest der Formel -C(=A²)-B² bedeutet und B² einen Rest der Formel -NR^{B}R^{C} bedeutet, werden beispielsweise nach Verfahren [Variante (i)] erhalten, dadurch gekennzeichnet, dass man eine Verbindung der Formel (I'), 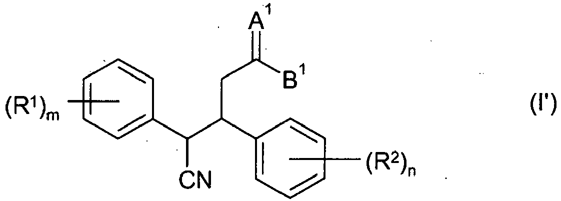 worin (R¹)ₘ und (R²)ₙ wie in Formel (I) definiert sind, A¹ einen Rest der Formel -NR^{B} und B¹ einen Rest der Formel (B1), (B2), (B3), (B4), (B5), (B6), (B7), (B8), (B9), (B11), (B12), (B13) oder (B14) wie oben für Verbindungen (I), in denen L einen Rest -C(=A¹)-B¹ bedeutet, definiert ist, wobei Q¹ bis Q⁹ in den Resten B¹ jeweils OH oder OM bedeuten,
   mit einer Verbindung der Formel Y'-R^{C}, worin R^{C} wie im Rest -NR^{B}R^{C} von B² definiert ist und Y' eine Abgangsgruppe, beispielsweise Chlor, bedeutet, zur Verbindung (I), in der B² den Rest -NR^{B}R^{C} bedeutet, umsetzt, wobei die Gruppe -C(=A²)- in der Verbindung (I) im Vergleich zur Gruppe -C-B¹ in Verbindung (I') das stabilere Tautomer oder vorzugsweise ein strukturell fixiertes Tautomer darstellt, in dem der Rest -C(=A²)- dem Rest =C(-B¹)- tautomer entspricht.

Mit "strukturell fixiertes Tautomer" ist hier gemeint, dass im Rest -C(=A²)-B² die Gruppe B² einen Rest der Formel -NR^{B}R^{C} (d. h. nicht OH, OM, SH oder SM) bedeutet.

Andere erfindungsgemäße Verbindungen der Formel (I) können analog bekannten Verfahren aus erfindungsgemäßen Verbindungen (I) durch Derivatisierung erhalten werden. Beispielsweise können Hydroxyliminoverbindungen der Formel (I), worin A¹ einen Rest der Formel =N-OH bedeutet, gegebenenfalls durch Umsetzung von Verbindungen (I), worin A¹ ein Sauerstoffatom ist, mit Hydroxylamin erhalten werden.

Die zur Herstellung der erfindungsgemäßen Verbindungen (I) nach Variante (a) verwendeten Verbindungen der Formel (II) mit der reaktiven Gruppe -C(=A¹)-Y können aus entsprechenden Carbonsäuren oder Carbonsäureestern, d.h. Cyanobutyraten (III), 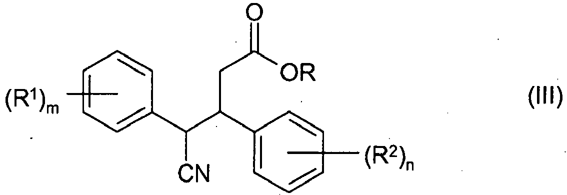 worin (R¹)ₘ und (R²)ₙ wie in Formel (I) definiert ist und R Wasserstoff oder einen hydrolysierbaren Rest bedeutet,
durch Derivatisierung, z.B. Herstellung der Halogenide, wenn Y ein Halogenatom darstellt, analog bekannten Methoden hergestellt werden.

Die Herstellung von Säurehalogeniden mit der reaktiven Gruppe -C(=O)-Hal, worin Hal ein Halogenatom, vorzugsweise Chloratom bedeutet, aus Carbonsäuren oder Carbonsäureestern sind dem Fachmann grundsätzlich bekannt; siehe Säurechlorid aus Carbonsäuren mit Thionylchlorid Houben-Weyl. Bd. VIII (1952), Seite 359-680 oder mit Oxalylchlorid gemäß Heterocycles, 26(5), 1291-302; 1987.
Analoge Verfahren zur Herstellung anderer reaktiver Gruppen -C(=A¹)-Y sind bekannt.

Falls die reaktive Gruppe -C(=A¹)-Y eine Gruppe der Formel -C(=N-R^{A})-Hal bedeutet, kann man ebenfalls entsprechende Carbonsäuren oder Carbonsäureester der Formel (III), worin A¹ ein Sauerstoffatom bedeutet, verwenden. Die Verbindungen mit der reaktiven Gruppe C(=O)-OR werden dabei zunächst zur Verbindung mit der funktionalen Alkohol-Gruppe (-CH₂-OH) reduziert und dann zur Verbindung mit der funktionalen Aldehyd-Gruppe (-C(=O)H) oxidiert oder sie werden zur Verbindung mit der funktionalen Aldehyd-Gruppe (-C(=O)H) direkt reduziert; siehe analog beispielsweise István E. Markó et al. Eur. J. Org. Chem. 2009, 1806. Zur Herstellung der Verbindungen mit der reaktiven Gruppe -C(=N-R^{A})-Hal können die Verbindungen mit der funktionalen Aldehyd-Gruppe (-C(=O)H) dann halogeniert werden; siehe analog beispielweise R. A. Whitney et al. J. Org. Chem. 1988, 53, 4074; S. Kim et al. Chem. Commun. 2007, 4507; Chemistry-An Asian Journal 2008, 3, 1692; Chem. Commun. 2010, 46, 7822.

Diastereomerengemische der Cyanobutyrate der genannten Formel (III) sind im Prinzip bekannt; siehe beispielsweise EP-A 5341, EP-A 266725, EP-A270 830, JP 04/297454, JP 04/297455, JP 05/058979, WO 2011/003775, WO 2011/003776. Analog den in den zitierten Schriften beschriebenen Syntheserouten lassen sich die Verbindungen nach Standardverfahren der organischen Chemie herstellen. Beispielsweise werden Verbindungen der Formel (III) erhalten, dadurch gekennzeichnet, dass man
(aa) Verbindungen der Formel (IV) ("Cyanomethylbenzole"/"Phenylacetonitrile") 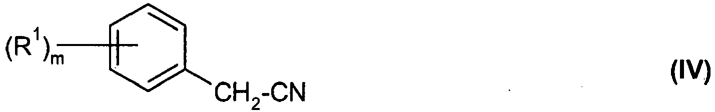 mit Verbindungen der Formel (V) (Zimtsäurederivaten) oder deren Salzen, 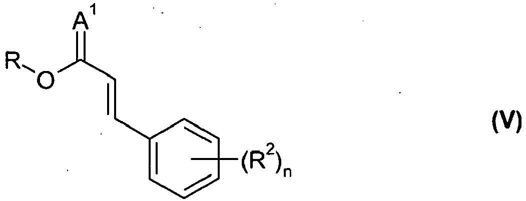 zu Verbindungen der Formel (III) (Diastereomere/racemisch)
   umsetzt, wobei A¹, R, R¹, R², m und n in den Verbindungen (IV) und (V) wie in der jeweils herzustellenden Verbindung der Formel (III) definiert sind.
   Die für die Herstellung der Verbindungen (III) benötigten Ausgangsstoffe (IV) und (V) sind gemäß der zitierten Literatur bekannt oder können analog der zitierten Literatur hergestellt werden.
   Die Umsetzung nach Variante (aa) kann beispielsweise nach Methoden und unter Bedingungen, wie sie für Michael-Additionen bekannt sind, durchgeführt werden. Die Umsetzung erfolgt beispielsweise bei Temperaturen von -100°C bis 150°C, vorzugsweise -78°C bis 100°C, in einem organischen oder anorganischen Lösungsmittel, in der Regel in Gegenwart einer Base oder eines Katalysators oder beidem [vgl. J. Chem. Soc. (1945), S. 438].
   Geeignete Lösungsmittel sind beispielsweise organische Lösungsmittel wie:
   - aliphatische Kohlenwasserstoffe wie Pentan, Hexan, Cyclohexan oder Petrolether;
   - aromatische Kohlenwasserstoffe wie Toluol, o-, m- oder p-Xylol,
   - halogenierte Kohlenwasserstoffe wie Methylenchlorid, Chloroform oder Chlorbenzol,
   - Ether wie Diethylether, Diisopropylether, tert.-Butylmethylether, Dioxan, Anisol und Tetrahydrofuran (THF),
   - Nitrile wie Acetonitril oder Propionitril,
   - Ketone wie Aceton, Methylethylketon, Diethylketon oder tert.-Butylmethylketon,
   - Alkohole wie Methanol, Ethanol, n-Propanol, Isopropanol, n-Butanol und tert.-Butanol, sowie
   - Dimethylsulfoxid, Dimethylformamid, Dimethylacetamid, Sulfolan,
   - Gemische aus den genannten organischen Lösungsmitteln.

   Geeignet sind in Einzelfällen auch anorganische Lösungsmittel wie Wasser oder Gemische organischer Lösungsmittel mit Wasser.
   Bevorzugte Lösungsmittel sind THF und Methanol und deren Gemische mit anderen organischen Lösungsmitteln.
   Die Umsetzung nach Herstellungsvariante (aa) erfolgt vorzugsweise in Gegenwart einer Base, beispielsweise aus der Gruppe anorganischer Verbindungen wie der Alkalimetall- und Erdalkalimetallhydroxide, zum Beispiel (z. B.) Lithiumhydroxid, Natriumhydroxid, Kaliumhydroxid oder Calziumhydroxid, der Alkalimetall- und Erdalkalimetalloxide, zum Beispiel Lithiumoxid, Natriumoxid, Calziumoxid oder Magnesiumoxid, der Alkalimetall- und Erdalkalimetallhydride, z. B. Lithiumhydrid, Natriumhydrid, Kaliumhydrid oder Calziumhydrid, der Alkalimetallamide, zum Beispiel Lithiumamid, Natriumamid oder Kaliumamid, der Alkalimetall- und Erdalkalimetallcarbonate, z. B. Lithiumcarbonat, Kaliumcarbonat oder Calziumcarbonat, der Alkalimetallhydrogencarbonate, z. B. Natriumhydrogencarbonat, oder der metallorganischen Verbindungen wie vorzugsweise der Alkalimetallalkyle, z. B. Methyllithium, Butyllithium oder Phenyllithium, der Alkylmagnesiumhalogenide, z. B. Methylmagnesiumchlorid, oder der Alkalimetall- und Erdalkalimetallalkoholate, z. B. Natriummethanolat, Natriumethanolat, Kaliumethanolat, Kalium- tert.Butanolat oder Dimethoxymagnesium.
   Auch können als Basen organische Basen eingesetzt werden, beispielsweise aus der Gruppe der tertiären aliphatischen Amine, z. B. Trimethylamin, Triethylamin, Tributylamin, Di-isopropylethylamin oder N-Methylpiperidin, oder der aromatischen tertiären Amine, z. B. Pyridin oder substituierte Pyridine wie Collidin, Lutidin oder 4-Dimethylaminopyridin, oder der bicyclische Amine wie 7-Methyl-1,5,7-triazabicyclo[4.4.0]- dec-5-en oder 1,8-Diazabicyclo[5.4.0]undec-7en (DBU). Bevorzugte Basen sind beispielsweise Kalium-tert.Butanolat, Lithium-bis(trimethylsilyl)amid oder 7-Methyl-1,5,7-triazabicyclo[4.4.0]- dec-5-en.
   Die Menge an Base kann im Allgemeinen breit variiert weren. Beispielsweise kann es sinnvoll sein, die Base in katalytischen Mengen, im Unterschuss, äquimolar oder im Überschuß einzusetzen. Eine vorzugsweise flüssige organische Base kann gegebenenfalls auch als Lösungsmittel verwendet werden.
   Geeignete Katalysatoren für die Michael-Addition nach Variante (aa) sind saure Katalysatoren, bespielsweise aus der Gruppe der anorganischen Säuren, z. B. Broensted-Säuren, wie Fluorwasserstoffsäure, Salzsäure, Bromwasserstoffsäure, Schwefelsäure oder Perchlorsäure, oder Lewis-Säuren wie Bortrifluorid, Aluminiumtrichlorid, Eisen-III-chlorid, Zinn-IV-chlorid, Titan-IV-chlorid, Scandium-III-triflat oder Zink-II-chlorid, sowie der organischen Säuren, z. B. Ameisensäure, Essigsäure, Propionsäure, Oxalsäure, Toluolsulfonsäure, Benzolsulfonsäure, Camphersulfonsäure, Zitronensäure oder Trifluoressigsäure.
   Die Menge an saurem Katalysator kann im Allgemeinen breit variiert weren. Beispielsweise kann es sinnvoll sein, die Säure in katalytischen Mengen, im Unterschuss, äquimolar oder im Überschuß einzusetzen. Eine vorzugsweise flüssige Säure kann gegebenenfalls auch als Lösungsmittel verwendet werden.
   Beispielsweise werden Verbindungen der Formel (III) auch durch Umesterung erhalten, dadurch gekennzeichnet, dass man
(bb) Verbindungen der Formel (III'), 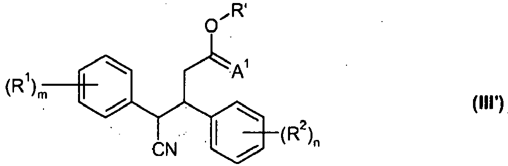 in denen R' einen Rest aus der Gruppe der für R möglichen Reste bedeutet aber von dem Rest R in der herzustellenden Verbindung (III) verschieden ist, mit einer Verbindung der Formel R-OH, in der R wie in Formel (III) definiert ist, zur Verbindung (III) umsetzt, wobei A¹, R¹, R², m und n in der Verbindung (III') wie in der jeweils herzustellenden Verbindung der Formel (III) definiert sind.

Die Umesterungen (bb) können beispielsweise mit einem geeigneten Alkohol R-OH in Gegenwart eines Katalysators, gegebenenfalls in Gegenwart eines aprotischen Lösungsmittels erfolgen. Weiterhin sind in der Regel Bedingungen vorteilhaft, mit denen das chemische Gleichgewicht auf die Seite des gewünschten Produkts verschoben wird, beispielsweise mit einem großen Überschuss des Alkohols R-OH unter praktisch wasserfreien Bedingungen, z. B. in Gegenwart eines Molekularsiebs.

Die Umsetzungen (Umesterungen) können in der Regel bei Temperaturen von 0°C bis180°C, vorzugsweise 20°C bis 100°C in Gegenwart einer Lewis- bzw. Broenstedt-Säure oder eines Enzyms durchgeführt werden [vgl. J. Org. Chem. 2002, 67, 431].

Geeignete Lösungsmittel sind z. B. folgende organische aprotische Lösungsmittel:
- aliphatische Kohlenwasserstoffe wie Pentan, Hexan, Cyclohexan oder Petrolether;
- aromatische Kohlenwasserstoffe wie Toluol, o-, m- oder p-Xylol,
- halogenierte Kohlenwasserstoffe wie Methylenchlorid (Dichlormethan), Chloroform oder Chlorbenzol,
- Ether wie Diethylether, Diisopropylether, tert.-Butylmethylether, Dioxan, Anisol oder Tetrahydrofuran (THF),
- Nitrile wie Acetonitril oder Propionitril,
- Ketone wie Aceton, Methylethylketon, Diethylketon oder tert.-Butylmethylketon,
- Dimethylsulfoxid, Dimethylformamid, Dimethylacetamid oder Sulfolan oder
- Gemische aus den genannten organischen Lösungsmitteln.

Bevorzugtes Lösungsmittel ist der Alkohol R-OH, der zugleich als Reaktant für die Umesterung verwendet wird, gegebenenfalls in Kombination mit einem der genannten aprotischen organischen Lösungsmittel.

Alternativ kann man den gewünschten Ester aus einem anderen Ester auch zweistufig durch saures oder basisches Verseifen des anderen Esters zur freien Säure, d. h. zu Verbindungen (III'), worin R' jeweils H bedeutet, und nachfolgender Veresterung mit einem Alkohol R-OH erhalten.

Die Veresterung aus der freien Säure der Formel (III')/R = H kann beispielsweise analog üblichen Methoden erfolgen, beispielsweise bei Temperaturen von 0°C bis 120°C, vorzugsweise 20°C bis 50°C, gegebenenfalls in Gegenwart eines Katalysators, in einem weitgehend wasserfreien Medium bzw. unter Bedingungen, in denen Wasser inklusive das bei der Veresterung entstehende Wasser gebunden oder anderweitig entfernt wird. Als Katalysator kommen wasserfreie Säuren und Basen, vorzugsweise organische Säuren oder Basen in Frage; siehe Handbücher für chemische Verfahren zur Veresterung von Carbonsäuren; siehe auch z. B. J. Am. Chem. Soc. 2007,129 (43),13321; J. Org. Chem. 1984,49 (22), 4287.

Geeignete Lösungsmittel für die Veresterung sind die oben zur Verfahrensvariante (bb) genannten aprotischen organischen Lösungsmittel geeignet, inklusive des Alkohols R-OH, der zugleich als Reaktant für die Veresterung verwendet wird, gegebenenfalls in Kombination mit einem der genannten aprotischen organischen Lösungsmittel.

Geeignete Katalysatoren für die Veresterung sind die zur genannten Verfahrensvariante (aa) (Michael-Addition) erwähnten Basen oder sauren oder basischen Katalysatoren in wasserfreier Form oder mit möglichst geringem Wasseranteil. Bevorzugte Katalysatoren sind die Basen Lithiumhydroxid, Kaliumcarbonat oder organische Amine wie Pyridine, subsitutierte Pyridine und DBU. Die der Veresterung gegebenenfalls vorgeschaltete Verseifung anderer Ester der Formeln (III'), jeweils mit R' ungleich H, kann analog üblichen Methoden erfolgen, beispielsweise bei Temperaturen von 0°C bis 120°C, vorzugsweise 20°C bis 50°C, gegebenenfalls in Gegenwart eines Katalysators, in einem wasserhaltigen Medium/Lösungsmittel; siehe Handbücher für chemische Verfahren zur Verseifung von Carbonsäureestern; siehe auch z. B. J. Am. Chem. Soc. 2007,129 (43),13321; J. Org. Chem. 1984,49 (22), 4287.

Geeignete Lösungsmittel für die Verseifung ist Wasser oder ein wasserhaltiges organisches Lösungsmittel, beispielsweise auf Basis der zur genannten Verfahrensvariante (aa)(Michael-Addition) erwähnten organischen Lösungmittel, vorzugsweise Wasser oder polare organische Lösungsmittel mit einem Wassergehalt wie THF.

Geeignete Katalysatoren für die Verseifung sind die zur genannten Verfahrensvariante (aa) (Michael-Addition) erwähnten Säuren, Basen oder sauren oder basischen Katalysatoren, jeweils mit einem Wassergehalt. Bevorzugte Katalysatoren sind wässrige Säuren und Basen, insbesondere Basen wie Lithiumhydroxid, Natriumhydroxid, Kaliumcarbonat, Pyridine, substituierte Pyridine und DBU in Gegenwart von organischen Lösungsmitteln.

Die Katalysatoren für die Veresterung oder die Verseifung können im Allgemeinen in katalytischen Mengen eingesetzt werden. Eine Verwendung in größeren Mengen, auch äquimolar oder im molaren Überschuß ist in der Regel auch möglich. Eine Verwendung als Lösungsmittel kommt oftmals auch in Frage.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden.

Sofern die Zwischen- und Endprodukte als Feststoffe erhalten werden, kann die Reinigung auch durch Umkristallisieren oder Digerieren erfolgen. Sofern einzelne Verbindungen (III') bzw. (III) nicht auf den voranstehend beschriebenen Wegen zugänglich sind, können sie durch Derivatisierung anderer Verbindungen (III') bzw. (III) hergestellt werden.

Für die Herstellung von stereochemisch angereicherten Verbindungen der Formel (I) können die zunächst als Diastereomerengemische erhaltenen Verbindungen (I) in die Stereoisomeren getrennt werden.

Alternativ bietet sich eine Trennung und Anreicherung der gewünschten Stereoisomeren auf der Stufe der Verbindungen (III) und nachfolgende enantioselektive Umsetzungen bis zur Stufe optisch aktiver Verbindungen (I) an.

Die Verbindungen der Formeln (I) bzw. (III) oder (III'), enthalten zwei Chiralitätszentren in Positionen 2 und 3 des Valeronitrilgerüstes bzw. Positionen 3 und 4 des Cyanobutyrat-Grundgerüsts und können je nach Substitutionsmuster ein oder mehrere weitere Chiralitätszentren enthalten.

Aufgrund der genannten zwei Chiralitätszentren existieren 4 Stereoisomere, und zwar zwei Erythro-Enantiomere und zwei Threo-Enantiomere

Zur Herstellung der erfindungsgemäßen Verbindungen (I) in Form der bevorzugten stereochemisch angereicherten Threo-Verbindungen oder Threo-2-Verbindungen ist es erforderlich die Threo-Verbindungen bzw. das Stereoisomer (Enantiomer) Threo-2 aus dem Gemisch der Stereoisomeren entsprechend anzureichern. Ein zweckmäßiges Verfahren beinhaltet daher zunächst eine Isolierung der Threo-Isomeren Threo-1 und Threo-2 aus dem Diastereomerengemisch (I), welches noch die Erythro-Isomeren enthält und anschließende Racemattrennung mit Isolierung oder Anreicherung des Enantionmeren Threo-2 aus dem Gemisch mit dem Enantiomeren Threo-1.

Die Isolierung der Threo-Isomeren als racemisches Gemisch kann analog der oben erwähnten üblichen Trenn- und Reinigungsverfahren erfolgen (Diastereomerentrennung).

Für die daran anschließende Herstellung von Verbindungen der Formel (I) in Form der stereochemisch angereicherten Threo-2-Isomeren kommen Racemattrennungsmethoden in Frage, die dem Fachmann aus analogen Fällen allgemein bekannt sind (vgl. Handbücher der Stereochemie), z. B. im Anschluss an Verfahren zur Trennung von Gemischen in Diastereomere, z. B. durch physikalische Verfahren wie Kristallisation, Chromatographieverfahren, vor allem Säulenchromatographie und Hochdruckflüssigchromatographie, Destillation, gegebenenfalls unter reduziertem Druck, Extraktion und andere Verfahren, können verbleibende Gemische von Enantiomeren in der Regel durch chromatographische Trennung an chiralen Festphasen getrennt werden. Für präparative Mengen oder im industriellen Maßstab kommen Verfahren wie die Kristallisation diastereomerer Salze, die aus den Diastereomerengemischen mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säure in Betracht; als optisch aktive Basen kommen z. B. Chinin, Cinchonin, Chinidin, Brucin, 1-(S)-oder 1-(R)-Phenylethylamin und andere analoge Basen in Frage.

Die Kristallisationen werden dann meist in wässrigen, alkoholischen oder wässrigorganischen Lösungsmittel durchgeführt, wobei das Diastereomer mit der geringeren Löslichkeit gegebenenfalls nach Animpfen zunächst ausfällt. Das eine Enantiomer der Verbindung der Formel (I) wird danach aus dem ausgefällten Salz oder das andere aus dem Kristallisat durch Ansäuern bzw. mit Base freigesetzt.

Gegenstand der Erfindung ist daher auch das Verfahren zur Herstellung der Threo-2-Isomere der Verbindungen (I), dadurch gekennzeichet, dass man mit Verbindungen (I) eine Racemattrennung durchführt und die Verbindung (I) in einer stereochemischen Reinheit von 60 bis 100 %, vorzugsweise 70 bis 100 %, mehr bevorzugt 80 bis 100 %, insbesondere 90 bis 100 %, bezogen auf das vorliegende Gemisch der threo-2-Enantiomeren isoliert.

Alternativ zu den genannten Racemattrennungsverfahren eignen sich zur Herstellung der Threo-2-Enantiomeren (I) prinzipiell auch enantioselektive Verfahren ausgehend von stereochemisch reinen Ausgangsstoffen.

Zur Herstellung der Säureadditionssalze der Verbindungen der Formel (I) kommen allgemein folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid öder Benzol und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat oder Kalium-tert.-butylat, oder Ammoniak, Ethanolamin oder quartäres Ammoniumhydroxid der Formel [NRR'R"R"']⁺ OH⁻.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Eine Kollektion aus Verbindungen der Formel (I), die nach den obengenannten Verfahren synthetisiert werden können, können zusätzlich in parallelisierter Weise hergestellt werden, wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen der Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.

Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß den hier beschriebenen Verfahren liefert Verbindungen der Formel (I) in Form von Substanzkollektionen oder-bibliotheken. Gegenstand der vorliegenden Erfindung sind daher auch Bibliotheken der Verbindungen der Formel (I), die mindestens zwei Verbindungen der Formel (I) enthalten, und deren Vorprodukte.

Die erfindungsgemäßen Verbindungen der Formel (I) (und/oder deren Salze), oben und im Folgenden zusammen als "erfindungsgemäße Verbindungen", "erfindungsgemäße Verbindungen (I)" oder kurz als "Verbindungen (I)" bezeichnet, weisen eine ausgezeichnete herbizide Wirksamkeit gegen ein breites Spektrum wirtschaftlich wichtiger mono und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono-oder dikotyle Unkräuter oder unerwünschte Kulturpflanzen), das Saatgut (z.B. Körner, Samen oder vegetative Vermehrungsorgane wie Knollen oder Sprossteile mit Knospen), den Boden, in dem oder auf dem die Pflanzen wachsen, (z. B. den Boden von Kulturland oder Nicht-Kulturland) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird. Obgleich die erfindungsgemäßen Verbindungen eine ausgezeichnete herbizide Aktivität gegenüber mono und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen und/oder deren Salze in wirtschaftlich bedeutenden transgenen Kulturen von Nutz und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die tränsgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Getreide wie Weizen, Gerste, Roggen, Hafer, Hirse und Reis im Vor- oder Nachauflauf, insbesondere im Weizen im Nachauflauf. Bevorzugt ist auch die Verwendung in Mais im Vor- oder Nachauflauf, insbesondere im Mais im Vorauflauf.

Bevorzugt ist auch die Verwendung in Soja im Vor- oder Nachauflauf, insbesondere Soja im Nachauflauf.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Gegenstand der Erfindung ist auch das Verfahren (Anwendungsverfahren) zur Bekämpfung von Schadpflanzen oder zur Wachstumsregulierung von Pflanzen, dadurch gekennzeichnet, dass man eine wirksame Menge von einer oder mehreren Verbindungen der Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Die erfindungsgemäßen Verbindungen (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) und/oder deren Salze enthalten.

Die Verbindungen der Formel (I) und/oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I) und/oder dessen Salze. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 15th edition, The British Crop Protection Council and the Royal Soc. of Chemistry, 2006 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt: Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminocyclopyrachlor-potassium, Aminocyclopyrachlor-methyl, Aminopyralid, Amitrole, Ammoniumsulfamat, Ancymidol, Anilofos, Asulam, Atrazine, Azafenidin, Azimsulfuron, Aziprotryn, Beflubutamid, Benazolin, Benazolin-ethyl, Bencarbazone, Benfluralin, Benfuresate, Bensulide, Bensulfuron, Bensulfuron-methyl, Bentazone, Benzfendizone, Benzobicyclon, Benzofenap, Benzofluor, Benzoylprop, Bicyclopyrone, Bifenox, Bilanafos, Bilanafos-sodium, Bispyribac, Bispyribac-sodium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop, Clodinafop-propargyl, Clofencet, Clofencet-potassium, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3-fluorpropyl)-4,5-dihydro-5-oxo-1H-tetrazol-1-yl]-phenyl]-ethansulfonamid, F-7967, d. h. 3-[7-Chlor-5-fluor-2-(trifluormethyl)-1H-benzimidazol-4-yl]-1-methyl-6-(trifluormethyl)pyrimidin-2,4(1H,3H)-dion, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumicloracpentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacetmethyl, Fluthiamide, Fomesafen, Foramsulfuron, Forchlorfenuron, Fosamine, Furyloxyfen, Gibberellinsäure, Glufosinate, Glufosinate-ammonium, Glufosinate-P, Glufosinate-P-ammonium, Glufosinate-P-sodium, Glyphosate, Glyphosateisopropylammonium, H-9201, d. h. O-(2,4-Dimethyl-6-nitrophenyl)-O-ethyl-isopropylphosphoramidothioat, Halosafen, Halosulfuron, Halosulfuron-methyl, Haloxyfop, Haloxyfop-P, Haloxyfop-ethoxyethyl, Haloxyfop-P-ethoxyethyl, Haloxyfopmethyl, Haloxyfop-P-methyl, Hexazinone, HW-02, d. h. 1-(Dimethoxyphosphoryl)-ethyl-(2,4-dichlorphenoxy)acetat, Imazamethabenz, Imazamethabenz-methyl, Imazamox, Imazamox-ammonium, Imazapic, Imazapyr, Imazapyr-isopropylammonium, Imazaquin, Imazaquin-ammonium, Imazethapyr, Imazethapyrammonium, Imazosulfuron, Inabenfide, Indanofan, Indaziflam, Indolessigsäure (IAA), 4-Indol-3-ylbuttersäure (IBA), lodosulfuron, lodosulfuron-methyl-sodium, lofensulfuron, lofensulfuron-sodium, loxynil, Ipfencarbazone, Isocarbamid, Isopropalin, Isoproturon, Isouron, Isoxaben, Isoxachlortole, Isoxaflutole, Isoxapyrifop, KUH-043, d. h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Karbutilate, Ketospiradox, Lactofen, Lenacil, Linuron, Maleinsäurehydrazid, MCPA, MCPB, MCPB-methyl, - ethyl und -sodium, Mecoprop, Mecoprop-sodium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuronmethyl, Mesotrione, Methabenzthiazuron, Metam, Metamifop, Metamitron, Metazachlor, Metazasulfuron, Methazole, Methiopyrsulfuron, Methiozolin, Methoxyphenone, Methyldymron, 1-Methylcyclopropen, Methylisothiocyanat, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monosulfuron-ester, Monuron, MT-128, d. h. 6-Chlor-N-[(2E)-3-chlorprop-2-en-1-yl]-5-methyl-N-phenylpyridazin-3-amin, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-Dichlorobenzoyl)-1-methyl-5-benzyloxypyrazole, Neburon, Nicosulfuron, Nipyraclofen, Nitralin, Nitrofen, Nitrophenolat-sodium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron, Pyrazosulfuron-ethyl, Pyrazoxyfen, Pyribambenz, Pyribambenz-isopropyl, Pyribambenz-propyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Saflufenacil, Secbumeton, Sethoxydim, Siduron, Simazine, Simetryn, SN-106279, d. h. Methyl-(2R)-2-({7-[2-chlor-4-(trifluormethyl)phenoxy]-2-naphthyl}oxy)propanoat, Sulcotrione, Sulfallate (CDEC), Sulfentrazone, Sulfometuron, Sulfometuron-methyl, Sulfosate (Glyphosatetrimesium), Sulfosulfuron, SW-065, SYN-523, SYP-249, d. h. 1-Ethoxy-3-methyl-1-oxobut-3-en-2-yl-5-[2-chlor-4-(trifluormethyl)phenoxy]-2-nitrobenzoat, SYP-300, d. h. 1-[7-Fluor-3-oxo-4-(prop-2-in-1-yl)-3,4-dihydro-2H-1,4-benzoxazin-6-yl]-3-propyl-2-thioxoimidazolidin-4,5-dion, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triafamone, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfurön-methyl, Trimeturon, Trinexapac, Trinexapac-ethyl, Tritosulfuron, Tsitodef, Uniconazole, Uniconazole-P, Vernolate, ZJ-0862, d. h. 3,4-Dichlor-N-{2-[(4,6-dimethoxypyrimidin-2-yl)oxy]benzyl}anilin, sowie die folgenden Verbindungen:

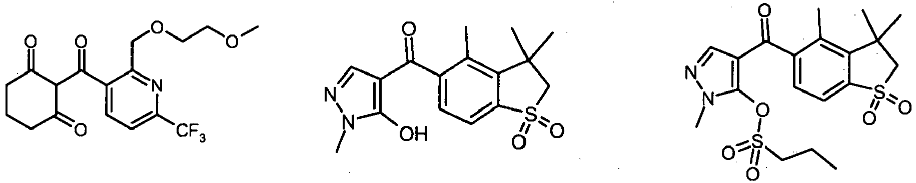

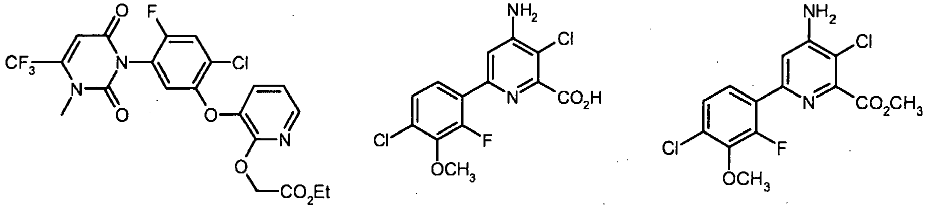

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
A) Verbindungen der Formel (S-I), 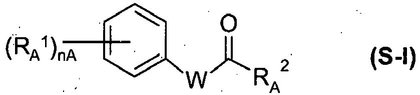 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W_{A}¹) bis (W_{A}⁴), 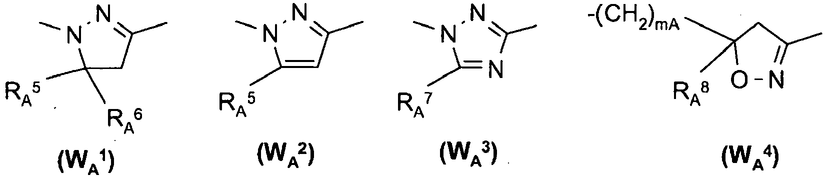
   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-I) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäure-ethylester (S1-1) ("Mefenpyr-diethyl", siehe Pestic. Man.), und verwandte Verbindungen, wie sie in der WO 91/07874 beschrieben sind;
   b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind;
   c) Verbindungen vom Typ der Triazolcarbonsäuren, vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-6), und verwandte Verbindungen wie sie in EP-A-174 562 und EP-A-346 620 beschrieben sind;
   d) Verbindungen vom Typ der 5-Benzyl- oder 5-Phenyl-2-isoxazolin-3- carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder -n-propylester (S1-10) oder der 5-(4-Fluorphenyl)-5-phenyl-2-isoxazolin-3-carbonsäureethylester (S1-11), wie sie in der Patentanmeldung WO-A-95/07897 beschrieben sind.
B) Chinolinderivate der Formel (S-II), 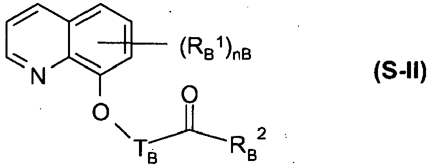 wobei die Symbole und Indizes folgende Bedeutungen haben:
   - R_{B}¹: Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: OR_{B}³, SR_{B}³ oder NR_{B}³R_{B}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2), vorzugsweise (5-Chlor-8-chinolinoxy)essigsäure-(1-methylhexyl)-ester (Common name "Cloquintocet-mexyl" (S2-1) (siehe Pestic. Man.), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie deren Hydrate und Salze wie sie in der WO-A-2002/034048 beschrieben sind.
   b) Verbindungen vom Typ der (5-Chlor-8-chinolinoxy)malonsäure, vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
C) Verbindungen der Formel (S-III) 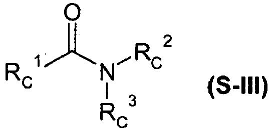 wobei die Symbole und Indizes folgende Bedeutungen haben:
   - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{C}², R_{C}³: ist gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B. "Dichlormid" (siehe Pestic.Man.) (= N,N-Diallyl-2,2-dichloracetamid), "R-29148" (= 3-Dichloracetyl-2,2,5-trimethyl-1,3-oxazolidin von der Firma Stauffer),
   "R-28725" (= 3-Dichloracetyl-2,2,-dimethyl-1,3-oxazolidin von der Firma Stauffer),
   "Benoxacor" (siehe Pestic. Man.) (= 4-Dichloracetyl-3,4-dihydro-3-methyl-2H-1,4-benzoxazin),
   "PPG-1292" (= N-Allyl-N-[(1,3-dioxolan-2-yl)methyl]-dichloracetamid von der Firma PPG Industries),
   "DKA-24" (= N-Allyl-N-[(allylaminocarbonyl)methyl]-dichloracetamid von der Firma Sagro-Chem),
   "AD-67" oder "MON 4660" (= 3-Dichloracetyl-1-oxa-3-aza-spiro[4,5]decan von der Firma Nitrokemia bzw. Monsanto),
   "TI-35" (= 1-Dichloracetyl-azepan von der Firma TRI-Chemical RT) "Diclonon" (Dicyclonon) oder "BAS145138" oder "LAB145138" (= 3-Dichloracetyl-2,5,5-trimethyl-1,3-diazabicyclo[4.3.0]nonan von der Firma BASF) und
   "Furilazol" oder "MON 13900" (siehe Pestic. Man.) (= (RS)-3-Dichloracetyl-5-(2-furyl)-2,2-dimethyloxazolidin)
D) N-Acylsulfonamide der Formel (S-IV) und ihre Salze, 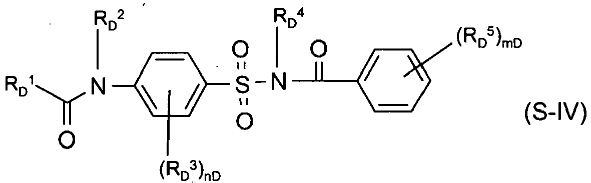 worin
   - R_{D}¹: Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
   wobei jeder Kohlenwasserstoffteil vorzugsweise 1 bis 20 C-Atome aufweist und ein C-haltiger Rest R_{D}¹ inklusive Substituenten vorzugsweise 1 bis 30 C-Atome aufweist;
   - R_{D}²: Wasserstoff oder (C₁-C₄)Alkyl, vorzugsweise Wasserstoff, oder
   - R_{D}¹ und R_{D}²: zusammen mit der Gruppe der Formel -CO-N- den Rest eines 3- bis 8-gliedrigen gesättigten oder ungesättigten Rings;
   - R_{D}³: gleich oder verschieden Halogen, Cyano, Nitro, Amino, Hydroxy, Carboxy, Formyl, CONH₂, SO₂NH₂ oder einen Rest der Formel -Z^{b}-R^{b} ;
   - R_{D}⁴: Wasserstoff oder (C₁-C₄)Alkyl, vorzugsweise H;
   - R_{D}⁵: gleich oder verschieden Halogen, Cyano, Nitro, Amino, Hydroxy, Carboxy, CHO, CONH₂, SO₂NH₂ oder einen Rest der Formel -Z^{c}-R^{c};
   - R^{a}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b},R^{c}: gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a}: eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-, -SO₂-NR*-oder -NR*-SO₂-, wobei die rechts angegebene Bindung der jeweiligen divalenten Gruppe die Bindung zum Rest R^{a} ist und wobei die R* in den letztgenannten 5 Resten unabhängig voneinander jeweils H, (C₁-C₄)-Alkyl oder Halo-(C₁-C₄)-alkyl bedeuten;
   - Z^{b},Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*-oder -NR*-CO-, wobei die rechts angegebene Bindung der jeweiligen divalenten Gruppe die Bindung zum Rest R^{b} bzw. R^{c} ist und wobei die R* in den letztgenannten 5 Resten unabhängig voneinander jeweils H, (C₁-C₄)-Alkyl oder Halo-(C₁-C₄)-alkyl bedeuten;
   - n_{D}: eine ganze Zahl von 0 bis 4, vorzugsweise 0, 1 oder 2, insbesondere 0 oder 1, und
   - m_{D}: eine ganze Zahl von 0 bis 5, vorzugsweise 0, 1, 2 oder 3, insbesondere 0, 1 oder 2;
   bedeuten;
E) Acylsulfamoylbenzoesäureamide der allgemeinen Formel (S-V), gegebenenfalls auch in Salzform, 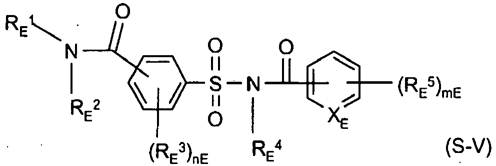 worin
   - X_{E}: CH oder N;
   - R_{E}¹: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
   - R_{E}²: Wasserstoff, Hydroxy, (C₁-C₆)Alkyl, (C₂-C₆)Alkenyl, (C₂-C₆)Alkinyl, (C₁-C₆)Alkoxy, (C₂-C₆)Alkenyloxy, wobei die fünf letztgenannten Reste gegebenenfalls durch einen oder mehrere, gleiche oder verschiedene Reste aus der Gruppe Halogen, Hydroxy, (C₁-C₄)Alkyl, (C₁-C₄)Alkoxy und (C₁-C₄)Alkylthio substituiert sind, oder
   - R_{E}¹ und R_{E}²: zusammen mit dem sie tragenden Stickstoffatom einen 3- bis 8-gliedrigen gesättigten oder ungesättigten Ring;
   - R_{E}³: Halogen, Cyano, Nitro, Amino, Hydroxy, Carboxy, CHO, CONH₂, SO₂NH₂ oder Z^{b}-R^{b};
   - R_{E}⁴: Wasserstoff, (C₁-C₄)Alkyl, (C₂-C₄)Alkenyl oder (C₂-C₄)Alkinyl;
   - R_{E}⁵: Halogen, Cyano, Nitro, Amino, Hydroxy, Carboxy, Phosphoryl, CHO, CONH₂, SO₂NH₂ oder Z^{c}-R^{c};
   - R^{a}: einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
   - R^{b}, R^{c}: gleich oder verschieden einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
   - Z^{a}: eine divalente Einheit aus der Gruppe O, S, CO, CS, C(O)O, C(O)S, SO, SO₂, NR^{d}, C(O)NR^{d} oder SO₂NR^{d};
   - Z^{b}, Z^{c}: gleich oder verschieden eine direkte Bindung oder eine divalente Einheit aus der Gruppe O, S, CO, CS, C(O)O, C(O)S, SO, SO₂, NR^{d}, SO₂NR^{d} oder C(O)NR^{d};
   - R^{d}: Wasserstoff, (C₁-C₄)-Alkyl oder (C₁-C₄)-Haloalkyl;
   - n_{E}: eine ganze Zahl von 0 bis 4, und
   - m_{E}: für den Fall, daß X für CH steht, eine ganze Zahl von 0 bis 5, und für den Fall, daß X für N steht, eine ganze Zahl von 0 bis 4
   bedeuten;
   davon bevorzugt sind Verbindungen (auch in Form ihrer Salze) vom Typ der Acylsulfamoylbenzoesäureamide, z.B. der nachfolgenden Formel (S-VI), die z.B. bekannt sind aus WO 99/16744, 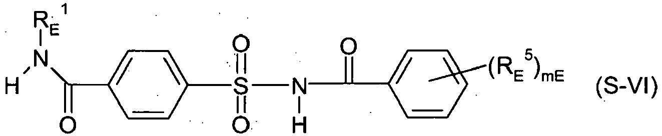 z.B. solche worin
   R_{E}¹ = Cyclopropyl und R_{E}⁵ = 2-OMe ist ("Cyprosulfamide", S3-1),
   R_{E}¹ = Cyclopropyl und R_{E}⁵ = 5-Cl-2-OMe ist (S3-2),
   R_{E}¹ = Ethyl und R_{E}⁵ = 2-OMe ist(S3-3),
   R_{E}¹ = Isopropyl und R_{E}⁵ = 5-Cl-2-OMe ist (S3-4) und
   R_{E}¹ = Isopropyl und R_{E}⁵ = 2-OMe ist (S3-5);
F) Verbindungen vom Typ der N-Acylsulfamoylphenylharnstoffe der Formel (S-VII), die z.B. bekannt sind aus der EP-A-365484, 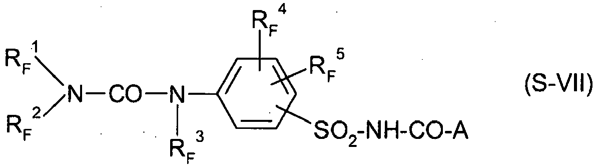 worin
   - A: für einen Rest aus der Gruppe 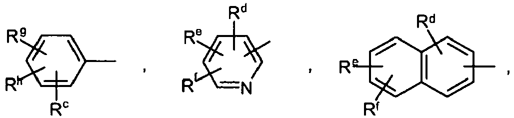 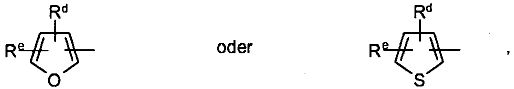
   - R_{F}¹ und R_{F}²: unabhängig voneinander für Wasserstoff, (C₁-C₈)Alkyl, (C₃-C₈)Cycloalkyl, (C₃-C₆)Alkenyl, (C₃-C₆)Alkinyl, 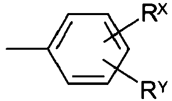 oder durch (C₁-C₄)Alkoxy oder 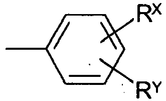 substituiertes (C₁-C₄)Alkoxy, oder
   - R_{F}¹ und R_{F}²: gemeinsam für eine (C₄-C₆)Alkylenbrücke oder eine durch Sauerstoff, Schwefel, SO, SO₂, NH oder -N(C₁-C₄-Alkyl)-unterbrochene (C₄-C₆)Alkylenbrücke,
   - R_{F}³: für Wasserstoff oder (C₁-C₄)Alkyl,
   - R_{F}⁴ und R_{F}⁵: unabhängig voneinander für Wasserstoff, Halogen, Cyan, Nitro, Trifluormethyl, C₁-C₄-Alkyl, C₁-C₄-Alkoxy, C₁-C₄-Alkylthio, C₁-C₄-Alkylsulfinyl, C₁-C₄-Alkylsulfonyl, -COOR^{j}, -CONR^{k}R^{m}, -CORⁿ, -SO₂NR^{k}R^{m} oder -OSO₂-C₁-C₄-Alkyl, oder R^{a} und R^{b} gemeinsam für eine (C₃-C₄)Alkylenbrücke, die durch Halogen oder C₁-C₄-Alkyl substituiert sein kann, oder eine (C₃-C₄)Alkenylenbrücke, die durch Halogen oder (C₁-C₄)Alkyl substituiert sein kann, oder eine C₄-Alkadienylenbrücke, die durch Halogen oder (C₁-C₄)Alkyl substituiert sein kann, und
   - R^{g} und R^{h}: unabhängig voneinander für Wasserstoff, Halogen, C₁-C₄-Alkyl, Trifluormethyl, Methoxy, Methylthio oder -COOR^{j} stehen, wobei
   - R^{c}: Wasserstoff, Halogen, (C₁-C₄)Alkyl oder Methoxy,
   - R^{d}: Wasserstoff, Halogen, Nitro, (C₁-C₄)Alkyl, (C₁-C₄)Alkoxy, (C₁-C₄)Alkylthio, (C₁-C₄)Alkylsulfinyl, (C₁-C₄)Alkylsulfonyl, -COOR^{j} oder -CONR^{k}R^{m},
   - R^{e}: Wasserstoff, Halogen, C₁-C₄-Alkyl, -COOR^{j}, Trifluormethyl oder Methoxy, oder R^{d} und R^{e} gemeinsam für eine (C₃-C₄)Alkylenbrücke,
   - R^{f}: Wasserstoff, Halogen oder (C₁-C₄)Alkyl,
   - R^{X} und R^{Y}: unabhängig voneinander Wasserstoff, Halogen, (C₁-C₄)Alkyl, (C₁-C₄)Alkoxy, (C₁-C₄)Alkylthio, -COOR⁴, Trifluormethyl, Nitro oder Cyan,
   - R^{j}, R^{k} und R^{m}: unabhängig voneinander Wasserstoff oder (C₁-C₄)Alkyl,
   - R^{k} und R^{m}: gemeinsam eine (C₄-C₆)Alkylenbrücke oder eine durch Sauerstoff, NH oder -N(C₁-C₄-Alkyl)- unterbrochene C₄-C₆-Alkylenbrücke, und
   - Rⁿ: (C₁-C₄)Alkyl, Phenyl oder durch Halogen, (C₁-C₄)Alkyl, Methoxy, Nitro oder Trifluormethyl substituiertes Phenyl bedeuten,
   davon bevorzugt sind:
   1-[4-(N-2-Methoxybenzoylsulfamoyl)phenyl]-3-methylharnstoff, 1-[4-(N-2-Methoxybenzoylsulfamoyl)phenyl]-3,3-dimethylharnstoff, 1-[4-(N-4,5-Dimethylbenzoylsulfamoyl)phenyl]-3-methylharnstoff, 1-[4-(N-Naphthoylsulfamoyl)phenyl]-3,3-dimethylharnstoff, einschließlich der Stereoisomeren und der in der Landwirtschaft gebräuchlichen Salze,
G) Wirkstoffe aus der Klasse der Hydroxyaromaten und der aromatischaliphatischen Carbonsäurederivate, z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 1,2-Dihydro-2-oxo-6-trifluoromethylpyridin-3-carboxamid, 2-Hydroxyzimtsäure, 2,4-Dichlorzimtsäure, wie sie in der WO 2004084631, WO 2005015994, WO 2006007981, WO 2005016001 beschrieben sind;
H) Wirkstoffe aus der Klasse der 1,2-Dihydrochinoxalin-2-one, z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO 2005112630 beschrieben sind,
I) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (siehe Pestic. Man.) (= Piperidin-1-thiocarbonsäure-S-1-methyl-1-phenylethylester), das als Safener für Reis gegen Schäden des Herbizids Molinate bekannt ist,
   "Daimuron" oder "SK 23" (siehe Pestic. Man.) (= 1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist,
   "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
K) Verbindungen der Formel (S-IX), wie sie in der WO-A-1998/38856 beschrieben sind 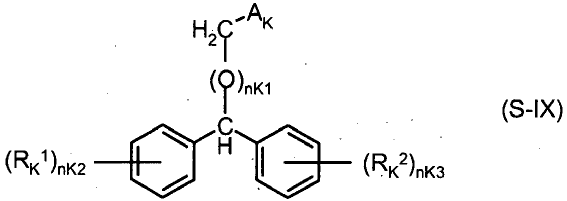 worin die Symbole und Indizes folgende Bedeutungen haben:
   - R_{K}¹, R_{K}²: unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - ^{A}K: COOR_{K}³ oder COOR_{K}⁴
   - R_{K}³, R_{K}⁴: unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{K}¹: 0 oder 1 und
   - n_{K}², n_{K}³: unabhängig voneinander 0, 1 oder 2;
   vorzugsweise:
   Methyl-(diphenylmethoxy)acetat (CAS-Regno: 41858-19-9),
L) Verbindungen der Formel (S-X),
   wie sie in der WO A-98/27049 beschrieben sind 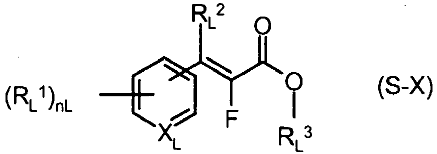 worin die Symbole und Indizes folgende Bedeutungen haben:
   - X_{L}: CH oder N,
   - n_{L}: für den Fall, dass X=N ist, eine ganze Zahl von 0 bis 4 und für den Fall, dass X=CH ist, eine ganze Zahl von 0 bis 5,
   - R_{L}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)Alkylsulfonyl, (C₁-C₄)Alkoxycarbonyl, ggf. substituiertes. Phenyl, ggf. substituiertes Phenoxy,
   - R_{L}²: Wasserstoff oder (C₁-C₄)Alkyl
   - R_{L}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze.
M) Wirkstoffe aus der Klasse der 3-(5-Tetrazolylcarbonyl)-2-chinolone, z.B. 1,2-Dihydro-4-hydroxy-1-ethyl-3-(5-tetrazolylcarbonyl)-2-chinolon (CAS-Regno: 219479-18-2), 1,2-Dihydro-4-hydroxy-1-methyl-3-(5-tetrazolylcarbonyl)-2-chinolon (CAS-Regno: 95855-00-8), wie sie in der WO-A-1999000020 beschrieben sind,
N) Verbindungen der Formeln (S-XI) oder (S-XII) wie sie in der WO-A-2007023719 und WO-A-2007023764 beschrieben sind 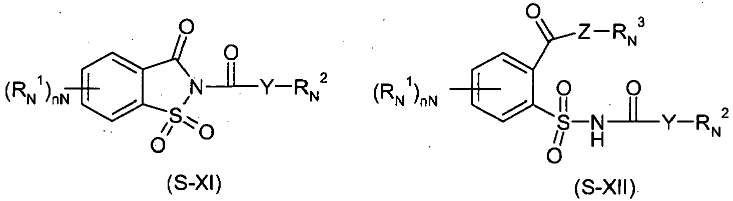 worin
   - R_{N}¹: Halogen, (C₁-C₄)Alkyl, Methoxy, Nitro, Cyano, CF₃, OCF₃
   - Y, Z: unabhängig voneinander O oder S,
   - n_{N}: eine ganze Zahl von 0 bis 4,
   - R_{N}²: (C₁-C₁₆)Alkyl, (C₂-C₆)Alkenyl, (C₃-C₆)Cycloalkyl, Aryl; Benzyl, Halobenzyl,
   - R_{N}³: Wasserstoff, (C₁-C₆)Alkyl bedeuten;
O) eine oder mehreren Verbindungen aus Gruppe:
   1,8-Naphthalsäureanhydrid,
   O,O-Diethyl S-2-ethylthioethyl phosphordithioat (Disulfoton),
   4-Chlorphenyl-methylcarbamat (Mephenate),
   O,O-Diethyl-O-phenylphosphorotioat (Dietholate),
   4-Carboxy-3,4-dihydro-2H-1-benzopyran-4-essigsäure (CL-304415, CAS-Regno: 31541-57-8),
   2-propenyl 1-oxa-4-azaspiro[4.5]decane-4-carbodithioate (MG-838, CAS-Regno: 133993-74-5),
   Methyl-[(3-oxo-1H-2-benzothiopyran-4(3H)-yliden)methoxy]acetate (aus WO-A-98/13361; CAS-Regno: 205121-04-6),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole), 2-Dichlormethyl-2-methyl-1,3-dioxolan (MG-191),
   einschließlich der jeweils möglichen Stereoisomeren und der in der Landwirtschaft gebräuchlichen Salze.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I) und/oder deren Salze. Sie kann innerhalb weiter Grenzen schwanken. Für die Anwendung als Herbizid zur Bekämpfung von Schadpflanzen liegt sie beispielsweise im Bereich von 0,001 bis 10,0 kg/ha oder mehr Aktivsubstanz, vorzugsweise im Bereich von 0,005 bis 5 kg/ha, insbesondere im Bereich von 0,01 bis 1 kg/ha Aktivsubstanz. Dies gilt sowohl für die Anwendung im Vorauflauf oder im Nachauflauf.

Bei der Anwendung als Pflanzenwachstumsregulator, beispielsweise als Halmverkürzer bei Kulturpflanzen, wie sie oben genannt worden sind, vorzugsweise Getreidepflanzen wie Weizen, Gerste, Roggen, Triticale, Hirse, Reis oder Mais, liegt die Aufwandmenge beispielsweise im Bereich von 0,001 bis 2 kg/ha oder mehr Aktivsubstanz, vorzugsweise im Bereich von 0,005 bis 1 kg/ha, insbesondere im Bereich von 10 bis 500 g/ha Aktivsubstanz, ganz besonders von 20 bis 250 g/ha Aktivsubstanz. (bitte prüfen, ob dies genannt werden soll) Dies gilt sowohl für die Anwendung im Vorauflauf oder im Nachauflauf, wobei die Nachauflaufbehandlung in der Regel bevorzugt ist.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Nachfolgend sind beispielhaft einige Synthesebeispiele von Verbindungen der allgemeinen Formel (I) beschrieben. In den Beispielen beziehen sich Mengenangaben (auch Prozentangaben) auf das Gewicht, sofern nichts anderes speziell angegeben ist.

Die Symbole ">" und "<" bedeuten "größer als" beziehungsweise "kleiner als". Das Symbol "≥" bedeutet "größer als oder gleich", das Symbol "≤" bedeutet "kleiner als oder gleich".

Wenn im Rahmen der Beschreibung und der Beispiele Bezeichnungen "R" und "S" für die absolute Konfiguration an einem Chiralitätszentrum der Stereoisomeren der Formel (I) angegeben ist, folgt diese der RS-Nomenklatur nach der Cahn-Ingold-Prelog-Regel, wenn nichts anderes näher definiert ist.

### (A) Synthesebeispiele

### Beispiel A1: Herstellung eines erythro-threo-Diastereomerengemischs des 5-(2,6-Dioxocyclohexyl)-2-(3-fluorphenyl)-3-(4-fluorphenyl)-5-oxopentannitrils [Tabelle 2, Beispiel Iaa20]

### Schritt 1: 4-Cyan-4-(3-fluorphenyl)-3-(4-fluorphenyl)-butansäuremethylester

Zu 0,820 g (4,55 mmol) 3-(4-Fluorphenyl)acrylsäuremethylester in 15,0 ml Toluol gab man unter Schutzgas (Ar) 0,767 g (5,0 mmol) (3-Fluorphenyl)acetonitril und 0,1 ml Natriummethylat-Lösung (30 % in Methanol) und rührte 15 h bei 65°C in einem geschlossenen Gefäß. Man entfernte das Lösemittel im Vakuum, nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Nach Chromatographie des Rückstands an Kieselgel (Ethylacetat / Heptan = 20:80) erhielt man 0,950 g (57% d. Th.) der diastereomeren 4-Cyan-4-(3-fluorphenyl)-3-(4-fluorphenyl)butansäuremethylester (erythro:threo = 54:46 gemäß Integration der Methyl-Singuletts im ¹H-NMR in CDCl₃ bei 3,66 ppm und 3,56 ppm).

### Schritt 2: 4-Cyan-3-(4-fluorphenyl)-4-(3-fluorphenyl)butansäure

Zu 1,75 g (5,55 mmol) 4-Cyan-4-(3-fluorphenyl)-3-(4-fluorphenyl)-butansäuremethylester in 50,0 ml Methanol gab man unter Schutzgas (Ar) 10 ml 2 molare wässrige Natronlauge und rührte 8 h bei 25°C. Man entfernte das Methanol im Vakuum. Der Rückstand wurde mit konzentrierter Salzsäure angesäuert (pH = 3) und dreimal mit jeweils 15 ml Dichlormethan extrahiert. Die vereinigten organischen Phasen wurden über Natriumsulfat getrocknet und das Lösemittel im Vakuum entfernt. Man erhielt 1,62 g (96.9 % d. Th.) der Titelverbindung in Form von farblosen Kristallen (erythro:threo = 49:51, Vergleich der Multipletts im ¹H-NMR in CDCl₃ bei 2,60 und 2,85 ppm).

### Schritt 3: 4-Cyan-4-(3-fluorphenyl)-3-(4-fluorphenyl)-butansäure-(3-oxocyclohex-1-en-1-yl)-ester

Zu 0,452 g (1,5 mmol) 4-Cyan-3-(4-fluorphenyl)-4-(3-fluorphenyl)butansäure gab man 1,79 g (15 mmol) Thionylchlorid und rührte 1,5 h bei 80°C. Man entfernte den Überschuß von Thionylchlorid im Vakuum. Das so konzentrierte Reaktionsgemisch (enthält Säurechlorid) wurde in 3 ml Dichlormethan gelöst und bei 0°C tropfenweise (ca 30 min.) zur Mischung von 0,252 g (2,25 mmol) 1,3-Cyclohexandion und 0,304 g (3 mmol) Triethylamin in Dichlormethan (10 ml) zugegeben. Nach 3 h Rühren bei 25 °C entfernte man das Lösemittel im Vakuum, nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Das erhaltene Rohprodukt wurde im Schritt 4 weiter umgesetzt.

### Schritt 4: 5-(2,6-Dioxocyclohexyl)-2-(3-fluorphenyl)-3-(4-fluorphenyl)-5-oxopentannitril

Zu 0,593 g (1,50 mmol) 4-Cyan-4-(3-fluorphenyl)-3-(4-fluorphenyl)-butansäure-(3-oxocyclohex-1-en-1-yl)-ester in 5,0 ml Acetonitril gab man 0,013 g (0,15 mmol) Acetocyanhydrin und 0,273 g (2,0 mmol) Triethylamin und rührte über Nacht bei 25°C in einem geschlossenen Gefäß. Man entfernte das Lösemittel im Vakuum, nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Durch präparative Chromatographie [(90 ml/min Acetonitril/Wasser (Trifluoressigsäure 0.05%)] an einer Festphase [Nucleodur C18, HTec C18, 10 µm, (250 x 50)-mm Säule] erhielt man 0,267 g (45% d. Th.) eines erythro-threo-Diastereomerengemischs von 5-(2,6-Dioxocyclohexyl)-2-(3-fluorphenyl)-3-(4-fluorphenyl)-5-oxopentannitril (erythro:threo = 63:37 gemäß Integration der Dubletts im ¹H-NMR in CDCl₃ bei 4,30 ppm und 4,03 ppm); ¹H-NMR in CDCl₃
Erythro: 17,47 (s, 1 H), 4,30 (d, 1 H), Threo: 17,46 (s, 1 H), 4,03 (d, 1 H)
Massenspektrum: Molpeak 394 [M-1]+
Retentionszeit bei HPLC: 1,58 min.

### Beispiel A2: Diastereomerengemisch von 2,2-Dimethylpropansäure-{2-[3-(4-Chlorphenyl)-4-cyan-4-(3-fluorphenyl)-butanoyl]-3-oxocyclohex-1-en-1-yl}-ester [Tabelle 2, Beispiel Iaa32]

Zu 0,180 g (0,43 mmol) 5-(2,6-Dioxocyclohexyl)-2-(3-fluorphenyl)-3-(4-chlorphenyl)-5-oxopentanonitril in 3,0 ml Dichlormethan gab man 0,091 g (0,656 mmol) Triethylamin und bei 0°C 0,069 g (0,568 mmol) 2,2-Dimethylpropanoylchlorid und rührte 3 h bei 25°C in einem geschlossenen Gefäß. Man entfernte das Lösemittel im Vakuum, nahm den Rückstand in Ethylacetat auf und wusch zweimal mit je 25 ml Wasser. Die vereinigten organischen Phasen wurden über Natriumsulfat getrocknet und.das Lösemittel im Vakuum entfernt. Durch präparative Chromatographie [(90 ml/min Acetonitril/Wasser (Trifluoressigsäure 0,05%)] an einer Festphase [Nucleodur C18, HTec C18, 10µm, (250 x 50)-mm Säule] erhielt man 114 mg (8% d. Th.) eines erythro-threo-Diastereomerengemischs von 2,2-Dimethylpropansäure-{2-[3-(4-Chlorphenyl)-4-cyan-4-(3-fluorphenyl)-butanoyl]-3-oxocyclohex-1-en-1-yl}-ester (erythro:threo = 70:30 gemäß Integration der tert-Butyl-Singuletts im ¹H-NMR in CDCl₃ bei 1,15 ppm und 1,06 ppm).

### Beispiel A3: Diastereomerengemisch von 2,6-Dicyan-6-(3-fluorphenyl)-5-(4-fluorphenyl)-3-oxohexansäuremethylester [Tabelle 26, Beispiel IB8aa1]

### Schritt 1: 4-Cyan-4-(3-fluorphenyl)-3-(4-fluorphenyl)butansäuremethylester

Zu 0,820 g (4,55 mmol) 3-(4-Fluorphenyl)acrylsäuremethylester in 15,0 ml Toluol gab man unter Schutzgas (Ar) 0,767 g (5,0 mmol) (3-Fluorphenyl)acetonitril und 0,1 ml Natriummethylat-Lösung (30 % in Methanol) und rührte 15 h bei 65°C in einem geschlossenen Gefäß. Man entfernte das Lösemittel im Vakuum, nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Nach Chromatographie des Rückstands an Kieselgel (Ethylacetat / Heptan = 20:80) erhielt man 0,950 g (57% d. Th.) eines erythro-threo-Diastereomerengemischs von 4-Cyan-4-(3-fluorphenyl)-3-(4-fluorphenyl)butansäuremethylester (erythro:threo = 54:46 gemäß Integration der Methyl-Singuletts im ¹H-NMR in CDCl₃ bei 3,66 ppm und 3,56 ppm).

### Schritt 2: 4-Cyan-3-(4-fluorphenyl)-4-(3-fluorphenyl)butansäure

Zu 1,75 g (5,55 mmol) 4-Cyan-3-(4-fluorphenyl)-4-(3-fluorphenyl)-butansäure-methylester (Beispiel A1) in 50,0 ml Methanol gab man unter Schutzgas (Ar) 10 ml 2 molare wässrige Natronlauge und rührte 8 h bei 25°C. Man entfernte das Methanol im Vakuum. Der Rückstand wurde mit konzentrierter Salzsäure angesäuert (pH = 3) und dreimal mit jeweils 15 ml Dichlormethan extrahiert. Die vereinigten organischen Phasen wurden über Natriumsulfat getrocknet und das Lösemittel im Vakuum entfernt. Man erhielt 1,62 g (96,9 % d. Th.) der Titelverbindung in Form von farblosen Kristallen (erythro:threo = 49:51 gemäß Vergleich der Multipletts im ¹H-NMR in CDCl₃ bei 2,60 und 2,85 ppm).

### Schritt 3: 2,6-Dicyan-6-(3-fluorphenyl)-5-(4-fluorphenyl)-3-oxohexansäuremethyl-ester

Zu 0,603 g (2,0 mmol) 4-Cyan-3-(4-fluorphenyl)-4-(3-fluorphenyl)butansäure gab man 1,79 g (20 mmol) Thionylchlorid und rührte 1,5 h bei 80°C. Man entfernte den Überschuß von Thionylchlorid im Vakuum (Rückstand = Säurechlorid). Zu 0,405 g (4,00 mmol) Triethylamin in 10 ml Dichlormethan gab man 0,297 g (3,00 mmol) Cyanessigsäuremethylester, 0,012 g (0,1 mmol) 4-Dimethylaminopyridin und das Säurechlorid gelöst in Dichlormethan (5 ml) und rührte 2 h bei 25 °C. Danach fügte man 50 mL Ethylacetat zu und extrahierte zweimal mit je 25 ml Wasser. Die vereinigten organischen Phasen wurden über Natriumsulfat getrocknet und das Lösemittel im Vakuum entfernt. Durch präparative Chromatographie [(90 ml/min Acetonitril/Wasser (Trifluoressigsäure 0,05%)] an einer Festphase [Nucleodur C18, HTec C18, 10 µm, (250 x 50)-mm Säule] erhielt man 0,019 g (8% d. Th.) eines erythro-threo-Diastereomerengemischs von 2,6-Dicyan-6-(3-fluorphenyl)-5-(4-fluorphenyl)-3-oxohexansäuremethylester (erythro:threo = 52:48 gemäß Integration der Dubletts im ¹H-NMR in CDCl₃ bei 4,09 ppm und 3,99 ppm).

### Beispiel A4: Diastereomere von 2-Acetyl-6-cyan-6-(3-fluorphenyl)-5-(4-fluorphenyl)-3-oxohexansäuremethylester [Tabelle 27, Beispiel IBXaa20]

### Schritt 1: 4-Cyan-4-(3-fluorphenyl)-3-(4-fluorphenyl)-butansäuremethylester

Zu 0,820 g (4,55 mmol) 3-(4-Fluorphenyl)acrylsäuremethylester in 15,0 ml Toluol gab man unter Schutzgas (Ar) 0,767 g (5,0 mmol) (3-Fluorphenyl)acetonitril und 0,1 ml Natriummethylat-Lösung (30 % in Methanol) und rührte 15 h bei 65°C in einem geschlossenen Gefäß. Man entfernte das Lösemittel im Vakuum, nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Nach Chromatographie des Rückstands an Kieselgel (Ethylacetat / Heptan = 20:80) erhielt man 0,950 g (57% d. Th.) eines erythro-threo-Diastereomerengemischs von 4-Cyan-4-(3-fluorphenyl)-3-(4-fluorphenyl)-butansäure-methylester (erythro:threo = 54:46 gemäß Integration der Methyl-Singuletts im ¹H-NMR in CDCl₃ bei 3,66 ppm und 3,56 ppm).

### Schritt 2: 4-Cyan-3-(4-fluorphenyl)-4-(3-fluorphenyl)butansäure

Zu 1,75 g (5,55 mmol) 4-Cyan-3-(4-fluorphenyl)-4-(3-fluorphenyl)-butansäure-methylester (Beispiel A1) in 50,0 ml Methanol gab man unter Schutzgas (Ar) 10 ml 2 molare wässrige Natronlauge und rührte 8 h bei 25°C. Man entfernte das Methanol im Vakuum. Der Rückstand wurde mit konzentrierter Salzsäure angesäuert (pH = 3) und dreimal mit jeweils 15 ml Dichlormethan extrahiert. Die vereinigten organischen Phasen wurden über Natriumsulfat getrocknet und das Lösemittel im Vakuum entfernt. Man erhielt 1,62 g (96,9 % d. Th.) der Titelverbindung in Form von farblosen Kristallen (erythro:threo = 49:51 gemäß Vergleich der Multipletts im ¹H-NMR in CDCl₃ bei 2,60 und 2,85 ppm).

### Schritt 3: 2-Acetyl-6-cyan-6-(3-fluorphenyl)-5-(4-fluorphenyl)-3-oxohexansäure-methylester

Zu 0,603 g (2,0 mmol) 4-Cyan-3-(4-fluorphenyl)-4-(3-fluorphenyl)butansäure gab man 2,379 g (20 mmol) Thionylchlorid und rührte 1,5 h bei 80°C. Man entfernte den Überschuß von Thionylchlorid im Vakuum und erhielt das Säurechlorid als Rückstand. Zu 0,190 g (2,00 mmol) Magnesiumchlorid und 0,464 g (4,00 mmol) 3-Oxobutansäuremethylester in Dichlormethan (5 ml) gab man 0,316 g (4,00 mmol) Pyridin bei 0°C, dann das Säurechlorid gelöst in Dichlormethan (5 mL) und rührte 1 h bei 25 °C. Man nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Durch präparative Chromatographie [(90 ml/min Acetonitril/Wasser (Trifluoressigsäure 0.05%)] an einer Festphase [Nucleodur C18, HTec C18, 10 µm, (250 x 50)-mm Säule] erhielt man 0,422 g (52% d. Th.) des erythro-threo-Diastereomeregemischs von 2-Acetyl-6-cyan-6-(3-fluorphenyl)-5-(4-fluorphenyl)-3-oxohexansäuremethylester (erythro:threo = 53:47 gemäß Integration der Singuletts im ¹H-NMR in CDCl₃ bei 17,74 ppm und 17,62 ppm).

### Beispiel A5: 2-(3,4-Difluorphenyl)-3-(3-fluorphenyl)-5-(5-methyl-1,2-oxazol-4-yl)-5-oxopentanonitril (siehe Tabelle 35, Verbindung Nr. IB10ga6)

### Schritt 1: 4-Cyan-4-(3,4-difluorphenyl)-3-(3-fluorphenyl)-butansäuremethylester

Zu 4,099 g (22,7 mmol) 3-(3-Fluorphenyl)acrylsäuremethylester in 25,0 ml Toluol gab man unter Schutzgas (Ar) 3,828 g (25,0 mmol) (3,4-Difluorphenyl)acetonitril und 0,281 mg Kalium tert Butylat und rührte 5 h bei 65°C in einem geschlossenen Gefäß. Man entfernte das Lösemittel im Vakuum, nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Nach Chromatographie des Rückstands an Kieselgel (Ethylacetat / Heptan = 20:80) erhielt man 7,3 g (87% d. Th.) der diastereömeren 4-Cyan-4-(3,4-difluorphenyl)-3-(3-fluorphenyl)-butansäuremethylester (erythro:threo = 65:35 gemäß Integration der Methyl-Singuletts im ¹H-NMR in CDCl₃ bei 3,68 ppm und 3,59 ppm).

### Schritt 2: 4-Cyan-4-(3,4-difluorphenyl)-3-(3-fluorphenyl)butansäure

Zu 4,7 g (14,1 mmol) 4-Cyan-4-(3,4-difluorphenyl)-3-(3-fluorphenyl)-butansäuremethylester in 95,0 ml Methanol gab man unter Schutzgas (Ar) 23,5 ml 2 molare wässrige Natronlauge und rührte über Nacht bei 25°C. Man entfernte das Methanol im Vakuum. Der Rückstand wurde mit konzentrierter Salzsäure angesäuert (pH = 3) und dreimal mit jeweils 100 ml Dichlormethan extrahiert. Die vereinigten organischen Phasen wurden über Natriumsulfat getrocknet und das Lösemittel im Vakuum entfernt. Man erhielt 4,50 g (99.9 % d. Th.) der Titelverbindung in Form von farblosen Kristallen (erythro:threo = 52:48, Vergleich der Dubletts im ¹H-NMR in CDCl₃ bei 4,34 und 4,04 ppm).

### Schritt 3: 2-(3,4-Difluorphenyl)-3-(3-fluorphenyl)-5-(5-methyl-1,2-oxazol-4-yl)-5-oxopentanonitril

In einem trockenen und mit Argon befüllten Schlenk-Rohr mit magnetischem Rührkern und Septum wurden Magnesiumspänen (291 mg, 11,9 mmol), Zink Chlorid (717 mg, 5,26 mmol) und Lithium Chlorid (254 mg, 5,98 mmol) in THF (5 ml) vorgelegt und durch Zugabe von DIBAL-H (0,2 ml, 1 M in THF) aktiviert. Nach 5 min Rühren wurde 4-Iod-5-methyl-1,2-oxazol (1,0 g, 4,78 mmol) in wasserfreiem THF (3 ml) bei 20 °C zugetropft. Danach wurde die Reaktionsmischung noch 30 min bei 25 °C gerührt. Die erhaltene Rohlösung wird dann mit dem untenen Säure Chlorid weiter umgesetzt.

Zu 1,83 g (5,75 mmol) 4-Cyan-4-(3,4-difluorphenyl)-3-(3-fluorphenyl)butansäure gab man 6,84 g (57,5 mmol) Thionylchlorid und rührte 1,5 h bei 80°C. Man entfernte den Überschuß von Thionylchlorid im Vakuum. Das so konzentrierte Reaktionsgemisch (enthält Säurechlorid) wurde in 3 ml Dichlormethan gelöst und bei -10°C tropfenweise (ca 30 min.) zu der obigen Rohlösung und dann bei 25 °C für 2 h weitergerührt. Man entfernte das Lösemittel im Vakuum, nahm den Rückstand in Ethylacetat auf und wusch zweimal mit je 25 ml Wasser. Die vereinigten organischen Phasen wurden über Natriumsulfat getrocknet und das Lösemittel im Vakuum entfernt. Nach Chromatographie des Rückstands an Kieselgel (Ethylacetat / Heptan = 20:80) erhielt man 810 mg (44% d. Th.) des Gemischs der diastereomeren 2-(3,4-Difluorphenyl)-3-(3-fluorphenyl)-5-(5-methyl-1,2-oxazol-4-yl)-5-oxopentanonitrile (erythro:threo = 63:37 gemäß Integration der Methyl-Singuletts im ¹H-NMR in CDCl₃ bei 8,54 ppm und 8,48 ppm).
NMR (Erythro): 8,54 (s, 1 H), 7,22 (m, 1 H), 7,09 (m, 1 H), 6,98 (m, 2H), 6,82 (m, 3H), 4,51 (d, 1 H), 3,71 (m, 1 H), 3,50 (dd, 1 H), 3,27 (dd, 1 H), 2,75 (s, 3H);
MS: 385 [M+1]+; HPLC (Rtz.): 1,57 min
Beispiel Nr. IB10ga6:
NMR (Threo): 8,48 (s, 1 H), 7,27 (m, 1 H), 7,12 (m, 1 H), 6,96 (m, 5H), 4,13 (d, 1 H), 3,82 (m, 1 H), 3,39 (dd, 1 H), 3,23 (dd, 1H), 2,65 (s, 3H);
MS: 385 [M+1]+; HPLC (Rtz.): 1,57 min

Die in den nachfolgenden Tabellen beschriebenen Verbindungen erhält man gemäß oder analog zu den oben beschriebenen Beispielen, gegebenenfalls unter ergänzender Anwendung von weiter oben beschriebenen allgemeinen Methoden.

In den nachfolgenden Tabellen bedeuten:
- H: = Wasserstoff(-atom)
- Me: = Methyl oder CH₃
- Et: = Ethyl
- n-Pr: = n-Propyl
- i-Pr: = Isopropyl
- n-Bu: = n-Butyl
- i-Bu: = Isobutyl
- Rtz: = Retentionszeit
- F, Cl, Br, I: = Fluor, Chlor, Brom bzw. Jod entsprechend den üblichen chemischen Atomsymbolen
- MeO oder OMe: = Methoxy
- CN: = Cyano
- NO₂: = Nitro
- Ph: = Phenyl
Die Position eines Substituenten am Phenylring, beispielsweise in Position 2, ist dem Symbol oder der Abkürzung des Restes vorangestellt, z.B.
- 2-Cl: = 2-Chlor
- 2-Me: = 2-Methyl
Nummerierungen der Substituentenpositionen bei di- oder trisubstituiertem Substitutionsmuster sind analog vorangestellt, z. B.
- 3,5-Me₂: = 3,5-Dimethyl (z. B. als Substitution am Phenylring)
- 2,3-Cl₂: = 2,3-Dichlor (z. B. als Substitution am Phenylring)
- 3,4-F₂: = 3,4-Difluor (z. B. als Substitution am Phenylring)
Andere Abkürzungen sind analog der oben angegebenen Beispiele zu verstehen.
- "(R¹)ₘ: = "H" = unsubstituierter Cyclus (m = 0)
- "(R²)ₙ: = "H" = unsubstituierter Cyclus (n = 0)

Im Übrigen gelten die üblichen chemischen Symbole und Formeln, wie z. B. CH₂ für Methylen oder CF₃ für Trifluormethyl oder OH für Hydroxyl. Zusammengesetzte Bedeutungen sind entsprechend aus den genannten Abkürzungen zusammengesetzt definiert.

**Tabelle 1: Definitionen von Strukturkombinationen der Gruppen Q, (R¹)ₘ und (R²)ₙ für die nachfolgenden Tabellen von erfindungsgemäßen Verbindungen der allgemeinen Formel (I)**

| Nr. | Q | (R¹)ₘ | (R**²**)**ₙ** |
|---|---|---|---|
| 1 | OH | 3-F | 4-Cl |
| 2 | OH | 3-F | 3-F |
| 3 | OH | 3,4-F₂ | 4-Cl |
| 4 | OH | 3-F | 4-Br |
| 5 | OH | 3-Cl | 3-F |
| 6 | OH | 3,4-F₂ | 3-F |
| 7 | OH | H | 3-Br |
| 8 | OH | 4-F | 3-F |
| 9 | OH | 3-F | 3-Cl |
| 10 | OH | 3,4-F₂ | 3-Cl |
| 11 | OH | 3-Br | 3-F |
| 12 | OH | 2,5-F₂ | 3-F |
| 13 | OH | 3-F | 2-F |
| 14 | OH | 3,4,5-F₃ | 4-Cl |
| 15 | OH | 4-F | 3-Cl |
| 16 | OH | 3,4-F₂ | 3-F, 4-Cl |
| 17 | OH | 3,4-F₂ | 2-F |
| 18 | OH | 3-F | 2,3-F₂ |
| 19 | OH | 4-F | 3,5-F₂ |
| 20 | OH | 3-F | 4-F |
| 21 | OH | 3,4-F₂ | 3-Cl, 5-F |
| 22 | OH | 3-F | 3-Cl, 5-F |
| 23 | OH | 3-F | 2,5-F₂ |
| 24 | OH | 3,4-F₂ | 2,3-F₂ |
| 25 | OH | 3,4-F₂ | 2,5-F₂ |
| 26 | OH | 3-Cl | 3-Cl, 5-F |
| 27 | OH | 3-F, 4-Cl | 3-Cl |
| 28 | OH | 3,4-F₂ | 3-NO₂ |
| 29 | OH | 3,4-F₂ | H |
| 30 | OH | 3-Cl, 4-F | 3-F |
| 31 | OH | 3,4-F₂ | 4-OMe |
| 32 | O-C(O)-t-Bu | 3-F | 4-Cl |
| 33 | O-C(O)Me | 3-F | 4-Cl |
| 34 | SPh | 3-F | 4-Cl |
| 35 | SMe | 3-F | 4-Cl |
| 36 | OSO₂(4-Me-Ph) | 3-F | 4-Cl |
| 37 | Cl | 3-F | 4-Cl |
| 38 | O-CO₂Me | 3-F | 4-Cl |
| 39 | O-C(O)-t-Bu | 3-F | 3-F |
| 40 | O-C(O)Me | 3-F | 3-F |
| 41 | SPh | 3-F | 3-F |
| 42 | SMe | 3-F | 3-F |
| 43 | OSO₂(4-Me-Ph) | 3-F | 3-F |
| 44 | Cl | 3-F | 3-F |
| 45 | O-CO₂Me | 3-F | 3-F |
| 46 | O-C(O)-t-Bu | 3,4-F₂ | 3-F |
| 47 | O-C(O)Me | 3,4-F₂ | 3-F |
| 48 | O-C(O)Et | 3,4-F₂ | 3-F |
| 49 | SMe | 3,4-F₂ | 3-F |
| 50 | OSO₂(4-Me-Ph) | 3,4-F₂ | 3-F |
| 51 | Cl | 3,4-F₂ | 3-F |
| 52 | CO₂Me | 3,4-F₂ | 3-F |
| 53 | O-C(O)-t-Bu | 3,4-F₂ | 3-Cl |
| 54 | O-C(O)Me | 3,4-F₂ | 3-Cl |
| 55 | SPh | 3,4-F₂ | 3-Cl |
| 56 | SMe | 3,4-F₂ | 3-Cl |
| 57 | OSO₂(4-Me-Ph) | 3,4-F₂ | 3-Cl |
| 58 | Cl | 3,4-F₂ | 3-Cl |
| 59 | O-CO₂Me | 3,4-F₂ | 3-Cl |
| 60 | O-C(O)-t-Bu | 3-Br | 3-F |
| 61 | O-C(O)Me | 2,5-F₂ | 3-F |
| 62 | SPh | 3-F | 2-F |
| 63 | O-C(O)-t-Bu | 3,4,5-F₃ | 4-Cl |
| 64 | O-C(O)Me | 4-F | 3-Cl |
| 65 | SPh | 3,4-F₂ | 3-F, 4-Cl |
| 66 | O-C(O)-t-Bu | 3,4-F₂ | 2-F |
| 67 | O-C(O)Me | 3-F | 2,3-F₂ |
| 68 | SPh | 4-F | 3,5-F₂ |
| 69 | O-C(O)-t-Bu | 3-F | 4-F |
| 70 | O-C(O)Me | 3,4-F₂ | 3-Cl, 5-F |
| 71 | SPh | 3-F | 3-Cl, 5-F |
| 72 | O-C(O)-t-Bu | 3-F | 2,5-F₂ |
| 73 | O-C(O)Me | 3,4-F₂ | 2,3-F₂ |
| 74 | SPh | 3,4-F₂ | 2,5-F₂ |
| 75 | O-C(O)-t-Bu | 3-CI | 3-Cl, 5-F |
| 76 | O-C(O)Me | 3-F, 4-Cl | 3-Cl |
| 77 | SPh | 3,4-F₂ | 3-NO₂ |
| 78 | O-C(O)-t-Bu | 3,4-F₂ | H |
| 79 | O-C(O)Me | 3-Cl, 4-F | 3-F |
| 80 | SPh | 3,4-F₂ | 4-OMe |
| 81 | OH | 3-F | H |
| 82 | OH | 3-F | 2,4-F₂ |
| 83 | OH | 3-F | 2,6-F₂ |
| 84 | OH | 3-F | 3,4-F₂ |
| 85 | OH | 3-F | 3,5-F₂ |
| 86 | OH | 3-F | 2-CI |
| 87 | OH | 3-F | 2,3-Cl₂ |
| 88 | OH | 3-F | 2,4-Cl₂ |
| 89 | O-C(O)-t-Bu | 3-F | 2,5-Cl₂ |
| 90 | SPh | 3-F | 2,6-Cl₂ |
| 91 | OH | 3-F | 3,4-Cl₂ |
| 92 | OH | 3-F | 3,5-Cl₂ |
| 93 | O-C(O)-t-Bu | 3,4-F₂ | 4-F |
| 94 | OH | 3,4-F₂ | 2,4-F₂ |
| 95 | OH | 3,4-F₂ | 2,6-F₂ |
| 96 | OH | 3,4-F₂ | 3,4-F₂ |
| 97 | OH | 3,4-F₂ | 3,5-F₂ |
| 98 | SPh | 3,4-F₂ | 2-Cl |
| 99 | O-C(O)-t-Bu | 3,4-F₂ | 2,3-Cl₂ |
| 100 | OH | 3,4-F₂ | 2,4-Cl₂ |
| 101 | O-C(O)-t-Bu | 3,4-F₂ | 2,5-Cl₂ |
| 102 | OH | 3,4-F₂ | 2,6-Cl₂ |
| 103 | SPh | 3,4-F₂ | 3,4-Cl₂ |
| 104 | OH | 3,4-F₂ | 3-Br |
| 105 | OH | 3-Cl | 3-NO₂ |
| 106 | OH | 3-Cl | 4-NO₂ |
| 107 | OH | 3-Cl | 4-CN |
| 108 | OH | 3-Cl | 2-Br |
| 109 | O-C(O)-t-Bu | 3-Cl | 2,4-F₂ |
| 110 | OH | 3-Br | 2,5-F₂ |
| 111 | OH | 3-Cl | 2,6-F₂ |
| 112 | O-C(O)-t-Bu | 3-Cl | 3,4-F₂ |
| 113 | OH | 3-Br | 3,5-F₂ |
| 114 | OH | 3-Cl | 2-CI |
| 115 | OH | 3-Br | 3-CI |
| 116 | SPh | 3-Cl | 4-Cl |
| 117 | OH | 3-Br | 2,3-Cl₂ |
| 118 | OH | 3-Br | 2,4-Cl₂ |
| 119 | OH | 3-Cl | 2,5-Cl₂ |
| 120 | SPh | 3-Cl | 2,6-Cl₂ |
| 121 | SPh | 3-Cl | 3,4-Cl₂ |
| 122 | OH | 3-Cl | 3,5-Cl₂ |
| 123 | O-C(O)Me | 3,4-Cl₂ | H |
| 124 | O-C(O)Me | 3,4-Cl₂ | 2-F |
| 125 | O-C(O)Me | 3,4-Cl₂ | 3-F |
| 126 | O-C(O)Me | 3,4-Cl₂ | 4-F |
| 127 | O-C(O)Me | 3,4-Cl₂ | 2,3-F₂ |
| 128 | O-C(O)Me | 3,4-Cl₂ | 2,4-F₂ |
| 129 | OH | 3,4-Cl₂ | 2,5-F₂ |
| 130 | O-C(O)Me | 3,4-Cl₂ | 2,6-F₂ |
| 131 | O-C(O)-t-Bu | 3,4-Cl₂ | 3,4-F₂ |
| 132 | O-C(O)Et | 3,4-Cl₂ | 3,5-F₂ |
| 133 | O-C(O)Et | 3,4-Cl₂ | 2-CI |
| 134 | O-C(O)Et | 3,4-Cl₂ | 3-Cl |
| 135 | O-C(O)-t-Bu | 3,4-Cl₂ | 4-Cl |
| 136 | SPh | 3,4-Cl₂ | 2,3-Cl₂ |
| 137 | O-C(O)-t-Bu | 3,4-Cl₂ | 2,4-Cl₂ |
| 138 | O-C(O)Me | 3,4-Cl₂ | 2,5-Cl₂ |
| 139 | O-C(O)Et | 3,4-Cl₂ | 2,6-Cl₂ |
| 140 | O-C(O)Me | 3,4-Cl₂ | 3,4-Cl₂ |
| 141 | O-C(O)Me | 3,4-Cl₂ | 3,5-Cl₂ |
| 142 | O-C(O)Et | 3-Cl, 4-F | H |
| 143 | SMe | 3-Cl, 4-F | 2-F |
| 144 | O-C(O)Me | 3-Cl, 4-F | 4-F |
| 145 | O-C(O)Et | 3-Cl, 4-F | 2,3-F₂ |
| 146 | O-C(O)-t-Bu | 3-Cl, 4-F | 2,4-F₂ |
| 147 | O-C(O)Me | 3-Cl, 4-F | 2,5-F₂ |
| 148 | SPh | 3-Cl, 4-F | 2,6-F₂ |
| 149 | O-C(O)Et | 3-Cl, 4-F | 3,4-F₂ |
| 150 | OSO₂(4-Me-Ph) | 3-Cl, 4-F | 3,5-F₂ |
| 151 | O-C(O)Me | 3-Cl, 4-F | 2-Cl |
| 152 | OH | 3-Cl, 4-F | 3-Br |
| 153 | O-C(O)Me | 3-Cl, 4-F | 4-Cl |
| 154 | O-C(O)Me | 3-Cl, 4-F | 2,3-Cl₂ |
| 155 | O-C(O)Et | 3-Cl, 4-F | 2,4-Cl₂ |
| 156 | O-C(O)Me | 3-Cl, 4-F | 2,5-Cl₂ |
| 157 | O-C(O)Me | 3-Cl, 4-F | 2,6-Cl₂ |
| 158 | O-C(O)Et | 3-Cl, 4-F | 3,4-Cl₂ |
| 159 | O-C(O)Me | 3-Cl, 4-F | 3,5-Cl₂ |
| 160 | O-C(O)Me | 3-Cl, 4-F | H |
| 161 | O-C(O)Et | 3-F, 4-Cl | 2-F |
| 162 | O-C(O)Me | 3-F, 4-Cl | 3-F |
| 163 | O-C(O)Me | 3-F, 4-Cl | 4-F |
| 164 | OH | 3-F, 4-Cl | 2,3-F₂ |
| 165 | O-C(O)Et | 3-F, 4-Cl | 2,4-F₂ |
| 166 | SPh | 3-F, 4-Cl | 2,5-F₂ |
| 167 | O-C(O)Me | 3-F, 4-Cl | 2,6-F₂ |
| 168 | O-C(O)Me | 3-F, 4-Cl | 3,4-F₂ |
| 169 | OH | 3-F, 4-Cl | 3,5-F₂ |
| 170 | O-C(O)Me | 3-F, 4-Cl | 2-Cl |
| 171 | O-C(O)Et | 3-F, 4-Cl | 4-Cl |
| 172 | SPh | 3-F, 4-Cl | 2,3-Cl₂ |
| 173 | SPh | 3-F, 4-Cl | 2,4-Cl₂ |
| 174 | SPh | 3-F, 4-Cl | 2,5-Cl₂ |
| 175 | SPh | 3-F, 4-Cl | 2,6-Cl₂ |
| 176 | SPh | 3-F, 4-CI | 3,4-Cl₂ |
| 177 | SPh | 3-F, 4-Cl | 3,5-Cl₂ |
| 178 | O-C(O)-i-Pr | 3-F | 2,6-F₂ |
| 179 | O-C(O)Me | 3-F | 2,6-F₂ |
| 180 | SEt | 3-F | 2,6-F₂ |
| 181 | OH | 3,4-F₂ | 3,4-Cl₂ |
| 182 | OH | 3,4-F₂ | 3,5-Cl₂ |
| 183 | OH | 3-Cl | H |
| 184 | OH | 3-CI | 2-F |
| 185 | OH | 3-CI | 4-F |
| 186 | OH | 3-Cl | 2,3-F₂ |
| 187 | OH | 3-Cl | 2,4-F₂ |
| 188 | OH | 3-Cl | 2,5-F₂ |
| 189 | O-C(O)-t-Bu | 3-CI | 2,6-F₂ |
| 190 | OH | 3-CI | 3,4-F₂ |
| 191 | OH | 3-Cl | 3,5-F₂ |
| 192 | SPh | 3-Cl | 2-Cl |
| 193 | OH | 3-Cl | 3-Cl |
| 194 | OH | 3-Cl | 4-Cl |
| 195 | OH | 3-Cl | 2,3-Cl₂ |
| 196 | OH | 3-Cl | 2,4-Cl₂ |
| 197 | O-C(O)-t-Bu | 3-Cl | 2,5-Cl₂ |
| 198 | OH | 3-Cl | 2,6-Cl₂ |
| 199 | O-C(O)-t-Bu | 3-Cl | 3,4-Cl₂ |
| 200 | O-C(O)Me | 3-Cl | 3,5-Cl₂ |
| 201 | OH | 3,4-Cl₂ | H |
| 202 | OH | 3,4-Cl₂ | 2-F |
| 203 | OH | 3,4-Cl₂ | 3-F |
| 204 | OH | 3,4-Cl₂ | 4-F |
| 205 | O-C(O)-t-Bu | 3,4-Cl₂ | 2,3-F₂ |
| 206 | O-C(O)Me | 3,4-Cl₂ | 2,4-F₂ |
| 207 | SPh | 3,4-Cl₂ | 2,5-F₂ |
| 208 | OH | 3,4-Cl₂ | 2,6-F₂ |
| 209 | OSO₂(4-Me-Ph) | 3,4-Cl₂ | 3,4-F₂ |
| 210 | OH | 3,4-Cl₂ | 3,5-F₂ |
| 211 | OH | 3,4-Cl₂ | 2-Cl |
| 212 | OH | 3,4-Cl₂ | 3-Cl |
| 213 | OH | 3,4-Cl₂ | 4-Cl |
| 214 | O-C(O)-t-Bu | 3,4-Cl₂ | 2,3-Cl₂ |
| 215 | OH | 3,4-Cl₂ | 2,4-Cl₂ |
| 216 | OH | 3,4-Cl₂ | 2,5-Cl₂ |
| 217 | OH | 3,4-Cl₂ | 2,6-Cl₂ |
| 218 | OH | 3,4-Cl₂ | 3,4-Cl₂ |
| 219 | OH | 3,4-Cl₂ | 3,5-Cl₂ |
| 220 | OH | 3-Cl, 4-F | H |
| 221 | OH | 3-Cl, 4-F | 2-F |
| 222 | OH | 3-Cl, 4-F | 4-F |
| 223 | OH | 3-Cl, 4-F | 2,3-F₂ |
| 224 | OH | 3-Cl, 4-F | 2,4-F₂ |
| 225 | OH | 3-Cl, 4-F | 2,5-F₂ |
| 226 | OH | 3-Cl, 4-F | 2,6-F₂ |
| 227 | OH | 3-Cl, 4-F | 3,4-F₂ |
| 228 | OH | 3-Cl, 4-F | 3,5-F₂ |
| 229 | OH | 3-Cl, 4-F | 2-Cl |
| 230 | OH | 3-Cl, 4-F | 3-Cl |
| 231 | OH | 3-Cl, 4-F | 4-Cl |
| 232 | OH | 3-Cl, 4-F | 2,3-Cl₂ |
| 233 | OH | 3-Cl, 4-F | 2,4-Cl₂ |
| 234 | OH | 3-Cl, 4-F | 2,5-Cl₂ |
| 235 | OH | 3-Cl, 4-F | 2,6-Cl₂ |
| 236 | OH | 3-Cl, 4-F | 3,4-Cl₂ |
| 237 | OH | 3-Cl, 4-F | 3,5-Cl₂ |
| 238 | OH | 3-Cl, 4-F | H |
| 239 | OH | 3-F, 4-Cl | 2-F |
| 240 | OH | 3-F, 4-Cl | 3-F |
| 241 | OH | 3-F, 4-Cl | 4-F |
| 242 | SPh | 3-F, 4-Cl | 2,3-F₂ |
| 243 | OH | 3-F, 4-Cl | 2,4-F₂ |
| 244 | OH | 3-F, 4-Cl | 2,5-F₂ |
| 245 | OH | 3-F, 4-Cl | 2,6-F₂ |
| 246 | OH | 3-F, 4-Cl | 3,4-F₂ |
| 247 | OH | 3-F, 4-Cl | 3,5-F₂ |
| 248 | OH | 3-F, 4-Cl | 2-Cl |
| 249 | OH | 3-F, 4-Cl | 4-Cl |
| 250 | SPh | 3-F, 4-Cl | 2,3-Cl₂ |
| 251 | OH | 3-F, 4-Cl | 2,4-Cl₂ |
| 252 | OH | 3-F, 4-Cl | 2,5-Cl₂ |
| 253 | OH | 3-F, 4-Cl | 2,6-Cl₂ |
| 254 | OH | 3-F, 4-Cl | 3,4-Cl₂ |
| 255 | OH | 3-F, 4-Cl | 3,5-Cl₂ |
| 256 | SPh | 3-F | 2,6-F₂ |
| 257 | O-C(O)-t-Bu | 3-F | 2,6-F₂ |
| 258 | SMe | 3-F | 2,6-F₂ |
| 259 | O-C(O)Et | 3,4-F₂ | 2,6-F₂ |
| 260 | O-C(O)-t-Bu | 3,4-F₂ | 2,6-F₂ |
| 261 | O-C(O)Me | 3,4-F₂ | 2,6-F₂ |
| 262 | SPh | 3,4-F₂ | 2,6-F₂ |
| 263 | SMe | 3,4-F₂ | 2,6-F₂ |
| 264 | OSO₂(4-Me-Ph) | 3,4-F₂ | 2,6-F₂ |
| 265 | Cl | 3,4-F₂ | 2,6-F₂ |
| 266 | O-CO₂Me | 3,4-F₂ | 2,6-F₂ |
| 267 | OH | 3-CN | H |
| 268 | OH | 3-CN | 2,5-F₂ |
| 269 | OH | 3-CN | 2,6-F₂ |
| 270 | OH | 3-CN | 2,3-F₂ |
| 271 | OH | 3-CN | 2,4-F₂ |
| 272 | OH | 3-CN | 3,4-F₂ |
| 273 | OH | 3-CN | 3,5-F₂ |
| 274 | OH | 3-CN | 4-Cl |
| 275 | OH | 3-CN | 3-Cl |
| 276 | OH | 3-CN | 2-Cl |
| 277 | OH | 3-CN | 4-F |
| 278 | OH | 3-CN | 3-F |
| 279 | OH | 3-CN | 2-F |
| 280 | OH | 2-CN | 3-F |
| 281 | OH | 2-CN | 4-F |
| 282 | OH | 2-CN | 4-Cl |
| 283 | OH | 3-NO₂ | H |
| 284 | OH | 3-NO₂ | 2,5-F₂ |
| 285 | OH | 3-NO₂ | 2,6-F₂ |
| 286 | OH | 3-NO₂ | 2,3-F₂ |
| 287 | OH | 3-NO₂ | 2,4-F₂ |
| 288 | OH | 3-NO₂ | 3,4-F₂ |
| 289 | OH | 3-NO₂ | 3,5-F₂ |
| 290 | OH | 3-NO₂ | 4-CI |
| 291 | OH | 3-NO₂ | 3-CI |
| 292 | OH | 3-NO₂ | 2-CI |
| 293 | OH | 3-NO₂ | 4-F |
| 294 | OH | 3-NO₂ | 3-F |
| 295 | OH | 3-NO₂ | 2-F |
| 296 | OH | H | 2,6-F₂ |
| 297 | OH | H | 3,4-F₂ |
| 298 | OH | H | 3,5-F₂ |
| 299 | OH | H | 2,3-F₂ |
| 300 | OH | H | 2,4-F₂ |
| 301 | OH | H | 2,5-F₂ |
| 302 | OH | H | 2-Cl |
| 303 | OH | H | 3-Cl |
| 304 | OH | H | 4-Cl |
| 305 | OH | H | 2-F |
| 306 | OH | H | 3-F |
| 307 | OH | H | 4-F |
| 308 | OH | H | 2,3-Cl₂ |
| 309 | OH | H | 2,4-Cl₂ |
| 310 | OH | H | 2,5-Cl₂ |
| 311 | OH | H | 2,6-Cl₂ |
| 312 | OH | H | 3,4-Cl₂ |
| 313 | OH | H | 3,5-Cl₂ |
| 314 | O-C(O)Et | 3-CN | 2,5-F₂ |
| 315 | O-C(O)-t-Bu | 3-CN | 2,5-F₂ |
| 316 | O-C(O)Me | 3-CN | 2,5-F₂ |
| 317 | SPh | 3-CN | 2,5-F₂ |
| 318 | SMe | 3-CN | 2,5-F₂ |
| 319 | OSO₂(4-Me-Ph) | 3-CN | 2,5-F₂ |
| 320 | Cl | 3-CN | 2,5-F₂ |
| 321 | O-CO₂Me | 3-CN | 2,5-F₂ |
| 322 | O-C(O)Et | 3-CN | 2,6-F₂ |
| 323 | O-C(O)-t-Bu | 3-CN | 2,6-F₂ |
| 324 | O-C(O)Me | 3-CN | 2,6-F₂ |
| 325 | SPh | 3-CN | 2,6-F₂ |
| 326 | SMe | 3-CN | 2,6-F₂ |
| 327 | OSO₂(4-Me-Ph) | 3-CN | 2,6-F₂ |
| 328 | Cl | 3-CN | 2,6-F₂ |
| 329 | O-CO₂Me | 3-CN | 2,6-F₂ |
| 330 | O-C(O)Et | 3-CN | 2,3-F₂ |
| 331 | O-C(O)-t-Bu | 3-CN | 2,3-F₂ |
| 332 | O-C(O)Me | 3-CN | 2,3-F₂ |
| 333 | SPh | 3-CN | 2,3-F₂ |
| 334 | SMe | 3-CN | 2,3-F₂ |
| 335 | OSO₂(4-Me-Ph) | 3-CN | 2,3-F₂ |
| 336 | Cl | 3-CN | 2,3-F₂ |
| 337 | O-CO₂Me | 3-CN | 2,3-F₂ |
| 338 | O-C(O)Et | 3-CN | 2,4-F₂ |
| 339 | O-C(O)-t-Bu | 3-CN | 2,4-F₂ |
| 340 | O-C(O)Me | 3-CN | 2,4-F₂ |
| 341 | SPh | 3-CN | 2,4-F₂ |
| 342 | SMe | 3-CN | 2,4-F₂ |
| 343 | OSO₂(4-Me-Ph) | 3-CN | 2,4-F₂ |
| 344 | Cl | 3-CN | 2,4-F₂ |
| 345 | O-CO₂Me | 3-CN | 2,4-F₂ |
| 346 | O-C(O)Et | 3-CN | 2,4-F₂ |
| 347 | O-C(O)-t-Bu | 3-CN | 2,4-F₂ |
| 348 | O-C(O)Me | 3-CN | 2,4-F₂ |
| 349 | SPh | 3-CN | 2,4-F₂ |
| 350 | SMe | 3-CN | 2,4-F₂ |
| 351 | OSO₂(4-Me-Ph) | 3-CN | 2,4-F₂ |
| 352 | Cl | 3-CN | 2,4-F₂ |
| 353 | O-CO₂Me | 3-CN | 2,4-F₂ |
| 354 | O-C(O)Et | 3-CN | 4-Cl |
| 355 | O-C(O)-t-Bu | 3-CN | 4-Cl |
| 356 | O-C(O)Me | 3-CN | 4-Cl |
| 357 | SPh | 3-CN | 4-Cl |
| 358 | SMe | 3-CN | 4-Cl |
| 359 | OSO₂(4-Me-Ph) | 3-CN | 4-Cl |
| 360 | Cl | 3-CN | 4-Cl |
| 361 | O-CO₂Me | 3-CN | 4-Cl |
| 362 | O-C(O)Et | 3-CN | 3-Cl |
| 363 | O-C(O)-t-Bu | 3-CN | 3-Cl |
| 364 | O-C(O)Me | 3-CN | 3-Cl |
| 365 | SPh | 3-CN | 3-Cl |
| 366 | SMe | 3-CN | 3-Cl |
| 367 | OSO₂(4-Me-Ph) | 3-CN | 3-Cl |
| 368 | Cl | 3-CN | 3-Cl |
| 369 | O-CO₂Me | 3-CN | 3-Cl |
| 370 | O-C(O)Et | 3-CN | 4-F |
| 371 | O-C(O)-t-Bu | 3-CN | 4-F |
| 372 | O-C(O)Me | 3-CN | 4-F |
| 373 | SPh | 3-CN | 4-F |
| 374 | SMe | 3-CN | 4-F |
| 375 | OSO₂(4-Me-Ph) | 3-CN | 4-F |
| 376 | Cl | 3-CN | 4-F |
| 377 | O-CO₂Me | 3-CN | 4-F |
| 378 | O-C(O)Et | 3-CN | 3-F |
| 379 | O-C(O)-t-Bu | 3-CN | 3-F |
| 380 | O-C(O)Me | 3-CN | 3-F |
| 381 | SPh | 3-CN | 3-F |
| 382 | SMe | 3-CN | 3-F |
| 383 | OSO₂(4-Me-Ph) | 3-CN | 3-F |
| 384 | Cl | 3-CN | 3-F |
| 385 | O-CO₂Me | 3-CN | 3-F |
| 386 | O-C(O)Et | 3-NO₂ | 2,5-F₂ |
| 387 | O-C(O)-t-Bu | 3-NO₂ | 2,5-F₂ |
| 388 | O-C(O)Me | 3-NO₂ | 2,5-F₂ |
| 389 | SPh | 3-NO₂ | 2,5-F₂ |
| 390 | SMe | 3-NO₂ | 2,5-F₂ |
| 391 | OSO₂(4-Me-Ph) | 3-NO₂ | 2,5-F₂ |
| 392 | Cl | 3-NO₂ | 2,5-F₂ |
| 393 | O-CO₂Me | 3-NO₂ | 2,5-F₂ |
| 394 | O-C(O)Et | 3-NO₂ | 2,6-F₂ |
| 395 | O-C(O)-t-Bu | 3-NO₂ | 2,6-F₂ |
| 396 | O-C(O)Me | 3-NO₂ | 2,6-F₂ |
| 397 | SPh | 3-NO₂ | 2,6-F₂ |
| 398 | SMe | 3-NO₂ | 2,6-F₂ |
| 399 | OSO₂(4-Me-Ph) | 3-NO₂ | 2,6-F₂ |
| 400 | Cl | 3-NO₂ | 2,6-F₂ |
| 401 | O-CO₂Me | 3-NO₂ | 2,6-F₂ |
| 402 | O-C(O)Et | 3-NO₂ | 2,3-F₂ |
| 403 | O-C(O)-t-Bu | 3-NO₂ | 2,3-F₂ |
| 404 | O-C(O)Me | 3-NO₂ | 2,3-F₂ |
| 405 | SPh | 3-NO₂ | 2,3-F₂ |
| 406 | SMe | 3-NO₂ | 2,3-F₂ |
| 407 | OSO₂(4-Me-Ph) | 3-NO₂ | 2,3-F₂ |
| 408 | Cl | 3-NO₂ | 2,3-F₂ |
| 409 | O-CO₂Me | 3-NO₂ | 2,3-F₂ |
| 410 | O-C(O)Et | 3-NO₂ | 2,4-F₂ |
| 411 | O-C(O)-t-Bu | 3-NO₂ | 2,4-F₂ |
| 412 | O-C(O)Me | 3-NO₂ | 2,4-F₂ |
| 413 | SPh | 3-NO₂ | 2,4-F₂ |
| 414 | SMe | 3-NO₂ | 2,4-F₂ |
| 415 | OSO₂(4-Me-Ph) | 3-NO₂ | 2,4-F₂ |
| 416 | Cl | 3-NO₂ | 2,4-F₂ |
| 417 | O-CO₂Me | 3-NO₂ | 2,4-F₂ |
| 418 | O-C(O)Et | 3-NO₂ | 2,4-F₂ |
| 419 | O-C(O)-t-Bu | 3-NO₂ | 2,4-F₂ |
| 420 | O-C(O)Me | 3-NO₂ | 2,4-F₂ |
| 421 | SPh | 3-NO₂ | 2,4-F₂ |
| 422 | SMe | 3-NO₂ | 2,4-F₂ |
| 423 | OSO₂(4-Me-Ph) | 3-NO₂ | 2,4-F₂ |
| 424 | Cl | 3-NO₂ | 2,4-F₂ |
| 425 | O-CO₂Me | 3-NO₂ | 2,4-F₂ |
| 426 | O-C(O)Et | 3-NO₂ | 4-Cl |
| 427 | O-C(O)-t-Bu | 3-NO₂ | 4-Cl |
| 428 | O-C(O)Me | 3-NO₂ | 4-Cl |
| 429 | SPh | 3-NO₂ | 4-Cl |
| 430 | SMe | 3-NO₂ | 4-Cl |
| 431 | OSO₂(4-Me-Ph) | 3-NO₂ | 4-Cl |
| 432 | Cl | 3-NO₂ | 4-Cl |
| 433 | O-CO₂Me | 3-NO₂ | 4-Cl |
| 434 | O-C(O)Et | 3-NO₂ | 3-Cl |
| 435 | O-C(O)-t-Bu | 3-NO₂ | 3-Cl |
| 436 | O-C(O)Me | 3-NO₂ | 3-Cl |
| 437 | SPh | 3-NO₂ | 3-Cl |
| 438 | SMe | 3-NO₂ | 3-Cl |
| 439 | OSO₂(4-Me-Ph) | 3-NO₂ | 3-Cl |
| 440 | Cl | 3-NO₂ | 3-Cl |
| 441 | O-CO₂Me | 3-NO₂ | 3-Cl |
| 442 | O-C(O)Et | 3-NO₂ | 4-F |
| 443 | O-C(O)-t-Bu | 3-NO₂ | 4-F |
| 444 | O-C(O)Me | 3-NO₂ | 4-F |
| 445 | SPh | 3-NO₂ | 4-F |
| 446 | SMe | 3-NO₂ | 4-F |
| 447 | OSO₂(4-Me-Ph) | 3-NO₂ | 4-F |
| 448 | Cl | 3-NO₂ | 4-F |
| 449 | O-CO₂Me | 3-NO₂ | 4-F |
| 450 | O-C(O)Et | 3-NO₂ | 3-F |
| 451 | O-C(O)-t-Bu | 3-NO₂ | 3-F |
| 452 | O-C(O)Me | 3-NO₂ | 3-F |
| 453 | SPh | 3-NO₂ | 3-F |
| 454 | SMe | 3-NO₂ | 3-F |
| 455 | OSO₂(4-Me-Ph) | 3-NO₂ | 3-F |
| 456 | Cl | 3-NO₂ | 3-F |
| 457 | O-CO₂Me | 3-NO₂ | 3-F |
| 458 | Me | 3-CN, 4-F | 3-F |
| 459 | Me | 3-CN, 4-F | 3-Cl |
| 460 | Me | 3-CN, 4-F | 3-CN |
| 461 | Me | 3-CN, 4-F | 4-F |
| 462 | Me | 3-CN, 4-F | 4-Cl |
| 463 | Me | 3-CN, 4-F | 2,5-F₂ |
| 464 | Me | 3-CN, 4-F | 2,6-F₂ |
| 465 | H | 3-CN, 4-F | 3-F |
| 466 | H | 3-CN, 4-F | 3-CI |
| 467 | H | 3-CN, 4-F | 3-CN |
| 468 | H | 3-CN, 4-F | 4-F |
| 469 | H | 3-CN, 4-F | 4-Cl |
| 470 | H | 3-CN, 4-F | 2,5-F₂ |
| 471 | H | 3-CN, 4-F | 2,6-F₂ |
| 472 | Me | 3-Br, 4-F | 3-F |
| 473 | Me | 3-Br, 4-F | 3-Cl |
| 474 | Me | 3-Br, 4-F | 3-CN |
| 475 | Me | 3-Br, 4-F | 4-F |
| 476 | Me | 3-Br, 4-F | 4-Cl |
| 477 | Me | 3-Br, 4-F | 2,5-F₂ |
| 478 | Me | 3-Br, 4-F | 2,6-F₂ |
| 479 | H | 3-Br, 4-F | 3-F |
| 480 | H | 3-Br, 4-F | 3-Cl |
| 481 | H | 3-Br, 4-F | 3-CN |
| 482 | H | 3-Br, 4-F | 4-F |
| 483 | H | 3-Br, 4-F | 4-Cl |
| 484 | H | 3-Br, 4-F | 2,5-F₂ |
| 485 | H | 3-Br, 4-F | 2,6-F₂ |
| 486 | Me | 3-F, 4-CN | 3-F |
| 487 | Me | 3-F, 4-CN | 3-Cl |
| 488 | Me | 3-F, 4-CN | 3-CN |
| 489 | Me | 3-F, 4-CN | 4-F |
| 490 | Me | 3-F, 4-CN | 4-CI |
| 491 | Me | 3-F, 4-CN | 2,5-F₂ |
| 492 | Me | 3-F, 4-CN | 2,6-F₂ |
| 493 | H | 3-F, 4-CN | 3-F |
| 494 | H | 3-F, 4-CN | 3-Cl |
| 495 | H | 3-F, 4-CN | 3-CN |
| 496 | H | 3-F, 4-CN | 4-F |
| 497 | H | 3-F, 4-CN | 4-Cl |
| 498 | H | 3-F, 4-CN | 2,5-F₂ |
| 499 | H | 3-F, 4-CN | 2,6-F₂ |

Definition der Beispiele in den nachfolgenden Tabellen 2 bis 24f:
Zu Referenzzwecken sind in den nachfolgenden Tabellen 2 bis 24f die einzelnen Verbindungen einzelnen Nummern (= Beispielnummern) zugeordnet, wobei die jeweilige Beispielnummer sich zusammensetzt aus der Nummer der chemischen Formel, die der jeweiligen Tabelle zugeordnet ist und einer "Zeilennummer" (Zeilennummer), die sich auf dieselbe Nummer in der Zeile der ersten Spalte der Tabelle 1 bezieht. Die chemische Struktur des Beispiels Nr. "(Formelnummer)(Zeilennummer)" ist damit durch die der jeweiligen Tabelle voran gestellten Formel gemäß Formelnummer und der Zeilennummer aus Tabelle 1 eindeutig definiert, zum Beispiel:
   Das Beispiel mit der Nr. "Iaa1" aus Tabelle 2 ist die Verbindung der Formel (la), worin A¹ = O bedeutet [= Formel (Iaa)] und Q = OH, (R¹)ₘ = 3-F und (R¹)ₙ = 4-Cl gemäß der Zeile 1 aus Tabelle 1 definiert ist, oder ein Tautomer davon.
   Das Beispiel mit der Nr. "Iad204" aus Tabelle 2 ist die Verbindung der Formel (la), worin A¹ = N-OH bedeutet [= Formel (lad)] und Q = OH, (R¹)ₘ = 3,4-Cl₂ und (R²)ₙ = 4-F gemäß der Zeile 204 aus Tabelle 1 definiert ist, oder ein Tautomer davon. Entsprechendes gilt für die Zuordnung von racemischen oder optisch aktiven threo-Stereoisomeren oder erythro-Stereoisomeren. Beispielsweise sind die Verbindungen der Tabelle 2a zu Referenzzwecken einzelnen Nummern (= Beispielnummern) zugeordnet, wobei die Nummer "threo-laa(Zeilennummer)" das racemische Gemisch der threo-Enantiomeren mit der chemischen Struktur der Formeln (threo-1-Iaa) und (threo-2-Iaa), welche jeweils die Strukturkombination der Gruppen Q, (R¹)ₘ und (R²)ₙ gemäß der Zeilennummer aus der Tabelle 1 aufweist, bezeichnet.

**Tabelle 2: Verbindungen der Formel (Ia), (Iaa), (Iab), (Iac), (Iad), (Iae), (Iaf), (Iag) und (Iah), worin jeweils Q, (R¹)ₘ und (R²)ₙ wie in Tabelle 1 definiert sind**

| | |
|---|---|
| 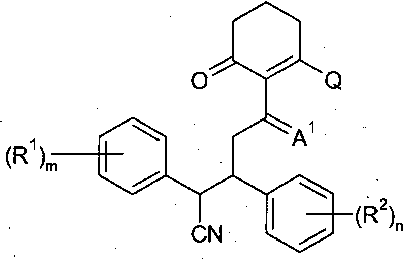 | **(Ia)** |
| | **(Iaa) A¹ ist (=O)** |
| | **(Iab) A¹ ist (=S)** |
| | **(Iac) A¹ ist (=N-CH₃)** |
| | **(Iad) A¹ ist (=N-OH)** |
| | **(Iae) A¹ ist** (**=N**-**OCH₃**) |
| | **(Iaf) A¹ ist (=N-NH₂)** |
| | **(Iag) A¹ ist [=N-N(CH₃)₂]** |
| | **(Iah) A¹ ist (=N-Benzyl)** |

Tabelle 2, Beispiele für erythro-threo-Gemische (Mengenverhältnis 70:30 bis 30:70):
Beispiele für Verbindungen der Formeln (Iaa) bis (Iah) sind die Verbindungen der betreffenden Formeln (Iaa) bis (Iah) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern, z. B. Iaa50 = Verbindung der Formel (Iaa) mit Q = OSO₂(4-Me-Ph), (R¹)ₘ = 3,4-Difluor und (R²)ₙ = 3-Fluor gemäß Zeile 50 aus Tabelle 1.

**Tabellen 2a bis 2f: Verbindungen der Formel (threo-Iaa) und (threo-2-Iaa), worin jeweils Q, (R¹)ₘ und (R²)ₙ wie in Tabelle 1 definiert sind:**

| | |
|---|---|
| 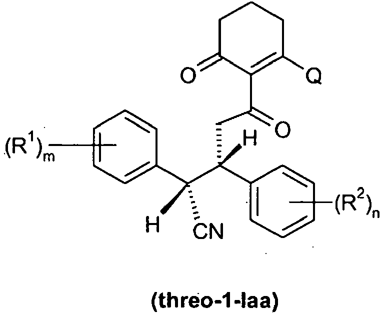 | 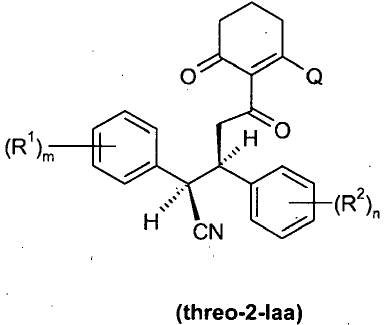 |
| **(threo-Iaa) = (threo-1-Iaa) + (threo-2-Iaa) (50:50) = (rac.)** | |

### Tabelle 2a (threo-Racemate), Beispiele:

Verbindungen der Formel (Iaa) in Form des racemischen Gemischs der threo-Isomeren [= Formel (threo-Iaa)], worin die Strukturkombination der Gruppen Q, (R¹)ₘ und (R²)ₙ gemäß einer Zeilennummer aus der Tabelle 1 definiert ist.

Die Nummerierung erfolgt gemäß "threo-Iaa(Zeilennummer)".

### Tabelle 2b (optisch aktive, threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-Iaa), worin die Strukturkombination der Gruppen Q, (R¹)ₘ und (R²)ₙ gemäß einer Zeilennummer aus der Tabelle 1 definiert ist.

Die Nummerierung erfolgt gemäß "threo-2-Iaa(Zeilennummer)".

### Tabelle 2c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Iaa), worin die Strukturkombination der Gruppen Q, (R¹)ₘ und (R²)ₙ gemäß einer Zeilennummer aus der Tabelle 1 definiert ist.

Einem einzelnen Enantiomer wird dabei die Nummer "threo-1-Iaa(Zeilennummer)" zugeordnet. Beispielsweise bezeichnet die Nr. threo-1-Iaa5 die Verbindung der Formel (threo-1-laa), worin Q = OH, (R¹)ₘ = 3-F und (R²)ₙ = 4-Br bedeuten.

### Tabelle 2d (erythro-Racemate), Beispiele:

Verbindungen der Formel (Iaa) in Form des racemischen Gemischs der erythro-Isomeren [= Formel (erythro-Iaa)], worin die Strukturkombination der Gruppen Q, (R¹)ₘ und (R²)ₙ gemäß einer Zeilennummer aus der Tabelle 1 definiert ist.

Die Nummerierung erfolgt gemäß "erythro-Iaa(Zeilennummer)".

### Tabelle 2e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Iaa) in der erythro-1-Form [= Formel (erythro-1-Iaa)], worin die Strukturkombination der Gruppen Q, (R¹)ₘ und (R²)ₙ gemäß einer Zeilennummer aus der Tabelle 1 definiert ist.

Die Nummerierung erfolgt gemäß "erythro-1-Iaa(Zeilennummer)".

### Tabelle 2f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Iaa) in der erythro-2-Form [= Formel (erythro-2-Iaa)], worin die Strukturkombination der Gruppen Q, (R¹)ₘ und (R²)ₙ gemäß einer Zeilennummer aus der Tabelle 1 definiert ist.

Die Nummerierung erfolgt gemäß "erythro-2-Iaa(Zeilennummer)".

**Tabelle 3: Verbindungen der Formel (Ib), (Iba), (Ibb), (Ibc), (Ibd), (Ibe), (Ibf), (Ibg) und (Ibh), worin jeweils Q, (R¹)ₘ und (R²)ₙ wie in Tabelle 1 definiert sind:**

| | |
|---|---|
| 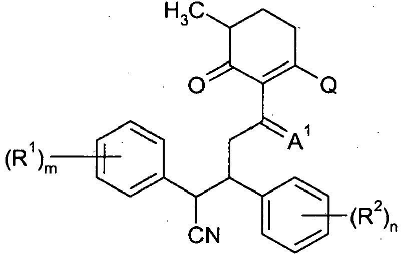 | **(Ib)** |
| | **(Iba) A¹ ist (=O)** |
| | **(Ibb) A¹ ist (=S)** |
| | **(Ibc) A¹ ist (=N-CH₃)** |
| | **(Ibd) A¹ ist (=N-OH)** |
| | **(Ibe) A¹ ist (=N-OCH₃)** |
| | **(Ibf) A¹** ist **(=N-NH₂)** |
| | **(Ibg) A¹ ist [=N-N(CH₃)₂]** |
| | **(Ibh) A¹ ist (=N-Benzyl)** |

### Tabelle 3, Beispiele für erythro-threo-Gemische (Mengenverhältnis 70:30 bis 30:70):

Beispiele sind die Verbindungen der betreffenden Formel (Iaa) bis (Iah) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 4: Verbindungen der Formel (Ic), (Ica), (Icb), (Icc), (Icd), (Ice), (Icf), (Icg) und (Ich), worin jeweils Q, (R¹)ₘ und (R²)ₙ wie in Tabelle 1 definiert sind:**

| | |
|---|---|
| 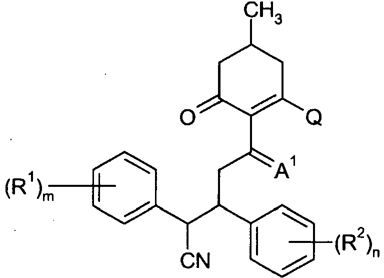 | **(Ic)** |
| | **(Ica) A¹ ist (=O)** |
| | **(Icb) A¹ ist** (=S) |
| | **(Icc) A¹ ist (=N-CH₃)** |
| | **(Icd) A¹ ist (=N-OH)** |
| | **(Ice) A¹ ist (=N-OCH₃)** |
| | **(Icf) A¹ ist (=N-NH₂)** |
| | **(Icg) A¹ ist [=N-N(CH₃)₂]** |
| | **(Ich) A¹ ist (=N-Benzyl)** |

### Tabelle 4, Beispiele für erythro-threo-Gemische (Mengenverhältnis 70:30 bis 30:70):

Beispiele sind die Verbindungen der betreffenden Formel (Ica) bis (Ich) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 5: Verbindungen der Formel (Id), (Ida), (Idb), (Idc), (Idd), (Ide), (Idf), (Idg) und (Idh), worin jeweils Q, (R¹)ₘ und (R²)ₙ wie in Tabelle 1 definiert sind:**

| | |
|---|---|
| 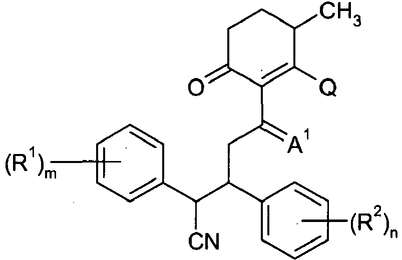 | **(Id**) |
| | **(Ida) A¹ ist (=O)** |
| | **(Idb) A¹ ist (=S)** |
| | **(Idc) A¹ ist (=N-CH₃)** |
| | **(Idd) A¹ ist (=N-OH)** |
| | **(Ide) A¹ ist (=N-OCH₃)** |
| | **(Idf) A¹ ist (=N-NH₂)** |
| | **(Idg) A¹ ist [=N-N(CH₃)₂]** |
| | **(Idh) A¹ ist (=N-Benzyl)** |

### Tabelle 5, Beispiele für erythro-threo-Gemische (Mengenverhältnis 70:30 bis 30:70):

Beispiele sind die Verbindungen der betreffenden Formel (Ida) bis (Idh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 6: Verbindungen der Formel (Ie), (lea), (leb), (Iec), (Ied), (lee), (Ief), (leg) und (Ieh), worin jeweils Q, (R¹)ₘ und (R²)ₙ wie in Tabelle 1 definiert sind:**

| | |
|---|---|
| 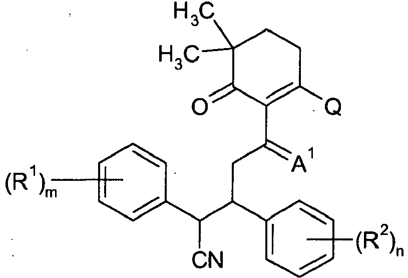 | **(Ie)** |
| | **(Iea) A¹ ist (=O)** |
| | **(Ieb) A¹ ist (=S)** |
| | **(Iec) A¹ ist (=N-CH₃)** |
| | **(Ied) A¹ ist (=N-OH)** |
| | **(Iee) A¹ ist (=N-OCH₃)** |
| | **(Ief) A¹ ist (=N-NH₂)** |
| | **(Ieg) A¹ ist [=N-N(CH₃)₂]** |
| | **(Ieh) A¹ ist (=N-Benzyl)** |

### Tabelle 6, Beispiele für erythro-threo-Gemische (Mengenverhältnis 70:30 bis 30:70):

Beispiele sind die Verbindungen der betreffenden Formel (Iea) bis (Ieh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabellen 6a bis 6f: Verbindungen der Formel (threo-Iea) und (threo-2-Iea), worin jeweils Q, (R¹)ₘ und (R²)ₙ wie in Tabelle 1 definiert sind:**

| | |
|---|---|
| 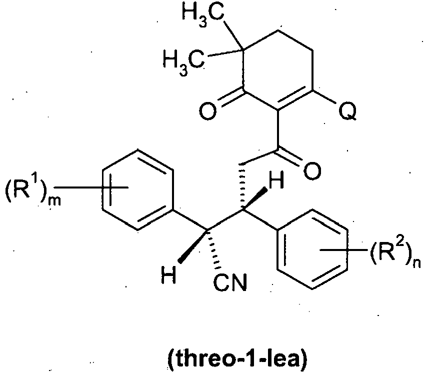 | 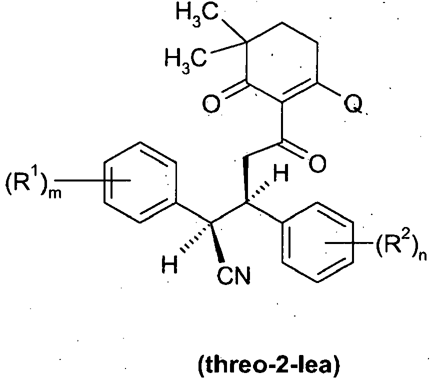 |
| **(threo-Iea) = (threo-1-Iea) + (threo-2-Iea) (50:50) = (rac.)** | |

### Tabelle 6a (Racemate), Beispiele:

Verbindungen der Formel (Iea) in Form des racemischen Gemischs der threo-Isomeren [= Formel (threo-Iea)], worin die Strukturkombination der Gruppen Q, (R¹)ₘ und (R²)ₙ gemäß einer Zeilennummer aus der Tabelle 1 definiert ist.

Die Nummerierung einzelner Verbindungen erfolgt gemäß "threo-Iea(Zeilennummer)".

### Tabelle 6b (optisch aktive threo-2-Enantiomere), Beispiele:

Optisch aktive threo-2-Enantiomere in angereicherter Form [= (2R,3R)-Form mit mehr als 90%ee] mit der chemischen Struktur der Formel (threo-2-lea), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-2-lea(Zeilennummer)".

### Tabelle 6c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-lea), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-1-lea(Zeilennummer)".

### Tabelle 6d (erythro-Racemate), Beispiele:

Verbindungen der Formel (lea) in Form des racemischen Gemischs der erythro-Isomeren [= Formel (erythro-lea)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-lea(Zeilennummer)".

### Tabelle 6e (erythro-1-Enantiomere), Beispiele:

Optisch aktive erythro-1-Enantiomere in angereicherter Form [= (2R,3S)-Form mit mehr als 90%ee] mit der chemischen Struktur der Formel (lea) in der erythro-1-Form [= Formel (erythro-1-lea)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-1-lea(Zeilennummer)".

### Tabelle 6f (erythro-2-Enantiomere), Beispiele:

Optisch aktive erythro-2-Enantiomere in angereicherter Form [= (2S,3R)-Form mit mehr als 90%ee] mit der chemischen Struktur der Formel (lea) in der erythro-2-Form [= Formel (erythro-2-lea)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-2-lea(Zeilennummer)".

**Tabelle 7: Verbindungen der Formel (If), (Ifa), (Ifb), (Ifc), (Ifd), (Ife), (Iff), (Ifg) und (Ifh), worin jeweils Q, (R¹)ₘ und (R²)ₙ wie in Tabelle 1 definiert sind:**

| | |
|---|---|
| 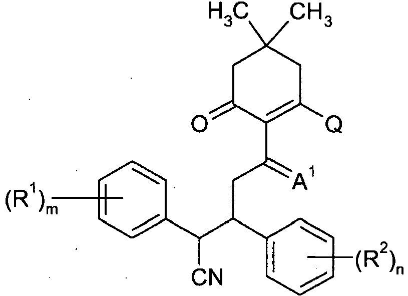 | **(If)** |
| | **(Ifa) A¹ ist (=O)** |
| | **(Ifb) A¹ ist (=S)** |
| | **(Ifc) A¹ ist (=N-CH₃)** |
| | **(Ifd) A¹ ist (=N-OH)** |
| | **(Ife) A¹ ist (=N-OCH₃)** |
| | **(Iff) A¹ ist (=N-NH₂)** |
| | **(Ifg) A¹ ist [=N-N(CH₃)₂]** |
| | **(Ifh) A¹ ist (=N-Benzyl)** |

### Tabelle 7, Beispiele für erythro-threo-Gemische (Mengenverhältnis 70:30 bis 30:70):

Beispiele sind die Verbindungen der betreffenden Formel (Ifa) bis (Ifh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabellen 7a bis 7f: Verbindungen der Formel (threo-Ifa) und (threo-2-Ifa), worin jeweils Q, (R¹)ₘ und (R²)ₙ wie in Tabelle 1 definiert sind:**

| | |
|---|---|
| 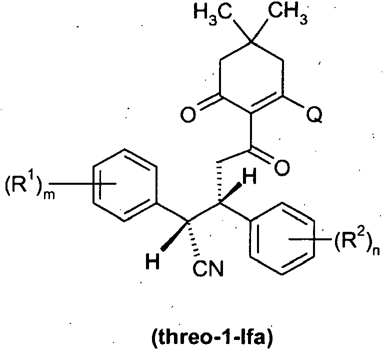 | 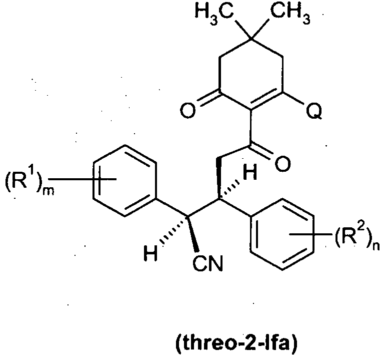 |
| **(threo-Ifa) = (threo-1-Ifa) + (threo-2-Ifa) (50:50) = (rac.)** | |

### Tabelle 7a (threo-Racemate), Beispiele:

Verbindungen der Formel (Ifa) in Form des racemischen Gemischs der threo-Isomeren [= Formel (threo-Ifa)], worin die Strukturkombination der Gruppen Q, (R¹)ₘ und (R²)ₙ gemäß einer Zeilennummer aus der Tabelle 1 definiert ist.

Die Nummerierung einzelner Verbindungen erfolgt gemäß "threo-Ifa(Zeilennummer)".

### Tabelle 7b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-Ifa), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-2-Ifa(Zeilennummer)".

### Tabelle 7c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Ifa), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-1-Ifa(Zeilennummer)".

### Tabelle 7d (erythro-Racemate), Beispiele:

Verbindungen der Formel (Ifa) in Form des racemischen Gemischs der erythro-Isomeren [= Formel (erythro-Ifa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-Ifa(Zeilennummer)".

### Tabelle 7e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Ifa) in der erythro-1-Form [= Formel (erythro-1-Ifa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-1-Ifa(Zeilennummer)".

### Tabelle 7f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Ifa) in der erythro-2-Form [= Formel (erythro-2-Ifa)], worin die Strukturkombination der Gruppen Q, (R¹)ₘ und (R²)ₙ gemäß einer Zeilennumnier aus der Tabelle 1 definiert ist. Die Nummerierung einzelner Verbindungen erfolgt gemäß "erythro-2-Ifa(Zeilennummer)".

**Tabelle 8: Verbindungen der Formel (Ig), (Iga), (Igb), (Igc), (Igd), (Ige), (Igf), (Igg) und (Igh), worin jeweils Q, (R¹)ₘ und (R²)ₙ wie in Tabelle 1 definiert sind:**

| | |
|---|---|
| 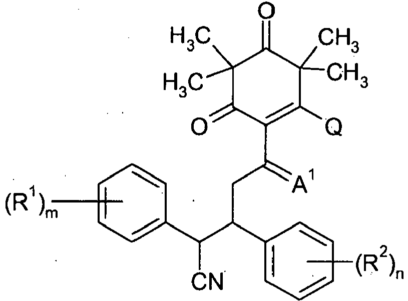 | **(Ig)** |
| | **Iga) A¹ ist (=O)** |
| | **(Igb) A¹ ist (=S)** |
| | **(Igc) A¹ ist (=N-CH₃)** |
| | **(Igd) A¹ ist (=N-OH)** |
| | **(Ige) A¹ ist (=N-OCH₃)** |
| | **(Igf) A¹ ist (=N-NH₂)** |
| | **(Igg) A¹ ist [=N=N(CH₃)₂]** |
| | **(Igh) A¹ ist (=N-Benzyl)** |

### Tabelle 8, Beispiele für erythro-threo-Gemische (Mengenverhältnis 70:30 bis 30:70):

Beispiele sind die Verbindungen der betreffenden Formel (Iga) bis (Igh) jeweils in Form eines racemischen erythro-threo-Gemischs (Mengenverhältnis 70:30 bis 30:70), worin die Bedeutung von Q, (R¹)ₘ und (R²ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 9: Verbindungen der Formel (Ih), (Iha), (Ihb), (Ihc), (Ihd), (Ihe), (Ihf), (Ihg) und (Ihh), worin jeweils Q, (R¹)ₘ und (R²)ₙ wie in Tabelle 1 definiert sind:**

| | |
|---|---|
| 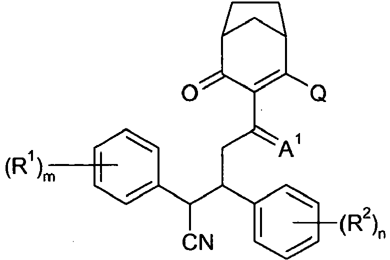 | **(Ih)** |
| | **Iha) A¹ ist (=O)** |
| | **(Ihb) A¹ ist (=S)** |
| | **(Ihc) A¹ ist (=N-CH₃)** |
| | **(Ihd) A¹ ist (=N-OH)** |
| | **(Ihe) A¹ ist (=N-OCH₃)** |
| | **(Ihr A¹ ist (=N-NH₂)** |
| | **(Ihg) A¹ ist [=N-N(CH₃)₂]** |
| | **(Ihh) A¹ ist (=N-Benzyl)** |

### Tabelle 9, Beispiele für erythro-threo-Gemische (Mengenverhältnis von 70:30 bis 30:70):

Beispiele sind die Verbindungen der betreffenden Formel (Iha) bis (Ihh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabellen 9a bis 9f: Verbindungen der Formel (threo-Iha) und (threo-2-Iha), worin jeweils Q, (R¹)ₘ und (R²)ₙ wie in Tabelle 1 definiert sind:**

| | |
|---|---|
| 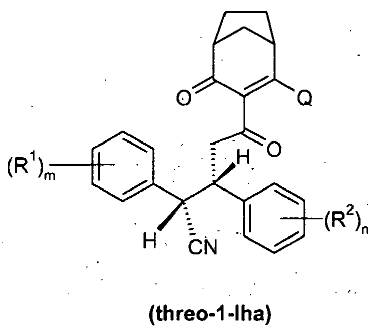 | 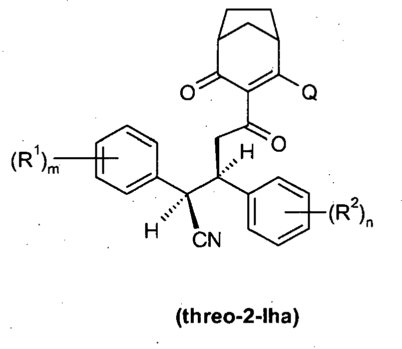 |
| **(threo-Iha) = (threo-1-Iha) + (threo-2-Iha) (50:50) = (rac.)** | |

### Tabelle 9a (threo-Racemate), Beispiele:

Verbindungen der Formel (Iha) in Form des racemischen Gemischs der threo-Isomeren [= Formel (threo-Iha)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-Iha(Zeilennummer)".

### Tabelle 9b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-Iha), worin die Strukturkombination der Gruppen Q, (R¹)ₘ und (R²)ₙ gemäß einer Zeilennummer aus der Tabelle 1 definiert ist. Die Nummerierung einzelner Beispiele erfolgt gemäß "threo-2-Iha(Zeilennummer)".

### Tabelle 9c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Iha), worin die Strukturkombination der Gruppen Q, (R¹)ₘ und (R²)ₙ gemäß einer Zeilennummer aus der Tabelle 1 definiert ist. Die Nummerierung einzelner Beispiele erfolgt gemäß "threo-1-Iha(Zeilennummer)".

### Tabelle 9d (erythro-Racemate), Beispiele:

Verbindungen der Formel (Iha) in Form des racemischen Gemischs der erythro-Isomeren [= Formel (erythro-Iha)], worin die Strukturkombination der Gruppen Q, (R¹)ₘ und (R²)ₙ gemäß einer Zeilennummer aus der Tabelle 1 definiert ist. Die Nummerierung erfolgt einzelner Beispiele gemäß "erythro-Iha(Zeilenhummer)".

### Tabelle 9e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Iha) in der erythro-1-Form [= Formel (erythro-1-Iha)], worin die Strukturkombination der Gruppen Q, (R¹)ₘ und (R²)ₙ gemäß einer Zeilennummer aus der Tabelle 1 definiert ist. Die Nummerierung einzelner Beispiele erfolgt gemäß "erythro-1-Iha(Zeilennummer)".

### Tabelle 9f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Iha) in der erythro-2-Form [= Formel (erythro-2-Iha)], worin die Strukturkombination der Gruppen Q, (R¹)ₘ und (R²)ₙ gemäß einer Zeilennummer aus der Tabelle 1 definiert ist. Die Nummerierung einzelner Beispiele erfolgt gemäß "erythro-2-Iha(Zeilennummer)".

**Tabelle 10: Verbindungen der Formel (Ii), (Iia), (Iib), (Iic), (Iid), (Iie), (Iif), (Iig) und (Iih), worin jeweils Q, (R¹)ₘ und (R²)ₙ wie in Tabelle 1 definiert sind:**

| | |
|---|---|
| 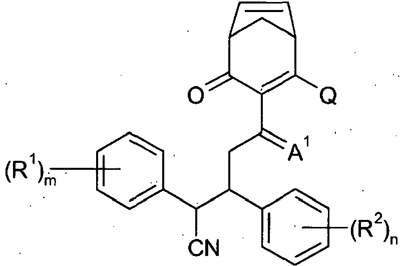 | **(Ii)** |
| | **Iia) A¹ ist (=O)** |
| | **(Iib) A¹ ist (=S)** |
| | **(Iic) A¹ ist (=N-CH₃)** |
| | **(Iid) A¹ ist (=N-OH)** |
| | **(Iie) A¹ ist (=N-OCH₃)** |
| | **(Iif) A¹ ist (=N-NH₂)** |
| | **(Iig) A¹ ist [=N-N(CH₃)₂]** |
| | **(Iih) A¹ ist (=N-Benzyl)** |

### Tabelle 10, Beispiele für erythro-threo-Gemische (Mengenverhältnis 70:30 bis 30:70):

Beispiele sind die Verbindungen der betreffenden Formel (Iia) bis (Iih) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 11: Verbindungen der Formel (Ik), (Ika), (Ikb), (Ikc), (Ikd), (Ike), (Ikf), (Ikg) und (Ikh), worin jeweils Q, (R¹)ₘ und (R²)ₙ wie in Tabelle 1 definiert sind:**

| | |
|---|---|
| 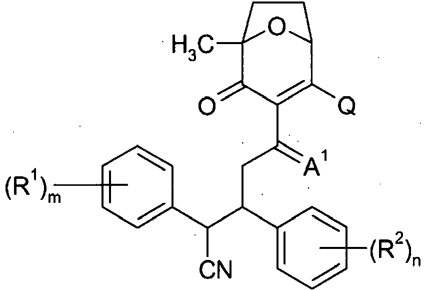 | **(Ik)** |
| | **(Ika) A¹ ist (=O)** |
| | **(Ikb) A¹ ist (=S)** |
| | **(Ikc) A¹ ist (=N-CH₃)** |
| | **(Ikd) A¹ ist (=N-OH)** |
| | **(Ike) A¹ ist (=N-OCH₃)** |
| | **(Ikf) A¹ ist (=N-NH₂)** |
| | **(Ikg) A¹ ist 1=N-N(CH₃)₂]** |
| | **(Ikh) A¹ ist (=N-Benzyl)** |

### Tabelle 11, Beispiele für erythro-threo-Gemische (Mengenverhältnis 70:30 bis 30:70):

Beispiele sind die Verbindungen der betreffenden Formel (Ika) bis (Ikh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 12: Verbindungen der Formel (IL), (ILa), (ILb), (ILc), (ILd), (ILe), (ILf), (ILg) und (ILh), worin jeweils Q, (R¹)ₘ und (R²)ₙ wie in Tabelle 1 definiert sind:**

| | |
|---|---|
| 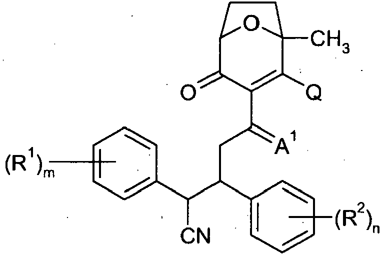 | **(IL)** |
| | **(ILa) A¹ ist (=O)** |
| | **(ILb) A¹ ist (=S)** |
| | **(ILc) A¹ ist (=N-CH₃)** |
| | **(ILd) A¹ ist (=N-OH)** |
| | **(ILe) A¹ ist (=N-OCH₃)** |
| | **(ILf) A¹ ist (=N-NH₂)** |
| | **(ILg) A¹ ist (=N-N(CH₃)₂]** |
| | **(ILh) A¹ ist (=N-Benzyl)** |

### Tabelle 12, Beispiele für erythro-threo-Gemische (Mengenverhältnis 70:30 bis 30:70):

Beispiele sind die Verbindungen der betreffenden Formel (ILa) bis (ILh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 13: Verbindungen der Formel (Im), (Ima), (Imb), (Imc), (Imd), (Ime), (Imf), (Img) und (Imh), worin jeweils Q, (R¹)ₘ und (R²)ₙ wie in Tabelle 1 definiert sind:**

| | |
|---|---|
| 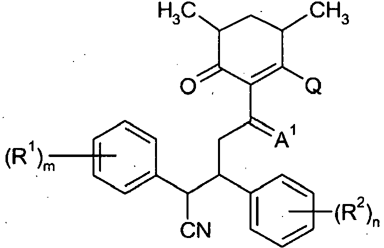 | **(Im)** |
| | **(Ima) A¹ ist (=O)** |
| | **(Imb) A¹ ist (=S)** |
| | **(Imc) A¹ ist (=N-CH₃)** |
| | **(Imd) A¹ ist (=N-OH)** |
| | **(Ime) A¹ ist (=N-OCH₃)** |
| | **(Imf) A¹ ist (=N-NH₂)** |
| | **(Img) A¹ ist (=N-N(CH₃)₂]** |
| | **(Imh) A¹ ist (=N-Benzyl)** |

### Tabelle 13, Beispiele für erythro-threo-Gemische (Mengenverhältnis 70:30 bis 30:70):

Beispiele sind die Verbindungen der betreffenden Formel (Ima) bis (Imh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 14: Verbindungen der Formel (In), (Ina), (Inb), (Inc), (Ind), (Ine), (Inf), (Ing) und (Inh), worin jeweils Q, (R¹)ₘ und (R²)ₙ wie in Tabelle 1 definiert sind:**

| | |
|---|---|
| 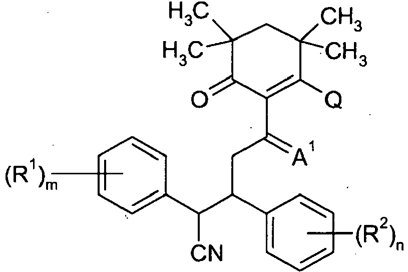 | **(In)** |
| | **(Ina) A¹ ist (=O)** |
| | **(Inb) A¹ ist (=S)** |
| | **(Inc) A¹ ist (=N-CH₃)** |
| | **(Ind) A¹ ist (=N-OH)** |
| | **(Ine) A¹ ist (=N-OCH₃)** |
| | **(Inf) A¹ ist (=N-NH₂)** |
| | **(Ing) A¹ ist [=N-N(CH₃)₂]** |
| | **(Inh) A¹ ist (=N-Benzyl)** |

### Tabelle 14, Beispiele für erythro-threo-Gemische (Mengenverhältnis 70:30 bis 30:70):

Beispiele sind die Verbindungen der betreffenden Formel (Ina) bis (Inh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 15: Verbindungen der Formel (Io), (Ioa), (Iob), (Ioc), (Iod), (Ioe), (Iof), (Iog) und (loh), worin jeweils Q, (R¹)ₘ und (R²)ₙ wie in Tabelle 1 definiert sind:**

| | |
|---|---|
| 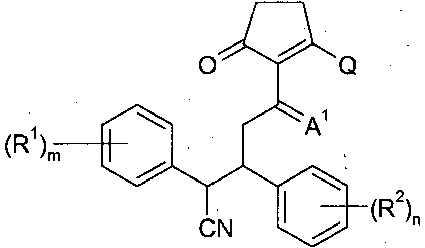 | **(Io)** |
| | **(Ioa) A¹ ist (=O)** |
| | **(Iob) A¹ ist (=S)** |
| | **(Ioc) A¹ ist (=N-CH₃)** |
| | **(Iod) A¹ ist (=N-OH)** |
| | **(Ioe) A¹ ist (=N=OCH₃)** |
| | **(Iot) A¹ ist (=N-NH₂)** |
| | **(Iog) A¹ ist [=N-N(CH₃)₂]** |
| | **(Ioh) A¹ ist (=N-Benzyl)** |

### Tabelle 15, Beispiele für erythro-threo-Gemische (Mengenverhältnis 70:30 bis 30:70):

Beispiele sind die Verbindungen der betreffenden Formel (Ioa) bis (loh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 16: Verbindungen der Formel (Ip), (Ipa), (Ipb), (Ipc), (Ipd), (Ipe), (Ipf), (Ipg) und (Iph), worin jeweils Q, (R¹)ₘ und (R²)ₙ wie in Tabelle 1 definiert sind:**

| | |
|---|---|
| 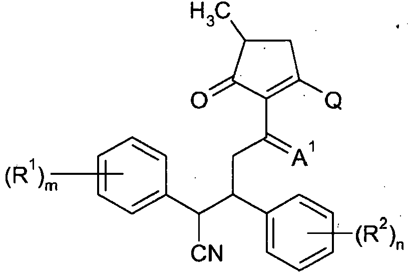 | **(Ip)** |
| | **(Ipa) A¹ ist (=O)** |
| | **(Ipb) A¹ ist (=S)** |
| | **(Ipc) A¹ ist (=N-CH₃)** |
| | **(Ipd) A¹ ist (=N-OH)** |
| | **(Ipe) A¹ ist (=N-OCH₃)** |
| | **(Ipf) A¹ ist (=N-NH₂)** |
| | **(Ipg) A¹ ist [=N-N(CH₃)₂]** |
| | **(Iph) A¹ ist (=N-Benzyl)** |

### Tabelle 16, Beispiele für erythro-threo-Gemische (Mengenverhältnis 70:30 bis 30:70):

Beispiele sind die Verbindungen der betreffenden Formel (Ipa) bis (Iph) jeweils in Form eines racemischen erythro-threo-Gemischs (Menenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 17: Verbindungen der Formel (Iq), (Iqa), (Iqb), (Iqc), (Iqd), (Iqe), (Iqf), (Iqg) und (Iqh), worin jeweils Q, (R¹)ₘ und (R²)ₙ wie in Tabelle 1 definiert sind:**

| | |
|---|---|
| 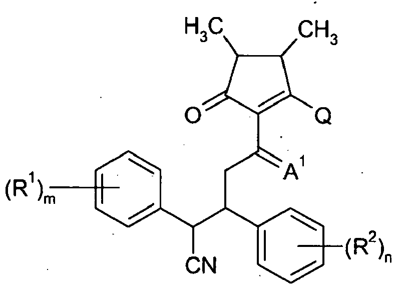 | **(Iq)** |
| | **(Iqa) A¹ ist (=O)** |
| | **(Iqb) A¹ ist (=S)** |
| | **(Iqc) A¹ ist (=N-CH₃)** |
| | **(Iqd) A¹ ist (=N-OH)** |
| | **(Iqe) A¹ ist (=N-OCH₃)** |
| | **(Iqf) A¹ ist (=N-NH₂)** |
| | **(Iqg) A¹ ist [=N-N(CH₃)₂]** |
| | **(Iqh) A¹ ist (=N-Benzyl)** |

### Tabelle 17, Beispiele für erythro-threo-Gemische (Mengenverhältnis 70:30 bis 30:70):

Beispiele sind die Verbindungen der betreffenden Formel (Iqa) bis (Iqh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 18: Verbindungen der Formel (Ir), (Ira), (Irb), (Irc), (Ird), (Ire), (Irf), (Irg) und (Irh), worin jeweils Q, (R¹)ₘ und (R²)ₙ wie in Tabelle 1 definiert sind:**

| | |
|---|---|
| 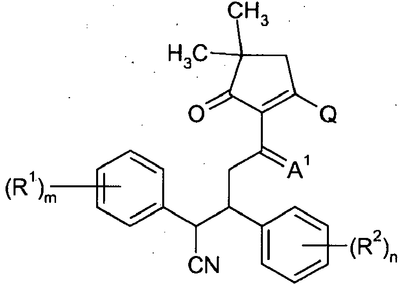 | **(Ir)** |
| | **(Ira) A¹ ist (=O)** |
| | **(Irb) A¹ ist (=S)** |
| | **(Irc) A¹ ist (=N-CH₃)** |
| | **(Ird) A¹ ist (=N-OH)** |
| | **(Ire) A¹ ist (=N-OCH₃)** |
| | **(Irf) A¹ ist (=N-NH₂)** |
| | **(Irg) A¹ ist [=N-N(CH₃)₂]** |
| | **(Irh) A¹ ist (=N-Benzyl)** |

### Tabelle 18, Beispiele für erythro-threo-Gemische (Mengenverhältnis 70:30 bis 30:70):

Beispiele sind die Verbindungen der betreffenden Formel (Ira) bis (Irh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 19: Verbindungen der Formel (Is), (Isa), (Isb), (Isc), (Isd), (Ise), (Isf), (Isg) und (Ish), worin jeweils Q, (R¹)ₘ und (R²)ₙ wie in Tabelle 1 definiert sind:**

| | |
|---|---|
| 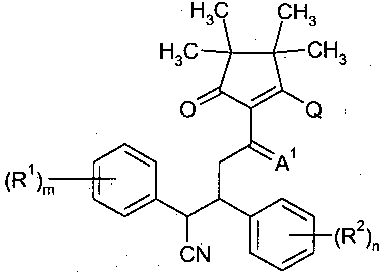 | **(Is)** |
| | **(Isa) A¹ ist (=O)** |
| | **(Isb) A¹ ist (=S)** |
| | **(Isc) A¹ ist (=N-CH₃)** |
| | **(Isd) A¹ ist (=N-OH)** |
| | **(Ise) A¹ ist (=N-OCH₃)** |
| | **(Isf) A¹ ist (=N-NH₂)** |
| | **(Isg) A¹ ist [=N-N(CH₃)₂]** |
| | **(Ish) A¹ ist (=N-Benzyl)** |

### Tabelle 19, Beispiele für erythro-threo-Gemische (Mengenverhältnis 70:30 bis 30:70):

Beispiele sind die Verbindungen der betreffenden Formel (Isa) bis (Ish) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabelle 20: Verbindungen der Formel (It), (Ita), (Itb), (Itc), (Itd), (Ite), (Itf), (Itg) und (Ith), worin jeweils Q, (R¹)ₘ und (R²)ₙ wie in Tabelle 1 definiert sind:**

| | |
|---|---|
| 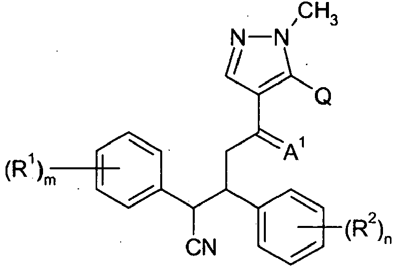 | **(It)** |
| | **(Ita) A¹ ist (=O)** |
| | **(Itb) A¹ ist (=S)** |
| | **(Itc) A¹ ist (=N-CH₃)** |
| | **(Itd) A¹ ist (=N-OH)** |
| | **(Ite) A¹ ist (=N-OCH₃)** |
| | **(Itf) A¹ ist (=N-NH₂)** |
| | **(Itg) A¹ ist [=N-N(CH₃)₂]** |
| | **(Ith) A¹ ist (=N-Benzyl)** |

### Tabelle 20, Beispiele für erythro-thireo-Gemische (Mengenverhältnis 70:30 bis 30:70):

Beispiele sind die Verbindungen der betreffenden Formel (Ita) bis (Ith) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabellen 20a bis 20f: Verbindungen der Formel (threo-Ita) und (threo-2-Ita), worin jeweils Q, (R¹)ₘ und (R²)ₙ wie in Tabelle 1 definiert sind:**

| | |
|---|---|
| 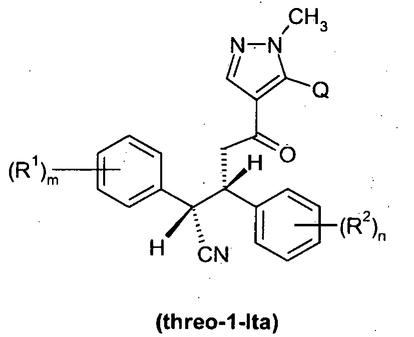 | 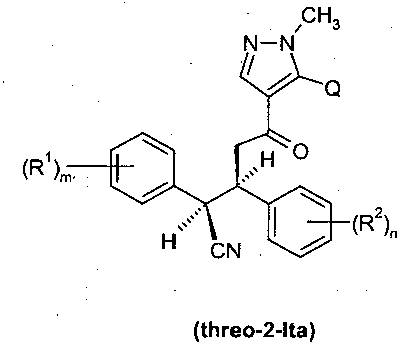 |
| **(threo-Ita) = (threo-1-Ita) + (threo-2-Ita) (50:50) = (rac.)** | |

### Tabelle 20a (threo-Racemate), Beispiele:

Verbindungen der Formel (Ita) in Form des racemischen Gemischs der threo-Isomeren [= Formel (threo-Ita)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-Ita(Zeilennummer)".

### Tabelle 20b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-Ita), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-2-Ita(Zeilennummer)".

### Tabelle 20c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Ita), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-1-Ita(Zeilennummer)".

### Tabelle 20d (erythro-Racemate), Beispiele:

Verbindungen der Formel (Ita) in Form des racemischen Gemischs der erythro-Isomeren [= Formel (erythro-Ita)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-Ita(Zeilennummer)".

### Tabelle 20e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Ita) in der erythro-1-Form [= Formel (erythro-1-Ita)], worin die Strukturkombination der Gruppen Q, (R¹⁺)ₘ und (R²)ₙ gemäß einer Zeilennummer aus der Tabelle 1 definiert ist. Die Nummerierung einzelner Verbindungen erfolgt gemäß "erythro-1-Ita(Zeilennummer)".

### Tabelle 20f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Ita) in der erythro-2-Form [= Formel (erythro-2-Ita)], worin die Strukturkombination der Gruppen Q, (R¹³)ₘ und (R²)ₙ gemäß einer Zeilennummer aus der Tabelle 1 definiert ist. Die Nummerierung einzelner Verbindungen erfolgt gemäß "erythro-2-Ita(Zeilennummer)" (analog Tab. 20b).

**Tabelle 21: Verbindungen der Formel (Iu), (Iua), (Iub), (Iuc), (Iud), (Iue), (Iuf), (Iug) und (Iuh), worin jeweils Q, (R¹)ₘ und (R²)ₙ wie in Tabelle 1 definiert sind:**

| | |
|---|---|
| 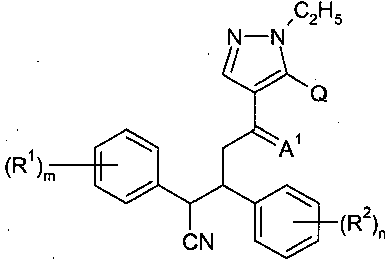 | **(Iu)** |
| | **(Iua) A¹ ist (=O)** |
| | **(Iub) A¹ ist (=S)** |
| | **(Iuc) A¹ ist (=N-CH₃)** |
| | **(Iud) A¹ ist (=N-OH)** |
| | **(Iue) A¹ ist (=N-OCH₃)** |
| | **(Iuf) A¹ ist (=N-NH₂)** |
| | **(Iug) A¹ ist (=N-N(CH₃)₂]** |
| | **(Iuh) A¹ ist (=N-Benzyl)** |

### Tabelle 21, Beispiele für erythro-threo-Gemische (Mengenverhältnis 70:30 bis 30:70):

Beispiele sind die Verbindungen der betreffenden Formel (Iua) bis (Iuh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabellen 21a bis 21f: Verbindungen der Formel (threo-Iua) und (threo-2-Iua), worin jeweils Q, (R¹)ₘ und (R²)ₙ wie in Tabelle 1 definiert sind:**

| | |
|---|---|
| 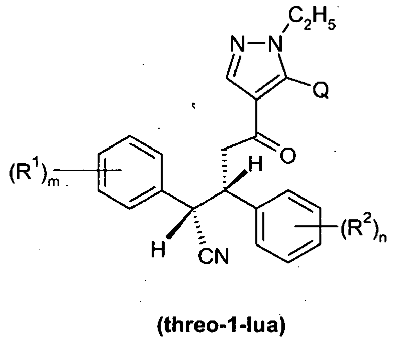 | 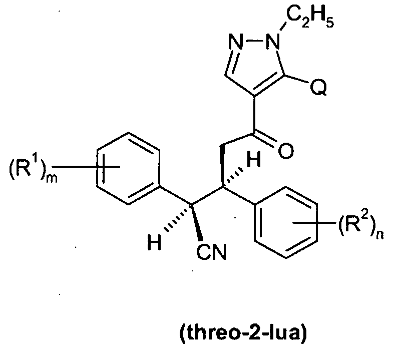 |
| **(threo-Iua) = (threo-1-Iua) + (threo-2-Iua) (50:50) = (rac.)** | |

### Tabelle 21 a (threo-Racemate), Beispiele:

Verbindungen der Formel (Iua) in Form des racemischen Gemischs der threo-Isomeren [= Formel (threo-Iua)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-Iua(Zeilennummer)".

### Tabelle 21 b (optisch aktive threo-2-Verbindungen), Beispiele:

Optisch aktive threo-2-Enantiomere in angereicherter Form [= (2R;3R)-Form mit mehr als 90%ee] mit der chemischen Struktur der Formel (threo-2-Iua), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-2-Iua(Zeilennummer)".

### Tabelle 21c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Iua), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-1-Iua(Zeilennummer)".

### Tabelle 21d (erythro-Racemate), Beispiele:

Verbindungen der Formel (Iua) in Form des racemischen Gemischs der erythro-Isomeren [= Formel (erythro-Iua)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-Iua(Zeilennummer)".

### Tabelle 21e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Iua) in der erythro-1-Form [= Formel (erythro-1-Iua)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-1-Iua(Zeilennummer)".

### Tabelle 21f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Iua) in der erythro-2-Form [= Formel (erythro-2-Iua)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-2-Iua(Zeilennummer)".

**Tabelle 22: Verbindungen der Formel (Iv), (Iva), (Ivb), (Ivc), (Ivd), (Ive), (Ivf), (Ivg) und (Ivh), worin jeweils Q, (R¹)ₘ und (R²)ₙ wie in Tabelle 1 definiert sind:**

| | |
|---|---|
| 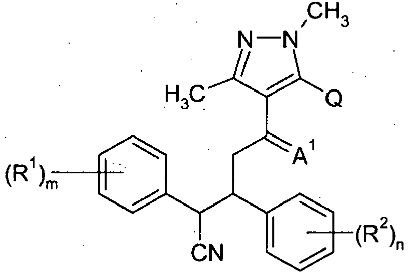 | **(Iv)** |
| | **(Iva) A¹ ist (=O)** |
| | **(Ivb) A¹ ist (=S)** |
| | **(Ivc) A¹ ist (=N-CH₃)** |
| | **(Ivd) A¹ ist (=N-OH)** |
| | **(Ive) A¹ ist (=N-OCH₃₃)** |
| | **(Ivf) A¹ ist (=N-NH₂)** |
| | **(Ivg) A¹ ist [=N-N(CH₃)₂]** |
| | **(Ivh) A¹ ist (=N-Benzyl)** |

### Tabelle 22, Beispiele für erythro-threo-Gemische (Mengenverhältnis 70:30 bis 30:70):

Beispiele sind die Verbindungen der betreffenden Formel (Iva) bis (Ivh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)." ohne Schreiben der Klammern.

**Tabellen 22a bis 22f: Verbindungen der Formel (threo-Iva) und (threo-2-Iva), worin jeweils Q, (R¹)ₘ und (R²)ₙ wie in Tabelle 1 definiert sind:**

| | |
|---|---|
| 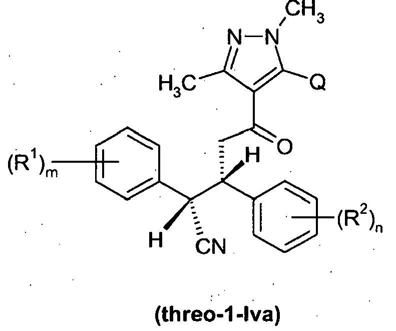 | 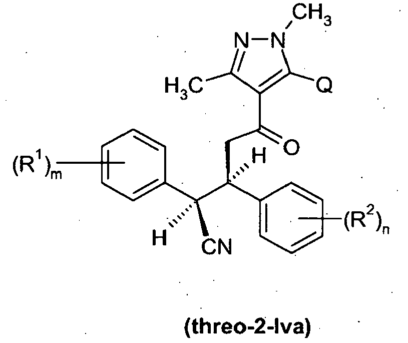 |
| **(threo-Iva) = (threo-1-Iva) + (threo-2-Iva) (50:50) = (rac.)** | |

### Tabelle 22a (threo-Racemate), Beispiele:

Verbindungen der Formel (Iva) in Form des racemischen Gemischs der threo-Isomeren [= Formel (threo-Iva)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-Iva(Zeilennummer)".

### Tabelle 22b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-Iva), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-2-Iva(Zeilennummer)".

### Tabelle 22c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Iva), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-1-Iva(Zeilennummer)".

### Tabelle 22d (erythro-Racemate), Beispiele:

Verbindungen der Formel (Iva) in Form des racemischen Gemischs der erythro-Isomeren [= Formel (erythro-Iva)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-Iva(Zeilennummer)".

### Tabelle 22e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Iva) in der erythro-1-Form [= Formel (erythro-1-Iva)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-1-Iva(Zeilennummer)".

### Tabelle 22f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Iva) in der erythro-2-Form [= Formel (erythro-2-Iva)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-2-Iva(Zeilennummer)".

**Tabelle 23: Verbindungen der Formel (Iw), (Iwa), (Iwb), (Iwc), (Iwd), (Iwe), (Iwf), (Iwg) und (Iwh), worin jeweils Q, (R¹)ₘ und (R²)ₙ wie in Tabelle 1 definiert sind:**

| | |
|---|---|
| 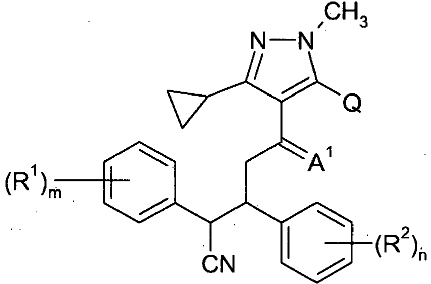 | **(Iw)** |
| | **(Iwa) A¹ ist (=O)** |
| | **(Iwb) A¹ ist (=S)** |
| | **(Iwc) A¹ ist (=N-CH₃]** |
| | **(Iwd) A¹ ist (=N-OH)** |
| | **(Iwe) A¹ ist (=N-OCH₃)** |
| | **(Iwf) A¹ ist (=N-NH₂)** |
| | **(Iwg) A¹ ist [=N-N(CH₃)₂]** |
| | **(Iwh) A¹ ist (=N-Benzyl)** |

### Tabelle 23, Beispiele für erythro-threo-Gemische (Mengenverhältnis 70:30 bis 30:70):

Beispiele sind die Verbindungen der betreffenden Formel (Iwa) bis (Iwh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabellen 23a bis 23f: Verbindungen der Formel (threo-Iwa) und (threo-2-Iwa), worin jeweils Q, (R¹)ₘ und (R²)ₙ wie in Tabelle 1 definiert sind:**

| | |
|---|---|
| 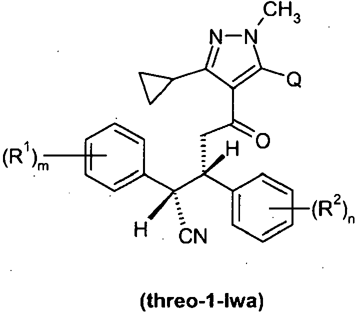 | 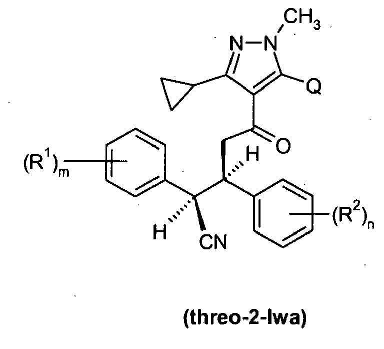 |
| **(threo-Iwa) = (threo-1-Iwa) + (threo-2-Iwa) (50:50) = (rac.)** | |

### Tabelle 23a (threo-Racemate), Beispiele:

Verbindungen der Formel (Iwa) in Form des racemischen Gemischs der threo-Isomeren [= Formel (threo-Iwa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-Iwa(Zeilennummer)".

### Tabelle 23b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-Iwa), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-2-Iwa(Zeilennummer)".

### Tabelle 23c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Iwa), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-1-Iwa(Zeilennummer)".

### Tabelle 23d (erythro-Racemate), Beispiele:

Verbindungen der Formel (Iwa) in Form des racemischen Gemischs der erythro-Isomeren [= Formel (erythro-Iwa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-Iwa(Zeilennummer)".

### Tabelle 23e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Iwa) in der erythro-1-Form [= Formel (erythro-1-Iwa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-1-Iwa(Zeilennummer)".

### Tabelle 23f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Iwa) in der erythro-2-Form [= Formel (erythro-2-Iwa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-2-Iwa(Zeilennummer)".

**Tabelle 24: Verbindungen der Formel (Ix), (Ixa), (Ixb), (Ixc), (Ixd), (Ixe), (Ixf), (Ixg) und (Ixh), worin jeweils Q, (R¹)ₘ und (R²)ₙ wie in Tabelle 1 definiert sind**

| | |
|---|---|
| 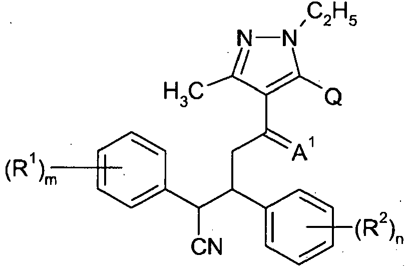 | **(Ix)** |
| | **(Ixa) A¹ ist (=O)** |
| | **(Ixb) A¹ ist (=S)** |
| | **(Ixc) A¹ ist (=N-CH₃)** |
| | **(Ixd) A¹ ist (=N-OH)** |
| | **(Ixe) A¹ ist (=N-OCH₃)** |
| | **(Ixf) A¹ ist (=N-NH₂)** |
| | **(Ixg) A¹ ist [=N-N(CH₃)₂]** |
| | **(Ixh) A¹ ist (=N-Benzyl)** |

### Tabelle 24, Beispiele für erythro-threo-Gemische (Mengenverhältnis 70:30 bis 30:70):

Beispiele sind die Verbindungen der betreffenden Formel (Ixa) bis (Ixh) jeweils in Form eines racemischen erythro-threo-Gemischs (Mengenverhältnis 70:30 bis 30:70), worin die Bedeutung von Q, (R¹)ₘ und (R²)ₙ gemäß einer Zeilennummer aus der Tabelle 1 definiert ist. Die Nummerierung einzelner Verbindungen erfolgt gemäß "(Formel)(Zeilennummer)" ohne Schreiben der Klammern.

**Tabellen 24a bis 24f: Verbindungen der Formel (threo-Ixa) und (threo-2-Ixa), worin jeweils Q, (R¹)ₘ und (R²)ₙ wie in Tabelle 1 definiert sind:**

| | |
|---|---|
| 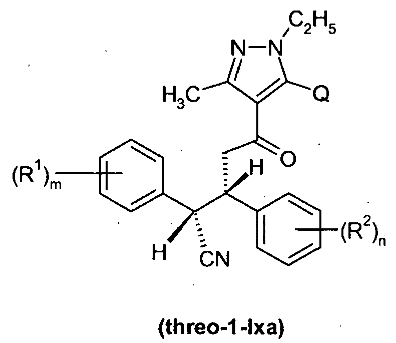 | 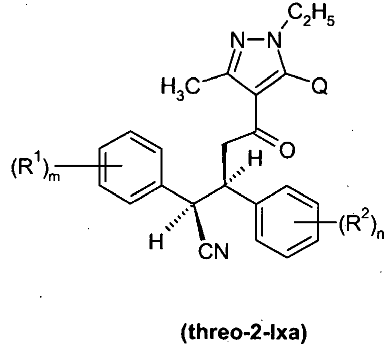 |
| **(threo-Ixa) = (threo-1-Ixa) + (threo-2-Ixa) (50:50) = (rac.)** | |

### Tabelle 24a (threo-Racemate), Beispiele:

Verbindungen der Formel (Ixa) in Form des racemischen Gemischs der threo-Isomeren [= Formel (threo-Ixa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-Ixa(Zeilennummer)".

### Tabelle 24b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-Ixa), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-2-Ixa(Zeilennummer)".

### Tabelle 24c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-Ixa), worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "threo-1-Ixa(Zeilennummer)".

### Tabelle 24d (erythro-Racemate), Beispiele:

Verbindungen der Formel (Ixa) in Form des racemischen Gemischs der erythro-Isomeren [= Formel (erythro-Ixa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-Ixa(Zeilennummer)".

### Tabelle 24e (erythro-1-Enantiomere), Beispiele:

Optisch aktive erythro-1-Enantiomere in angereicherter Form [= (2R,3S)-Form mit mehr als 90%ee] mit der chemischen Struktur der Formel (Ixa) in der erythro-1-Form [= Formel (erythro-1-Ixa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-1-Ixa(Zeilennummer)".

### Tabelle 24f (erythro-2-Enantiomere), Beispiele:

Optisch aktive erythro-2-Enantiomere in angereicherter Form [= (2S,3R)-Form mit mehr als 90%ee] mit der chemischen Struktur der Formel (Ixa) in der erythro-2-Form [= Formel (erythro-2-Ixa)], worin die Strukturkombination der Gruppen Q, (R¹)ₘ und (R²)ₙ gemäß einer Zeilennummer aus der Tabelle 1 definiert ist. Die Nummerierung einzelner Verbindungen erfolgt gemäß "erythro-2-Ixa(Zeilennummer)".

**Tabelle 25: Definitionen von Strukturkombinationen der Gruppen (R¹)ₘ und (R²)ₙ für die nachfolgenden Tabellen von erfindungsgemäßen Verbindungen der allgemeinen Formel (I)**

| Nr. | (R¹)ₘ | (R²)ₙ |
|---|---|---|
| 1 | 3-F | 4-Cl |
| 2 | 3-F | 3-F |
| 3 | 3,4-F₂ | 4-Cl |
| 4 | 3-F | 4-Br |
| 5 | 3-Cl | 3-F |
| 6 | 3,4-F₂ | 3-F |
| 7 | H | 3-F |
| 8 | 4-F | 3-F |
| 9 | 3-F | 3-Cl |
| 10 | 3,4-F₂ | 3-Cl |
| 11 | 3-Br | 3-F |
| 12 | 2,5-F₂ | 3-F |
| 13 | 3-F | 2-F |
| 14 | 3,4,5-F₃ | 4-Cl |
| 15 | 4-F | 3-Cl |
| 16 | 3,4-F₂ | 3-F, 4-Cl |
| 17 | 3,4-F₂ | 2-F |
| 18 | 3-F | 2,3-F₂ |
| 19 | 4-F | 3,5-F₂ |
| 20 | 3-F | 4-F |
| 21 | 3,4-F₂ | 3-Cl, 5-F |
| 22 | 3-F | 3-Cl, 5-F |
| 23 | 3-F | 2,5-F₂ |
| 24 | 3,4-F₂ | 2,3-F₂ |
| 25 | 3,4-F₂ | 2,5-F₂ |
| 26 | 3-CI | 3-Cl, 5-F |
| 27 | 3-F, 4-Cl | 3-Cl |
| 28 | 3,4-F2 | 3-NO₂ |
| 29 | 3,4-F₂ | H |
| 30 | 3-Cl, 4-F | 3-F |
| 31 | 3,4-F₂ | 4-OMe |
| 32 | 3-F | H |
| 33 | 3-F | 2,4-F₂ |
| 34 | 3-F | 2,6-F₂ |
| 35 | 3-F | 3,4-F₂ |
| 36 | 3-F | 3,5-F₂ |
| 37 | 3-F | 2-CI |
| 38 | 3-F | 2,3-Cl₂ |
| 39 | 3-F | 2,4-Cl₂ |
| 40 | 3-F | 3,4-Cl₂ |
| 41 | 3-F | 3,5-Cl₂ |
| 42 | 3,4-F₂ | 2,4-F₂ |
| 43 | 3,4-F₂ | 2,6-F₂ |
| 44 | 3,4-F₂ | 3,4-F₂ |
| 45 | 3,4-F₂ | 3,5-F₂ |
| 46 | 3,4-F₂ | 2,4-Cl₂ |
| 47 | 3,4-F₂ | 2,6-Cl₂ |
| 48 | 3,4-F₂ | 3,5-Cl₂ |
| 49 | 3-Cl | H |
| 50 | 3-Cl | 2-F |
| 51 | 3-Cl | 4-F |
| 52 | 3-Cl | 2,3-F₂ |
| 53 | 3-Cl | 2,5-F₂ |
| 54 | 3-Cl | 2,6-F₂ |
| 55 | 3-Cl | 3,5-F₂ |
| 56 | 3-Cl | 2-Cl |
| 57 | 3-Cl | 3-Cl |
| 58 | 3-Cl | 2,3-Cl₂ |
| 59 | 3-Cl | 2,4-Cl₂ |
| 60 | 3-Cl | 2,5-Cl₂ |
| 61 | 3-Cl | 3,5-Cl₂ |
| 62 | 3,4-Cl₂ | 2,5-F₂ |
| 63 | 3-Cl, 4-F | 3-CI |
| 64 | 3-F, 4-Cl | 2,3-F₂ |
| 65 | 3-F, 4-Cl | 3,5-F₂ |
| 66 | 3,4-F₂ | 3,4-Cl₂ |
| 67 | 3,4-F₂ | 3,5-Cl₂ |
| 68 | 3-Cl | H |
| 69 | 3-Cl | 2,4-F₂ |
| 70 | 3-Cl | 3,4-F₂ |
| 71 | 3-Cl | 4-Cl |
| 72 | 3-Cl | 2,6-Cl₂ |
| 73 | 3,4-Cl₂ | H |
| 74 | 3,4-Cl₂ | 2-F |
| 75 | 3,4-Cl₂ | 3-F |
| 76 | 3,4-Cl₂ | 4-F |
| 77 | 3,4-Cl₂ | 2,6-F₂ |
| 78 | 3,4-Cl₂ | 3,5-F₂ |
| 79 | 3,4-Cl₂ | 2-Cl |
| 80 | 3,4-Cl₂ | 3-Cl |
| 81 | 3,4-Cl₂ | 4-Cl |
| 82 | 3,4-Cl₂ | 2,4-Cl₂ |
| 83 | 3,4-Cl₂ | 2,5-Cl₂ |
| 84 | 3,4-Cl₂ | 2,6-Cl₂ |
| 85 | 3,4-Cl₂ | 3,4-Cl₂ |
| 86 | 3,4-Cl₂ | 3,5-Cl₂ |
| 87 | 3-Cl, 4-F | H |
| 88 | 3-Cl, 4-F | 2-F |
| 89 | 3-Cl, 4-F | 4-F |
| 90 | 3-Cl, 4-F | 2,3-F₂ |
| 91 | 3-Cl, 4-F | 2,4-F₂ |
| 92 | 3-Cl, 4-F | 2,5-F₂ |
| 93 | 3-Cl, 4-F | 2,6-F₂ |
| 94 | 3-Cl, 4-F | 3,4-F₂ |
| 95 | 3-Cl, 4-F | 3,5-F₂ |
| 96 | 3-Cl, 4-F | 2-Cl |
| 97 | 3-Cl, 4-F | 3-Cl |
| 98 | 3-Cl, 4-F | 4-Cl |
| 99 | 3-Cl, 4-F | 2,3-Cl₂ |
| 100 | 3-Cl, 4-F | 2,4-Cl₂ |
| 101 | 3-Cl, 4-F | 2,5-Cl₂ |
| 102 | 3-Cl, 4-F | 2,6-Cl₂ |
| 103 | 3-Cl, 4-F | 3,4-Cl₂ |
| 104 | 3-Cl, 4-F | 3,5-Cl₂ |
| 105 | 3-Cl, 4-F | H |
| 106 | 3-F, 4-Cl | 2-F |
| 107 | 3-F, 4-Cl | 3-F |
| 108 | 3-F, 4-Cl | 4-F |
| 109 | 3-F, 4-Cl | 2,4-F₂ |
| 110 | 3-F, 4-Cl | 2,5-F₂ |
| 111 | 3-F, 4-Cl | 2,6-F₂ |
| 112 | 3-F, 4-Cl | 3,4-F2 |
| 113 | 3-F, 4-Cl | 2-Cl |
| 114 | 3-F, 4-Cl | 4-CI |
| 115 | 3-F, 4-Cl | 2,4-Cl₂ |
| 116 | 3-F, 4-Cl | 2,5-Cl₂ |
| 117 | 3-F, 4-Cl | 2,6-Cl₂ |
| 118 | 3-F, 4-Cl | 3,4-Cl₂ |
| 119 | 3-F, 4-Cl | 3,5-Cl₂ |
| 120 | 3-CN | H |
| 121 | 3-CN | 2,5-F₂ |
| 122 | 3-CN | 2,6-F₂ |
| 123 | 3-CN | 2,3-F₂ |
| 124 | 3-CN | 2,4-F₂ |
| 125 | 3-CN | 3,4-F₂ |
| 126 | 3-CN | 3,5-F₂ |
| 127 | 3-CN | 4-Cl |
| 128 | 3-CN | 3-Cl |
| 129 | 3-CN | 2-Cl |
| 130 | 3-CN | 4-F |
| 131 | 3-CN | 3-F |
| 132 | 3-CN | 2-F |
| 133 | 2-CN | 3-F |
| 134 | 2-CN | 4-F |
| 135 | 2-CN | 4-Cl |
| 136 | 3-NO₂ | H |
| 137 | 3-NO₂ | 2,5-F₂ |
| 138 | 3-NO₂ | 2,6-F₂ |
| 139 | 3-NO₂ | 2,3-F₂ |
| 140 | 3-NO₂ | 2,4-F₂ |
| 141 | 3-NO₂ | 3,4-F₂ |
| 142 | 3-NO₂ | 3,5-F₂ |
| 143 | 3-NO₂ | 4-Cl |
| 144 | 3-NO₂ | 3-Cl |
| 145 | 3-NO₂ | 2-Cl |
| 146 | 3-NO₂ | 4-F |
| 147 | 3-NO₂ | 3-F |
| 148 | 3-NO₂ | 2-F |
| 149 | H | 2,6-F₂ |
| 150 | H | 3,4-F₂ |
| 151 | H | 3,5-F₂ |
| 152 | H | 2,3-F₂ |
| 153 | H | 2,4-F₂ |
| 154 | H | 2,5-F₂ |
| 155 | H | 2-Cl |
| 156 | H | 3-Cl |
| 157 | H | 4-Cl |
| 158 | H | 2-F |
| 159 | H | 4-F |
| 160 | H | 2,3-Cl₂ |
| 161 | H | 2,4-Cl₂ |
| 162 | H | 2,5-Cl₂ |
| 163 | H | 2,6-Cl₂ |
| 164 | H | 3,4-Cl₂ |
| 165 | H | 3,5-Cl₂ |
| 166 | 3-CN, 4-F | 3-F |
| 167 | 3-CN, 4-F | 3-Cl |
| 168 | 3-CN, 4-F | 3-CN |
| 169 | 3-CN, 4-F | 4-F |
| 170 | 3-CN, 4-F | 4-Cl |
| 171 | 3-CN, 4-F | 2,5-F₂ |
| 172 | 3-CN, 4-F | 2,6-F₂ |
| 173 | 3-CN, 4-F | 3-F |
| 174 | 3-CN, 4-F | 3-Cl |
| 175 | 3-CN, 4-F | 3-CN |
| 176 | 3-CN, 4-F | 4-F |
| 177 | 3-CN, 4-F | 4-Cl |
| 178 | 3-CN, 4-F | 2,5-F₂ |
| 179 | 3-CN, 4-F | 2,6-F₂ |
| 180 | 3-Br, 4-F | 3-F |
| 181 | 3-Br, 4-F | 3-Cl |
| 182 | 3-Br, 4-F | 3-CN |
| 183 | 3-Br, 4-F | 4-F |
| 184 | 3-Br, 4-F | 4-Cl |
| 185 | 3-Br, 4-F | 2,5-F₂ |
| 186 | 3-Br, 4-F | 2,6-F₂ |
| 187 | 3-Br, 4-F | 3-F |
| 188 | 3-Br, 4-F | 3-Cl |
| 189 | 3-Br, 4-F | 3-CN |
| 190 | 3-Br, 4-F | 4-F |
| 191 | 3-Br, 4-F | 4-Cl |
| 192 | 3-Br, 4-F | 2,5-F₂ |
| 193 | 3-Br, 4-F | 2,6-F₂ |
| 194 | 3-F, 4-CN | 3-F |
| 195 | 3-F, 4-CN | 3-Cl |
| 196 | 3-F, 4-CN | 3-CN |
| 197 | 3-F, 4-CN | 4-F |
| 198 | 3-F, 4-CN | 4-Cl |
| 199 | 3-F, 4-CN | 2,5-F₂ |
| 200 | 3-F, 4-CN | 2,6-F₂ |
| 201 | 3-F, 4-CN | 3-F |
| 202 | 3-F, 4-CN | 3-Cl |
| 203 | 3-F, 4-CN | 3-CN |
| 204 | 3-F, 4-CN | 4-F |
| 205 | 3-F, 4-CN | 4-Cl |
| 206 | 3-F, 4-CN | 2,5-F₂ |
| 207 | 3-F, 4-CN | 2,6-F₂ |

Zu Referenzzwecken sind in den nachfolgenden Tabellen 26 bis 35 die einzelnen Verbindungen einzelnen Nummern (= Beispielnummern) zugeordnet, wobei die jeweilige Beispielnummer sich zusammensetzt aus der Nummer der chemischen Formel, die der jeweiligen Tabelle zugeordnet ist und einer "Zeilennummer" (Zeilennummer), die sich auf dieselbe Nummer in der Zeile der ersten Spalte der Tabelle 25 bezieht. Die chemische Struktur des Beispiels Nr. "(Formelnummer)(Zeilennummer)" ist damit durch die der jeweiligen Tabelle voran gestellten Formel gemäß Formelnummer und der Zeilennummer aus Tabelle 25 eindeutig definiert, zum Beispiel:
Das Beispiel mit der Nr. "IB8aa1" aus Tabelle 26 ist die Verbindung der Formel (IB8), worin R = Methyl und A¹ = O bedeuten [= Formel (IB8aa), siehe nachstehende Tabelle der Unterformeln zu Formel (IB8)] und (R¹)ₘ = 3-F und (R²)ₙ = 4-CI gemäß der Zeile 1 aus Tabelle 25 definiert ist, oder ein Tautomer davon.
Das Beispiel mit der Nr. "IB8af136" aus Tabelle 26 ist die Verbindung der Formel (IB8), worin R¹ = Methyl und A¹ = N-NH₂ bedeuten [= Formel (IB8af)] und (R¹)ₘ = 3-NO₂ und (R²)ₙ = H (n = 0, unsubstituiert) gemäß der Zeile 136 aus Tabelle 25 definiert ist, oder ein Tautomer davon.

Tabelle 26: Verbindungen der Formel (IB8), worin jeweils (R¹)ₘ und (R²)ₙ wie in Tabelle 25 definiert sind:

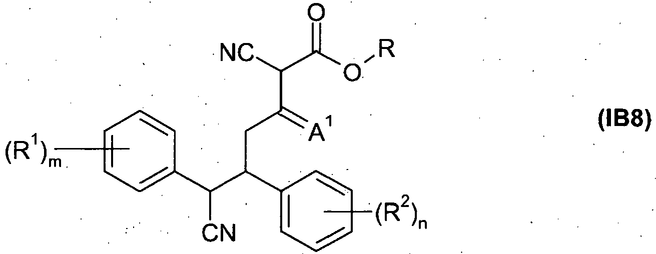

Definition der Unterformeln zur Formel (IB8) mit festgelegten Resten R und A¹:

| Unterformel zu (IB8) | R | A¹ |
|---|---|---|
| (IB8a) | CH₃ | A¹ |
| (IB8aa) | CH₃ | O |
| (IB8ab) | CH₃ | S |
| (IB8ac) | CH₃ | =N-CH₃ |
| (IB8ad) | CH₃ | =N-OH |
| (IB8ae) | CH₃ | =N-OCH₃ |
| (IB8af) | CH₃ | =N-NH₂ |
| (IB8ag) | CH₃ | =N-N(CH₃)₂ |
| (IB8ah) | CH₃ | =N-CH₂-C₆H₅ |
| (IB8b) | C₂H₅ | A¹ |
| (IB8ba) | C₂H₅ | O |
| (IB8bb) | C₂H₅ | S |
| (IB8bc) | C₂H₅ | =N-CH₃ |
| (IB8bd) | C₂H₅ | =N-OH |
| (IB8be) | C₂H₅ | =N-OCH₃ |
| (IB8bf) | C₂H₅ | =N-NH₂ |
| (IB8bg) | C₂H₅ | =N-N(CH₃)₂ |
| (IB8bh) | C₂H₅ | =N-CH₂-C₆H₅ |
| (IB8c) | n-C₃H₇ | A¹ |
| (IB8ca) | n-C₃H₇ | O |
| (IB8cb) | n-C₃H₇ | S |
| (IB8cc) | n-C₃H₇ | =N-CH₃ |
| (IB8cd) | n-C₃H₇ | =N-OH |
| (IB8ce) | n-C₃H₇ | =N-OCH₃ |
| (IB8cf) | n-C₃H₇ | =N-NH₂ |
| (IB8cg) | n-C₃H₇ | =N-N(CH₃)₂ |
| (IB8cH) | n-C₃H₇ | =N-CH₂-C₆H₅ |
| (IB8d) | i-C₃H₇ | A¹ |
| (IB8da) | i-C₃H₇ | O |
| (IB8db) | i-C₃H₇ | S |
| (IB8dc) | i-C₃H₇ | =N-CH₃ |
| (IB8dd) | i-C₃H₇ | =N-OH |
| (IB8de) | i-C₃H₇ | =N-OCH₃ |
| (IB8df) | i-C₃H₇ | =N-NH₂ |
| (IB8dg) | i-C₃H₇ | =N-N(CH₃)₂ |
| (IB8dh) | i-C₃H₇ | =N-CH₂-C₆H₅ |

### Tabelle 26, Beispiele für erythro-threo-Gemische (Mengenverhältnis 70:30 bis 30:70):

Beispiele sind die Verbindungen der betreffenden Formeln (IB8aa) bis (IB8ah), (IB8ba) bis (IB8bh), (IB8ca) bis (IB8ch) und (IB8da) bis (IB8dh), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabellen 26a bis 26f: Verbindungen der Formel (threo-IB8), (threo-1-IB8) und (threo-2-IB8), worin jeweils (R¹)ₘ und (R²)ₙ wie in Tabelle 25 definiert sind:**

| | |
|---|---|
| 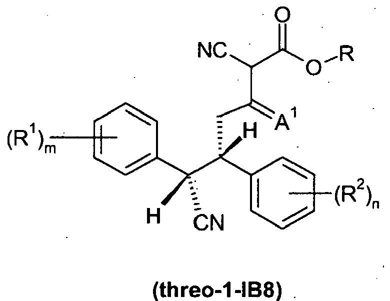 | 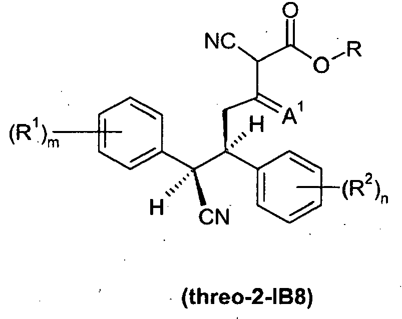 |
| **(threo-IB8) = (threo-1-IB8) + (threo-2-IB8) (50:50) = (rac.)** | |

Definition der Unterformeln zu Formel threo-(IB8), threo-1-(IB8) und threo-2-(IB8) mit festgelegten Resten R und A¹:

| Unterformel | R | A¹ | Stereochemie |
|---|---|---|---|
| threo-(IB8a) | CH₃ | A¹ | (2S,3S)/(2R,3R), racemisch |
| threo-1-(IB8a) | CH₃ | A¹ | (2S,3S), optisch aktiv |
| threo-2-(IB8a) | CH₃ | A¹ | (2R,3R), optisch aktiv |
| threo-(IB8aa) | CH₃ | O | (2S,3S)/(2R,3R), racemisch |
| threo-1-(IB8aa) | CH₃ | O | (2S,3S), optisch aktiv |
| threo-2-(IB8aa) | CH₃ | O | (2R,3R), optisch aktiv |
| threo-(IB8b) | C₂H₅ | A¹ | (2S,3S)/(2R,3R), racemisch |
| threo-1-(IB8b) | C₂H₅ | A¹ | (2S,3S), optisch aktiv |
| threo-2-(IB8b) | C₂H₅ | A¹ | (2R,3R), optisch aktiv |
| threo-(IB8ba) | C₂H₅ | O | (2S,3S)/(2R,3R), racemisch |
| threo-1-(IB8ba) | C₂H₅ | O | (2S,3S), optisch aktiv |
| threo-2-(IB8ba) | C₂H₅ | O | (2R,3R), optisch aktiv |
| threo-(IB8c) | n-C₃H₇ | A¹ | (2S,3S)/(2R,3R), racemisch |
| threo-1-(IB8c) | n-C₃H₇ | A¹ | (2S,3S), optisch aktiv |
| threo-2-(IB8c) | n-C₃H₇ | A¹ | (2R,3R), optisch aktiv |
| threo-(IB8ca) | n-C₃H₇ | O | (2S,3S)/(2R,3R), racemisch |
| threo-1-(IB8ca) | n-C₃H₇ | O | (2S,3S), optisch aktiv |
| threo-2-(IB8ca) | n-C₃H₇ | O | (2R,3R), optisch aktiv |
| threo-(IB8d) | i-C₃H₇ | A¹ | (2S,3S)/(2R,3R), racemisch |
| threo-1-(IB8d) | i-C₃H₇ | A¹ | (2S,3S), optisch aktiv |
| threo-2-(IB8d) | i-C₃H₇ | A¹ | (2R,3R), optisch aktiv |
| threo-(IB8da) | i-C₃H₇ | O | (2S,3S)/(2R,3R), racemisch |
| threo-1-(IB8da) | i-C₃H₇ | O | (2S,3S), optisch aktiv |
| threo-2-(IB8da) | i-C₃H₇ | O | (2R,3R), optisch aktiv |

### Tabelle 26a (threo-Racemate), Beispiele:

Verbindungen der Formeln threo-(IB8aa), threo-(IB8ba), threo-(IB8ca) und threo-(IB8da), jeweils in Form des racemischen Gemischs der threo-Isomeren, worin die Strukturkombination der Gruppen (R¹)ₘ und (R²)ₙ gemäß einer Zeilennummer aus der Tabelle 25 definiert ist.

Die Nummerierung einzelner Verbindungen erfolgt gemäß
"threo-IB8aa(Zeilennummer)", "threo-IB8ba(Zeilennummer)",
"threo-IB8ca(Zeilennummer)" und "threo-IB8da(Zeilennummer)".

### Tabelle 26b (optisch aktive threo-2-Verbindungen), Beispiele:

Verbindungen der Formeln threo-2-(IB8aa), threo-2-(IB8ba), threo-2-(IB8ca) und threo-2-(IB8da), jeweils in stereochemisch angereicherter Form [= (2R,3R)-Form mit mehr als 90%ee], worin die Strukturkombination der Gruppen (R¹)ₘ und (R²)ₙ gemäß einer Zeilennummer aus der Tabelle 25 definiert ist.

Die Nummerierung einzelner Verbindungen erfolgt gemäß
"threo-2-IB8aa(Zeilennummer)", "threo-2-IB8ba(Zeilennummer)",
"threo-2-IB8ca(Zeilennummer)" und "threo-2-IB8da(Zeilennummer)".

### Tabelle 26c (optisch aktive threo-1-Enantiomere), Beispiele:

Optisch aktive enantiomere Verbindungen der Formeln threo-1-(IB8aa), threo-1-(IB8ba), threo-1-(IB8ca) und threo-1-(IB8da), jeweils in stereochemisch angereicherter Form [= (2S,3S)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "threo-1-IB8aa(Zeilennummer)", "threo-1-IB8ba(Zeilennummer)", "threo-1-IB8ca(Zeilennummer)" und "threo-1-IB8da(Zeilennummer)".

### Tabelle 26d (erythro-Racemate), Beispiele:

Verbindungen der Formeln erythro-(IB8aa), erythro-(IB8ba), erythro-(IB8ca) und erythro-(IB8da), jeweils in Form des racemischen Gemischs der erythro-Isomeren, worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-IB8aa(Zeilennummer)", "erythro-IB8ba(Zeilennummer)", "erythro-IB8ca(Zeilennummer)" und "erythro-IB8da(Zeilennummer)".

### Tabelle 26e (erythro-1-Enantiomere), Beispiele:

Optisch aktive enantiomere Verbindungen der Formeln erythro-1-(IB8aa), erythro-1-(IB8ba), erythro-1-(IB8ca) und erythro-1-(IB8da), jeweils in stereochemisch angereicherter Form [= (2R,3S)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-1-IB8aa(Zeilennummer)", "erythro-1-IB8ba(Zeilennummer)", "erythro-1-IB8ca(Zeilennummer)" und "erythro-1-IB8da(Zeilennummer)".

### Tabelle 26f (erythro-2-Enantiomere), Beispiele:

Optisch aktive enantiomere Verbindungen der Formeln erythro-2-(IB8aa), erythro-2-(IB8ba), erythro-2-(IB8ca) und erythro-2-(IB8da), jeweils in stereochemisch angereicherter Form [= (2S,3R)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-2-IB8aa(Zeilennummer)", "erythro-2-IB8ba(Zeilennummer)", "erythro-2-IB8ca(Zeilennummer)" und "erythro-2-IB8da(Zeilennummer)".

Tabelle 27: Verbindungen der Formel (IBX), worin jeweils (R¹)ₘ und (R²)ₙ wie in Tabelle 25 definiert sind:

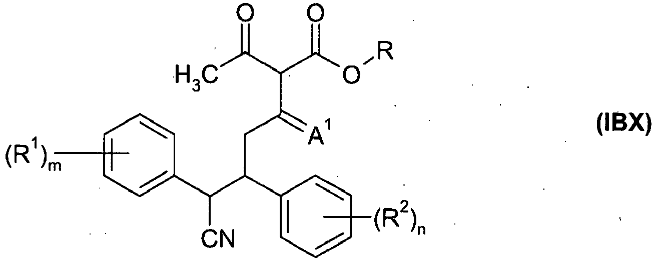

Definition der Unterformeln zu Formel (IBX) mit festgelegten Resten R und A¹:

| Unterformel zu (IBX) | R | A¹ |
|---|---|---|
| (IBXa) | CH₃ | A¹ |
| (IBXaa) | CH₃ | O |
| (IBXab) | CH₃ | S |
| (IBXac) | CH₃ | =N-CH₃ |
| (IBXad) | CH₃ | =N-OH |
| (IBXae) | CH₃ | =N-OCH₃ |
| (IBXaf) | CH₃ | =N-NH₂ |
| (IBXag) | CH₃ | =N-N(CH₃)₂ |
| (IBXah) | CH₃ | =N-CH₂-C₆H₅ |
| (IBXb) | C₂H₅ | A¹ |
| (IBXba) | C₂H₅ | O |
| (IBXbb) | C₂H₅ | S |
| (IBXbc) | C₂H₅ | =N-CH₃ |
| (IBXbd) | C₂H₅ | =N-OH |
| (IBXbe) | C₂H₅ | =N-OCH₃ |
| (IBXbf) | C₂H₅ | =N-NH₂ |
| (IBXbg) | C₂H₅ | =N-N(CH₃)₂ |
| (IBXbh) | C₂H₅ | =N-CH₂-C₆H₅ |
| (IBXc) | n-C₃H₇ | A¹ |
| (IBXca) | n-C₃H₇ | O |
| (IBXcb) | n-C₃H₇ | S |
| (IBXcc) | n-C₃H₇ | =N-CH₃ |
| (IBXcd) | n-C₃H₇ | =N-OH |
| (IBXce) | n-C₃H₇ | =N-OCH₃ |
| (IBXcf) | n-C₃H₇ | =N-NH₂ |
| (IBXcg) | n-C₃H₇ | =N-N(CH₃)₂ |
| (IBXch) | n-C₃H₇ | =N-CH₂-C₆H₅ |
| (IBXd) | i-C₃H₇ | A¹ |
| (IBXda) | i-C₃H₇ | O |
| (IBXdb) | i-C₃H₇ | S |
| (IBXdc) | i-C₃H₇ | =N-CH₃ |
| (IBXdd) | i-C₃H₇ | =N-OH |
| (IBXde) | i-C₃H₇ | =N-OCH₃ |
| (IBXdf) | i-C₃H₇ | =N-NH₂ |
| (IBXdg) | i-C₃H₇ | =N-N(CH₃)₂ |
| (IBXdh) | i-C₃H₇ | =N-CH₂-C₆H₅ |

### Tabelle 27, Beispiele für erythro-threo-Gemische (Mengenverhältnis 70:30 bis 30:70):

Beispiele sind die Verbindungen der betreffenden Formeln (IBXaa) bis (IBXah), (IBXba) bis (IBXbh), (IBXca) bis (IBXch) und (IBXda) bis (IBXdh), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabellen 27a bis 27f: Verbindungen der Formel (threo-IBX), (threo-1-IBX) und (threo-2-IBX), worin jeweils (R¹)ₘ und (R²)ₙ wie in Tabelle 25 definiert sind:**

| | |
|---|---|
| 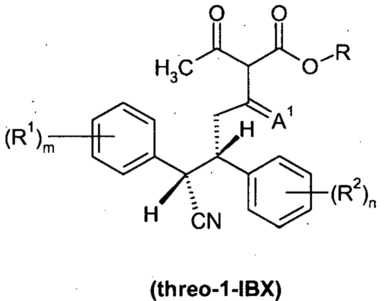 | 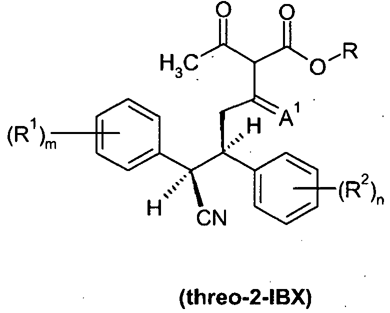 |
| **(threo-IBX) = (threo-1-IBX) + (threo-2-IBX) (50:50) = (rac.)** | |

Definition der Unterformeln zu Formel threo-(IBX), threo-1-(IBX) und threo-2-(IBX) mit festgelegten Resten R und A¹:

| Unterformel | R | A¹ | Stereochemie |
|---|---|---|---|
| threo-(IBXa) | CH₃ | A¹ | (2S,3S)/(2R,3R), racemisch |
| threo-1-(IBXa) | CH₃ | A¹ | (2S,3S), optisch aktiv |
| threo-2-(IBXa) | CH₃ | A¹ | (2R,3R), optisch aktiv |
| threo-(IBXaa) | CH₃ | O | (2S,3S)/(2R,3R), racemisch |
| threo-1-(IBXaa) | CH₃ | O | (2S,3S), optisch aktiv |
| threo-2-(IBXaa) | CH₃ | O | (2R,3R), optisch aktiv |
| threo-(IBXb) | C₂H₅ | A¹ | (2S,3S)/(2R,3R), racemisch |
| threo-1-(IBXb) | C₂H₅ | A¹ | (2S,3S), optisch aktiv |
| threo-2-(IBXb) | C₂H₅ | A¹ | (2R,3R), optisch aktiv |
| threo-(IBXba) | C₂H₅ | O | (2S,3S)/(2R,3R), racemisch |
| threo-1-(IBXba) | C₂H₅ | O | (2S,3S), optisch aktiv |
| threo-2-(IBXba) | C₂H₅ | O | (2R,3R), optisch aktiv |
| threo-(IBXc) | n-C₃H₇ | A¹ | (2S,3S)/(2R,3R), racemisch |
| threo-1-(IBXc) | n-C₃H₇ | A¹ | (2S,3S), optisch aktiv |
| threo-2-(IBXc) | n-C₃H₇ | A¹ | (2R,3R), optisch aktiv |
| threo-(IBXca) | n-C₃H₇ | O | (2S,3S)/(2R,3R), racemisch |
| threo-1-(IBXca) | n-C₃H₇ | O | (2S,3S), optisch aktiv |
| threo-2-(IBXca) | n-C₃H₇ | O | (2R,3R), optisch aktiv |
| threo-(IBXd) | i-C₃H₇ | A¹ | (2S,3S)/(2R,3R), racemisch |
| threo-1-(IBXd) | i-C₃H₇ | A¹ | (2S,3S), optisch aktiv |
| threo-2-(IBXd) | i-C₃H₇ | A¹ | (2R,3R), optisch aktiv |
| threo-(IBXda) | i-C₃H₇ | O | (2S,3S)/(2R,3R), racemisch |
| threo-1-(IBXda) | i-C₃H₇ | O | (2S,3S), optisch aktiv |
| threo-2-(IBXda) | i-C₃H₇ | O | (2R,3R), optisch aktiv |

### Tabelle 27a (threo-Racemate), Beispiele:

Verbindungen der Formeln threo-(IBXaa), threo-(IBXba), threo-(IBXca) und threo-(IBXda), jeweils in Form des racemischen Gemischs der threo-Isomeren, worin die Strukturkombination der Gruppen (R¹)ₘ und (R²)ₙ gemäß einer Zeilennummer aus der Tabelle 25 definiert ist.

Die Nummerierung einzelner Verbindungen erfolgt gemäß "threo-IBXaa(Zeilennummer)", "threo-IBXba(Zeilennummer)", "threo-IBXca(Zeilennummer)" und "threo-IBXda(Zeilennummer)".

### Tabelle 27b (optisch aktive threo-2-Verbindungen), Beispiele:

Verbindungen der Formeln threo-2-(IBXaa), threo-2-(IBXba), threo-2-(IBXca) und threo-2-(IBXda), jeweils in stereochemisch angereicherter Form [= (2R,3R)-Form mit mehr als 90%ee], worin die Strukturkombination der Gruppen (R¹)ₘ und (R₂)ₙ gemäß einer Zeilennummer aus der Tabelle 25 definiert ist.

Die Nummerierung einzelner Verbindungen erfolgt gemäß "threo-2-IBXaa(Zeilennummer)", "threo-2-IBXba(Zeilennummer)", "threo-2-IBXca(Zeilennummer)" und "threo-2-IBXda(Zeilennummer)".

### Tabelle 27c (optisch aktive threo-1-Enantiomere), Beispiele:

Optisch aktive enantiomere Verbindungen der Formeln threo-1-(IBXaa), threo-1-(IBXba), threo-1-(IBXca) und threo-1-(IBXda), jeweils in stereochemisch angereicherter Form [= (2S,3S)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "threo-1-IBXaa(Zeilennummer)", "threo-1-IBXba(Zeilennummer)", "threo-1-IBXca(Zeilennummer)" und "threo-1-IBXda(Zeilennummer)".

### Tabelle 27d (erythro-Racemate), Beispiele:

Verbindungen der Formeln erythro-(IBXaa), erythro-(IBXba), erythro-(IBXca) und erythro-(IBXda), jeweils in Form des racemischen Gemischs der erythro-Isomeren, worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-IBXaa(Zeilennummer)", "erythro-IBXba(Zeilennummer)", "erythro-IBXca(Zeilennummer)" und "erythro-IBXda(Zeilennummer)".

### Tabelle 27e (erythro-1-Enantiomere), Beispiele:

Optisch aktive enantiomere Verbindungen der Formeln erythro-1-(IBXaa), erythro-1-(IBXba), erythro-1-(IBXca) und erythro-1-(IBXda), jeweils in stereochemisch angereicherter Form [= (2R,3S)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-1-IBXaa(Zeilennummer)", "erythro-1-IBXba(Zeilennummer)", "erythro-1-IBXca(Zeilennummer)" und "erythro-1-IBXda(Zeilennummer)".

### Tabelle 27f (erythro-2-Enantiomere), Beispiele:

Optisch aktive enantiomere Verbindungen der Formeln erythro-2-(IBXaa), erythro-2-(IBXba), erythro-2-(IBXca) und erythro-2-(IBXda), jeweils in stereochemisch angereicherter Form [= (2S,3R)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-2-IBXaa(Zeilennummer)", "erythro-2-IBXba(Zeilennummer)", "erythro-2-IBXca(Zeilennummer)" und "erythro-2-IBXda(Zeilennummer)".

Tabelle 28: Verbindungen der Formel (IB9), worin jeweils (R¹)ₘ und (R²)ₙ wie in Tabelle 25 definiert sind:

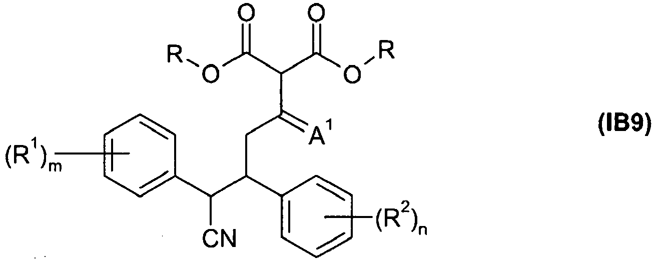

Definition der Unterformeln zu Formel (IB9) mit festgelegten Resten R und A¹:

| Unterformel zu (IB9) | R | A¹ |
|---|---|---|
| (IB9a) | CH₃ | A¹ |
| (IB9aa) | CH₃ | O |
| (IB9ab) | CH₃ | S |
| (IB9ac) | CH₃ | =N-CH₃ |
| (IB9ad) | CH₃ | =N-OH |
| (IB9ae) | CH₃ | =N-OCH₃ |
| (IB9af) | CH₃ | =N-NH₂ |
| (IB9ag) | CH₃ | =N-N(CH₃)₂ |
| (IB9ah) | CH₃ | =N-CH₂-C₆H₅ |
| (IB9b) | C₂H₅ | A¹ |
| (IB9ba) | C₂H₅ | O |
| (IB9bb) | C₂H₅ | S |
| (IB9bc) | C₂H₅ | =N-CH₃ |
| (IB9bd) | C₂H₅ | =N-OH |
| (IB9be) | C₂H₅ | =N-OCH₃ |
| (IB9bf) | C₂H₅ | =N-NH₂ |
| (IB9bg) | C₂H₅ | =N-N(CH₃)₂ |
| (IB9bh) | C₂H₅ | =N-CH₂-C₆H₅ |
| (IB9c) | n-C₃H₇ | A¹ |
| (IB9ca) | n-C₃H₇ | O |
| (IB9cb) | n-C₃H₇ | S |
| (IB9cc) | n-C₃H₇ | =N-CH₃ |
| (IB9cd) | n-C₃H₇ | =N-OH |
| (IB9ce) | n-C₃H₇ | =N-OCH₃ |
| (IB9cf) | n-C₃H₇ | =N-NH₂ |
| (IB9cg) | n-C₃H₇ | =N-N(CH₃)₂ |
| (IB9ch) | n-C₃H₇ | =N-CH₂-C₆H₅ |
| (IB9d) | i-C₃H₇ | A¹ |
| (IB9da) | i-C₃H₇ | O |
| (IB9db) | i-C₃H₇ | S |
| (IB9dc) | i-C₃H₇ | =N-CH₃ |
| (IB9dd) | i-C₃H₇ | =N-OH |
| (IB9de) | i-C₃H₇ | =N-OCH₃ |
| (IB9df) | i-C₃H₇ | =N-NH₂ |
| (IB9dg) | i-C₃H₇ | =N-N(CH₃)₂ |
| (IB9dh) | i-C₃H₇ | =N-CH₂-C₆H₅ |

### Tabelle 27, Beispiele für erythro-threo-Gemische (Mengenverhältnis 70:30 bis 30:70):

Beispiele sind die Verbindungen der betreffenden Formeln (IB9aa) bis (IB9ah), (IB9ba) bis (IB9bh), (IB9ca) bis (IB9ch) und (IB9da) bis (IB9dh), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabellen 28a bis 28f: Verbindungen der Formel (threo-IB9), (threo-1-IB9) und (threo-2-IB9), worin jeweils (R¹)ₘ und (R²)ₙ wie in Tabelle 25 definiert sind:**

| | |
|---|---|
| 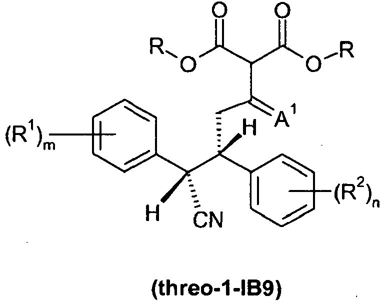 | 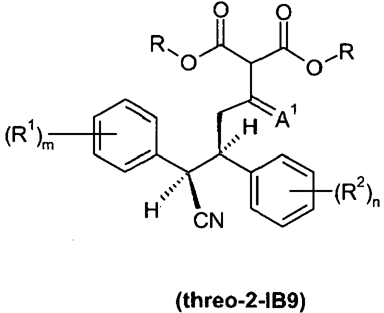 |
| **(threo-IB9) = (threo-1-IB9) + (threo-2-IB9) (50:50) = (rac.)** | |

Definition der Unterformeln zu Formel threo-(IB9), threo-1-(IB9) und threo-2-(IB9) mit festgelegten Resten R und A¹:

| Unterformel | R | A¹ | Stereochemie |
|---|---|---|---|
| threo-(IB9a) | CH₃ | A¹ | (2S,3S)/(2R,3R), racemisch |
| threo-1-(IB9a) | CH₃ | A¹ | (2S,3S), optisch aktiv |
| threo-2-(IB9a) | CH₃ | A¹ | (2R,3R), optisch aktiv |
| threo-(IB9aa) | CH₃ | O | (2S,3S)/(2R,3R), racemisch |
| threo-1-(IB9aa) | CH₃ | O | (2S,3S), optisch aktiv |
| threo-2-(IB9aa) | CH₃ | O | (2R,3R), optisch aktiv |
| threo-(IB9b) | C₂H₅ | A¹ | (2S,3S)/(2R,3R), racemisch |
| threo-1-(IB9b) | C₂H₅ | A¹ | (2S,3S), optisch aktiv |
| threo-2-(IB9b) | C₂H₅ | A¹ | (2R,3R), optisch aktiv |
| threo-(IB9ba) | C₂H₅ | O | (2S,3S)/(2R,3R), racemisch |
| threo-1-(IB9ba) | C₂H₅ | O | (2S,3S), optisch aktiv |
| threo-2-(IB9ba) | C₂H₅ | O | (2R,3R), optisch aktiv |
| threo-(IB9c) | n-C₃H₇ | A¹ | (2S,3S)/(2R,3R), racemisch |
| threo-1-(IB9c) | n-C₃H₇ | A¹ | (2S,3S), optisch aktiv |
| threo-2-(IB9c) | n-C₃H₇ | A¹ | (2R,3R), optisch aktiv |
| threo-(IB9ca) | n-C₃H₇ | O | (2S,3S)/(2R,3R), racemisch |
| threo-1-(IB9ca) | n-C₃H₇ | O | (2S,3S), optisch aktiv |
| threo-2-(IB9ca) | n-C₃H₇ | O | (2R,3R), optisch aktiv |
| threo-(IB9d) | i-C₃H₇ | A¹ | (2S,3S)/(2R,3R), racemisch |
| threo-1-(IB9d) | i-C₃H₇ | A¹ | (2S,3S), optisch aktiv |
| threo-2-(IB9d) | i-C₃H₇ | A¹ | (2R,3R), optisch aktiv |
| threo-(IB9da) | i-C₃H₇ | O | (2S,3S)/(2R,3R), racemisch |
| threo-1-(IB9da) | i-C₃H₇ | O | (2S,3S), optisch aktiv |
| threo-2-(IB9da) | i-C₃H₇ | O | (2R,3R), optisch aktiv |

### Tabelle 28a (threo-Racemate), Beispiele:

Verbindungen der Formeln threo-(IB9aa), threo-(IB9ba), threo-(IB9ca) und threo-(IB9da), jeweils in Form des racemischen Gemischs der threo-Isomeren, worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "threo-IB9aa(Zeilennummer)", "threo-IB9ba(Zeilennummer)", "threo-IB9ca(Zeilennummer)" und "threo-IB9da(Zeilennummer)".

### Tabelle 28b (optisch aktive threo-2-Verbindungen), Beispiele:

Verbindungen der Formeln threo-2-(IB9aa), threo-2-(IB9ba), threo-2-(IB9ca) und threo-2-(IB9da), jeweils in stereochemisch angereicherter Form [= (2R,3R)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "threo-2-IB9aa(Zeilennummer)", "threo-2-IB9ba(Zeilennummer)", "threo-2-IB9ca(Zeilennummer)" und "threo-2-IB9da(Zeilennummer)".

### Tabelle 28c (optisch aktive threo-1-Enantiomere), Beispiele:

Optisch aktive enantiomere Verbindungen der Formeln threo-1-(IB9aa), threo-1-(IB9ba), threo-1-(IB9ca) und threo-1-(IB9da), jeweils in stereochemisch angereicherter Form [= (2S,3S)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "threo-1-IB9aa(Zeilennummer)", "threo-1-IB9ba(Zeilennummer)", "threo-1-IB9ca(Zeilennummer)" und "threo-1-IB9da(Zeilennummer)".

### Tabelle 28d (erythro-Racemate), Beispiele:

Verbindungen der Formeln erythro-(IB9aa), erythro-(IB9ba), erythro-(IB9ca) und erythro-(IB9da), jeweils in Form des racemischen Gemischs der erythro-Isomeren, worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-IB9aa(Zeilennummer)", "erythro-IB9ba(Zeilennummer)", "erythro-IB9ca(Zeilennummer)" und "erythro-IB9da(Zeilennummer)".

### Tabelle 28e (erythro-1-Enantiomere), Beispiele:

Optisch aktive enantiomere Verbindungen der Formel einzelner Verbindungen erythro-1-(IB9aa), erythro-1-(IB9ba), erythro-1-(IB9ca) und erythro-1-(IB9da), jeweils in stereochemisch angereicherter Form [= (2R,3S)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-1-IB9aa(Zeilennummer)", "erythro-1-IB9ba(Zeilennummer)", "erythro-1-IB9ca(Zeilennummer)" und "erythro-1-IB9da(Zeilennummer)".

### Tabelle 28f (erythro-2-Enantiomere), Beispiele:

Optisch aktive enantiomere Verbindungen der Formeln erythro-2-(IB9aa), erythro-2-(IB9ba), erythro-2-(IB9ca) und erythro-2-(IB9da), jeweils in stereochemisch angereicherter Form [= (2S,3R)-Form mit mehr als 90%ee], worin die Strukturkombination der Gruppen (R¹)ₘ und (R²)ₙ gemäß einer Zeilennummer aus der Tabelle 25 definiert ist. Die Nummerierung einzelner Verbindungen erfolgt gemäß "erythro-2-IB9aa(Zeilennummer)", "erythro-2-IB9ba(Zeilennummer)", "erythro-2-IB9ca(Zeilennummer)" und "erythro-2-IB9da(Zeilennummer)".

**Tabelle 29: Verbindungen der Formel (IB10a), (IB10aa), (IB10ab), (IB10ac), (IB10ad), (IB10ae), (IB10af), (IB10ag) und (IB10ah), worin jeweils (R¹)ₘ und (R²)ₙ wie in Tabelle 25 definiert sind:**

| | |
|---|---|
| 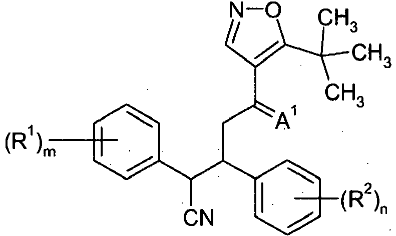 | **(IB10a)** |
| | **(IB10aa) A¹ ist (=O)** |
| | **(IB10ab) A¹ ist (=S)** |
| | **(IB10ac) A¹ ist (=N-CH₃)** |
| | **(IB10ad) A¹ ist (=N-OH)** |
| | **(IB10ae) A¹ ist (=N-OCH₃)** |
| | **(IB10af) A¹ ist (=N-NH₂)** |
| | **(IB10ag) A¹ ist [=N-N(CH₃)₂]** |
| | **(IB10ah) A¹ ist (=N-Benzyl)** |

### Tabelle 29, Beispiele für erythro-threo-Gemische (Mengenverhältnis 70:30 bis 30:70):

Beispiele sind die Verbindungen der betreffenden Formeln (IB10aa) bis (IB10ah), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabellen 29a bis 29f: Verbindungen der Formel (threo-IB10aa) und (threo-2-IB10aa), worin jeweils (R¹)ₘ und (R²)ₙ wie in Tabelle 25 definiert sind:**

| | |
|---|---|
| 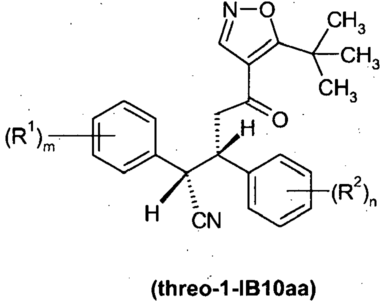 | 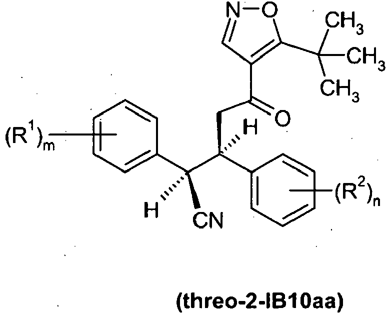 |
| **(threo-IB10aa) = (threo-1-IB10aa) + (threo-2-IB10aa) (50:50) = (rac.)** | |

### Tabelle 29a (threo-Racemate), Beispiele:

Verbindungen der Formel (IB10aa) in Form des racemischen Gemischs der threo-Isomeren [= Formel (threo-IB10aa)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-IB10aa(Zeilennummer)".

### Tabelle 29b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-IB10aa), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-2-IB10aa(Zeilennummer)".

### Tabelle 29c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-IB10aa), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-1-IB10aa(Zeilennummer)".

### Tabelle 29d (erythro-Racemate), Beispiele:

Verbindungen der Formel (IB10aa) in Form des racemischen Gemischs der erythro-Isomeren [= Formel (erythro-IB10aa)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-IB10aa(Zeilennummer)".

### Tabelle 29e (erythro-1-Enantiomere), Beispiele:

Optisch aktive erythro-1-Enantiomere in angereicherter Form [= (2R,3S)-Form mit mehr als 90%ee] mit der chemischen Struktur der Formel (IB10aa) in der erythro-1-Form [= Formel (erythro-1-IB10aa)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-1-IB10aa(Zeilennummer)".

### Tabelle 29f (erythro-2-Enantiomere), Beispiele:

Optisch aktive erythro-2-Enantiomere in angereicherter Form [= (2S,3R)-Form mit mehr als 90%ee] mit der chemischen Struktur der Formel (IB10aa) in der erythro-2-Form [= Formel (erythro-2-IB10aa)], worin die Strukturkombination der Gruppen Q, (R¹)ₘ und (R²)ₙ gemäß einer Zeilennummer aus der Tabelle 25 definiert ist. Die Nummerierung erfolgt gemäß "erythro-2-IB10aa(Zeilennummer)".

**Tabelle 30: Verbindungen der Formel (IB10b), (IB10ba), (IB10bb), (IB10bc), (IB10bd), (IB10be), (IB10bf), (IB10bg) und (IB10bh), worin jeweils (R¹)ₘ und (R²)ₙ wie in Tabelle 25 definiert sind**

| | |
|---|---|
| 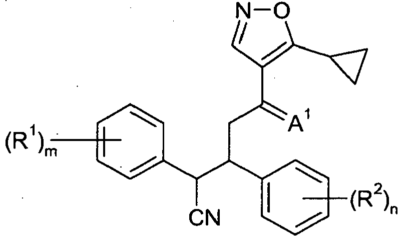 | **(IB10b)** |
| | **(IB10ba) A¹ ist (=O)** |
| | **(IB10bb) A¹ ist (=S)** |
| | **(IB10bc) A¹ ist (=N-CH₃)** |
| | **(IB10bd) A¹ ist (=N-OH)** |
| | **(IB10be) A¹ ist (=N-OCH₃)** |
| | **(IB10bf) A¹ ist (=N-NH₂)** |
| | **(IB10bg) A¹ ist [=N-N(CH₃)₂]** |
| | **(IB10bh) A¹ ist (=N-Benzyl)** |

### Tabelle 30, Beispiele für erythro-threo-Gemische (Mengenverhältnis 70:30 bis 30:70):

Beispiele sind die Verbindungen der betreffenden Formeln (IB10ba) bis (IB10bh), jeweils in Form eines racemischen erythro-threo-Gemischs (Mengenverhältnis 70:30 bis 30:70), worin die Bedeutung von (R¹)ₘ und (R¹)ₙ gemäß einer-Zeilennummer aus der Tabelle 25 definiert ist. Die Nummerierung einzelner Verbindungen erfolgt gemäß "(Formel)(Zeilennummer)" ohne Schreiben der Klammern.

**Tabellen 30a bis 30f: Verbindungen der Formel (threo-IB10ba) und (threo-2-IB10ba), worin jeweils (R¹)ₘ und (R²)ₙ wie in Tabelle 25 definiert sind:**

| | |
|---|---|
| 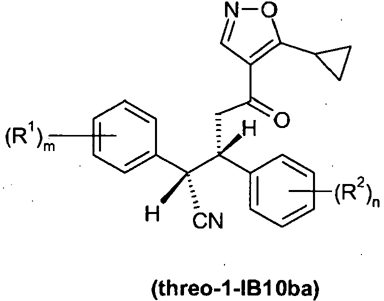 | 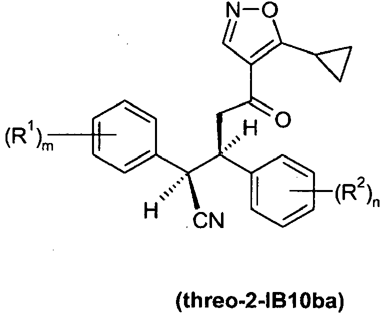 |
| **(threo-IB10ba) = (threo-1-IB10ba) + (threo-2-IB10ba) (50:50) = (rac.)** | |

### Tabelle 30a (threo-Racemate), Beispiele:

Verbindungen der Formel (IB10ba) in Form des racemischen Gemischs der threo-Isomeren [= Formel (threo-IB10ba)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-IB10ba(Zeilennummer)".

### Tabelle 30b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-IB10ba), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-2-IB10ba(Zeilennummer)".

### Tabelle 30c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-IB10ba), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-1-IB10ba(Zeilennummer)".

### Tabelle 30d (erythro-Racemate), Beispiele:

Verbindungen der Formel (IB10ba) in Form des racemischen Gemischs der erythro-Isomeren [= Formel (erythro-IB10ba)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-IB10ba(Zeilennummer)".

### Tabelle 30e (erythro-1-Enantiomere), Beispiele:

Optisch aktive erythro-1-Enantiomere in angereicherter Form [= (2R,3S)-Form mit mehr als 90%ee] mit der chemischen Struktur der Formel (IB10ba) in der erythro-1-Form [= Formel (erythro-1-IB10ba)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-1-IB10ba(Zeilennummer)".

### Tabelle 30f (erythro-2-Enantiomere), Beispiele:

Optisch aktive erythro-2-Enantiomere in angereicherter Form [= (2S,3R)-Form mit mehr als 90%ee] mit der chemischen Struktur der Formel (IB10ba) in der erythro-2-Form [= Formel (erythro-2-IB10ba)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-2-IB10ba(Zeilennummer)".

**Tabelle 31: Verbindungen der Formel (IB10c), (IB10ca), (IB10cb), (IB10cc), (IB10cd), (IB10ce), (IB10cf), (IB10cg) und (IB10ch), worin jeweils (R¹)ₘ und (R²)ₙ wie in Tabelle 25 definiert sind**

| | |
|---|---|
| 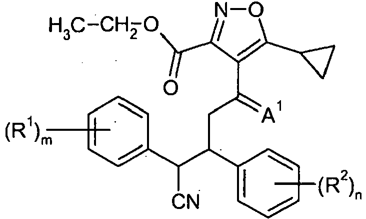 | **(IB10c)** |
| | **(IB10ca) A¹ ist (=O)** |
| | **(IB10cb) A¹ ist (=S)** |
| | **(IB10cc) A¹ ist (=N-CH₃)** |
| | **(IB10cd) A¹ ist (=N-OH)** |
| | **(IB10ce) A¹ ist (=N-OCH₃)** |
| | **(IB10cf) A¹ ist (=N-NH₂)** |
| | **(IB10cg) A¹ ist [=N-N(CH₃)₂]** |
| | **(IB10ch) A¹ ist (=N-Benzyl)** |

### Tabelle 31, Beispiele für erythro-threo-Gemische (Mengenverhältnis 70:30 bis 30:70):

Beispiele sind die Verbindungen der betreffenden Formeln (IB10ca) bis (IB10ch), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabellen 31a bis 31f: Verbindungen der Formel (threo-IB10ca) und (threo-2-IB10ca), worin jeweils (R¹)ₘ und (R²)ₙ wie in Tabelle 25 definiert sind:**

| | |
|---|---|
| 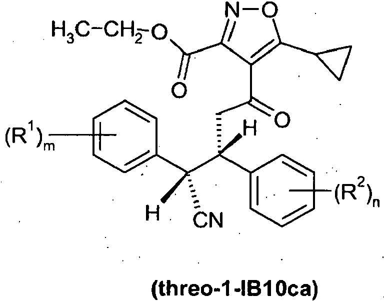 | 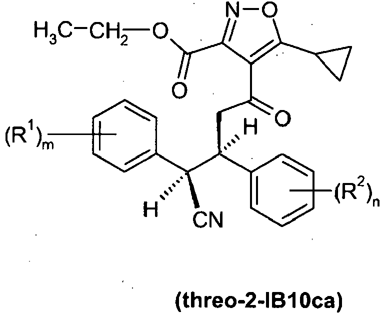 |
| **(threo-IB10ca) = (threo-1-IB10ca) + (threo-2-IB10ca) (50:50) = (rac.)** | |

### Tabelle 31 a (threo-Racemate), Beispiele:

Verbindungen der Formel (IB10ca) in Form des racemischen Gemischs der threo-Isomeren [= Formel (threo-IB10ca)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-IB10ca(Zeilennummer)".

### Tabelle 31 b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-IB10ca), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-2-IB10ca(Zeilennummer)".

### Tabelle 31c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-IB10ca), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-1-IB10ca(Zeilennummer)".

### Tabelle 31d (erythro-Racemate), Beispiele:

Verbindungen der Formel (IB10ca) in Form des racemischen Gemischs der erythro-Isomeren [= Formel (erythro-IB10ca)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-IB10ca(Zeilennummer)".

### Tabelle 31 e (erythro-1-Enantiomere), Beispiele:

Optisch aktive erythro-1-Enantiomere in angereicherter Form [= (2R,3S)-Form mit mehr als 90%ee] mit der chemischen Struktur der Formel (IB10ca) in der erythro-1-Form [= Formel (erythro-1-IB10ca)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-1-IB10ca(Zeilennummer)".

### Tabelle 31f (erythro-2-Enantiomere), Beispiele:

Optisch aktive erythro-2-Enantiomere in angereicherter Form [= (2S,3R)-Form mit mehr als 90%ee] mit der chemischen Struktur der Formel (IB10ca) in der erythro-2-Form [= Formel (erythro-2-IB10ca)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-2-IB10ca(Zeilennummer)".

**Tabelle 32: Verbindungen der Formel (IB10d), (IB10da), (IB10db), (IB10dc), (IB10dd), (IB10de), (IB10df), (IB10dg) und (IB10dh), worin jeweils (R¹)ₘ und (R²)ₙ wie in Tabelle 25 definiert sind**

| | |
|---|---|
| 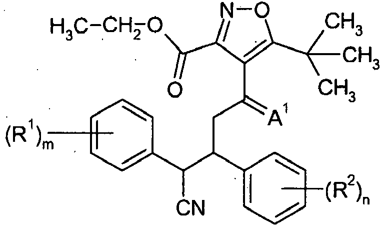 | **(IB10d)** |
| | **(IB10da) A¹ ist (=O)** |
| | **(IB10db) A¹ ist (=S)** |
| | **(IB10dc) A¹ ist (=N-CH₃)** |
| | **(IB10dd) A¹ ist (=N-OH)** |
| | **(IB10de) A¹ ist (=N-OCH₃)** |
| | **(IB10df) A¹ ist (=N-NH₂)** |
| | **(IB10dg) A¹ ist [=N-N(CH₃)₂]** |
| | **(IB10dh) A¹ ist (=N-Benzyl)** |

### Tabelle 32, Beispiele für erythro-threo-Gemische (Mengenverhältnis 70:30 bis 30:70):

Beispiele sind die Verbindungen der betreffenden Formeln (IB10da) bis (IB10dh), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabellen 32a bis 32f: Verbindungen der Formel (threo-IB10da) und (threo-2-IB10da), worin jeweils (R¹)ₘ und (R²)ₙ wie in Tabelle 25 definiert sind:**

| | |
|---|---|
| 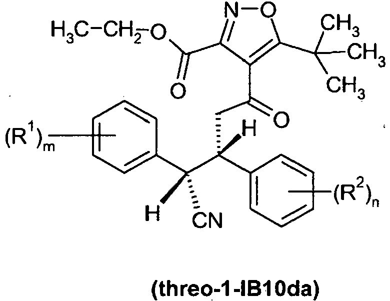 | 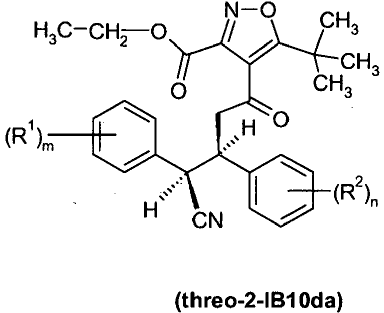 |
| **(threo-IB10da) = (threo-1-IB10da) + (threo-2-IB10da) (50:50) = (rac.)** | |

### Tabelle 32a (threo-Racemate), Beispiele:

Verbindungen der Formel (IB10da) in Form des racemischen Gemischs der threo-Isomeren [= Formel (threo-IB10da)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-IB10da (Zeilennummer)".

### Tabelle 32b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2=IB10da), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-2-IB10da(Zeilennummer)".

### Tabelle 32c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-IB10da), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-1-IB10da(Zeilennummer)".

### Tabelle 32d (erythro-Racemate), Beispiele:

Verbindungen der Formel (IB10da) in Form des racemischen Gemischs der erythro-Isomeren [= Formel (erythro-IB10da)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-IB10da(Zeilennummer)".

### Tabelle 32e (erythro-1-Enantiomere), Beispiele:

Optisch aktive erythro-1-Enantiomere in angereicherter Form [= (2R,3S)-Form mit mehr als 90%ee] mit der chemischen Struktur der Formel (IB10da) in der erythro-1-Form [= Formel (erythro-1-IB10da)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-1-IB10da(Zeilennummer)".

### Tabelle 32f (erythro-2-Enantiomere), Beispiele:

Optisch aktive erythro-2-Enantiomere in angereicherter Form [= (2S,3R)-Form mit mehr als 90%ee] mit der chemischen Struktur der Formel (IB10da) in der erythro-2-Form [= Formel (erythro-2-IB10da)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-2-IB10da(Zeilennummer)".

**Tabelle 33: Verbindungen der Formel (IB10e), (IB10ea), (IB10eb), (IB10ec), (IB10ed), (IB10ee), (IB10ef), (IB10eg) und (IB10eh), worin jeweils (R¹)ₘ und (R²)ₙ wie in Tabelle 25 definiert sind**

| | |
|---|---|
| 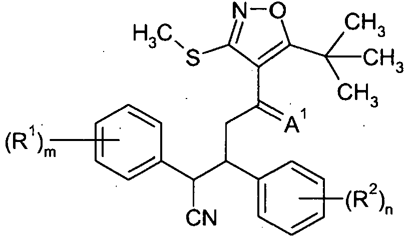 | **(IB10e)** |
| | **(IB10ea) A¹ ist (=O)** |
| | **(IB10eb) A¹ ist (=S)** |
| | **(IB10ec) A¹ ist (=N-CH₃)** |
| | **(IB10ed) A¹ ist (=N-OH)** |
| | **(IB10ee) A¹ ist (=N-OCH₃)** |
| | **(IB10ef) A¹ ist (=N-NH₂)** |
| | **(IB10eg) A¹ ist [=N-N(CH₃)₂]** |
| | **(IB10eh) A¹ ist (=N-Benzyl)** |

### Tabelle 33, Beispiele für erythro-threo-Gemische (Mengenverhältnis 70:30 bis 30:70):

Beispiele sind die Verbindungen der betreffenden Formeln (IB10ea) bis (IB10eh), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabelle 34: Verbindungen der Formel (IB10f), (IB10fa), (IB10fb), (IB10fc), (IB10fd), (IB10fe), (IB10ff), (IB10fg) und (IB10fh), worin jeweils (R¹)ₘ und (R²)ₙ wie in Tabelle 25 definiert sind**

| | |
|---|---|
| 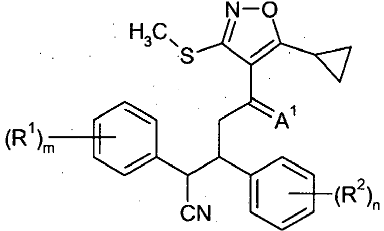 | **(IB10f)** |
| | **(IB10fa) A¹ ist (=O)** |
| | **(IB10fb) A¹ ist (=S)** |
| | **(IB10fc) A¹ ist (=N-CH₃)** |
| | **(IB10fd) A¹ ist (=N-OH)** |
| | **(IB10fe) A¹ ist (=N-OCH₃)** |
| | **(IB10ff) A¹ ist (=N-NH₂)** |
| | **(IB10fg) A¹ ist [=N-N(CH₃)₂]** |
| | **(IB10fh) A¹ ist (=N-Benzyl)** |

### Tabelle 34, Beispiele für erythro-threo-Gemische (Mengenverhältnis 70:30 bis 30:70):

Beispiele sind die Verbindungen der betreffenden Formeln (IB10fa) bis (IB10fh), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabelle 35: Verbindungen der Formel (IB10g), (IB10ga), (IB10gb), (IB10gc), (IB10gd), (IB10ge), (IB10gf), (IB10gg) und (IB10gh), worin jeweils (R¹)ₘ und (R²)ₙ wie in Tabelle 25 definiert sind**

| | |
|---|---|
| 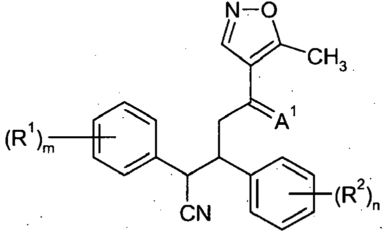 | **(IB10g)** |
| | **(IB10ga) A¹ ist (=0)** |
| | **(IB10gb) A¹ ist (=S)** |
| | **(IB10gc) A¹ ist (=N-CH₃)** |
| | **(IB10gd) A¹ ist (=N-OH)** |
| | **(IB10ge) A¹ ist (=N-OCH₃)** |
| | **(IB10gf) A¹ ist (=N-NH₂)** |
| | **(IB10gg) A¹ ist [=N-N(CH₃)₂]** |
| | **(IB10gh) A¹ ist (=N-Benzyl)** |

### Tabelle 35, Beispiele für erythro-threo-Gemische (Mengenverhältnis 70:30 bis 30:70):

Beispiele sind die Verbindungen der betreffenden Formeln (IB10ga) bis (IB10gh), jeweils in Form eines racemischen erythro-threo-Gemischs (Mengenverhältnis 70:30 bis 30:70), worin die Bedeutung von (R¹)ₘ und (R²)ₙ gemäß einer Zeilennummer aus der Tabelle 25 definiert ist. Die Nummerierung einzelner Verbindungen erfolgt gemäß "(Formel)(Zeilennummer)" ohne Schreiben der Klammern.

**Tabellen 35a bis 35f: Verbindungen der Formel (threo-IB10ga) und (threo-2-IB10ga), worin jeweils (R¹)ₘ und (R²)ₙ wie in Tabelle 25 definiert sind:**

| | |
|---|---|
| 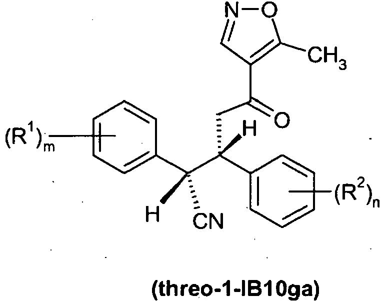 | 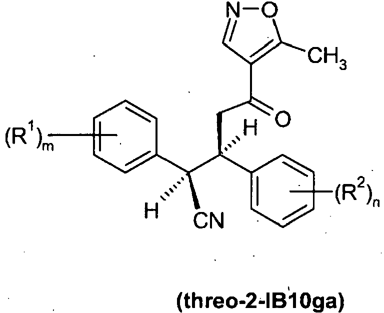 |
| **(threo-IB10ga) = (threo-1-IB10ga) + (threo-2-IB10ga) (50:50) = (rac.)** | |

### Tabelle 35a (threo-Racemate), Beispiele:

Verbindungen der Formel (IB10ga) in Form des racemischen Gemischs der threo-Isomeren [= Formel (threo-IB10ga)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-IB10ga(Zeilennummer)".

### Tabelle 35b (optisch aktive threo-2-Verbindungen), Beispiele:

Optisch aktive threo-2-Enantiomere in angereicherter Form [= (2R,3R)-Form mit mehr als 90%ee] mit der chemischen Struktur der Formel (threo-2-IB10ga), worin die Strukturkömbination der Gruppen Q, (R¹)ₘ und (R²)ₙ gemäß einer Zeilennummer aus der Tabelle 25 definiert ist. Die Nummerierung einzelner Verbindungen erfolgt gemäß "threo-2-IB10ga(Zeilennummer)".

### Tabelle 35c (optisch aktive threo-1-Enantiomere), Beispiele:

Optisch aktive threo-1-Enantiomere in angereicherter Form [= (2S,3S)-Form mit mehr als 90%ee] mit der chemischen Struktur der Formel (threo-1-IB10ga), worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "threo-1-IB10ga(Zeilennummer)".

### Tabelle 35d (erythro-Racemate), Beispiele:

Verbindungen der Formel (IB10ga) in Form des racemischen Gemischs der erythro-Isomeren [= Formel (erythro-IB10ga)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-IB10ga(Zeilennummer)".

### Tabelle 35e (erythro-1-Enantiomere), Beispiele:

Optisch aktive erythro-1-Enantiomere in angereicherter Form [= (2R,3S)-Form mit mehr als 90%ee] mit der chemischen Struktur der Formel (IB10ga) in der erythro-1-Form [= Formel (erythro-1-IB10ga)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-1-IB10ga(Zeilennummer)".

### Tabelle 35f (erythro-2-Enantiomere), Beispiele:

Optisch aktive erythro-2-Enantiomere in angereicherter Form [= (2S,3R)-Form mit mehr als 90%ee] mit der chemischen Struktur der Formel (IB10ga) in der erythro-2-Form [= Formel (erythro-2-IB10ga)], worin die Strukturkombination der Gruppen Q, (R¹)ₘ und (R²)ₙ gemäß einer Zeilennummer aus der Tabelle 25 definiert ist. Die Nummerierung einzelner Verbindungen erfolgt gemäß "erythro-2-IB10ga(Zeilennummer)".

**Tabelle 36: Definitionen von Strukturkombinationen der Gruppen B², (R¹)ₘ und (R²)ₙ für die nachfolgenden Tabellen von erfindungsgemäßen Verbindungen der allgemeinen Formel (I)**

| Bsp. | B² | (R¹)ₘ | (R²)ₙ |
|---|---|---|---|
| 1 | O-C(O)Et | 3-F | 4-Cl |
| 2 | O-C(O)Et | 3-F | 3-F |
| 3 | O-C(O)-i-Pr | 3,4-F₂ | 4-CI |
| 4 | O-C(O)Me | 3-F | 4-Br |
| 5 | NHOH | 3-Cl | 3-F |
| 6 | NHOH | 3,4-F₂ | 3-F |
| 7 | NHOH | H | 3-F |
| 8 | NHOMe | 4-F | 3-F |
| 9 | NHOMe | 3-F | 3-Cl |
| 10 | NHOH | 3,4-F₂ | 3-Cl |
| 11 | NHNMe₂ | 3-Br | 3-F |
| 12 | O-C(O)Me | 2,5-F₂ | 3-F |
| 13 | O-C(O)Me | 3-F | 2-F |
| 14 | O-C(O)Me | 3,4,5-F₃ | 4-CI |
| 15 | OSO₂Me | 4-F | 3-CI |
| 16 | OSO₂Me | 3,4-F₂ | 3-F, 4-Cl |
| 17 | O-C(O)Me | 3,4-F₂ | 2-F |
| 18 | O-C(O)Me | 3-F | 2,3-F₂ |
| 19 | SMe | 4-F | 3,5-F₂ |
| 20 | NHOH | 3-F | 4-F |
| 21 | NHOMe | 3,4-F₂ | 3-Cl, 5-F |
| 22 | OCH₂OMe | 3-F | 3-Cl, 5-F |
| 23 | OSO₂(4-Me-Ph) | 3-F | 2,5-F₂ |
| 24 | O-C(O)Me | 3,4-F₂ | 2,3-F₂ |
| 25 | O-C(O)Me | 3,4-F₂ | 2,5-F₂ |
| 26 | SMe | 3-CI | 3-Cl, 5-F |
| 27 | OSO₂Me | 3-F, 4-Cl | 3-Cl |
| 28 | O-C(O)Et | 3,4-F₂ | 3-NO₂ |
| 29 | O-C(O)Me | 3,4-F₂ | H |
| 30 | NHOMe | 3-Cl, 4-F | 3-F |
| 31 | NHOH | 3,4-F₂ | 4-OMe |
| 32 | O-C(O)-t-Bu | 3-F | 4-Cl |
| 33 | O-C(O)Me | 3-F | 4-Cl |
| 34 | OCH₂OMe | 3-F | 4-Cl |
| 35 | SMe | 3-F | 4-Cl |
| 36 | OSO₂(4-Me-Ph) | 3-F | 4-Cl |
| 37 | Cl | 3-F | 4-Cl |
| 38 | OCO₂Me | 3-F | 4-Cl |
| 39 | NHOMe | 3-F | 4-Cl |
| 40 | NHOH | 3-F | 4-Cl |
| 41 | O-C(O)-t-Bu | 3-F | 3-F |
| 42 | O-C(O)Me | 3-F | 3-F |
| 43 | OCH₂Ph | 3-F | 3-F |
| 44 | SMe | 3-F | 3-F |
| 45 | OSO₂(4-Me-Ph) | 3-F | 3-F |
| 46 | Cl | 3-F | 3-F |
| 47 | OCO₂Me | 3-F | 3-F |
| 48 | O-C(O)-t-Bu | 3,4-F₂ | 3-F |
| 49 | O-C(O)Me | 3,4-F₂ | 3-F |
| 50 | O-C(O)Et | 3,4-F₂ | 3-F |
| 51 | OSO₂Me | 3,4-F₂ | 3-F |
| 52 | OCH₂OMe | 3,4-F₂ | 3-F |
| 53 | OSO₂(4-Me-Ph) | 3,4-F₂ | 3-F |
| 54 | Cl | 3,4-F₂ | 3-F |
| 55 | OCO₂Me | 3,4-F₂ | 3-F |
| 56 | NHOMe | 3,4-F₂ | 3-F |
| 57 | O-C(O)-t-Bu | 3,4-F₂ | 3-Cl |
| 58 | O-C(O)Me | 3,4-F₂ | 3-Cl |
| 59 | O-C(O)Et | 3,4-F₂ | 3-Cl |
| 60 | OCH₂OMe | 3,4-F₂ | 3-Cl |
| 61 | SMe | 3,4-F₂ | 3-Cl |
| 62 | OSO₂(4-Me-Ph) | 3,4-F₂ | 3-Cl |
| 63 | Cl | 3,4-F₂ | 3-Cl |
| 64 | OCO₂Me | 3,4-F₂ | 3-Cl |
| 65 | NHOMe | 3,4-F₂ | 3-Cl |
| 66 | O-C(O)-t-Bu | 3-Br | 3-F |
| 67 | O-C(O)Me | 2,5-F₂ | 3-F |
| 68 | SPh | 3-F | 2-F |
| 69 | O-C(O)-t-Bu | 3,4,5-F₃ | 4-Cl |
| 70 | O-C(O)Me | 4-F | 3-Cl |
| 71 | SO₂Ph | 3,4-F₂ | 3-F, 4-Cl |
| 72 | O-C(O)-t-Bu | 3,4-F₂ | 2-F |
| 73 | O-C(O)Me | 3-F | 2,3-F₂ |
| 74 | NHNH₂ | 4-F | 3,5-F₂ |
| 75 | O-C(O)-t-Bu | 3-F | 4-F |
| 76 | O-C(O)Me | 3,4-F₂ | 3-Cl, 5-F |
| 77 | NHNHMe | 3-F | 3-Cl, 5-F |
| 78 | O-C(O)-t-Bu | 3-F | 2,5-F₂ |
| 79 | O-C(O)Me | 3,4-F₂ | 2,3-F₂ |
| 80 | SPh | 3,4-F₂ | 2,5-F₂ |
| 81 | O-C(O)-t-Bu | 3-Cl | 3-Cl, 5-F |
| 82 | O-C(O)Me | 3-F, 4-Cl | 3-Cl |
| 83 | NHOMe | 3,4-F₂ | 3-NO₂ |
| 84 | O-C(O)-t-Bu | 3,4-F₂ | H |
| 85 | O-C(O)Me | 3-Cl, 4-F | 3-F |
| 86 | SPh | 3,4-F₂ | 4-OMe |
| 87 | O-C(O)Et | 3-F | H |
| 88 | O-C(O)Et | 3-F | 2,4-F₂ |
| 89 | O-C(O)Et | 3-F | 2,6-F₂ |
| 90 | O-C(O)Et | 3-F | 3,4-F₂ |
| 91 | O-C(O)-i-Pr | 3-F | 3,5-F₂ |
| 92 | NHOMe | 3-F | 2-CI |
| 93 | NHOH | 3-F | 2,3-Cl₂ |
| 94 | NHOH | 3-F | 2,4-Cl₂ |
| 95 | O-C(O)-t-Bu | 3-F | 2,5-Cl₂ |
| 96 | SMe | 3-F | 2,6-Cl₂ |
| 97 | NHOMe | 3-F | 3,4-Cl₂ |
| 98 | O-C(O)Et | 3-F | 3,5-Cl₂ |
| 99 | O-C(O)-t-Bu | 3,4-F₂ | 4-F |
| 100 | O-C(O)Me | 3,4-F₂ | 2,4-F₂ |
| 101 | O-C(O)-i-Pr | 3,4-F₂ | 2,6-F₂ |
| 102 | NHOMe | 3,4-F₂ | 3,4-F₂ |
| 103 | NHOH | 3,4-F₂ | 3,5-F₂ |
| 104 | SPh | 3,4-F₂ | 2-Cl |
| 105 | O-C(O)-t-Bu | 3,4-F₂ | 2,3-Cl₂ |
| 106 | O-C(O)-i-Pr | 3,4-F₂ | 2,4-Cl₂ |
| 107 | O-C(O)-t-Bu | 3,4-F₂ | 2,5-Cl₂ |
| 108 | O-C(O)Et | 3,4-F₂ | 2,6-Cl₂ |
| 109 | NHNH₂ | 3,4-F₂ | 3,4-Cl₂ |
| 110 | NHCH₂Ph | 3,4-F₂ | 3,5-Cl₂ |
| 111 | O-C(O)-i-Pr | 3-Cl | H |
| 112 | O-C(O)-i-Pr | 3-Cl | 2-F |
| 113 | OSO₂Me | 3-Cl | 4-F |
| 114 | OSO₂Me | 3-Cl | 2,3-F₂ |
| 115 | O-C(O)-t-Bu | 3-Cl | 2,4-F₂ |
| 116 | NHOMe | 3-Cl | 2,5-F₂ |
| 117 | NHCH₂Ph | 3-Cl | 2,6-F₂ |
| 118 | O-C(O)-t-Bu | 3-Cl | 3,4-F₂ |
| 119 | OCH₂OMe | 3-Cl | 3,5-F₂ |
| 120 | OSO₂Me | 3-Cl | 2-Cl |
| 121 | NHOMe | 3-Cl | 3-Cl |
| 122 | SPh | 3-Cl | 4-Cl |
| 123 | O-C(O)-i-Pr | 3-Cl | 2,3-Cl₂ |
| 124 | O-C(O)-i-Pr | 3-Cl | 2,4-Cl₂ |
| 125 | SMe | 3-Cl | 2,5-Cl₂ |
| 126 | OSO₂Ph | 3-Cl | 2,6-Cl₂ |
| 127 | SPh | 3-Cl | 3,4-Cl₂ |
| 128 | OCH₂OMe | 3-Cl | 3,5-Cl₂ |
| 129 | O-C(O)-i-Pr | 3,4-Cl₂ | H |
| 130 | O-C(O)Me | 3,4-Cl₂ | 2-F |
| 131 | O-C(O)Me | 3,4-Cl₂ | 3-F |
| 132 | O-C(O)Et | 3,4-Cl₂ | 4-F |
| 133 | O-C(O)Me | 3,4-Cl₂ | 2,3-F₂ |
| 134 | O-C(O)Et | 3,4-Cl₂ | 2,4-F₂ |
| 135 | O-C(O)Et | 3,4-Cl₂ | 2,5-F₂ |
| 136 | O-C(O)Me | 3,4-Cl₂ | 2,6-F₂ |
| 137 | NHOMe | 3,4-Cl₂ | 3,4-F₂ |
| 138 | O-C(O)Et | 3,4-Cl₂ | 3,5-F₂ |
| 139 | O-C(O)-i-Pr | 3,4-Cl₂ | 2-Cl |
| 140 | O-C(O)Et | 3,4-Cl₂ | 3-Cl |
| 141 | O-C(O)-t-Bu | 3,4-Cl₂ | 4-Cl |
| 142 | SPh | 3,4-Cl₂ | 2,3-Cl₂ |
| 143 | O-C(O)-t-Bu | 3,4-Cl₂ | 2,4-Cl₂ |
| 144 | O-C(O)Me | 3,4-Cl₂ | 2,5-Cl₂ |
| 145 | O-C(O)Et | 3,4-Cl₂ | 2,6-Cl₂ |
| 146 | O-C(O)Me | 3,4-Cl₂ | 3,4-Cl₂ |
| 147 | O-C(O)Me | 3,4-Cl₂ | 3,5-Cl₂ |
| 148 | O-C(O)Et | 3-Cl, 4-F | H |
| 149 | SMe | 3-Cl, 4-F | 2-F |
| 150 | O-C(O)Me | 3-Cl, 4-F | 4-F |
| 151 | O-C(O)Et | 3-Cl, 4-F | 2,3-F₂ |
| 152 | O-C(O)-t-Bu | 3-Cl, 4-F | 2,4-F₂ |
| 153 | O-C(O)Me | 3-Cl, 4-F | 2,5-F₂ |
| 154 | OSO₂Me | 3-Cl, 4-F | 2,6-F₂ |
| 155 | O-C(O)Et | 3-Cl, 4-F | 3,4-F₂ |
| 156 | OSO₂(4-Me-Ph) | 3-Cl, 4-F | 3,5-F₂ |
| 157 | O-C(O)Me | 3-Cl, 4-F | 2-Cl |
| 158 | OCH₂OMe | 3-Cl, 4-F | 3-Cl |
| 159 | O-C(O)Me | 3-Cl, 4-F | 4-Cl |
| 160 | O-C(O)Me | 3-Cl, 4-F | 2,3-Cl₂ |
| 161 | O-C(O)Et | 3-Cl, 4-F | 2,4-Cl₂ |
| 162 | O-C(O)Me | 3-Cl, 4-F | 2,5-Cl₂ |
| 163 | O-C(O)-i-Pr | 3-Cl, 4-F | 2,6-Cl₂ |
| 164 | O-C(O)Et | 3-Cl, 4-F | 3,4-Cl₂ |
| 165 | O-C(O)Me | 3-Cl, 4-F | 3,5-Cl₂ |
| 166 | O-C(O)Me | 3-Cl, 4-F | H |
| 167 | NHOH | 3-F, 4-Cl | 2-F |
| 168 | O-C(O)Me | 3-F, 4-Cl | 3-F |
| 169 | O-C(O)Me | 3-F, 4-Cl | 4-F |
| 170 | NHOMe | 3-F, 4-Cl | 2,3-F₂ |
| 171 | O-C(O)Et | 3-F, 4-Cl | 2,4-F₂ |
| 172 | SPh | 3-F, 4-Cl | 2,5-F₂ |
| 173 | O-C(O)Me | 3-F, 4-Cl | 2,6-F₂ |
| 174 | O-C(O)Me | 3-F, 4-Cl | 3,4-F₂ |
| 175 | O-C(O)Et | 3-F, 4-Cl | 3,5-F₂ |
| 176 | O-C(O)Me | 3-F, 4-Cl | 2-Cl |
| 177 | O-C(O)Et | 3-F, 4-Cl | 4-Cl |
| 178 | SPh | 3-F, 4-Cl | 2,3-Cl₂ |
| 179 | NHOMe | 3-F, 4-Cl | 2,4-Cl₂ |
| 180 | NHOMe | 3-F, 4-Cl | 2,5-Cl₂ |
| 181 | O-C(O)-i-Pr | 3-F, 4-Cl | 2,6-Cl₂ |
| 182 | SMe | 3-F, 4-Cl | 3,4-Cl₂ |
| 183 | SPh | 3-F, 4-Cl | 3,5-Cl₂ |
| 184 | O-C(O)Et | 3-F | 2,5-F₂ |
| 185 | O-C(O)Me | 3-F | 2,6-F₂ |
| 186 | SPh | 3-F | 2,6-F₂ |
| 187 | NHNH₂ | 3,4-F₂ | 3,4-Cl₂ |
| 188 | NHCH2Ph | 3,4-F₂ | 3,5-Cl₂ |
| 189 | SPh | 3-F | 2,6-F₂ |
| 190 | O-C(O)-t-Bu | 3-F | 2,6-F₂ |
| 191 | SMe | 3-F | 2,6-F₂ |
| 192 | O-C(O)Et | 3,4-F₂ | 2,6-F₂ |
| 193 | O-C(O)-t-Bu | 3,4-F₂ | 2,6-F₂ |
| 194 | O-C(O)Me | 3,4-F₂ | 2,6-F₂ |
| 195 | SPh | 3,4-F₂ | 2,6-F₂ |
| 196 | SMe | 3,4-F₂ | 2,6-F₂ |
| 197 | OSO₂(4-Me-Ph) | 3,4-F₂ | 2,6-F₂ |
| 198 | Cl | 3,4-F₂ | 2,6-F₂ |
| 199 | OCO₂Me | 3,4-F₂ | 2,6-F₂ |
| 200 | OCH₂OMe | 3,4-F₂ | 2,6-F₂ |
| 201 | O-C(O)Et | H | 2,6-F₂ |
| 202 | O-C(O)Et | H | 3,4-F₂ |
| 203 | O-C(O)Me | H | 3,5-F₂ |
| 204 | NHOMe | H | 2-Cl |
| 205 | NHOH | H | 3-Cl |
| 206 | O-C(O)Et | H | 4-Cl |
| 207 | SMe | H | 2,3-Cl₂ |
| 208 | SPh | H | 2,4-Cl₂ |
| 209 | NHCH₂Ph | H | 2,5-Cl₂ |
| 210 | O-C(O)Et | H | 2,6-Cl₂ |
| 211 | OCH₂OMe | H | 3,4-Cl₂ |
| 212 | OCH₂OMe | H | 3,5-Cl₂ |
| 213 | O-C(O)Et | 3-CN | 2,5-F₂ |
| 214 | O-C(O)-t-Bu | 3-CN | 2,5-F₂ |
| 215 | O-C(O)Me | 3-CN | 2,5-F₂ |
| 216 | NHOMe | 3-CN | 2,5-F₂ |
| 217 | SMe | 3-CN | 2,5-F₂ |
| 218 | OSO₂(4-Me-Ph) | 3-CN | 2,5-F₂ |
| 219 | Cl | 3-CN | 2,5-F₂ |
| 220 | OCO₂Me | 3-CN | 2,5-F₂ |
| 221 | O-C(O)Et | 3-CN | 2,6-F₂ |
| 222 | NHOMe | 3-CN | 2,6-F₂ |
| 223 | O-C(O)Me | 3-CN | 2,6-F₂ |
| 224 | NHOH | 3-CN | 2,6-F₂ |
| 225 | SMe | 3-CN | 2,6-F₂ |
| 226 | OSO₂(4-Me-Ph) | 3-CN | 2,6-F₂ |
| 227 | Cl | 3-CN | 2,6-F₂ |
| 228 | OCO₂Me | 3-CN | 2,6-F₂ |
| 229 | O-C(O)Et | 3-CN | 2,3-F₂ |
| 230 | O-C(O)-t-Bu | 3-CN | 2,3-F₂ |
| 231 | O-C(O)Me | 3-CN | 2,3-F₂ |
| 232 | SPh | 3-CN | 2,3-F₂ |
| 233 | SMe | 3-CN | 2,3-F₂ |
| 234 | NHOH | 3-CN | 2,3-F₂ |
| 235 | Cl | 3-CN | 2,3-F₂ |
| 236 | OCO₂Me | 3-CN | 2,3-F₂ |
| 237 | O-C(O)Et | 3-CN | 2,4-F₂ |
| 238 | NHNH₂ | 3-CN | 2,4-F₂ |
| 239 | O-C(O)Me | 3-CN | 2,4-F₂ |
| 240 | NHNH₂ | 3-CN | 2,4-F₂ |
| 241 | SMe | 3-CN | 2,4-F₂ |
| 242 | OSO₂(4-Me-Ph) | 3-CN | 2,4-F₂ |
| 243 | Cl | 3-CN | 2,4-F₂ |
| 244 | OCO₂Me | 3-CN | 2,4-F₂ |
| 245 | O-C(O)Et | 3-CN | 2,4-F₂ |
| 246 | O-C(O)-t-Bu | 3-CN | 2,4-F₂ |
| 247 | O-C(O)Me | 3-CN | 2,4-F₂ |
| 248 | SPh | 3-CN | 2,4-F₂ |
| 249 | SMe | 3-CN | 2,4-F₂ |
| 250 | OSO₂(4-Me-Ph) | 3-CN | 2,4-F₂ |
| 251 | Cl | 3-CN | 2,4-F₂ |
| 252 | OCO₂Me | 3-CN | 2,4-F₂ |
| 253 | O-C(O)Et | 3-CN | 4-Cl |
| 254 | NHOMe | 3-CN | 4-Cl |
| 255 | O-C(O)Me | 3-CN | 4-Cl |
| 256 | NHOMe | 3-CN | 4-Cl |
| 257 | SMe | 3-CN | 4-Cl |
| 258 | OSO₂(4-Me-Ph) | 3-CN | 4-Cl |
| 259 | Cl | 3-CN | 4-Cl |
| 260 | OCO₂Me | 3-CN | 4-Cl |
| 261 | O-C(O)Et | 3-CN | 3-Cl |
| 262 | NHOMe | 3-CN | 3-Cl |
| 263 | O-C(O)Me | 3-CN | 3-Cl |
| 264 | NHOH | 3-CN | 3-Cl |
| 265 | SMe | 3-CN | 3-Cl |
| 266 | OSO₂(4-Me-Ph) | 3-CN | 3-Cl |
| 267 | Cl | 3-CN | 3-Cl |
| 268 | OCO₂Me | 3-CN | 3-Cl |
| 269 | O-C(O)Et | 3-CN | 4-F |
| 270 | NHOMe | 3-CN | 4-F |
| 271 | O-C(O)Me | 3-CN | 4-F |
| 272 | NHOMe | 3-CN | 4-F |
| 273 | SMe | 3-CN | 4-F |
| 274 | OSO₂(4-Me-Ph) | 3-CN | 4-F |
| 275 | Cl | 3-CN | 4-F |
| 276 | OCO₂Me | 3-CN | 4-F |
| 277 | O-C(O)Et | 3-CN | 3-F |
| 278 | NHOMe | 3-CN | 3-F |
| 279 | O-C(O)Me | 3-CN | 3-F |
| 280 | NHOH | 3-CN | 3-F |
| 281 | SMe | 3-CN | 3-F |
| 282 | OSO₂(4-Me-Ph) | 3-CN | 3-F |
| 283 | Cl | 3-CN | 3-F |
| 284 | OCO₂Me | 3-CN | 3-F |
| 285 | O-C(O)Et | 3-NO₂ | 2,5-F₂ |
| 286 | NHCH₂Ph | 3-NO₂ | 2,5-F₂ |
| 287 | O-C(O)Me | 3-NO₂ | 2,5-F₂ |
| 288 | NHOMe | 3-NO₂ | 2,5-F₂ |
| 289 | SMe | 3-NO₂ | 2,5-F₂ |
| 290 | OSO₂(4-Me-Ph) | 3-NO₂ | 2,5-F₂ |
| 291 | Cl | 3-NO₂ | 2,5-F₂ |
| 292 | OCO₂Me | 3-NO₂ | 2,5-F₂ |
| 293 | O-C(O)Et | 3-NO₂ | 2,6-F₂ |
| 294 | O-C(O)-t-Bu | 3-NO₂ | 2,6-F₂ |
| 295 | O-C(O)Me | 3-NO₂ | 2,6-F₂ |
| 296 | NHOMe | 3-NO₂ | 2,6-F₂ |
| 297 | SMe | 3-NO₂ | 2,6-F₂ |
| 298 | NHOH | 3-NO₂ | 2,6-F₂ |
| 299 | Cl | 3-NO₂ | 2,6-F₂ |
| 300 | OCO₂Me | 3-NO₂ | 2,6-F₂ |
| 301 | O-C(O)Et | 3-NO₂ | 2,3-F₂ |
| 302 | O-C(O)-t-Bu | 3-NO₂ | 2,3-F₂ |
| 303 | O-C(O)Me | 3-NO₂ | 2,3-F₂ |
| 304 | SPh | 3-NO₂ | 2,3-F₂ |
| 305 | SMe | 3-NO₂ | 2,3-F₂ |
| 306 | OSO₂(4-Me-Ph) | 3-NO₂ | 2,3-F₂ |
| 307 | Cl | 3-NO₂ | 2,3-F₂ |
| 308 | OCO₂Me | 3-NO₂ | 2,3-F₂ |
| 309 | O-C(O)Et | 3-NO₂ | 2,4-F₂ |
| 310 | O-C(O)-t-Bu | 3-NO₂ | 2,4-F₂ |
| 311 | O-C(O)Me | 3-NO₂ | 2,4-F₂ |
| 312 | NHOMe | 3-NO₂ | 2,4-F₂ |
| 313 | SMe | 3-NO₂ | 2,4-F₂ |
| 314 | OSO₂(4-Me-Ph) | 3-NO₂ | 2,4-F₂ |
| 315 | Cl | 3-NO₂ | 2,4-F₂ |
| 316 | OCO₂Me | 3-NO₂ | 2,4-F₂ |
| 317 | O-C(O)Et | 3-NO₂ | 2,4-F₂ |
| 318 | O-C(O)-t-Bu | 3-NO₂ | 2,4-F₂ |
| 319 | O-C(O)Me | 3-NO₂ | 2,4-F₂ |
| 320 | SPh | 3-NO₂ | 2,4-F₂ |
| 321 | SMe | 3-NO₂ | 2,4-F₂ |
| 322 | OSO₂(4-Me-Ph) | 3-NO₂ | 2,4-F₂ |
| 323 | Cl | 3-NO₂ | 2,4-F₂ |
| 324 | OCO₂Me | 3-NO₂ | 2,4-F₂ |
| 325 | O-C(O)Et | 3-NO₂ | 4-Cl |
| 326 | NHOMe | 3-NO₂ | 4-Cl |
| 327 | O-C(O)Me | 3-NO₂ | 4-Cl |
| 328 | SPh | 3-NO₂ | 4-Cl |
| 329 | SMe | 3-NO₂ | 4-Cl |
| 330 | NHOH | 3-NO₂ | 4-Cl |
| 331 | Cl | 3-NO₂ | 4-Cl |
| 332 | OCO₂Me | 3-NO₂ | 4-Cl |
| 333 | O-C(O)Et | 3-NO₂ | 3-Cl |
| 334 | O-C(O)-t-Bu | 3-NO₂ | 3-Cl |
| 335 | O-C(O)Me | 3-NO₂ | 3-Cl |
| 336 | SPh | 3-NO₂ | 3-Cl |
| 337 | SMe | 3-NO₂ | 3-Cl |
| 338 | NHOMe | 3-NO₂ | 3-Cl |
| 339 | Cl | 3-NO₂ | 3-Cl |
| 340 | OCO₂Me | 3-NO₂ | 3-Cl |
| 341 | O-C(O)Et | 3-NO₂ | 4-F |
| 342 | O-C(O)-t-Bu | 3-NO₂ | 4-F |
| 343 | O-C(O)Me | 3-NO₂ | 4-F |
| 344 | SPh | 3-NO₂ | 4-F |
| 345 | SMe | 3-NO₂ | 4-F |
| 346 | NHOMe | 3-NO₂ | 4-F |
| 347 | Cl | 3-NO₂ | 4-F |
| 348 | OCO₂Me | 3-NO₂ | 4-F |
| 349 | O-C(O)Et | 3-NO₂ | 3-F |
| 350 | NHOMe | 3-NO₂ | 3-F |
| 351 | O-C(O)Me | 3-NO₂ | 3-F |
| 352 | SPh | 3-NO₂ | 3-F |
| 353 | SMe | 3-NO₂ | 3-F |
| 354 | NHOH | 3-NO₂ | 3-F |
| 355 | Cl | 3-NO₂ | 3-F |
| 356 | OCO₂Me | 3-NO₂ | 3-F |
| 357 | O-C(O)Et | 3-CN, 4-F | 2,6-F₂ |
| 358 | NHOMe | 3-CN, 4-F | 2,6-F₂ |
| 359 | O-C(O)Me | 3-CN, 4-F | 2,6-F₂ |
| 360 | SPh | 3-CN, 4-F | 2,6-F₂ |
| 361 | SMe | 3-CN, 4-F | 2,6-F₂ |
| 362 | NHOH | 3-CN, 4-F | 2,6-F₂ |
| 363 | Cl | 3-CN, 4-F | 2,6-F₂ |
| 364 | OCO₂Me | 3-CN, 4-F | 2,6-F₂ |
| 365 | O-C(O)Me | 3-CN, 4-F | 3-Cl |
| 366 | O-C(O)Me | 3-CN, 4-F | 3-CN |
| 367 | O-C(O)Me | 3-CN, 4-F | 4-F |
| 368 | O-C(O)Me | 3-CN, 4-F | 4-Cl |
| 369 | O-C(O)Me | 3-CN, 4-F | 2,5-F₂ |
| 370 | O-C(O)Me | 3-CN, 4-F | 2,6-F₂ |
| 371 | O-C(O)Et | 3-Br, 4-F | 2,6-F₂ |
| 372 | NHOMe | 3-Br, 4-F | 2,6-F₂ |
| 373 | O-C(O)Me | 3-Br, 4-F | 2,6-F₂ |
| 374 | SPh | 3-Br, 4-F | 2,6-F₂ |
| 375 | SMe | 3-Br, 4-F | 2,6-F₂ |
| 376 | NHOH | 3-Br, 4-F | 2,6-F₂ |
| 377 | Cl | 3-Br, 4-F | 2,6-F₂ |
| 378 | OCO₂Me | 3-Br, 4-F | 2,6-F₂ |
| 379 | O-C(O)Me | 3-Br, 4-F | 3-Cl |
| 380 | O-C(O)Me | 3-Br, 4-F | 3-CN |
| 381 | O-C(O)Me | 3-Br, 4-F | 4-F |
| 382 | O-C(O)Me | 3-Br, 4-F | 4-Cl |
| 383 | O-C(O)Me | 3-Br, 4-F | 2,5-F₂ |
| 384 | O-C(O)Me | 3-Br, 4-F | 2,6-F₂ |
| 385 | O-C(O)Et | 3-F, 4-CN | 3-F |
| 386 | NHOMe | 3-F, 4-CN | 3-Cl |
| 387 | O-C(O)Me | 3-F, 4-CN | 3-CN |
| 388 | SPh | 3-F, 4-CN | 4-F |
| 389 | SMe | 3-F, 4-CN | 4-Cl |
| 390 | NHOH | 3-F, 4-CN | 2,5-F₂ |
| 391 | Cl | 3-F, 4-CN | 2,6-F₂ |
| 392 | OCO₂Me | 3-F, 4-CN | 3-F |
| 393 | O-C(O)Me | 3-F, 4-CN | 3-Cl |
| 394 | O-C(O)Me | 3-F, 4-CN | 3-CN |
| 395 | O-C(O)Me | 3-F, 4-CN | 4-F |
| 396 | O-C(O)Me | 3-F, 4-CN | 4-Cl |
| 397 | O-C(O)Me | 3-F, 4-CN | 2,5-F₂ |
| 398 | O-C(O)Me | 3-F, 4-CN | 2,6-F₂ |

Zu Referenzzwecken sind in den nachfolgenden Tabellen 37 bis 42f die einzelnen Verbindungen einzelnen Nummern (= Beispielnummern) zugeordnet, wobei die jeweilige Beispielnummer sich zusammensetzt aus der Nummer der chemischen Formel, die der jeweiligen Tabelle zugeordnet ist und einer "Zeilennummer" (Zeilennummer), die sich auf dieselbe Nummer in der Zeile der ersten Spalte der Tabelle 36 bezieht. Die chemische Struktur des Beispiels Nr. "(Formelnummer)(Zeilennummer)" ist damit durch die der jeweiligen Tabelle voran gestellten Formel gemäß Formelnummer und der Zeilennummer aus Tabelle 36 eindeutig definiert, zum Beispiel:
Das Beispiel mit der Nr. "IA5a1" aus Tabelle 37 ist die Verbindung der Formel (IA5a), worin B² = -O-C(O)-Et, (R¹)ₘ = 3-F und (R²)ₙ = 4-Cl gemäß der Zeile 1 aus Tabelle 36 definiert ist, oder ein Tautomer davon.
Das Beispiel mit der Nr. "IA12ab344" aus Tabelle 38 ist die Verbindung der Formel (IA12a), worin R = Ethyl bedeutet [= Unterformel (IA12ab)] und dabei B² = Phenylthio, (R¹)ₘ = 3-NO₂ und (R²)ₙ = 4-F gemäß der Zeile 344 aus Tabelle 36 definiert ist, oder ein Tautomer davon.
Tabelle 37: Verbindungen der Formel (IA5a), worin B², (R¹)ₘ und (R²)ₙ wie in Tabelle 36 definiert sind:

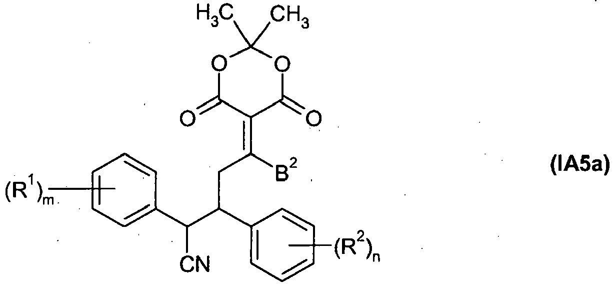

### Tabelle 37, Beispiele:

Beispiele sind die Verbindungen der Formel (IA5a), jeweils in Form eines racemischen erythro-threo-Gemischs (Mengenverhältnis 70:30 bis 30:70), worin die Bedeutungen von B², (R¹)ₘ und (R²)ₙ gemäß einer Zeilennummer aus der Tabelle 36 definiert ist. Die Nummerierung einzelner Verbindungen erfolgt gemäß "(Formel)(Zeilennummer)" ohne Schreiben der Klammern.

**Tabelle 38: Verbindungen der Formel (IA12a), (IA12aa), (IA12ab), (IA12ac) bzw. (IA12ad), worin B², (R¹)ₘ und (R²)ₙ wie in Tabelle 36 definiert sind:**

| | |
|---|---|
| 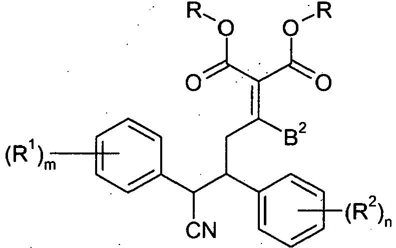 | **(IA12a)** |
| | **(IA12aa) R ist Methyl** |
| | **(IA12ab) R ist Ethyl** |
| | **(IA12ac) R ist n-Propyl** |
| | **(IA12ad) R ist i-Propyl** |

### Tabelle 38, Beispiele für erythro-threo-Gemische (Mengenverhältnis 70:30 bis 30:70):

Beispiele sind die Verbindungen der Formeln (IA12aa) bis (IA12ad), jeweils in Form eines racemischen erythro-threo-Gemischs (Mengenverhältnis 70:30 bis 30:70), worin die Bedeutungen von B², (R¹)ₘ und (R²)ₙ gemäß einer Zeilennummer aus der Tabelle 36 definiert ist. Die Nummerierung einzelner Verbindungen erfolgt gemäß "(Formel)(Zeilennummer)" ohne Schreiben der Klammern.

**Tabellen 38a bis 38f: Verbindungen der Formel (threo-IA12aa), (threo-IA12ab), (threo-IA12ac), (threo-IA12ad), (threo-2-IA12aa), (threo-2-IA12ab), (threo-2-IA12ac) und (threo-2-IA12ad), worin jeweils B², (R¹)ₘ und (R²)ₙ wie in Tabelle 36 definiert sind:**

| | |
|---|---|
| 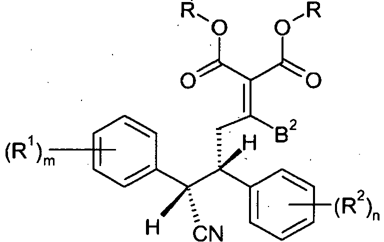 | 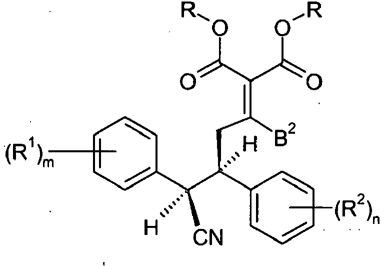 |
| **(threo-1-IA12aa) R = OCH₃** | **(threo-2-IA12aa) R = OCH₃** |
| **(threo-1-IA12ab) R = OC₂H₅** | **(threo-2-IA12ab) R = OC₂H₅** |
| **(threo-1-IA12ac) R = n-C₃H₇** | **(threo-2-IA12ac) R = n-C₃H₇** |
| **(threo-1-IA12ad) R = i-C₃H₇** | **(threo-2-IA12ad) R = i-C₃H₇** |
| **(threo-IA12aa) = (threo-1-IA12aa) + (threo-2-IA12aa) (50:50) = (rac.)** | |
| **(threo-IA12ab) = (threo-1-IA12ab) + (threo-2-IA12ab) (50:50) = (rac.)** | |
| **(threo-IA12ac) = (threo-1-IA12ac) + (threo-2-IA12ac) (50:50) = (rac.)** | |
| **(threo-IA12ad) = (threo-1-IA12ad) + (threo-2-IA12ad) (50:50) = (rac.)** | |

### Tabelle 38a (threo-Racemate), Beispiele:

Verbindungen der Formeln threo-(IA12aa), threo-(IA12ab), threo-(IA12ac) und threo-(IA12ad), jeweils in Form des racemischen Gemischs der threo-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-IA12aa(Zeilennummer)", "threo-IA12ab(Zeilennummer)", "threo-IA12ac(Zeilennummer)" und "threo-IA12ad(Zeilennummer)".

### Tabelle 38b, Beispiele (optisch aktive threo-2-Enantiomere):

Optisch aktive threo-2-Enantiomere in angereicherter Form [= (2R,3R)-Form mit mehr als 90%ee] mit der chemischen Struktur der Formel threo-2-(IA12aa), threo-2-(IA12ab), threo-2-(IA12ac) oder threo-2-(IA12ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-2-IA12aa(Zeilennummer)", threo-2-IA12ab(Zeilennummer)", "threo-2-IA12ac(Zeilennummer)" und "threo-2-IA12ad(Zeilennummer)".

### Tabelle 38c, Beispiele (optisch aktive threo-1-Enantiomere):

Optisch aktive threo-1-Enantiomere in angereicherter Form [= (2S,3S)-Form mit mehr als 90%ee] mit der chemischen Struktur der Formel threo-1-(IA12aa), threo-1-(IA12ab), threo-1-(IA12ac) oder threo-1-(IA12ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-1-IA12aa(Zeilennummer)", threo-1-IA12ab(Zeilennummer)", "threo-1-IA12ac(Zeilennummer)" und "threo-1-IA12ad(Zeilennummer)".

### Tabelle 38d (erythro-Raceniate), Beispiele:

Verbindungen der Formeln erythro-(IA12aa), erythro-(IA12ab), erythro-(IA12ac) und erythro-(IA12ad), jeweils in Form des racemischen Gemischs der erythro-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-IA12aa(Zeilennummer)", "erythro-IA12ab(Zeilennummer)", "erythro-IA12ac(Zeilennummer)" und "erythro-IA12ad(Zeilennummer)".

### Tabelle 38e (erythro-1-Enantiomere), Beispiele:

Optisch aktive erythro-2-Enantiomere in angereicherter Form [= (2R,3S)-Form mit mehr als 90%ee] mit der chemischen Struktur der Formel erythro-1-(IA12aa), erythro-1-(IA12ab), erythro-1-(IA12ac) oder erythro-1-(IA12ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-1-IA12aa(Zeilennummer)", erythro-1-IA12ab(Zeilennummer)", "erythro-1-IA12ac(Zeilennummer)" und "erythro-1-IA12ad(Zeilennummer)".

### Tabelle 38f (erythro-2-Enantiomere), Beispiele:

Optisch aktive erythro-2-Enantiomere in angereicherter Form [= (2S,3R)-Form mit mehr als 90%ee] mit der chemischen Struktur der Formel erythro-2-(IA12aa), erythro-2-(IA12ab), erythro-2-(IA12ac) oder erythro-2-(IA12ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-2-IA12aa(Zeilennummer)", erythro-2-IA12ab(Zeilennummer)", "erythro-2-IA12ac(Zeilennummer)" und "erythro-2-IA12ad(Zeilennummer)".

**Tabelle 39: Verbindungen der Formel (IA10a), (IA10aa), (IA10ab), (IA10ac) bzw. (IA10ad), worin B², (R¹)ₘ und (R²)ₙ wie in Tabelle 36 definiert sind:**

| | |
|---|---|
| 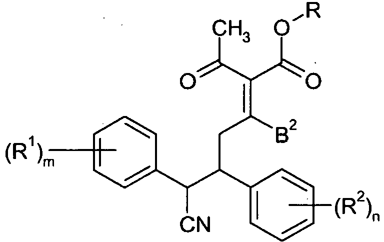 | **(IA10a)** |
| | **(IA10aa) R ist Methyl** |
| | **(IA10ab) R ist Ethyl** |
| | **(IA10ac) R ist n-Propyl** |
| | **(IA10ad) R ist i-Propyl** |

### Tabelle 39, Beispiele für erythro-threo-Gemische (Mengenverhältnis 70:30 bis 30:70):

Beispiele sind die Verbindungen der Formeln (IA10aa) bis (IA10ad), jeweils in Form eines racemischen erythro-threo-Gemischs (Mengenverhältnis 70:30 bis 30:70), worin die Bedeutungen von B², (R¹)ₘ und (R²)ₙ gemäß einer Zeilennummer aus der Tabelle 36 definiert ist. Die Nummerierung einzelner Verbindungen erfolgt gemäß "(Formel)(Zeilennummer)" ohne Schreiben der Klammern.

**Tabellen 39a bis 39f: Verbindungen der Formel (threo-IA10aa), (threo-IA10ab), (threo-IA10ac), (threo-IA10ad), (threo-2-IA10aa), (threo-2-IA10ab), (threo-2-IA10ac) und (threo-2-IA10ad), worin jeweils B², (R¹)ₘ und (R²)ₙ wie in Tabelle 36 definiert sind:**

| | |
|---|---|
| 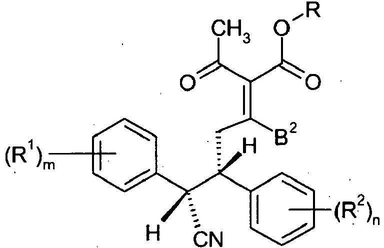 | 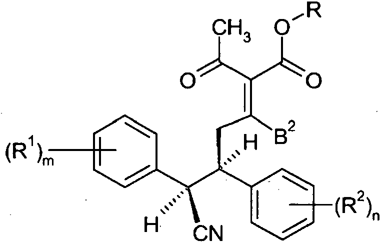 |
| **(threo-1-IA10aa) R = OCH₃** | **(threo-2-IA10aa) R = OCH₃** |
| **(threo-1-IA10ab) R = OC₂H₅** | **(threo-2-IA10ab) R = OC₂H₅** |
| **(threo-1-IA10ac) R = n-C₃H₇** | **(threo-2-IA10ac) R = n-C₃H7** |
| **(threo-1-IA10ad) R = i-C₃H₇** | **(threo-2-IA10ad) R = i-C₃H₇** |
| | |
| **(threo-IA10aa) = (threo-1-IA10aa) + (threo-2-IA10aa) (50:50) = (rac.)** | |
| **(threo-IA10ab) = (threo-1-IA10ab) + (threo-2-IA10ab) (50:50) = (rac.)** | |
| **(threo-IA10ac) = (threo-1-IA10ac) + (threo-2-IA10ac) (50:50) = (rac.)** | |
| **(threo-IA10ad) = (threo-1-IA10ad) + (threo-2-IA10ad) (50:50) = (rac.)** | |

### Tabelle 39a (threo-Racemate), Beispiele:

Verbindungen der Formeln threo-(IA10aa), threo-(IA10ab), threo-(IA10ac) und threo-(IA10ad), jeweils in Form des racemischen Gemischs der threo-Isomeren, worin die Strukturkombination der Gruppen B², (R¹)ₘ und (R²)ₙ gemäß einer Zeilennummer aus der Tabelle 36 definiert ist.

Die Nummerierung einzelner Verbindungen erfolgt gemäß "threo-IA10aa(Zeilennummer)", "threo-IA10ab(Zeilennummer)", "threo-IA10ac(Zeilennummer)" und "threo-IA10ad(Zeilennummer)".

### Tabelle 39b (optisch aktive threo-2-Enantiomere), Beispiele:

Optisch aktive threo-2-Enantiomere in angereicherter Form [= (2R,3R)-Form mit mehr als 90%ee] mit der chemischen Struktur der Formel threo-2-(IA10aa), threo-2-(IA10ab), threo-2-(IA10ac) oder threo-2-(IA10ad), worin jeweils die Strukturkombination der Gruppen B², (R¹)ₘ und (R²)ₙ gemäß einer Zeilennummer aus der Tabelle 36 definiert.ist. Die Nummerierung einzelner Verbindungen erfolgt gemäß "threo-2-IA10aa(Zeilennummer)", threo-2-IA10ab(Zeilennummer)", "threo-2-IA10ac(Zeilennummer)" und "threo-2-IA10ad(Zeilennummer)".

### Tabelle 39c, Beispiele (optisch aktive threo-1-Enantiomere):

Optisch aktive threo-1-Enantiomere in angereicherter Form [= (2S,3S)-Form mit mehr als 90%ee] mit der chemischen Struktur der Formel threo-1-(IA10aa), threo-1-(IA10ab), threo-1-(IA10ac) oder threo-1-(IA10ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-1-IA10aa(Zeilennummer)", threo-1-IA10ab(Zeilennummer)", "threo-1-IA10ac(Zeilennummer)" und "threo-1-IA10ad(Zeilennummer)".

### Tabelle 39d (erythro-Racemate), Beispiele:

Verbindungen der Formel erythro-(IA10aa), erythro-(IA10ab), erythro-(IA10ac) und erythro-(IA10ad), jeweils in Form des racemischen Gemischs der erythro-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-IA10aa(Zeilennummer)", "erythro-IA10ab(Zeilennummer)", "erythro-IA10ac(Zeilennummer)" und "erythro-IA10ad(Zeilennummer)".

### Tabelle 39e (erythro-1-Enantiomere), Beispiele:

Optisch aktive erythro-2-Enantiomere in angereicherter Form [= (2R,3S)-Form mit mehr als 90%ee] mit der chemischen Struktur der Formel erythro-1-(IA10aa), erythro-1-(IA10ab), erythro-1-(IA10ac) oder erythro-1-(IA10ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-1-IA10aa(Zeilennummer)", erythro-1-IA10ab(Zeilennummer)", "erythro-1-IA10ac(Zeilennummer)" und "erythro-1-IA10ad(Zeilennummer)".

### Tabelle 39f (erythro-2-Enantiomere), Beispiele:

Optisch aktive erythro-2-Enantiomere in angereicherter Form [= (2S,3R)-Form mit mehr als 90%ee] mit der chemischen Struktur der Formel erythro-2-(IA10aa), erythro-2-(IA10ab), erythro-2-(IA10ac) oder erythro-2-(IA10ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-2-IA10aa(Zeilennummer)", erythro-2-IA10ab(Zeilennummer)", "erythro-2-IA10ac(Zeilennummer)" und "erythro-2-IA10ad(Zeilennummer)".

**Tabelle 40: Verbindungen der Formel (IA10b), (IA10ba), (IA10bb), (IA10bc) bzw. (IA10bd), worin B², (R¹)ₘ und (R²)ₙ wie in Tabelle 36 definiert sind:**

| | |
|---|---|
| 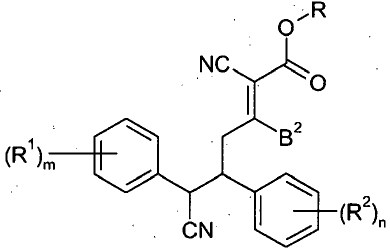 | **(IA10b)** |
| | **(IA10ba) Rist Methyl** |
| | **(IA10bb) R ist Ethyl** |
| | **(IA10bc) R ist n-Propyl** |
| | **(IA10bd) R ist i-Propyl** |

### Tabelle 40, Beispiele für erythro-threo-Gemische (Mengenverhältnis 70:30 bis 30:70):

Beispiele sind die Verbindungen der Formeln (IA10ba) bis (IA10bd), jeweils in Form eines racemischen erythro-threo-Gemischs (Mengenverhältnis 70:30 bis 30:70), worin die Bedeutungen von B², (R¹)ₘ und (R²)ₙ gemäß einer Zeilennummer aus der Tabelle 36 definiert ist. Die Nummerierung einzelner Verbindungen erfolgt gemäß "(Formel)(Zeilennummer)" ohne Schreiben der Klammern.

**Tabellen 40a bis 40f: Verbindungen der Formel (threo-IA10ba), (threo-IA10bb), (threo-IA10bc), (threo-IA10bd), (threo-2-IA10ba), (threo-2-IA10bb), (threo-2-IA10bc) und (threo-2-IA10bd), worin jeweils B², (R¹)ₘ und (R²)ₙ wie in Tabelle 36 definiert sind:**

| | |
|---|---|
| 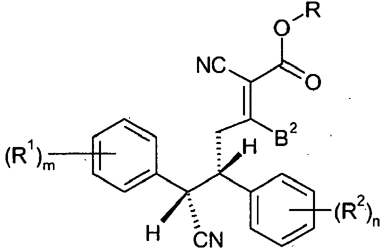 | 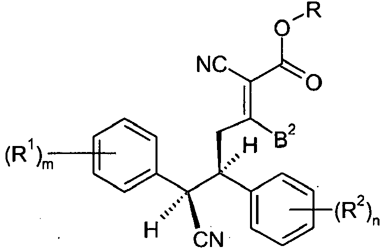 |
| **(threo-1-IA10ba) R = OCH₃** | **(threo-2-IA10ba) R = OCH₃** |
| **(threo-1-IA10bb) R = OC₂H₅** | **(threo-2-IA10bb) R = OC₂H₅** |
| **(threo-1-IA10bc) R = n-C₃H₇** | **(threo-2-IA10bc) R = n-C₃H₇** |
| **(threo=1-IA10bd) R = i-C₃H₇** | **(threo-2-IA10bd) R = i-C₃H₇** |
| | |
| **(threo-IA10ba) = (threo-1-IA10ba) + (threo-2-IA10ba) (50:50) = (rac.)** | |
| **(threo-IA10bb) = (threo-1-IA10bb) + (threo-2-IA10bb) (50:50) = (rac.)** | |
| **(threo-IA10bc) = (threo-1-IA10bc) + (threo-2-IA10bc) (50:50) = (rac.)** | |
| **(threo-IA10bd) = (threo-1-IA10bd) + (threo-2-IA10bd) (50:50) = (rac.)** | |

### Tabelle 40a (threo-Racemate), Beispiele:

Verbindungen der Formeln threo-(IA10ba), threo-(IA10bb), threo-(IA10bc) und threo-(IA10bd), jeweils in Form des racemischen Gemischs der threo-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-IA10ba(Zeilennummer)", "threo-IA10bb(Zeilennummer)", "threo-IA10bc(Zeilennummer)" und "threo-IA10bd(Zeilennummer)".

### Tabelle 40b (optisch aktive threo-2-Enantiomere), Beispiele:

Optisch aktive threo-2-Enantiomere in angereicherter Form [= (2R,3R)-Form mit mehr als 90%ee] mit der chemischen Struktur der Formel threo-2-(IA10ba), threo-2-(IA10bb), threo-2-(IA10bc) oder threo-2-(IA10bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-2-IA10ba(Zeilennummer)", threo-2-IA10bb(Zeilennummer)", "threo-2-IA10bc(Zeilennummer)" und "threo-2-IA10bd(Zeilennummer)".

### Tabelle 40c, Beispiele (optisch aktive threo-1-Enantiomere):

Optisch aktive threo-1-Enantiomere in angereicherter Form [= (2S,3S)-Form mit mehr als 90%ee] mit der chemischen Struktur der Formel threo-1-(IA10ba), threo-1-(IA10bb), threo-1-(IA10bc) oder threo-1-(IA10bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-1-IA10ba(Zeilennummer)", threo-1-IA10bb(Zeilennummer)", "threo-1-IA10bc(Zeilennummer)" und "threo-1-IA10bd(Zeilennummer)".

### Tabelle 40d (erythro-Racemate), Beispiele:

Verbindungen der Formeln erythro-(IA10ba), erythro-(IA10bb), erythro-(IA10bc) und erythro-(IA10bd), jeweils in Form des racemischen Gemischs der erythro-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-IA10ba(Zeilennummer)", "erythro-IA10bb(Zeilennummer)", "erythro-IA10bc(Zeilennummer)" und "erythro-IA10bd(Zeilennummer)".

### Tabelle 40e (erythro-1-Enantiomere), Beispiele:

Optisch aktive erythro-2-Enantiomere in angereicherter Form [= (2R,3S)-Form mit mehr als 90%ee] mit der chemischen Struktur der Formel erythro-1-(IA10ba), erythro-1-(IA10bb), erythro-1-(IA10bc) oder erythro-1-(IA10bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-1-IA10ba(Zeilennummer)", erythro-1-IA10bb(Zeilennummer)", "erythro-1-IA10bc(Zeilennummer)" und "erythro-1-IA10bd(Zeilennummer)".

### Tabelle 40f (erythro-2-Enantiomere), Beispiele:

Optisch aktive erythro-2-Enantiomere in angereicherter Form [= (2S,3R)-Form mit mehr als 90%ee] mit der chemischen Struktur der Formel erythro-2-(IA10ba), erythro-2-(IA10bb), erythro-2-(IA10bc) oder erythro-2-(IA10bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-2-IA10ba(Zeilennummer)", erythro-2-IA10bb(Zeilennummer)", "erythro-2-IA10bc(Zeilennummer)" und "erythro-2-IA10bd(Zeilennummer)".

**Tabelle 41: Verbindungen der Formel (IA11a), (IA11aa), (IA11 ab), (IA11ac) bzw. (IA11ad), worin B², (R¹)ₘ und (R²)ₙ wie in Tabelle 36 definiert sind:**

| | |
|---|---|
| 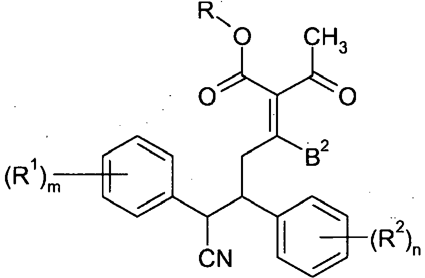 | **(IA11a)** |
| | **(IA11aa) R ist Methyl** |
| | **(IA11ab) R ist Ethyl** |
| | **(IA11ac) R ist n-Propyl** |
| | **(IA11ad) R ist i-Propyl** |

### Tabelle 41, Beispiele für erythro-threo-Gemische (Mengenverhältnis 70:30 bis 30:70):

Beispiele sind die Verbindungen der Formeln (IA11aa) bis (IA11ad), jeweils in Form eines racemischen erythro-threo-Gemischs (Mengenverhältnis 70:30 bis 30:70), worin die Bedeutungen von B², (R¹)ₘ und (R²)ₙ gemäß einer Zeilennummer aus der Tabelle 36 definiert ist. Die Nummerierung einzelner Verbindungen erfolgt gemäß "(Formel)(Zeilennummer)" ohne Schreiben der Klammern.

**Tabellen 41 a bis 41 f: Verbindungen der Formel (threo-IA11aa), (threo-IA11ab), (threo-IA11ac), (threo-IA11ad), (threo-2-IA11aa), (threo-2-IA11ab), (threo-2-IA11ac) und (threo-2-IA11ad), worin jeweils B², (R¹)ₘ und (R²)ₙ wie in Tabelle 36 definiert sind:**

| | |
|---|---|
| 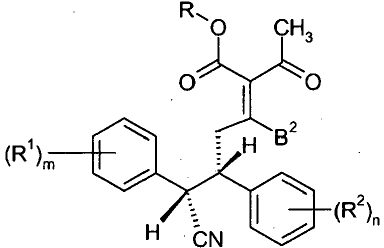 | 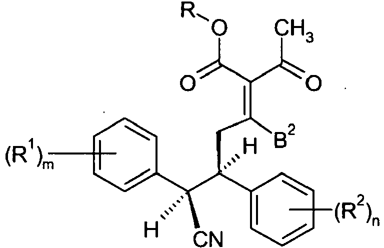 |
| **(threo-1-IA11aa) R = OCH₃** | **(threo-2-IA11aa) R= OCH₃** |
| **(threo-1-IA11ab) R = OC₂H₅** | **(threo-2-IA11ab) R = OC₂H₅** |
| **(threo-1-IA11ac) R = n-C₃H₇** | **(threo-2-IA11ac) R = n-C₃H₇** |
| **(threo-1-IA11ad) R = i-C₃H₇** | **(threo-2-IA11ad) R = i-C₃H₇** |
| | |
| **(threo-IA11aa) = (threo-1-IA11aa) + (threo-2-IA11aa) (50:50) = (rac.)** | |
| **(threo-IA11ab) = (threo-1-IA11ab) + (threo-2-IA11ab) (50:50) = (rac.)** | |
| **(threo-IA11ac) = (threo-1-IA11ac) + (threo-2-IA11ac) (50:50) = (rac.)** | |
| **(threo-IA11ad) = (threo-1-IA11ad) + (threo-2-IA11ad) (50:50) = (rac.)** | |

### Tabelle 41 a (threo-Racemate), Beispiele:

Verbindungen der Formeln threo-(IA11aa), threo-(IA11ab), threo-(IA11ac) und threo-(IA11ad), jeweils in Form des racemischen Gemischs der threo-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-IA11aa(Zeilennummer)", "threo-IA11ab(Zeilennummer)", "threo-IA11ac(Zeilennummer)" und "threo-IA11ad(Zeilennummer)",

### Tabelle 41 b (optisch aktive threo-2-Enantiomere), Beispiele:

Optisch aktive threo-2-Enantiomere in angereicherter Form [= (2R,3R)-Form mit mehr als 90%ee] mit der chemischen Struktur der Formel threo-2-(IA11aa), threo-2-(IA11ab), threo-2-(IA11ac) oder threo-2-(IA11ad), worin jeweils die Strukturkombination der Gruppen B², (R¹)ₘ und (R²)ₙ, gemäß einer Zeilennummer aus der Tabelle 36 definiert ist. Die Nummerierung einzelner Verbindungen erfolgt gemäß "threo-2-IA11aa(Zeilennummer)", threo-2-IA11ab(Zeilennummer)", "threo-2-IA11ac(Zeilennummer)" und "threo-2-IA11ad(Zeilennummer)".

### Tabelle 41 c, Beispiele (optisch aktive threo-1-Enantiomere):

Optisch aktive threo-1-Enantiomere in angereicherter Form [= (2S,3S)-Form mit mehr als 90%ee] mit der chemischen Struktur der Formel threo-1-(IA11aa), threo-1-(IA11ab), threo-1-(IA11 ac) oder threo-1-(IA11 ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-1-IA11aa(Zeilennummer)", threo-1-IA11ab(Zeilennummer)", "threo-1-IA11ac(Zeilennummer)" und "threo-1-IA11ad(Zeilennummer)".

### Tabelle 41 d (erythro-Racemate), Beispiele:

Verbindungen der Formel erythro-(IA11aa), erythro-(IA11ab), erythro-(IA11ac) und erythro-(IA11ad), jeweils in Form des racemischen Gemischs der erythro-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-IA11aa(Zeilennummer)", "erythro-IA11ab(Zeilennummer)", "erythro-IA11ac(Zeilennummer)" und "erythro-IA11ad(Zeilennummer)".

### Tabelle 41 e (erythro-1-Enantiomere), Beispiele:

Optisch aktive erythro-2-Enantiomere in angereicherter Form [= (2R,3S)-Form mit mehr als 90%ee] mit der chemischen Struktur der Formel erythro-1-(IA11aa), erythro-1-(IA11ab), erythro-1-(IA11ac) oder erythro-1-(IA11ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-1-IA11aa(Zeilennummer)", erythro-1-IA11ab(Zeilennummer)", "erythro-1-IA11ac(Zeilennummer)" und "erythro-1-IA11ad(Zeilennummer)".

### Tabelle 41f (erythro-2-Enantiomere), Beispiele:

Optisch aktive erythro-2-Enantiomere, in angereicherter Form [= (2S,3R)-Form mit mehr als 90%ee] mit der chemischen Struktur der Formel erythro-2-(IA11aa), erythro-2-(IA11ab), erythro-2-(IA11ac) oder erythro-2-(IA11ad), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-2-IA11aa(Zeilennummer)", erythro-2-IA11ab(Zeilennummer)", "erythro-2-IA11ac(Zeilennummer)" und "erythro-2-IA11ad(Zeilennummer)".

**Tabelle 42: Verbindungen der Formel (IA11b), (IA11ba), (IA11bb), (IA11bc) bzw. (IA11bd), worin B², (R¹)ₘ und (R²)ₙ wie in Tabelle 35 definiert sind:**

| | |
|---|---|
| 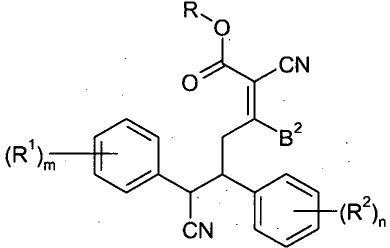 | **(IA11b)** |
| | **(IA11 ba) R ist Methyl** |
| | **(IA11 bb) R ist Ethyl** |
| | **(IA11 bc) R ist n-Propyl** |
| | **(IA11 bd) R ist i-Propyl** |

### Tabelle 42, Beispiele für erythro-threo-Gemische (Mengenverhältnis 70:30 bis 30:70):

Beispiele sind die Verbindungen der Formeln (IA11ba) bis (IA11bd), jeweils in Form eines racemischen erythro-threo-Gemischs (Mengenverhältnis 70:30 bis 30:70), worin die Bedeutungen von B², (R¹)ₘ und (R²)ₙ gemäß einer Zeilennummer aus der Tabelle 36 definiert ist. Die Nummerierung einzelner Verbindungen erfolgt gemäß "(Formel)(Zeilennummer)" ohne Schreiben der Klammern.

**Tabellen 42a bis 42f: Verbindungen der Formel (threo-IA11ba), (threo-IA11bb), (threo-IA11bc), (threo-IA11bd), (threo-2-IA11ba), (threo-2-IA11bb), (threo-2-IA11bc) und (threo-2-IA11bd), worin jeweils B², (R¹)ₘ und (R²)ₙ wie in Tabelle 36 definiert sind:**

| | |
|---|---|
| 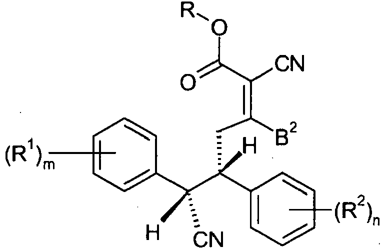 | 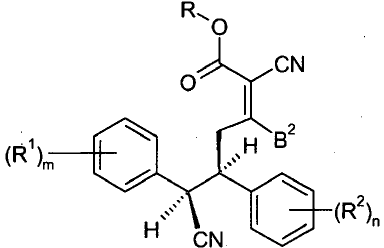 |
| **(threo-1-IA11ba) R = OCH₃** | **(threo-2-IA11ba) R = OCH₃** |
| **(threo-1-IA11bb) R = OC₂H₅** | (**threo-2-IA11bb) R = OC₂H₅** |
| **(threo-1-IA11bc) R = n-C₃H₇** | **(threo-2-IA11bc) R = n-C₃H₇** |
| **(threo-1-IA11bd) R = i-C₃H₇** | **(threo-2-IA11bd) R = i-C₃H₇** |
| | |
| **(threo-IA11ba) = (threo-1-IA11ba) + (threo-2-IA11ba) (50:50) = (rac.)** | |
| **(threo-IA11bb) = (threo-1-IA11bb) + (threo-2-IA11bb) (50:50) = (rac.)** | |
| **(threo-IA11bc) = (threo-1-IA11bc) + (threo-2-IA11bc) (50:50) = (rac.)** | |
| **(threo-IA11bd) = (threo-1-IA11bd) + (threo-2-IA11bd) (50:50) = (rac.)** | |

Zu Referenzzwecken sind die Verbindungen der Tabelle 42a einzelnen Nummern (= Beispielnummern) zugeordnet, wobei die Nummer "threo-IA11ba(Zeilennummer)", "threo-IA11bb(Zeilennummer)", threo-IA11bc(Zeilennummer)" und "threo-IA11bd(Zeilennummer)" das racemische Gemisch der threo-Enantiomeren mit der chemischen Struktur der Formeln (threo-1-IA11ba) und (threo-2-IA11ba), (threo-1-IA11bb) und (threo-2-IA11bb), (threo-1-IA11bc) und (threo-2-IA11bc) bzw. (threo-1-IA11bd) und (threo-2-IA11bd), welche jeweils die Strukturkombination der Gruppen B², (R¹)ₘ und (R²)ₙ gemäß der Zeilennummer aus der Tabelle 36 aufweist, bezeichnet.

Entsprechend bezeichnet in Tabelle 42b die Nummer "threo-2-IA11ba(Zeilennummer)", "threo-2-IA11bb(Zeilennummer)", "threo-2-IA11bc(Zeilennummer)" oder "threo-2-IA11bd(Zeilennummer)" das optisch aktive threo-2-Enantiomer in angereicherter Form [= (2R,3R)-Form mit mehr als 90%ee] mit der chemischen Struktur der Formel (threo-2-IA11ba(Zeilennummer)", (threo-2-IA11bb(Zeilennummer)", (threo-2-IA11bc(Zeilennummer)" bzw. (threo-2-IA11bd(Zeilennummer)", welche jeweils die Strukturkombination der Gruppen B², (R¹)ₘ und (R²)ₙ gemäß der Zeilennummer aus der Tabelle 36 aufweist.

### Tabelle 42a (threo-Racemate), Beispiele:

Verbindungen der Formel threo-(IA11ba), threo-(IA11bb), threo-(IA11bc) und threo-(IA11bd), jeweils in Form des racemischen Gemischs der threo-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-IA11ba(Zeilennummer)", "threo-IA11bb(Zeilennummer)", "threo-IA11bc(Zeilennummer)" und "threo-IA11bd(Zeilennummer)".

### Tabelle 42b (optisch aktive threo-2-Enantiomere), Beispiele:

Optisch aktive threo-2-Enantiomere in angereicherter Form [= (2R,3R)-Form mit mehr als 90%ee] mit der chemischen Struktur der Formel threo-2-(IA11ba), threo-2-(IA11bb), threo-2-(IA11bc) oder threo-2-(IA11bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-2-IA11ba(Zeilennummer)", threo-2-IA11bb(Zeilennummer)", "threo-2-IA11bc(Zeilennummer)" und "threo-2-IA11bd(Zeilennummer)".

### Tabelle 42c, Beispiele (optisch aktive threo-1-Enantiomere):

Optisch aktive threo-1-Enantiomere in angereicherter Form [= (2S,3S)-Form mit mehr als 90%ee] mit der chemischen Struktur der Formel threo-1-(IA11ba), threo-1-(IA11bb), threo-1-(IA11bc) oder threo-1-(IA11bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "threo-1-IA11ba(Zeilennummer)", threo-1-IA11bb(Zeilennummer)", "threo-1-IA11bc(Zeilennummer)" und "threo-1-IA11bd(Zeilennummer)".

### Tabelle 42d (erythro-Racemate), Beispiele:

Verbindungen der Formel erythro-(IA11ba), erythro-(IA11bb), erythro-(IA11 bc) und erythro-(IA11bd), jeweils in Form des racemischen Gemischs der erythro-Isomeren, worin die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-IA11ba(Zeilennummer)", "erythro-IA11bb(Zeilennummer)", "erythro-IA11bc(Zeilennummer)" und "erythro-IA11bd(Zeilennummer)".

### Tabelle 42e (erythro-1-Enantiomere), Beispiele:

Optisch aktive erythro-2-Enantiomere in angereicherter Form [= (2R,3S)-Form mit mehr als 90%ee] mit der chemischen Struktur der Formel erythro-1-(IA11ba), erythro-1-(IA11bb), erythro-1-(IA11bc) oder erythro-1-(IA11bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-1-IA11ba(Zeilennummer)", erythro-1-IA11bb(Zeilennummer)", "erythro-1-IA11bc(Zeilennummer)" und "erythro-1-IA11bd(Zeilennummer)".

### Tabelle 42f (erythro-2-Enantiomere), Beispiele:

Optisch aktive erythro-2-Enantiomere in angereicherter Form [= (2S,3R)-Form mit mehr als 90%ee] mit der chemischen Struktur der Formel erythro-2-(IA11ba), erythro-2-(IA11bb), erythro-2-(IA11bc) oder erythro-2-(IA11bd), worin jeweils die Strukturkombination der Gruppen B², (R¹)ₘ und (R²)ₙ gemäß einer Zeilennummer aus der Tabelle 36 definiert ist. Die Nummerierung einzelner Verbindungen erfolgt gemäß "erythro-2-IA11ba(Zeilennummer)", erythro-2-IA11bb(Zeilennummer)", "erythro-2-IA11bc(Zeilennummer)" und "erythro-2-IA11bd(Zeilennummer)".

Ausgewählte physikalische Daten zu Beispielen der Tabellen 1 bis 42f:
Testmethoden:
   1) ¹H-NMR-Daten (400 MHz, CDCl₃); charakteristische chemische Verschiebungen [in ppm] sind zum jeweiligen Beispiel angegeben,
   2) MS = Massenspektrum, gemessen mit Quadrupolgerät; Elektrospraylonisierung (+-), Massenbereich 100-1000; Molpeak M bzw [M+H]+ oder [M-1]+ oder [M-2]+ oder [M+1]+ beim jeweiligen Beispiel angegeben,
   3) HPLC = Hochdruckflüssigchromatographie (High Performance Liquid Chromatography), Säule: Zorbax Eclipse, 50x3,0, C18 1,8 ym, Laufmittel: Wasser + 0,06% Ameisensäure / Acrylnitril + 0,06% Ameisensäure, Gradient: 90:10, nach 2min 5:95; Detektor: DAD (210 - 400 nm); Retentionszeit (Rtz.) beim jeweiligen Beispiel angegeben,
   4) HPLC Chiral = HPLC an chiraler Säule, Säule: Chiralpak IC, 250 x 4,6mm, 5µm DAIC 83325, Detektorwellenlänge: 210 nm; Säulentemperatur, Fluß und Laufmittel beim jeweiligen Beispiel angegeben,

Daten zu Beispielen:
Beispiel Nr. Iaa1 (erythro-threo-Isomerengemisch 62:38):
   NMR (Threo): 17,41 (s, 1 H), 4,02 (d, 1H); NMR (Erythro): 17,44 (s, 1H), 4,29 (d, 1H); MS: 412 [M+H]+; HPLC (Rtz.): 1,67 min
Beispiel Nr. IB9aa6 (erythro-threo-Isomerengemisch 54:46):
   NMR (Threo): 13,54 (s, 1 H); NMR (Erythro): 13,64 (s, 1 H); MS: 432 [M-1]+; HPLC (Rtz.): 1,53 min
Beispiel Nr. IB9aa1 (erythro-threo-Isomerengemisch 53:47):
   NMR (Threo): 13,50 (s, 1H); NMR (Erythro): 13,61 (s, 1H); MS: 430 [M-1]+; HPLC (Rtz.): 1,59 min
Beispiel Nr. IB9aa10 (erythro-threo-Isomerengemisch 60:40):
   NMR (Threo): 13,54 (s, 1 H); NMR (Erythro): 13,63 (s, 1 H); MS: 448 [M-2]+; HPLC (Rtz.): 1,85 min
Beispiel Nr. Iaa20 (erythro-threo-Isomerengemisch 63:37):
   NMR (Threo): 17,46 (s, 1 H), 4,03 (d, 1 H); NMR (Erythro): 17,47 (s, 1 H), 4,30 (d, 1 H); MS: 394 [M-1]+; HPLC (Rtz.): 1,58 min
Beispiel Nr. IB8aa10 (erythro-threo-Isomerengemisch 57:43):
   NMR (Threo): 13,56 (breites s, 1H), 3,99 (d, 1H), 3,83 (s, 3H); NMR (Erythro): 13,56 (breites s, 1H), 4,09 (d, 1H), 3,85 (s, 3H); MS: 417 [M+1]+; HPLC (Rtz.): 1,53 min
Beispiel Nr. IB8aa1 (erythro-threo-Isomerengemisch 52:48):
   NMR (Threo): 13,52 (breites s, 1H), 4,00 (d, 1 H), 3,82 (s, 3H); NMR (Erythro): 13,58 (breites s, 1H), 4,09 (d, 1H), 3,84 (s, 3H); MS: 383 [M+1]+; HPLC (Rtz.): 1,44 min
Beispiel Nr. IB8aa43 (erythro-threo-Isomerengemisch 59:41):
   NMR (Erythro): 13,46 (breites s, 1 H), 3,81 (s, 3H); NMR (Threo): 13,55 (breites s, 1H), 3,84 (s, 3H); MS: 419 [M+1]+; HPLC (Rtz.): 1,46 min
Beispiel Nr. IB9aa20 (erythro-threo-Isomerengemisch 59:41):
   NMR (Threo): 13,51 (s, 1 H); NMR (Erythro): 13,62 (s, 1 H); MS: 414 [M-1]+; HPLC (Rtz.): 1,51 min
Beispiel Nr. IBXaa20 (erythro-threo-Isomerengemisch 53:47):
   NMR (Threo): 17,62 (s, 1 H), 4,04 (d, 1 H), 3,82 (s, 3H), 2,30 (s, 3H); NMR (Erythro): 17,74 (s, 1H), 4,30 (d, 1 H), 3,83 (s, 3H), 2,36 (s, 3H); MS: 398 [M-1]+; HPLC (Rtz.): 1,65 min
Beispiel Nr. IB8aa6 (erythro-threo-Isomerengemisch 57:43):
   NMR (Threo): 17,62 (s, 1 H), 4,04 (d, 1 H), 3,82 (s, 3H), 2,30 (s, 3H); NMR (Erythro): 13,62 (breites s, 1H), 4,10 (d, 1 H), 3,85 (s, 3H); MS: 399 [M-1]+; HPLC (Rtz.): 1,45 min
Beispiel Nr. IB9aa25 (erythro-threo-Isomerengemisch 63:37):
   NMR (Threo): 13,38 (s, 1H); NMR (Erythro): 13,41 (s, 1H); MS: 430 [M-1]+; HPLC (Rtz.): 1,58 min
Beispiel Nr. IA12a57 (erythro-threo-Isomerengemisch 63:37):
   NMR (Threo): 3,99 (d, 1H), 3,76 (s, 3H), 3,67 (s, 3H), 1,11 (s, 9H); NMR (Erythro): 4,30 (d, 1H), 3,84 (s, 3H), 3,73 (s, 3H), 1,17 (s, 9H);
Beispiel Nr. Iaa95 (erythro-threo-Isomerengemisch 57:43):
   NMR (Threo): 17,27 (s, 1 H); NMR (Erythro): 17,35 (s, 1 H); MS: 430 [M-1]+; HPLC (Rtz.): 1,59 min
Beispiel Nr. IB9aa23 (erythro-threo-Isomerengemisch 58:42):
   NMR (Threo): 13,43 (s, 1 H); NMR (Erythro): 13,53 (s, 1 H); MS: 432 [M-1]+; HPLC (Rtz.): 1,50 min
Beispiel Nr. Iaa23 (erythro-threo-Isomerengemisch 52:48):
   NMR (Threo): 17,26 (s, 1 H); NMR (Erythro): 17,31 (s, 1 H); MS: 414 [M+1]+; HPLC (Rtz.): 1,58 min
Beispiel Nr. Iaa10 (erythro-threo-Isomerengemisch 61:39):
   NMR (Threo): 17,36 (s, 1H), 4,01 (d, 1H); NMR (Erythro): 17,38 (s, 1H), 4,28 (d, 1H); MS: 430 [M+1]+; HPLC (Rtz.): 1,68 min
Beispiel Nr. IB9ba6 (erythro-threo-Isomerengemisch 57:43):
   NMR (Threo): 13,53 (s, 1 H); NMR (Erythro): 13,63 (s, 1 H); MS: 460 [M-1]+; HPLC (Rtz.): 1,67 min
Beispiel Nr. IBXaa6 (erythro-threo-Isomerengemisch 57:43):
   NMR (Threo): 17,59 (s, 1 H), 4,02 (d, 1 H), 3,83 (s, 3H), 2,31 (s, 3H); NMR (Erythro): 17,67 (s, 1 H), 4,30 (d, 1H), 3,83 (s, 3H), 2,36 (s, 3H); MS: 416 [M-1]+; HPLC (Rtz.): 1,67 min
Beispiel Nr. Iaa32 (erythro-threo-Isomerengemisch 70:30):
   NMR (Threo): 4,08 (d, 1 H), 1,06 (s, 9H); NMR (Erythro): 4,35 (d, 1 H), 1,15 (s, 9H)
Beispiel Nr. IA12aa23 (erythro-threo-Isomerengemisch 50:50):
   NMR (Threo):4,05 (d, 1 H), 2,47 (s, 3H); NMR (Erythro): 4,17 (d, 1 H), 2,50 (s, 3H); MS: 588 [M+1]+; HPLC (Rtz.): 1,71 min
Beispiel Nr. IA12ab53 (erythro-threo-Isomerengemisch 50:50):
   NMR (Threo): 2,47 (s, 3H); NMR (Erythro): 2,49 (s, 3H); MS: 616 [M+1]+; HPLC (Rtz.): 1,85 min
Beispiel Nr. Ioa95 (erythro-threo-Isomerengemisch 50:50):
   NMR (Threo): 13,8 (breites s, 1 H), 3,80 (d, 1 H); NMR (Erythro): 13,8 (breites s, 1 H), 4,18 (d, 1H); MS: 416 [M+1]+; HPLC (Rtz.): 1,39 min
Beispiel Nr. Iaa53 (erythro-threo-Isomerengemisch 60:34):
   NMR (Threo): 4,07 (d, 1H), 1,08 (s, 9H); NMR (Erythro): 4,35 (d, 1H), 1,16 (s, 9H)
Beispiel Nr. IA12aa193 (erythro-threo-Isomerengemisch 57:43):
   NMR (Threo): 3,74 (s, 3H), 3,67 (s, 3H), 1,15 (s, 9H); NMR (Erythro): 3,78 (s, 3H), 3,68 (s, 3H), 1,19 (s, 9H);
Beispiel Nr. IA12aa1 (erythro-threo-Isomerengemisch 67:33):
   NMR (Threo): 4,01 (d, 1 H), 3,73 (s, 3H), 3,66 (s, 3H), 1,11 (s, 9H); NMR (Erythro): 4,31 (d, 1H), 3,83 (s, 3H), 3,73 (s, 3H), 1,17 (s, 9H)
Beispiel Nr. IA12aa78 (erythro-threo-Isomerengemisch 66:34):
   NMR (Threo): 4,11 (d, 1H), 3,79 (s, 3H), 3,66 (s, 3H), 1,14 (s, 9H); NMR (Erythro): 4,30 (d, 1 H), 3,81 (s, 3H), 3,71 (s, 3H), 1,19 (s, 9H)
Beispiel Nr. IA12aa48 (erythro-threo-Isomerengemisch 66:34):
   NMR (Threo): 3,99 (d, 1H), 3,74 (s, 3H), 3,67 (s, 3H), 1,11 (s, 9H); NMR (Erythro): 4,31 (d, 1 H), 3,84 (s, 3H), 3,73 (s, 3H), 1,17 (s, 9H)
Beispiel Nr. Ioa6 (erythro-threo-Isomerengemisch 59:41):
   NMR (Threo): 14,0 (breites s, 1H), 4,03 (d, 1H); NMR (Erythro): 14,0 (breites s, 1H), 4,30 (d, 1H); MS: 400 [M+1]+; HPLC (Rtz.): 1,40 min
Beispiel Nr. threo-IA12aa57 (threo-Racemat)
   NMR (Threo): 3,99 (d, 1H), 3,76 (s, 3H), 3,67 (s, 3H), 3,39 (m, 3H), 1,11 (s, 9H)
   HPLC Chiral: Fluß: 1 mL/min; Säulentemperatur: 30 °C; Laufmittel: A = n-Heptan (80), B = Isopropanol (20), Retentionszeit: Threo 1: 10,751 min, Threo 2: 10,801 min
Beispiel Nr. erythro-2-IA12aa57 (optisch aktives erythro-2-Enantiomer)
   NMR (Erythro): 4,30 (d, 1 H), 3,84 (s, 3H), 3,73 (s, 3H), 3,35 (m, 1 H), 3,21 (m, 1 H), 3,07 (m, 1H), 1,17 (s, 9H); HPLC Chiral: Fluß: 1 mL/min; Säulentemperatur: 30 °C; Laufmittel: A = n-Heptan (80), B = Isopropanol (20), Retentionszeit: 7,481 min
Beispiel Nr. erythro-1-IA12aa57 (optisch aktives erythro-1-Enantiomer)
   NMR (Erythro): 4,30 (d, 1 H), 3,84 (s, 3H), 3,73 (s, 3H), 3,35 (m, 1 H), 3,21 (m, 1 H), 3,07 (m, 1H), 1,17 (s, 9H); HPLC Chiral: Fluß: 1 mL/min; Säulentemperatur: 30 °C; Laufmittel: A = n-Heptan (80), B = Isopropanol (20), Retentionszeit: 6,036 min
Beispiel Nr. IBXaa10 (erythro-threo-Isomerengemisch 58:42):
   NMR (Threo): 17,59 (s, 1 H), 4,02 (d, 1 H), 3,83 (s, 3H), 2,29 (s, 3H); NMR (Erythro): 17,67 (s, 1 H), 4,29 (d, 1 H), 3,84 (s, 3H), 2,36 (s, 3H); MS: 432 [M+1]+; HPLC (Rtz.): 1,74 min
Beispiel Nr. IBXaa43 (erythro-threo-Isomerengemisch 73:27):
   NMR (Threo): 17,54 (s, 1 H), 4,18 (d, 1 H), 3,79 (s, 3H), 2,32 (s, 3H); NMR (Erythro): 17,59 (s, 1 H), 4,30 (d, 1 H), 3,88 (s, 3H), 2,35 (s, 3H); MS: 434 [M+1]+; HPLC (Rtz.): 1,66 min
Beispiel Nr. Iaa83 (erythro-threo-Isomerengemisch 61:39):
   NMR (Threo): 17,26 (s, 1H); NMR (Erythro): 17,37 (s, 1H); MS: 414 [M+1]+; HPLC (Rtz.): 1,57 min
Beispiel Nr. Iea6 (erythro-threo-Isomerengemisch 58:42):
   NMR (Threo): 17,89 (m, 1H), 17,29 (s, 1H); NMR (Erythro): 17,92 (m, 1H), 17,31 (s, 1H); MS: 442 [M+1]+; HPLC (Rtz.): 1,79 min
Beispiel Nr. Ica6 (erythro-threo-Isomerengemisch 64:36):
   NMR (Threo): 17,35 (breites s, 1 H), 4,01 (d, 1 H); NMR (Erythro): 17,36 (breites s, 1 H), 4,29 (d, 1 H); MS: 428 [M+1]+; HPLC (Rtz.): 1,70 min
Beispiel Nr. threo-IA12aa48 (threo-Racemat)
   NMR (Threo): 3,99 (d, 1 H), 3,75 (s, 3H), 3,67 (s, 3H), 3,39 (m, 3H), 1,11 (s, 9H) HPLC Chiral: Fluß: 0,6 mL/min; Säulentemperatur: 25 °C; Laufmittel: A = n-Heptan (90), B = Isopropanol (10), Rtz.: Threo-1: 34,839 min, Threo-2: 36,287 min
Beispiel Nr. erythro-IA12aa48 (erythro-Racemat)
   NMR (Erythro): 4,31 (d, 1 H), 3,84 (s, 3H), 3,73 (s, 3H), 3,39 (m, 1 H), 3,23 (m, 1 H), 3,08 (m, 1 H), 1,17 (s, 9H); HPLC Chiral: Fluß: 0,6 mL/min; Säulentemperatur: 25 °C; Laufmittel: A = n-Heptan (90), B = Isopropanol (10), Rtz.: Erythro 1: 14,380 min, Erythro 2: 17,766 min
Beispiel Nr. Ifa6 (erythro-threo-Isomerengemisch 64:36):
   NMR (Threo): 17,41 (s, 1H), 4,00 (d, 1H), 1,06 (s, 3H), 1,03 (s, 3H); NMR (Erythro): 17,42 (s, 1H), 4,29 (d, 1H), 1,08 (s, 3H), 1,05 (s, 3H); MS: 442 [M+1]+; HPLC (Rtz.): 1,75 min
Beispiel Nr. threo-Iaa46 (threo-Racemat)
   NMR (Threo): 4,35 (d, 1H), 1,16 (s, 9H); HPLC Chiral: Fluß: 1 mL/min; Säulentemperatur: 25 °C; Laufmittel: A = n-Heptan (80), B = Isopropanol (20), Rtz.: Threo 1: 13,092 min, Threo 2: 13,876 min
Beispiel Nr. erythro-Iaa46 (erythro-Racemat)
   NMR (Threo): 4,08 (d, 1H), 1,09 (s, 9H); HPLC Chiral: Fluß: 1 mL/min; Säulentemperatur: 25 °C; Laufmittel: A = n-Heptan (80), B = Isopropanol (20), Rtz.: Erythro 1: 10,151 min, Erythro 2: 10,355
Beispiel Nr. Iva6 (erythro-threo-Isomerengemisch 62:38):
   NMR (Threo): 4,17 (d, 1 H), 3,55 (s, 3H), 2,40 (s, 3H); NMR (Erythro): 4,52 (d, 1 H), 3,59 (s, 3H), 2,44 (s, 3H); MS: 414 [M+1]+; HPLC (Rtz.): 1,24 min
Beispiel Nr. Ita6 (erythro-threo-Isomerengemisch 66:34):
   NMR (Threo): 7,60 (s, 1 H), 4,15 (d, 1 H), 3,62 (s, 3H); NMR (Erythro): 7,66 (s, 1 H), 4,45 (d, 1H), 3,66 (s, 3H); MS: 400 [M+1]+; HPLC (Rtz.): 1,31 min
Beispiel Nr. IA12aa50 (erythro-threo-Isomerengemisch 57:43):
   NMR (Threo): 2,15 (q, 2H), 1,03 (t, 3H); NMR (Erythro): 2,36 (q, 2H), 1,13 (t, 3H);
Beispiel Nr. IB9aa49 (erythro-threo-Isomerengemisch 59:4-1):
   NMR (Threo): 1,91 (s, 3H); NMR (Erythro): 2,12 (s, 3H);
Beispiel Nr. Iea95 (erythro-threo-Isomerengemisch 58:42):
   NMR (Threo): 17,20 (m, 1 H); NMR (Erythro): 17,83 (m, 1 H); MS: 460 [M+1]+; HPLC (Rtz.): 1,78 min
Beispiel Nr. threo-lva48 (threo-Racemat)
   NMR (Threo): 4,17 (d, 1H), 3,85 (m, 1H), 3,59 (s, 3H), 2,78 (q, 2H), 2,36 (s, 3H), 1,33 (t, 3H)
Beispiel Nr. erythro-lva48 (erythro-Racemat)
   NMR (Threo): 6,72 - 7,20 (m, 7H), 4,50 (d, 1H), 3,71 (m, 1 H), 3,62 (s, 3H), 3,41 (m, 1H), 3,11 (m, 1H), 2,79 (q, 2H), 2,47 (s, 3H), 1,33 (t, 3H)
Beispiel Nr. Ifa95 (erythro-threo-Isomerengemisch 61:39):
   NMR (Threo): 17,30 (s, 1 H), 2,47 (s, 3H), 2,30 (s, 3H); NMR (Erythro): 17,39 (s, 1H), 2,51 (s, 3H), 2,37 (s, 3H); MS: 460 [M+1]+; HPLC (Rtz.): 1,74 min
Beispiel Nr. Ita95 (erythro-threo-Isomerengemisch 73:27):
   NMR (Threo): 7,80 (s, 1 H), 4,17 (d, 1 H), 3,67 (s, 3H), 2,72 (q, 2H), 1,30 (t, 3H); NMR (Erythro): 7,90 (s, 1 H), 4,45 (d, 1 H), 3,71 (s, 3H), 2,80 (q, 2H), 1,35 (t, 3H);
Beispiel Nr. Iva6 (erythro-threo-Isomerengemisch 51:49):
   NMR (Threo): 4,18 (d, 1 H), 2,35 (s, 3H); NMR (Erythro): 4,38 (d, 1 H), 2,45 (s, 3H); MS: 432 [M+1]+; HPLC (Rtz.): 1,67 min
Beispiel Nr. Ica95 (erythro-threo-Isomerengemisch 58:42):
   NMR (Threo):17,23 (m, 1 H); NMR (Erythro): 17,31 (m, 1H); MS: 446 [M+1]+; HPLC (Rtz.): 1,22 min
Beispiel Nr. Iga95 (erythro-threo-Isomerengemisch 57:43):
   NMR (Threo): 17,62 (s, 1 H); NMR (Erythro): 17,69 (s, 1H); MS: 502 [M+1]+; HPLC (Rtz.): 1,68 min
Beispiel Nr. Iva259 (erythro-threo-Isomerengemisch 52:48):
   NMR (Threo): 4,29 - 4,37 (m, 1 H), 4,13 (d, 1 H), 3,59 (s, 3H), 2,80 (q, 2H), 2,37 (s, 3H), 1,35 (t, 3H); NMR (Erythro): 4,37 (d, 1 H), 3,60 (s, 3H), 2,80 (q, 2H), 2,42 (s, 3H), 1,35 (t, 3H);
Beispiel Nr. Iaa6 (erythro-threo-Isomerengemisch 61:39):
   NMR (Threo): 17,38 (s, 1 H), 4,01 (d, 1H); NMR (Erythro): 17,39 (s, 1 H), 4,30 (d, 1H); MS: 413 [M+1]+; HPLC (Rtz.): 1,52 min
Beispiel Nr. IB9aa43 (erythro-threo-Isomerengemisch 62:38):
   NMR (Threo): 13,37 (s, 1H); NMR (Erythro): 13,41 (s, 1H); MS: 450 [M+1]+; HPLC (Rtz.): 1,51 min
Beispiel Nr. Threo-2-IB8aa43 (threo-Racemat):
   NMR (Threo): 13,55 (s, 1 H), 7,17 (m, 1H), 7,02 (m, 2H), 6,92 (m, 1 H), 6,77 (t, 2H), 4,23 (d, 1H), 4,19 (m, 1H), 3,84 (s, 3H), 3,47 (m, 2H); MS: 419 [M+1]+; HPLC (Rtz.): 1,51 min; Drehwert: [a]_{D}²⁰ = -52,295 in CHCl₃. (Konzentration = 2,157 g/mL).
Beispiel Nr. Ioa83 (erythro-threo-Isomerengemisch 53:47):
   NMR (Threo): 13,8 (breites s, 1 H), 6,72 (t, 1 H); NMR (Erythro): 13,8 (breites s, 1 H), 6,89 (t, 1H); MS: 400 [M+1]+; HPLC (Rtz.): 1,39 min
Beispiel Nr. Ioa29 (erythro-threo-Isomerengemisch 60:40):
   NMR (Threo): 13,8 (breites s, 1H), 4,27 (d, 1H); NMR (Erythro): 13,8 (breites s, 1 H), 4,01 (d, 1H); MS: 382 [M+1]+; HPLC (Rtz.): 1,43 min
Beispiel Nr. Ioa24 (erythro-threo-Isomerengemisch 60:40):
   NMR (Threo): 13,8 (breites s, 1 H), 4,36 (d, 1 H); NMR (Erythro): 13,8 (breites s, 1 H), 4,71 (d, 1H); MS: 418 [M+1]+; HPLC (Rtz.): 1,44 min
Beispiel Nr. Ioa3 (erythro-threo-Isomerengemisch 60:40):
   NMR (Threo): 13,8 (breites s, 1H), 4,26 (d, 1H); NMR (Erythro): 13,8 (breites s, 1H), 3,99 (d, 1H); MS: 416 [M+1]+; HPLC (Rtz.): 1,54 min
Beispiel Nr. Ioa17 (erythro-threo-Isomerengemisch 58:42):
   NMR (Threo): 13,8 (breites s, 1H), 4,22 (d, 1H); NMR (Erythro): 13,8 (breites s, 1H), 4,00 (d, 1H); MS: 400 [M+1]+; HPLC (Rtz.): 1,54 min
Beispiel Nr. Ioa2 (erythro-threo-Isomerengemisch 58:42):
   NMR (Threo): 13,8 (breites s, 1 H), 4,27 (d, 1 H); NMR (Erythro): 13,8 (breites s, 1 H), 4,04 (d, 1 H); MS: 382 [M+1]+; HPLC (Rtz.): 1, 40 min
Beispiel Nr. Ioa9 (erythro-threo-Isomerengemisch 63:37):
   NMR (Threo): 13,8 (breites s, 1 H), 4,25 (d, 1 H); NMR (Erythro): 13,8 (breites s, 1 H), 4,03 (d, 1 H); MS: 398 [M+1]+; HPLC (Rtz.): 1, 49 min
Beispiel Nr. Ioa20 (erythro-threo-Isomerengemisch 63:37):
   NMR (Threo): 13,8 (breites s, 1 H), 4,26 (d, 1 H); NMR (Erythro): 13,8 (breites s, 1 H), 4,02 (d, 1H); MS: 382 [M+1]+; HPLC (Rtz.): 1, 40 min
Beispiel Nr. Ioa1 (erythro-threo-Isomerengemisch 63:37):
   NMR (Threo): 13,8 (breites s, 1 H), 4,26 (d, 1 H); NMR (Erythro): 13,8 (breites s, 1 H), 4,03 (d, 1 H); MS: 398 [M+1]+; HPLC (Rtz.): 1, 50 min
Beispiel Nr. Ica83 (erythro-threo-Isomerengemisch 63:37):
   NMR (Threo): 6,70 (t, 2H); NMR (Erythro): 6,88 (t, 2H); MS: 428 [M+1]+; HPLC (Rtz.): 1,68 min
Beispiel Nr. Ifa83 (erythro-threo-Isomerengemisch 64:36):
   NMR (Threo): 6,70 (t, 2H); NMR (Erythro): 6,88 (t, 2H); MS: 442 [M+1]+; HPLC (Rtz.): 1,74 min
Beispiel Nr. Iea83 (erythro-threo-Isomerengemisch 67:33):
   NMR (Threo): 6,70 (t, 2H); NMR (Erythro): 6,88 (t, 2H); MS: 442 [M+1]+; HPLC (Rtz.): 1,79 min
Beispiel Nr. Ica29 (erythro-threo-Isomerengemisch 63:37):
   NMR (Threo): 4,01 (m, 1 H); NMR (Erythro): 4,30 (m, 1 H); MS: 410 [M+1]+; HPLC (Rtz.): 1,73 min
Beispiel Nr. Ifa29 (erythro-threo-Isomerengemisch 62:38):
   NMR (Threo): 4,01 (d, 1 H); NMR (Erythro): 4,30 (m, 1 H); MS: 424 [M+1]+; HPLC (Rtz.): 1,79 min
Beispiel Nr. Iea29 (erythro-threo-Isomerengemisch 61:39):
   NMR (Threo): 4,01 (d, 1 H); NMR (Erythro): 4,31 (dd, 1 H); MS: 424 [M+1]+; HPLC (Rtz.): 1,83 min
Beispiel Nr. Iaa29 (erythro-threo-Isomerengemisch 62:38):
   NMR (Threo): 4,01 (d, 1 H); NMR (Erythro): 4,31 (m, 1 H); MS: 396 [M+1]+; HPLC (Rtz.): 1,63 min
Beispiel Nr. Ica3 (erythro-threo-Isomerengemisch 62:38):
   NMR (Threo): 3,98 (dd, 1 H); NMR (Erythro): 4,29 (d, 1 H); MS: 444 [M+1]+; HPLC (Rtz.): 1,82 min
Beispiel Nr. Ifa3 (erythro-threo-Isomerengemisch 62:38):
   NMR (Threo): 3,99 (dd, 1 H); NMR (Erythro): 4,29 (d, 1 H);
   MS: 458 [M+1]+; HPLC (Rtz.): 1,88 min
Beispiel Nr. Iea3 (erythro-threo-Isomerengemisch 64:36):
   NMR (Threo): 4,00 (dd, 1H); NMR (Erythro): 4,31 (dd, 1 H); MS: 458 [M+1]+; HPLC (Rtz.): 1,91 min
Beispiel Nr. Iaa3 (erythro-threo-Isomerengemisch 64:36):
   NMR (Threo): 4,00 (d, 1H); NMR (Erythro): 4,29 (d, 1H); MS: 430 [M+1]+; HPLC (Rtz.): 1,73 min
Beispiel Nr. Ica25 (erythro-threo-Isomerengemisch 63:37):
   NMR (Threo): 4,08 (m, 1 H); NMR (Erythro): 4,34 (d, 1 H); MS: 446 [M+1]+; HPLC (Rtz.): 1,73 min
Beispiel Nr. Ifa25 (erythro-threo-Isomerengemisch 64:36):
   NMR (Threo): 4,08 (m, 1H), 2,35 (s, 2H); NMR (Erythro): 4,34 (d, 1H), 2,51 (s, 2H); MS: 460 [M+1]+; HPLC (Rtz.): 1,78 min
Beispiel Nr. Iea25 (erythro-threo-Isomerengemisch 59:41):
   NMR (Threo): 4,08 (m, 1 H), 2,51 (m, 2H); NMR (Erythro): 4,34 (d, 1H), 2,68 (m, 2H); MS: 460 [M+1]+; HPLC (Rtz.): 1,82 min
Beispiel Nr. Ica17 (erythro-threo-Isomerengemisch 54:46):
   NMR (Threo): 4,09 (m, 1 H), 2,55 (m, 2H); NMR (Erythro): 4,36 (d, 1H), 2,65 (m, 2H); MS: 428 [M+1]+; HPLC (Rtz.): 1,72 min
Beispiel Nr. Ifa17 (erythro-threo-Isomerengemisch 57:43):
   NMR (Threo): 2,48 (s, 2H); NMR (Erythro): 4,36 (d, 1H), 2,52 (s, 2H); MS: 442 [M+1]+; HPLC (Rtz.): 1,78 min
Beispiel Nr. Iea17 (erythro-threo-Isomerengemisch 56:44):
   NMR (Threo): 2,51 (s, 2H); NMR (Erythro): 2,66 (s, 2H); MS: 443 [M+2]+; HPLC (Rtz.): 1,82 min
Beispiel Nr. Iaa17 (erythro-threo-Isomerengemisch 51:49):
   NMR (Threo): 2,48 (m, 2H); NMR (Erythro): 4,36 (d, 1H), 2,63 (m, 2H); MS: 414 [M+1]+; HPLC (Rtz.): 1,63 min
Beispiel Nr. Ica23 (erythro-threo-Isomerengemisch 54:46):
   NMR (Threo): 4,12 (d, 1H), 2,53 (m, 2H); NMR (Erythro): 4,32 (d, 1H), 2,67 (m, 2H); MS: 428 [M+1]+; HPLC (Rtz.): 1,70 min
Beispiel Nr. Ifa23 (erythro-threo-Isomerengemisch 56:44):
   NMR (Threo): 4,13 (d, 1H), 2,49 (s, 2H); NMR (Erythro): 4,33 (d, 1H), 2,51 (s, 2H); MS: 442 [M+1]+; HPLC (Rtz.): 1,76 min
Beispiel Nr. Iea23 (erythro-threo-Isomerengemisch 60:40):
   NMR (Threo): 2,51 (m, 2H); NMR (Erythro): 2,68 (m, 2H); MS: 442 [M+1]+; HPLC (Rtz.): 1,79 min
Beispiel Nr. Ica2 (erythro-threo-Isomerengemisch 65:35):
   NMR (Threo): 4,03 (dd, 1H); NMR (Erythro): 4,30 (d, 1H); MS: 410 [M+1]+; HPLC (Rtz.): 1,70 min
Beispiel Nr. Iea2 (erythro-threo-Isomerengemisch 60:40):
   NMR (Threo): 4,02 (d, 1H), 2,48 (s, 2H); NMR (Erythro): 4,31 (d, 1H), 2,52 (s, 2H); MS: 424 [M+1]+; HPLC (Rtz.): 1,76 min
Beispiel Nr. Iea9 (erythro-threo-Isomerengemisch 62:38):
   NMR (Threo): 4,03 (dd, 1H), 2,51 (m, 2H); NMR (Erythro): 4,31 (dd, 1H), 2,67 (m, 2H); MS: 440 [M+1]+; HPLC (Rtz.): 1,87 min
Beispiel Nr. Ifa9 (erythro-threo-Isomerengemisch 62:38):
   NMR (Threo): 4,03 (d, 1H), 2,49 (s, 2H); NMR (Erythro): 4,29 (d, 1H), 2,52 (s, 2H); MS: 440 [M+1]+; HPLC (Rtz.): 1,83 min
Beispiel Nr. Iaa9 (erythro-threo-Isomerengemisch 60:40):
   NMR (Threo): 4,03 (d, 1H), 2,48 (m, 2H); NMR (Erythro): 4,29 (d, 1H), 2,64 (m, 2H); MS: 412 [M+1]+; HPLC (Rtz.): 1,68 min
Beispiel Nr. Ica20 (erythro-threo-Isomerengemisch 64:36):
   NMR (Threo): 4,02 (dd, 1H), 2,54 (m, 2H); NMR (Erythro): 4,29 (d, 1H), 2,66 (m, 2H); MS: 410 [M+1]+; HPLC (Rtz.): 1,71 min
Beispiel Nr. Ifa20 (erythro-threo-Isomerengemisch 64:36):
   NMR (Threo): 4,02 (d, 1H), 2,48 (s, 2H); NMR (Erythro): 4,30 (d, 1H), 2,52 (s, 2H); MS: 424 [M+1]+; HPLC (Rtz.): 1,76 min
Beispiel Nr. Iea20 (erythro-threo-Isomerengemisch 59:41):
   NMR (Threo): 4,02 (dd, 1 H), 2,51 (m, 2H); NMR (Erythro): 4,32 (dd, 1H), 2,68 (m, 2H); MS: 424 [M+1]+; HPLC (Rtz.): 1,81 min
Beispiel Nr. Ica1 (erythro-threo-Isomerengemisch 63:37):
   NMR (Threo): 4,02 (dd, 1 H); NMR (Erythro): 4,29 (d, 1 H); MS: 426 [M+1]+; HPLC (Rtz.): 1,79 min
Beispiel Nr. Ifa1 (erythro-threo-Isomerengemisch 62:38):
   NMR (Threo): 4,01 (d, 1H), 2,48 (s, 2H); NMR (Erythro): 4,29 (d, 1H), 2,52 (s, 2H); MS: 440 [M+1]+; HPLC (Rtz.): 1,85 min
Beispiel Nr. Iea1 (erythro-threo-Isomerengemisch 68:32):
   NMR (Threo): 4,02 (dd, 1H), 2,50 (m, 2H); NMR (Erythro): 4,32 (dd, 1H), 2,67 (m, 2H); MS: 440 [M+1]+; HPLC (Rtz.): 1,89 min
Beispiel Nr. Iga95 (erythro-threo-Isomerengemisch 68:32):
   NMR (Threo): 4,02 (dd, 1H), 2,50 (m, 2H); NMR (Erythro): 4,32 (dd, 1H), 2,67 (m, 2H); MS: 502 [M+1]+; HPLC (Rtz.): 1,83 min
Beispiel Nr. Iva83 (erythro-threo-Isomerengemisch 51:49):
   NMR (Threo): 4,21 (d, 1 H), 3,53 (s, 3H), 2,31 (s, 3H); NMR (Erythro): 3,53 (s, 3H), 2,45 (s, 3H); MS: 414 [M+1]+; HPLC (Rtz.): 1,20 min
Beispiel Nr. Iva24 (erythro-threo-Isomerengemisch 64:36):
   NMR (Threo): 3,58 (s, 3H), 2,45 (s, 3H); NMR (Erythro): 4,55 (d, 1 H), 3,54 (s, 3H), 2,31 (s, 3H); MS: 432 [M+1]+; HPLC (Rtz.): 1,27 min
Beispiel Nr. Iva3 (erythro-threo-Isomerengemisch 72:28):
   NMR (Threo): 4,17 (d, 1H), 3,54 (s, 3H), 2,39 (s, 3H); NMR (Erythro): 4,51 (d, 1 H), 3,59 (s, 3H), 2,44 (s, 3H); MS: 430 [M+1]+; HPLC (Rtz.): 1,36 min
Beispiel Nr. Iva17 (erythro-threo-Isomerengemisch 72:28):
   NMR (Threo): 4,24 (d, 1 H), 3,51 (s, 3H), 2,42 (s, 3H); NMR (Erythro): 4,55 (d, 1 H), 3,55 (s, 3H), 2,41 (s, 3H); MS: 414 [M+1]+; HPLC (Rtz.): 1,25 min
Beispiel Nr. Iva2 (erythro-threo-Isomerengemisch 71:29):
   NMR (Threo): 4,19 (d, 1H), 3,54 (s, 3H), 2,40 (s, 3H); NMR (Erythro): 4,51 (d, 1H), 3,59 (s, 3H), 2,44 (s, 3H); MS: 397 [M+2]+; HPLC (Rtz.): 1,22 min
Beispiel Nr. Iva9 (erythro-threo-Isomerengemisch 71:29):
   NMR (Threo): 4,19 (d, 1 H), 3,54 (s, 3H), 2,40 (s, 3H); NMR (Erythro): 4,51 (d, 1 H), 3,58 (s, 3H), 2,44 (s, 3H); MS: 412 [M+1]+; HPLC (Rtz.): 1,26 min
Beispiel Nr. Iva20 (erythro-threo-Isomerengemisch 71:29):
   NMR (Threo): 4,19 (d, 1 H), 3,54 (s, 3H), 2,39 (s, 3H); NMR (Erythro): 4,50 (d, 1 H), 3,59 (s, 3H), 2,44 (s, 3H); MS: 397 [M+2]+; HPLC (Rtz.): 1,22 min
Beispiel Nr. Iva1 (erythro-threo-Isomerengemisch 71:29):
   NMR (Threo): 4,18 (d, 1 H), 3,53 (s, 3H), 2,38 (s, 3H); NMR (Erythro): 4,51 (d, 1 H), 3,58 (s, 3H), 2,43 (s, 3H); MS: 412 [M+1]+; HPLC (Rtz.): 1,32 min
Beispiel Nr. IB10ga6 (erythro-threo-Isomerengemisch 62:38):
   NMR (Threo): 8,48 (s, 1 H), 7,27 (m, 1H), 7,12 (m, 1H), 6,96 (m, 5H), 4,13 (d, 1H), 3,82 (m, 1 H), 3,39 (dd, 1 H), 3,23 (dd, 1 H), 2,65 (s, 3H); MS: 385 [M+1]+; HPLC (Rtz.): 1,57 min; NMR (Erythro): 8,54 (s, 1 H), 7,22 (m, 1 H), 7,09 (m, 1 H), 6,98 (m, 2H), 6,82 (m, 3H), 4,51 (d, 1 H), 3,71 (m, 1 H), 3,50 (dd, 1 H), 3,27 (dd, 1 H), 2,75 (s, 3H); MS: 385 [M+1]+; HPLC (Rtz.): 1,57 min
Beispiel Nr. Erythro-2-IB10ga6 (optisch aktives erythro-2-Isomer):
   NMR (Erythro): 8,54 (s, 1 H), 7,22 (m, 1 H), 7,09 (m, 1 H), 6,98 (m, 2H), 6,82 (m, 3H), 4,51 (d, 1 H), 3,71 (m, 1 H), 3,50 (dd, 1 H), 3,27 (dd, 1 H), 2,75 (s, 3H); MS: 385 [M+1]+; HPLC (Rtz.): 1,57 min
Beispiel Nr. Erythro-1-IB10ga6 (optisch aktives erythro-1-Isomer):
   NMR (Erythro): 8,54 (s, 1 H), 7,22 (m, 1 H), 7,09 (m, 1 H), 6,98 (m, 2H), 6,82 (m, 3H), 4,51 (d, 1H), 3,71 (m, 1H), 3,50 (dd, 1 H), 3,27 (dd, 1 H), 2,75 (s, 3H); MS: 385 [M+1]+; HPLC (Rtz.): 1,57 min
Beispiel Nr. Threo-2- IB10ga6 (optisch aktives threo-2-Isomer):
   NMR (Threo): 8,48 (s, 1H), 7,27 (m, 1 H), 7,12 (m, 1H), 6,96 (m, 5H), 4,13 (d, 1 H), 3,82 (m, 1H), 3,39 (dd, 1 H), 3,23 (dd, 1 H), 2,65 (s, 3H); MS: 385 [M+1]+; HPLC (Rtz.): 1,57 min
Beispiel Nr. IA12aa194 (erythro-threo-Isomerengemisch 80:20):
   NMR (Threo): 4,24 (d, 1 H), 2,04 (s, 3H); NMR (Erythro): 4,16 (d, 1H), 2,02 (s, 3H); MS: 452 [M-42]+; HPLC (Rtz.): 1,57 min
Beispiel Nr. Erythro-IA12aa49:
   NMR (Erythro): 4,33 (d, 1H), 3,87 (s, 3H), 3,76 (s, 3H), 2,12 (s, 3H); MS: 434 [M-42]+; HPLC (Rtz.): 1,57 min
Beispiel Nr. Threo-1-IA12aa49:
   NMR (Threo1): 3,95 (d, 1H), 3,75 (s, 3H), 3, 96 (s, 3H), 1,91 (s, 3H); MS: 434 [M-42]+; HPLC (Rtz.): 1,57 min
Beispiel Nr. Threo-2-IB12aa49:
   NMR (Threo2): 3,95 (d, 1 H), 3,75 (s, 3H), 3, 96 (s, 3H), 1,91 (s, 3H); MS: 434 [M-42]+; HPLC (Rtz.): 1,57 min

### (B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| 75 Gewichtsteile einer Verbindung der Formel (I), | | |
|---|---|---|
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| 25 Gewichtsteile einer Verbindung der Formel (I), | | |
|---|---|---|
| 5 | " | 2,2'-dinaphthylmethan-6,6'-disulfonsaures Natrium |
| 2 | " | oleoylmethyltaurinsaures Natrium, |
| 1 | Gewichtsteil | Polyvinylalkohol, |
| 17 | Gewichtsteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Herbizide Wirkung im Vorauflauf

Samen von mono- bzw. dikotylen Unkraut- bzw. Kulturpflanzen wurden in Holzfasertöpfen in sandiger Lehmerde ausgelegt und mit Erde abgedeckt. Die in Form von benetzbaren Pulvern (WP) formulierten erfindungsgemäßen Verbindungen (I) wurden dann als wäßrige Suspension bzw. Emulsion mit einer Wasseraufwandmenge von umgerechnet 600 l/ha unter Zusatz von 0,2% Netzmittel auf die Oberfläche der Abdeckerde appliziert.

Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen. Erfindungsgemäße Verbindungen (I), wie beispielsweise die Verbindungen Nr. IA12a57, IA12aa1, IA12aa193, IA12aa23, IA12aa48, IA12aa50, IA12aa78, IA12ab53, Iaa1, Iaa10, Iaa20, Iaa23, Iaa32, Iaa53, Iaa6, Iaa83, Iaa95, IB8aa1, IB8aa10, IB8aa43, IB8aa6, IB9aa1, IB9aa10, IB9aa20, IB9aa23, IB9aa25, IB9aa43, IB9aa49, IB9aa6, IB9ba6, IBXaa10, IBXaa20, IBXaa43, IBXaa6, Ica6, Ica95, lea6, Iea95, Ifa6, Ifa95, Iga95, Ioa6, Ioa95, Ita6, Ita95, Iva259, Iva6, Iva6, erythro-1-IB10ga6, erythro-2-IB10ga6, erythro-IA12aa49, IA12aa194, Iaa17, Iaa29, Iaa3, Ica83, Iaa9, IB10ga6, threo-2-IB8aa43, Ica1, Ica17, Ica2, Ica20, Ica23, Ica25, Ica29, Ica3, Iea1, Iea17, Iea2, Iea20, Iea23, Iea25, Iea29, Iea3, Iea83, Iea9, Ifa1, Ifa17, Ifa20, Ifa23, Ifa25, Ifa29, Ifa3, Ifa83, Ifa9, Iga95, Ioa1, Ioa17, Ioa2, Ioa20, Ioa24, Ioa29, Ioa3, Ioa83, Ioa9, Iva1, Iva17, Iva2, Iva20, Iva24, Iva3, Iva83, Iva9, threo-1-IA12aa49, threo-2- IB10ga6 und threo-2-IB12aa49 aus den obigen Tabellen 1 bis 42f, als erythro-threo-Gemische oder als threo-Racemate oder threo-2-Enantiomere, eine gute herbizide Wirksamkeit (70% bis 100% Wirkung) gegen mehrere Schadpflanzen bei einer Aufwandmenge von 320 g oder weniger Aktivsubstanz pro Hektar im Vorauflauf auf.

Beispielsweise haben die Verbindungen Nr. Iaa20, Iaa23, Iaa53, Iaa95, Ioa95, IA12aa78, IB9aa6, IB9ba6, IB9aa10, IB9aa23, IB9aa25, IB9aa43, threo-2-IB8aa43, threo-1-IA12aa49, IA12aa194, Iaa17, Ifa1, Iva20 und Ica20 eine sehr gute Wirkung (90-100%) gegen Schadpflanzen wie Viola tricolor im Vorauflaufverfahren bei einer Aufwandmenge von 0,32 kg Aktivsubstanz pro Hektar.

Beispielsweise haben auch die Verbindungen Nr. IA12aa78, IB9aa6, IB9aa25, IB9aa43, IB9ba6, threo-2-IB8aa43, IVa9 und IA12aa194 eine sehr gute Wirkung (90-100%) gegen Schadpflanzen wie Stellaria media im Vorauflaufverfahren bei einer Aufwandmenge von 0,32 kg Aktivsubstanz pro Hektar.

Beispielsweise haben auch die Verbindungen Nr. Iaa95, IA12aa78, IB9aa6, IB9aa10, IB9aa25, threo-2-IB8aa43, IB9aa43 und IB9ba6 eine sehr gute Wirkung (80-100%) gegen Schadpflanzen wie Stellaria media im Vorauflaufverfahren bei einer Aufwandmenge von 0,32 kg Aktivsubstanz pro Hektar.

Beispielsweise haben auch die Verbindungen Nr. Iaa23, Iaa53, IB9aa1, IB9aa10, IB9aa25, IB9aa23, IB9aa43, Ioa95, Ioa2, Iea1, Ioa9, threo-2-IB8aa43, threo-1-IA12aa49 und IA12aa194 eine sehr gute Wirkung (80-100%) gegen Schadpflanzen wie Polygonum convolvulus im Vorauflaufverfahren bei einer Aufwandmenge von 0,32 kg Aktivsubstanz pro Hektar.

Beispielsweise haben auch die Verbindungen Nr. Iaa95, IB9aa6, IB9aa10, IB9aa25, IB9aa1, IB9aa43, Iea29, threo-2-IB8aa43, threo-1-IA12aa49 und IA12aa194 eine sehr gute Wirkung (80-100%) gegen Schadpflanzen wie Amarantus retroflexus im Vorauflaufverfahren bei einer Aufwandmenge von 0,32 kg Aktivsubstanz pro Hektar. Beispielsweise haben auch die Verbindungen Nr. Iaa23, Iaa95, IB9aa23, IB9aa25, IB9aa6, IB9ba6 und IB9aa43 eine sehr gute Wirkung (80-100%) gegen Schadpflanzen wie Setaria viridis im Vorauflaufverfahren bei einer Aufwandmenge von 0,32 kg Aktivsubstanz pro Hektar.

Beispielsweise haben auch die Verbindungen Nr. Iaa95, IB9aa25, IB9aa1, IB9aa43, Ica2, threo-2-IB8aa43, threo-1-IA12aa49 und IA12aa194 eine sehr gute Wirkung (80-100%) gegen Schadpflanzen wie Alopecurus myosuroides im Vorauflaufverfahren bei einer Aufwandmenge von 0,32 kg Aktivsubstanz pro Hektar.

Beispielsweise haben auch die Verbindungen Nr. Ioa83 und threo-1-IA12aa49 eine sehr gute Wirkung (80-100%) gegen Schadpflanzen wie Abuthilon theophrasti im Vorauflaufverfahren bei einer Aufwandmenge von 0,32 kg Aktivsubstanz pro Hektar. Beispielsweise haben auch die Verbindungen Nr. Ica2, Iea23, Iaa17, IA12aa194 und threo-1-IA12aa49 eine sehr gute Wirkung (80-100%) gegen Schadpflanzen wie Veronica persica im Vorauflaufverfahren bei einer Aufwandmenge von 0,32 kg Aktivsubstanz pro Hektar.

Beispielsweise haben auch die Verbindungen Nr. Ica17, Iva25 und threo-1-IA12aa49 eine sehr gute Wirkung (80-100%) gegen Schadpflanzen wie Veronica persica im Vorauflaufverfahren bei einer Aufwandmenge von 0,32 kg Aktivsubstanz pro Hektar.

### 2. Herbizide Wirkung im Nachauflauf

Samen von mono- bzw. dikotylen Unkraut- bzw. Kulturpflanzen wurden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat wurden die Versuchspflanzen im Einblattstadium behandelt, wobei die in Form von benetzbaren Pulvern (WP) formulierten erfindungsgemäßen Verbindungen (I) als wäßrige Suspension bzw. Emulsion mit einer Wasseraufwandmenge von umgerechnet 600 l/ha unter Zusatz von 0,2% Netzmittel auf die grünen Pflanzenteile gesprüht wurden. Nach ca. 3 Wochen Standzeit der Versuchspflanzen im Gewächshaus unter optimalen Wachstumsbedingungen wurde die Wirkung der Präparate visuell im Vergleich zu unbehandelten Kontrollen in Prozentwerten bonitiert. Beispielsweise bedeutet 100% Wirkung = Pflanzen sind abgestorben, 50% herbizide Wirkung oder Schaden = Pflanzen zu 50% reduziert bzw. Pflanzenmasse um 50% reduziert, 0 % Wirkung = wie Kontrollpflanzen.

Wie die Ergebnisse zeigen, weisen erfindungsgemäße Verbindungen (I), wie beispielsweise die Verbindungen Nr. IA12a57, IA12aa1, IA12aa193, IA12aa23, IA12aa48, IA12aa50, IA12aa78, IA12ab53, Iaa1, Iaa10, Iaa20, Iaa23, Iaa32, Iaa53, Iaa6, Iaa83, Iaa95, IB8aa1, IB8aa10, IB8aa43, IB8aa6, IB9aa1, IB9aa10, IB9aa20, IB9aa23, IB9aa25, IB9aa43, IB9aa49, IB9aa6, IB9ba6, IBXaa10, IBXaa20, IBXaa43, IBXaa6, Ica6, Ica95, Iea6, Iea95, Ifa6, Ifa95, Iga95, Ioa6, Ioa95, Ita6, Ita95, Iva259, Iva6, Iva6, erythro-1-IB10ga6, erythro-2-IB10ga6, erythro-IA12aa49, IA12aa194, Iaa17, Iaa29, Iaa3, Ica83, Iaa9, IB10ga6, threo-2-IB8aa43, Ica1, Ica17, Ica2, Ica20, Ica23, Ica25, Ica29, Ica3, Iea1, Iea17, Iea2, Iea20, Iea23, Iea25, Iea29, Iea3, Iea83, Iea9, Ifa1, Ifa17, Ifa20, Ifa23, Ifa25, Ifa29, Ifa3, Ifa83, Ifa9, Iga95, Ioa1, Ioa17, Ioa2, Ioa20, Ioa24, Ioa29, Ioa3, Ioa83, Ioa9, Iva1, Iva17, Iva2, Iva20, Iva24, Iva3, Iva83, Iva9, threo-1-IA12aa49, threo-2- IB10ga6 und threo-2-IB12aa49 aus den obigen Tabellen 1 bis 42f als erythro-threo-Gemische oder als threo-Racemate oder threo-2-Enantiomere, eine gute herbizide Wirksamkeit (70 bis 100% Wirkung) gegen mehrere Schadpflanzen bei einer Aufwandmenge von 320 g oder weniger Aktivsubstanz pro Hektar im Nachauflauf auf.

Beispielsweise haben dabei die Verbindungen Nr. IB9aa6, Iaa1, Iaa20, IB9ba6, IB9aa10, IB9aa1, IB9aa23, IB9aa25, IB9aa43, Iaa10, Iaa23, Iaa95, Ioa95, Iea9, Ifa9, Iaa29, threo-2-IB8aa43 und IA12aa194 eine sehr gute Wirkung (90-100%) gegen Schadpflanzen wie Polygonum convolvulus galli im Nachauflaufverfahren bei einer Aufwandmenge von 0,32 kg Aktivsubstanz pro Hektar.

Beispielsweise haben dabei auch die Verbindungen Nr. IB9aa6, Iaa1, Iaa20, Iaa23, IB9aa1, IB9ba6, IB9aa10, IB9aa23, IB9aa25, IB9aa43 und Iaa10 eine sehr gute Wirkung (80-100%) gegen Schadpflanzen wie Veronica repens im Nachauflaufverfahren bei einer Aufwandmenge von 0,32 kg Aktivsubstanz pro Hektar.

Beispielsweise haben dabei auch die Verbindungen Nr. Iaa95, Iaa23, IB9aa1, IB9aa10, IB9aa23, IB9aa43, IB9aa25, IB9aa6, Iaa10, Iaa17, Ica83, threo-2-IB8aa43 und IA12aa194 eine sehr gute Wirkung (80-100%) gegen Schadpflanzen wie Viola tricolor im Nachauflaufverfahren bei einer Aufwandmenge von 0,32 kg Aktivsubstanz pro Hektar.

Beispielsweise haben dabei auch die Verbindungen Nr. Iaa23, IB9aa1, Iaa1, IB9aa23, IB9aa43 und IB9aa25 eine sehr gute Wirkung (80-100%) gegen Schadpflanzen wie Abuthilon theophrasti im Nachauflaufverfahren bei einer Aufwandmenge von 0,32 kg Aktivsubstanz pro Hektar.

Beispielsweise haben dabei auch die Verbindungen Nr. IB9ba6, IB9aa43, IB9aa6, IB9aa23, IB9aa25, Ifa20, Ica20, Iva2, Ioa2, Iea2, Ica2, threo-2-IB8aa43 und Iva17 eine sehr gute Wirkung (80-100%) gegen Schadpflanzen wie Setaria viridis im Nachauflaufverfahren bei einer Aufwandmenge von 0,32 kg Aktivsubstanz pro Hektar auf.

Beispielsweise haben dabei auch die Verbindungen Nr. IB9aa43, IB9aa1, IB9ba6, IB9aa23, IB9aa6, threo-2-IB8aa43 und IB9aa25 eine sehr gute Wirkung (80-100%) gegen Schadpflanzen wie Lolium multiflorum im Nachauflaufverfahren bei einer Aufwandmenge von 0,32 kg Aktivsubstanz pro Hektar.

Beispielsweise haben dabei auch die Verbindungen Nr. IB9aa1, IB9ba6, IB9aa43, IB9aa25, IB9aa6, Iaa95, Iaa23, Ioa95, IB9aa23, Iva1, Iea1, Ica1, Iea20, Ica20, Iva9, Ioa9, Ioa2, Iea2, Iea23, Ica23, Iva17, Ioa17, Ioa83, Ica83, threo-2-IB8aa43, threo-1-IA12aa49 und IA12aa194 eine sehr gute Wirkung (80-100%) gegen Schadpflanzen wie Echinochloa crus-galli im Nachauflaufverfahren bei einer Aufwandmenge von 0,32 kg Aktivsubstanz pro Hektar.

Beispielsweise haben dabei auch die Verbindungen Nr. IB9aa1, IB9ba6, IB9aa43, IB9aa25, IB9aa6, IB9aa10, Iaa95, Iaa23, Ioa95, threo-2-IB8aa43 und IB9aa23 eine sehr gute Wirkung (80-100%) gegen Schadpflanzen wie Avena fatua im Nachauflaufverfahren bei einer Aufwandmenge von 0,32 kg Aktivsubstanz pro Hektar.

Beispielsweise haben dabei auch die Verbindungen Nr. IB9aa1, IB9ba6, IB9aa43, IB9aa25, Ioa95, threo-2-IB8aa43 und IB9aa23 eine sehr gute Wirkung (80-100%) gegen Schadpflanzen wie Alopecurus myosuroides im Nachauflaufverfahren bei einer Aufwandmenge von 0,32 kg Aktivsubstanz pro Hektar.

Beispielsweise haben dabei auch die Verbindungen Nr. Iaa9, Iea9, Ifa9, Iva2, Ioa2, Iea2, Iaa17, Iva24, Ioa24, Iaa29, Ica29, IA12aa194 und IB10ga6 eine sehr gute Wirkung (80-100%) gegen Schadpflanzen wie Veronica persica im Nachauflaufverfahren bei einer Aufwandmenge von 0,32 kg Aktivsubstanz pro Hektar.

### 3. Herbizide Wirkung und Kulturpflanzenverträglichkeit

3.1 In weiteren Versuchen im Gewächshaus werden Samen von Kulturpflanzen in Töpfen in sandigem Lehmboden ausgelegt, mit Erde abgedeckt und unter guten Wachstumsbedingungen angezogen und analog den Schadpflanzen in Abschnitt 1 im Vorauflaufverfahren behandelt und ausgewertet. Die Ergebnisse zeigen, dass die erfindungsgemäßen Verbindungen zweikeimblättrige Kulturen wie z.B. Soja, Baumwolle, Raps, Zuckerrüben und Kartoffeln im Vorauflaufverfahren selbst bei hohen Wirkstoffdosierungen ungeschädigt lassen. Einige Substanzen schonen darüber hinaus aus Gramineen-Kulturen wie z.B. Gerste, Weizen, Roggen, Sorghum-Hirsen, Mais oder Reis.

## Patentansprüche

1. Verbindungen der Formel (I) oder deren Salze, 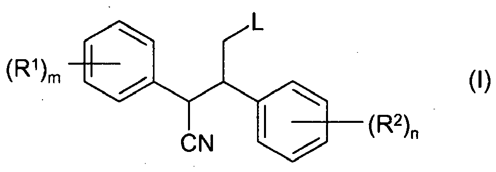 in welcher
L einen Rest der Formel 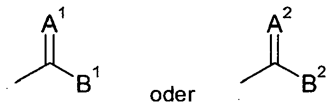 bedeutet,
A¹ Sauerstoff, Schwefel oder =N-R^{A} bedeutet,
B¹ einen Rest der Formeln (B1 bis (B14) bedeutet, 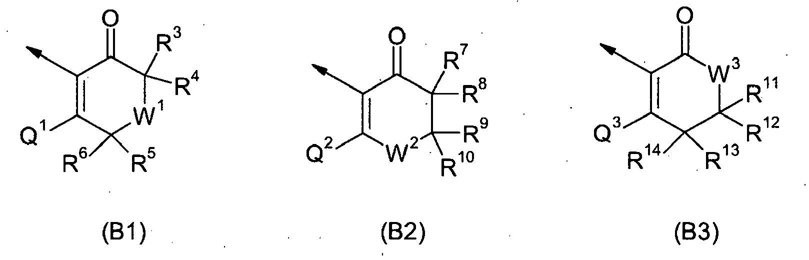 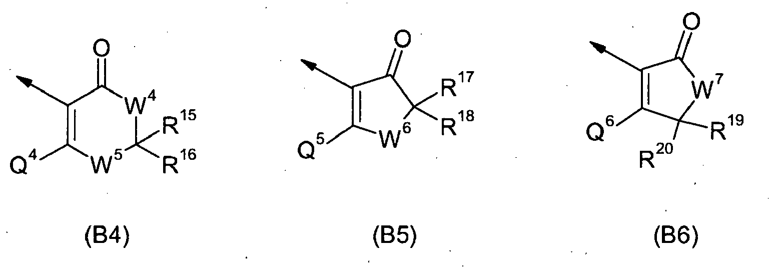 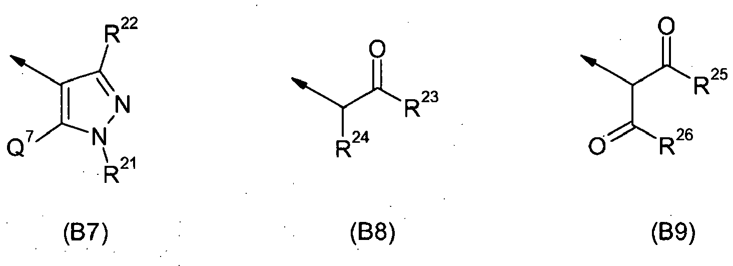 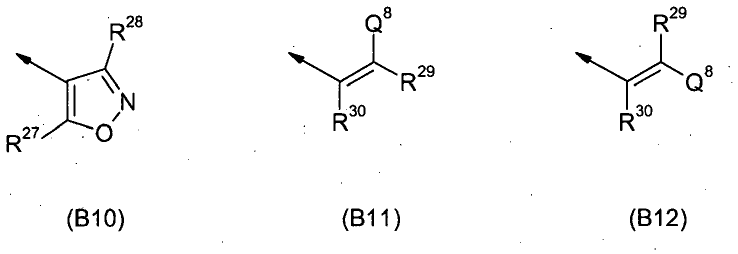 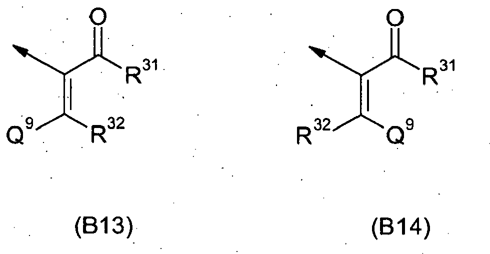
A² einen Rest der Formeln (A1) bis (A12) bedeutet, 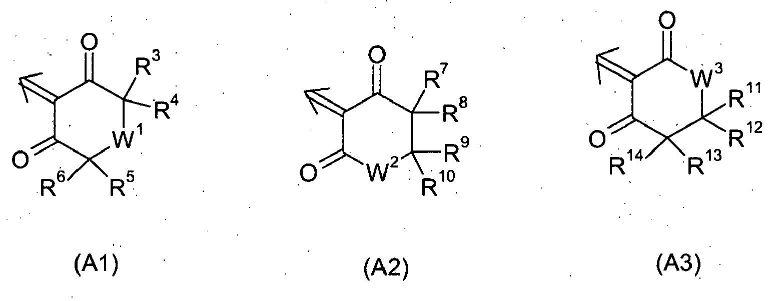 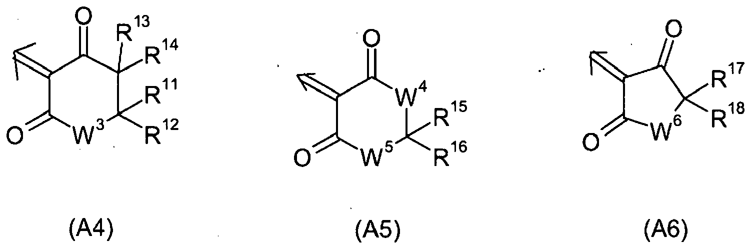 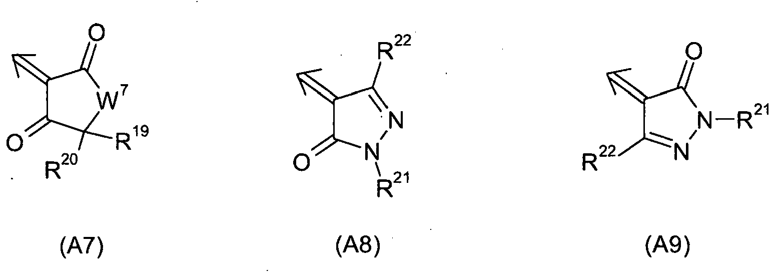 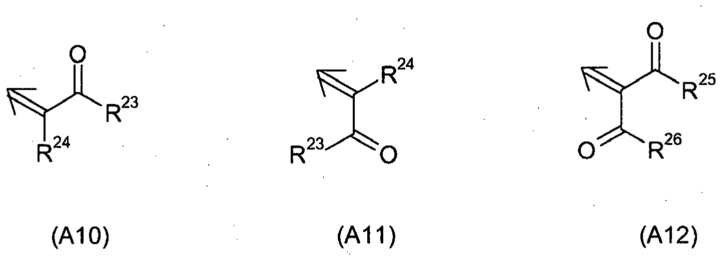
B² Hydroxy, Thio, Halogen oder eine Gruppe der Formel OR³³, OM, SR³⁴, SM oder -NR^{B}R^{C} bedeutet,
W¹ und W⁶ jeweils unabhängig voneinander die divalente Gruppe Sauerstoff, Schwefel oder eine Gruppe der Formel NH, N-[(C₁-C₄)Alkyl], C=O (Carbonyl), S=O (Sulfinyl), SO₂ (Sulfonyl) oder CR³⁵R³⁶ bedeuten,
W², W³, W⁴, W⁵ und W⁷ jeweils unabhängig voneinander die divalente Gruppe Sauerstoff, Schwefel oder eine Gruppe der Formel NH, N-[(C₁-C₄)Alkyl], C=O (Carbonyl), S=O (Sulfinyl) oder SO₂ (Sulfonyl) bedeuten,
Q¹, Q², Q³, Q⁴, Q⁵, Q⁶, Q⁷, Q⁸ und Q⁹ jeweils unabhängig voneinander Hydroxy, Thio, Halogen oder eine Gruppe der Formel OR³⁷, SR³⁸, SM oder OM bedeuten,
M ein Äquivalent eines Kations bedeutet,
R^{A}, R^{B} und R^{C} jeweils unabhängig voneinander Wasserstoff, Hydroxy, (C₁-C₄)Alkoxy, (C₁-C₄)Alkyl, (C₁-C₄)Haloalkyl, Phenyl oder Benzyl, wobei jeder der letztgenannten beiden Reste gegebenenfalls ein- oder mehrfach, gleich oder verschieden substituiert ist,
oder -NR*R**, wobei R*, R** jeweils unabhängig voneinander H, (C₁-C₈)Alkyl, (C₂-C₈)Alkenyl, (C₂-C₈)Alkinyl, (C₁-C₄)Alkoxy-(C₁-C₄)alkyl, (C₁-C₆)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy oder im Fall Cycloalkyl auch Oxo substituiert ist, bedeuten oder
R* und R** zusammen mit dem N-Atom einen 3- bis 8-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten,
(R¹)ₘ m Substituenten R¹ bedeutet,
wobei R¹, wenn m = 1, oder jeder der Substituenten R¹, wenn m größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio, (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR³⁹, C(O)NR⁴⁰R⁴¹, C(O)-Het¹, NR⁴²R⁴³ oder Het² bedeutet
oder wobei jeweils zwei am Ring ortho-ständige Gruppen R¹ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden,
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₈)Alkyl, (C₂-C₆)Alkenyl, (C₂-C₆)Alkinyl, (C₁-C₈)Alkoxy, (C₁-C₈)Alkylthio; (C₁-C₈)Alkylsulfinyl, (C₁-C₈)Alkylsulfonyl, (C₁-C₆)Haloalkyl, (C₁-C₆)Haloalkoxy, (C₁-C₆)Haloalkylthio, (C₁-C₆)Haloalkylsulfinyl, (C₁-C₆)Haloalkylsulfonyl, (C₂-C₆)Haloalkenyl, (C₂-C₆)Haloalkinyl, (C₁-C₆)Alkoxy-(C₁-C₄)alkyl, (C₁-C₆)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR⁴⁴, C(O)NR⁴⁵R⁴⁶, C(O)-Het³, NR⁴⁷R⁴⁸ oder Het⁴ bedeutet
oder
wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹ und R²⁰ jeweils unabhängig voneinander Wasserstoff, Halogen, Cyano, Nitro, Hydroxy, (C₁-C₄)Alkyl, (C₁-C₄)Haloalkyl, (C₁-C₄)Alkoxy, (C₁-C₄)Haloalkoxy, (C₁-C₄)Alkoxy-(C₁-C₄)alkyl, (C₁-C₄)Haloalkoxy-(C₁-C₄)alkyl, (C₃-C₆)Cycloalkyl, (C₃-C₆)Halocycloalkyl, (C₃-C₆)Cycloalkoxy, (C₂-C₆)Alkenyl, (C₂-C₆)Haloalkenyl, (C₂-C₆)Alkinyl, (C₂-C₆)Haloalkinyl, (C₁-C₄)Alkylthio, (C₁-C₄)Alkylsulfonyl, (C₁-C₄)Alkylsulfinyl, Phenyl, das gegebenenfalls substituiert ist, oder eine Gruppe C(O)OR⁴⁹, C(O)NR⁵⁰R⁵¹, C(O)Het⁵, NR⁵²R⁵³ oder Het⁶ bedeuten oder
R³ und R⁵ in der Gruppe der Formel (B1) oder (A1) gemeinsam eine divalente Brücke aus einer geradkettigen Alkylen- oder Alkenylen-gruppe mit 2 oder 3 C-Atomen, wobei eine CH₂-Gruppe in der Kette noch durch ein Sauerstoffatom ersetzt sein kann und wobei eine CH₂-Gruppe oder CH-Gruppe in der Kette gegebenenfalls durch ein oder mehrere Reste aus der Gruppe (C₁-C₄)Alkyl, (C₁-C₄)Haloalkyl, (C₁-C₄)Alkoxy und (C₁-C₄)Haloalkoxy substituiert ist,
R²¹ Wasserstoff, (C₁-C₄)Alkyl, (C₁-C₄)Halogenalkyl, (C₃-C₆)Cycloalkyl, (C₃-C₆)Halogencycloalkyl oder eine Gruppe C(O)OR⁵⁴, C(O)NR⁵⁵R⁵⁶, C(O)Het⁷, NR⁵⁷R⁵⁸ oder Het⁸ bedeutet,
R²² Wasserstoff, (C₁-C₄)Alkyl, (C₁-C₄)Halogenalkyl, (C₃-C₆)Cycloalkyl, (C₃-C₆)Halogencycloalkyl, (C₁-C₄)Alkylthio, (C₁₋C₄)Alkylsulfonyl, (C₁-C₄)Alkylsulfinyl oder eine Gruppe der Formel C(O)OR⁵⁹, C(O)NR⁶⁰R⁶¹, C(O)Het⁹, NR⁶²R⁶³ oder Het¹⁰ bedeutet,
R²³ und R²⁹ jeweils unabhängig voneinander Wasserstoff, (C₁-C₄)Alkyl, (C₁-C₄)Halogenalkyl, (C₃-C₆)Cycloalkyl, (C₃-C₆)Halogencycloalkyl, (C₁-C₄)Alkoxy oder (C₁-C₄)Halogenalkoxy bedeuten,
R²⁴ und R³⁰ jeweils unabhängig voneinander Wasserstoff, Halogen, Cyano, (C₁-C₄)Alkyl, (C₁-C₄)Halogenalkyl, (C₃-C₆)Cycloalkyl, (C₃-C₆)Halogencycloalkyl, (C₁-C₄)Alkoxy oder (C₁-C₄)Halogenalkoxy bedeuten,
R²⁵, R²⁶, R³¹ und R³² jeweils unabhängig voneinander Wasserstoff, (C₁-C₄)Alkyl, (C₁-C₄)Halogenalkyl, (C₁-C₄)Alkoxy, (C₁-C₄)Halogenalkoxy, (C₁-C₄)Alkoxy-(C₁-C₄)alkyl, (C₁-C₄)Halogenalkoxy-(C₁-C₄)alkyl, (C₃-C₆)Cycloalkyl, (C₃-C₆)Halogencycloalkyl, (C₃-C₆)Cycloalkoxy, (C₂-C₆)Alkenyl, (C₂-C₆)Halogenalkenyl, (C₂-C₆)Alkinyl, (C₂-C₆)Halogenalkinyl, (C₁-C₄)Alkylthio, (C₁-C₄)Alkylsulfonyl, (C₁-C₄)Alkylsulfinyl, NR⁶⁴R⁶⁵ oder Het¹¹ bedeuten,
R²⁷ Wasserstoff, (C₁-C₆)Alkyl, (C₁-C₄)Halogenalkyl, (C₃-C₆)Cycloalkyl oder (C₃-C₆)Halogencycloalkyl bedeutet,
R²⁸ Wasserstoff, (C₁-C₆)Alkyl, (C₁-C₄)Halogenalkyl oder einen Rest der Formel C(O)OR⁶⁶ bedeutet,
R³³, R³⁴, R³⁷ und R³⁸ jeweils unabhängig voneinander (C₁-C₈)Alkyl, (C₃-C₆)Cycloalkyl, Phenyl, wobei jeder der letztgenannten drei Reste jeweils unabhängig voneinander unsubstituiert oder durch einen oder mehrere Reste aus der Gruppe Halogen, Cyano, Nitro, Hydroxy, (C₁-C₄)Alkoxy, (C₁-C₄)Alkylthio und im Falle eines Cycloalkyl- oder Phenylrestes als Basisrest auch (C₁-C₄)Alkyl und (C₁-C₄)Haloalkyl substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
R³⁵ und R³⁶ jeweils unabhängig voneinander wie R³ definiert sind,
R³⁹, jeweils unabhängig voneinander Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder die genannte Gruppe M bedeuten,
R⁴⁰, R⁴¹, R⁴², R⁴³, R⁴⁵, R⁴⁶, R⁴⁷, R⁴⁸, R⁵⁰, R⁵¹, R⁵², R⁵³, R⁵⁵, R⁵⁶, R⁵⁷, R⁵⁸, R⁶⁰, R⁶¹,R⁶², R⁶³, R⁶⁴, R⁶⁵, R⁶⁹ und R⁷⁰ jeweils unabhängig voneinander Wasserstoff, (C₁-C₆)Alkyl, (C₂-C₆)Alkenyl oder (C₂-C₆)Alkinyl, wobei jeder der letztgenannten 3 Reste jeweils unabhängig voneinander unsubstituiert oder durch einen oder mehrere Reste aus der Gruppe Halogen, Nitro, Cyano und Phenyl, das gegebenenfalls substituiert ist, substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, Phenyl und Benzyl, wobei jeder der letztgenannten 2 Reste gegebenenfalls substituiert ist, substituiert ist, bedeuten,
R⁶⁷ Wasserstoff, (C₁-C₈)Alkyl oder (C₁-C₈)Halogenalkyl, wobei jeder der letztgenannten 2 Reste unabhängig voneinander gegebenenfalls im Alkylteil durch Sauerstoff oder Schwefel unterbrochen ist, oder Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl, (C₂-C₄)Alkinyl, (C₃-C₆)Cycloalkyl, (C₁-C₄)Halogenalkyl und (C₃-C₆)Halogencycloalkyl substituiert ist, bedeutet,
R⁶⁸ (C₁-C₈)Alkyl, (C₁-C₈)Halogenalkyl, (C₃-C₆)Cycloalkyl, (C₃-C₆)Halogencycloalkyl, (C₂-C₈)Alkenyl, (C₂-C₈)Haloalkenyl oder (C₂-C₄)Alkinyl bedeutet,
R⁷¹ (C₁-C₆)Alkyl, (C₁-C₆)Halogenalkyl oder Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl, (C₂-C₄)Alkinyl, (C₃-C₆)Cycloalkyl, (C₁-C₄)Halogenalkyl und (C₃-C₆)Halogencycloalkyl substituiert ist, bedeutet,
Het¹, Het², Het³, Het⁴, Het⁵, Het⁶, Het⁷, Het⁸, Het⁹, Het¹⁰, Het¹¹ und Het¹² jeweils unabhängig voneinander einen gesättigten oder teilungesättigten Rest eines Heterocyclus mit 3 bis 9 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, bedeuten und
m, n jeweils unabhängig voneinander 0, 1, 2, 3, 4 oder 5 bedeuten.

2. Verbindungen oder deren Salze gemäß Anspruch 1, **dadurch gekennzeichnet, dass**
(R¹)ₘ m Substituenten R¹ bedeutet,
wobei R¹, wenn m = 1, oder jeder der Substituenten R¹, wenn m größer als 1, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR³⁹, C(O)NR⁴⁰R⁴¹, C(O)-Het¹, NR⁴²R⁴³ oder Het² bedeutet
oder wobei jeweils zwei am Ring ortho-ständige Gruppen R¹ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht, wobei die Gruppe -Z¹-A*-Z² zusammen mit den an die Gruppe gebundenen C-Atomen des Phenylrings einen ankondensierten 5 oder 6 Ring bilden,
R³⁹ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder das Äquivalent eines Kations bedeutet,
R⁴⁰, R⁴¹, R⁴², R⁴³ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, bedeuten,
Het¹ und Het² jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, bedeuten und
m 0, 1, 2, 3, 4 oder 5 bedeutet.

3. Verbindungen oder deren Salze gemäß Anspruch 1 oder 2, **dadurch gekennzeichnet, dass**
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₆)Alkyl, (C₂-C₄)Alkenyl, (C₂-C₄)Alkinyl, (C₁-C₆)Alkoxy, (C₁-C₆)Alkylthio, (C₁-C₆)Alkylsulfinyl, (C₁-C₆)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₂-C₄)Haloalkenyl, (C₂-C₄)Haloalkinyl, (C₁-C₄)Alkoxy-(C₁-C₄)alkyl, (C₁-C₄)Haloalkoxy-(C₁-C₄)alkyl, (C₃-C₆)Cycloalkyl, das gegebenenfalls durch einen oder mehrere Reste aus der Gruppe Halogen und (C₁-C₄)Alkyl substituiert ist, (C₃-C₆)Cycloalkoxy, das gegebenenfalls durch einen oder mehrere Reste aus der Gruppe Halogen und (C₁-C₄)Alkyl substituiert ist, oder einen Rest der Formel C(O)OR⁴⁴, C(O)NR⁴⁵R⁴⁶, C(O)-Het³, NR⁴⁷R⁴⁸ oder Het⁴ bedeutet
oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht, wobei die Gruppe -Z³-A**-Z⁴ zusammen mit den an die Gruppe gebundenen C-Atomen des Phenylrings einen ankondensierten 5 oder 6 Ring bilden,
R⁴⁴ Wasserstoff, (C₁-C₄)Alkyl, (C₁-C₄)Halogenalkyl, (C₃-C₆)Cycloalkyl, (C₃-C₆)Halogencycloalkyl, (C₂-C₄)Alkenyl, (C₂-C₄)Halogenalkenyl, (C₂-C₄)Alkinyl oder das Äquivalent eines Kations bedeutet,
R⁴⁵, R⁴⁶, R⁴⁷ und R⁴⁸ jeweils unabhängig voneinander Wasserstoff oder (C₁-C₄)Alkyl, das unsubstituiert oder durch einen oder mehrere Reste aus der Gruppe Halogen, Nitro, Cyano und Phenyl substituiert ist, oder (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, (C₁-C₄)Alkyl, (C₁-C₄)Haloalkyl, Phenyl und Benzyl substituiert ist, bedeuten,
Het³ und Het⁴ jeweils unabhängig voneinander einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, und Oxo substituiert ist, bedeuten und
n 0, 1, 2, 3, 4 oder 5 bedeutet.

4. Verbindungen oder deren Salze gemäß einem der Ansprüche 1 bis 3, **dadurch gekennzeichnet, dass**
(R¹)ₘ m Substituenten R¹ bedeutet,
wobei R¹, wenn m = 1, oder jeder der Substituenten R¹, wenn m größer als 1, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio, (C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl, oder einen Rest der Formel C(O)OR³⁹, C(O)NR⁴⁰R⁴¹, C(O)-Het¹, NR⁴²R⁴³ oder Het² bedeutet,
oder wobei jeweils zwei am Ring ortho-ständige Gruppen R¹ gemeinsam eine Gruppe der Formel -Z¹-A*-Z² bedeuten, in welcher
A* für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z¹ für eine direkte Bindung, O oder S steht und
Z² für eine direkte Bindung, O oder S steht,
wobei die Gruppe -Z¹-A*-Z² zusammen mit den an die Gruppe gebundenen C-Atomen des Phenylrings einen ankondensierten 5 oder 6 Ring bilden,
R³⁹ Wasserstoff, (C₁-C₄)Alkyl oder dasÄquivalent eines Kations bedeutet,
R⁴⁰, R⁴¹, R⁴² und R⁴³ jeweils unabhängig voneinander Wasserstoff, (C₁-C₄)Alkyl, (C₁-C₄)Haloalkyl, Benzyl, (C₃-C₆)Cycloalkyl oder Phenyl bedeuten, insbesondere Wasserstoff, Methyl oder Ethyl bedeuten,
Het¹ und Het² jeweils unabhängig voneinandereine Morpholino- Piperidino- oder Pyrrolidinogruppe bedeuten,
m 0, 1, 2, 3, 4 oder 5 bedeutet,
(R²)ₙ n Substituenten R² bedeutet,
wobei R², wenn n = 1, oder jeder der Substituenten R², wenn n größer als 1 ist, unabhängig voneinander Halogen, Cyano, Nitro, Hydroxy, (C₁-C₄)Alkyl, (C₁-C₄)Alkoxy, (C₁-C₄)Alkylthio, (C₁-C₄)Alkylsulfinyl, (C₁-C₄)Alkylsulfonyl, (C₁-C₄)Haloalkyl, (C₁-C₄)Haloalkoxy, (C₁-C₄)Haloalkylthio,
(C₁-C₄)Haloalkylsulfinyl, (C₁-C₄)Haloalkylsulfonyl, (C₁-C₄)Alkoxy-(C₁-C₄)alkyl, (C₃-C₆)Cycloalkyl oder einen Rest der Formel C(O)OR⁴⁴, C(O)NR⁴⁵R⁴⁶, C(O)-Het³, NR⁴⁷R⁴⁸ oder Het⁴ bedeutet
oder wobei jeweils zwei am Ring ortho-ständige Gruppen R² gemeinsam eine Gruppe der Formel -Z³-A**-Z⁴ bedeuten, in welcher
A** für eine Alkylengruppe mit 1 bis 4 C-Atomen steht, die gegebenenfalls durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
Z³ für eine direkte Bindung, O oder S steht und
Z⁴ für eine direkte Bindung, O oder S steht,
wobei die Gruppe -Z³-A**-Z⁴ zusammen mit den an die Gruppe gebundenen C-Atomen des Phenylrings einen ankondensierten 5 oder 6 Ring bilden,
R⁴⁴ Wasserstoff, (C₁-C₄)Alkyl oder dasÄquivalent eines Kations bedeutet,
R⁴⁵, R⁴⁶, R⁴⁷ und R⁴⁸ jeweils unabhängig voneinander Wasserstoff oder (C₁-C₄)Alkyl, (C₁-C₄)Haloalkyl, Benzyl, (C₃-C₆)Cycloalkyl oder Phenyl bedeuten,
Het³ und Het⁴ jeweils unabhängig voneinander eine Morpholino- Piperidino- oder Pyrrolidinogruppe bedeuten und
n 0, 1, 2, 3, 4 oder 5 bedeutet.

5. Verbindungen oder deren Salze gemäß einem der Ansprüche 1 bis 4, **dadurch gekennzeichnet, dass**
L einen Rest der Formel 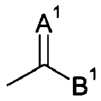 bedeutet,
worin A¹ und B¹ wie in Formel (I) definiert ist.

6. Verbindungen oder deren Salze gemäß Anspruch 5, **dadurch gekennzeichnet, dass**
A¹ Sauerstoff, Schwefel oder =N-R^{A} bedeutet,
B¹ einen Rest der genannten Formeln (B1) bis (B14) bedeutet,
R^{A} Wasserstoff, Hydroxy, (C₁-C₄)Alkoxy, (C₁-C₄)Alkyl, (C₁-C₄)Haloalkyl, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder Benzyl, das im Phenylteil unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder -NR*R**, wobei R*, R** jeweils unabhängig voneinander H, (C₁-C₄)Alkyl, (C₂-C₄)Alkenyl, (C₂-C₄)Alkinyl, (C₁-C₄)Alkoxy-(C₁-C₄)alkyl, (C₁-C₄)Alkanoyl, [(C₁-C₄)Haloalkyl]-carbonyl, [(C₁-C₄)Alkoxy]-carbonyl, [(C₁-C₄)Haloalkoxyl-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy oder im Fall Cycloalkyl auch Oxo substituiert ist, bedeuten oder
R* und R** zusammen mit dem N-Atom einen 3- bis 6-gliedrigen Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten, bedeutet,
(R¹)ₘ, (R²)ₙ, R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹,R²⁰, R²¹, R²², R²³, R²⁴, R²⁵, R²⁶, R²⁷, R²⁸, R²⁹, R³⁰, R³¹ und R³² die obengenannten Bedeutungen haben,
W¹ und W⁶ jeweils unabhängig voneinander die divalente Gruppe Sauerstoff, Schwefel oder eine Gruppe der Formel NH, N-[(C₁-C₄)Alkyl], C=O, S=O, SO₂ oder CR³⁵R³⁶ bedeuten,
W², W³, W⁴, W⁵ und W⁷ jeweils unabhängig voneinander die divalente Gruppe der Formel O, S, NH, N-[(C₁-C₄)Alkyl], C=O, S=O oder SO₂ bedeuten,
Q¹, Q², Q³, Q⁴, Q⁵, Q⁶, Q⁷, Q⁸ und Q⁹ jeweils unabhängig voneinander Hydroxy, Thio, Halogen oder eine Gruppe der Formel OR³⁷, SR³⁸, SM oder OM bedeuten,
M das Äquivalent eines Kations,
R³⁵ und R³⁶ jeweils unabhängig voneinander wie R³ definiert sind oder vorzugsweise unabhängig voneinander H, (C₁-C₄)Alkyl oder (C₁-C₄)Halogenalkyl, vorzugsweise H oder (C₁-C₂)Alkyl, insbesondere H, Methyl oder Ethyl bedeuten und
R³⁷, R³⁸, R⁶⁶, R⁶⁷, R⁶⁸, R⁶⁹, R⁷⁰, R⁷¹, Het¹² und M die obengenannten Bedeutungen haben.

7. Verbindungen oder deren Salze gemäß Anspruch 5 oder 6, **dadurch gekennzeichnet, dass**
R³⁷ und R³⁸ jeweils unabhängig voneinander (C₁-C₆)Alkyl, (C₁-C₆)Haloalkyl, (C₁-C₄)Alkoxy-(C₁-C₄)alkyl, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, Phenyl, das unsubstituiert oder durch einen oder mehrere Reste aus der Gruppe Halogen, Cyano, Hydroxy, Nitro, (C₁-C₄)Alkyl, (C₁-C₄)Haloalkyl und (C₁-C₄)Alkoxy substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
R⁶⁷ Wasserstoff, (C₁-C₆)Alkyl, (C₁-C₄)Haloalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl und (C₁-C₄)Haloalkyl substituiert ist bedeutet,
R⁶⁸ (C₁-C₆)Alkyl oder (C₁-C₄)Haloalkyl bedeutet,
R⁶⁹ und R⁷⁰ jeweils unabhängig voneinander Wasserstoff, (C₁-C₄)Alkyl, Benzyl, (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, substituiert ist, bedeuten,
R⁷¹ (C₁-C₄)Alkyl, (C₁-C₄)Halogenalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl und (C₁-C₄)Haloalkyl substituiert ist, bedeutet, Het¹² einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, und
M ein Metallionenäquivalent, ein Ammoniumion, dass gegebenenfalls durch 1 bis 4 gleiche oder verschiedene Reste aus der Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl substituiert ist, oder ein tertiäres Sulfoniumion, das durch 3 gleiche oder verschiedene Reste aus der Gruppe (C₁-C₄)Alkyl, (C₃-C₆)Cycloalkyl, Phenyl, (C₃-C₆)Cycloalkyl-(C₁-C₄)alkyl und Phenyl(C₁-C₄)alkyl bedeutet.

8. Verbindungen oder deren Salze gemäß einem der Ansprüche 1 bis 7, **dadurch gekennzeichnet, dass**
Bevorzugt sind auch Verbindungen (I), worin
L einen Rest der Formel 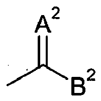 bedeutet,
worin A² und B² die genannten Bedeutungen haben.

9. Verbindungen oder deren Salze gemäß Anspruch 8, **dadurch gekennzeichnet, dass**
A² einen Rest der genannten Formeln (A1) bis (A12) bedeutet,
B² Hydroxy, Thio, Halogen oder eine Gruppe der Formel OR³³, OM, SR³⁴, SM oder -NR^{B}R^{C} bedeutet,
R^{B} und R^{C} unabhängig voneinander Wasserstoff, Hydroxy, (C₁-C₄)Alkoxy, (C₁-C₄)Alkyl, (C₁-C₄)Haloalkyl, Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder Benzyl, das im Phenylteil unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy substituiert ist, oder -NR*R**, wobei R*, R** jeweils unabhängig voneinander H, (C₁-C₄)Alkyl, (C₂-C₄)Alkenyl, (C₂-C₄)Alkinyl, (C₁-C₄)Alkoxy-(C₁-C₄)alkyl, (C₁-C₄)Alkanoyl, [(C₁-C₄)Haloalkyl]-carbonyl, [(C₁-C₄)Alkoxy]-carbonyl, [(C₁-C₄)Haloalkoxy]-carbonyl, (C₃-C₆)Cycloalkyl, (C₃-C₆)Cycloalkyl-(C₁-C₄)alkyl, Phenyl, Phenyl-(C₁-C₄)alkyl, wobei jeder der letztgenannten 4 Reste im Cyclus unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy oder im Fall Cycloalkyl auch Oxo substituiert ist, bedeuten oder
R* und R** zusammen mit dem N-Atom einen 3- bis 6-gliedrigen und vorzugsweise gesättigten Heterocyclus, welcher zusätzlich zum N-Atom ein oder zwei weitere Heteroringatome aus der Gruppe N, O und S enthalten kann und welcher unsubstituiert oder durch einen oder mehrere Reste aus der Gruppe (C₁-C₄)Alkyl, (C₁-C₄)Haloalkyl und Oxo substituiert ist, bedeuten, (R¹)ₘ, (R²)ₙ, R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹,R²⁰, R²¹, R²², R²³, R²⁴, R²⁵ und R²⁶ die obengenannten Bedeutungen haben,
W¹ und W⁶ jeweils unabhängig voneinander die divalente Gruppe Sauerstoff, Schwefel oder eine Gruppe der Formel NH, N-[(C₁-C₄)Alkyl], C=O, S=O, SO₂ oder CR³⁵R³⁶ bedeuten, W², W³, W⁴, W⁵ und W⁷ jeweils unabhängig voneinander die divalente Gruppe der Formel O, S, NH, N-[(C₁-C₄)Alkyl], C=O, S=O oder SO₂ bedeuten,
R³³ und R³⁴ jeweils unabhängig voneinander (C₁-C₄)Alkyl, (C₁-C₄)Haloalkyl, (C₁-C₄)Alkoxy-(C₁-C₄)alkyl, (C₃-C₆)Cycloalkyl, das unsubstituiert oder durch einen oder mehrere Reste aus der Gruppe Halogen, (C₁-C₄)Alkyl und (C₁-C₄)Alkoxy substituiert ist, Phenyl, das unsubstituiert oder durch einen oder mehrere Reste aus der Gruppe Halogen, Cyano, Hydroxy, Nitro, (C₁-C₄)Alkyl, (C₁-C₄)Haloalkyl und (C₁-C₄)Alkoxy substituiert ist, oder eine Gruppe der Formel -C(O)R⁶⁷, -C(O)OR⁶⁸, -C(O)NR⁶⁹R⁷⁰, -C(O)Het¹² oder -SO₂R⁷¹ bedeuten,
R³⁵ und R³⁶ jeweils unabhängig voneinander wie R³ definiert sind,
R⁶⁷ Wasserstoff, (C₁-C₆)Alkyl, (C₁-C₄)Haloalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl und (C₁-C₄)Haloalkyl substituiert ist, insbesondere H oder (C₁-C₄)Alkyl bedeutet,
R⁶⁸ (C₁-C₆)Alkyl oder (C₁-C₄)Haloalkyl, insbesondere (C₁-C₄)Alkyl bedeutet,
R⁶⁹ und R⁷⁰ jeweils unabhängig voneinander Wasserstoff, (C₁-C₄)Alkyl, Benzyl, (C₃-C₆)Cycloalkyl oder Phenyl, wobei jeder der letztgenannten 2 Reste jeweils unabhängig voneinander unsubstituiert oder durch einen oder mehrere Reste aus der Gruppe Halogen, Nitro, Cyano, (C₁-C₄)Alkyl, (C₁-C₄)Haloalkyl, substituiert ist, bedeuten oder insbesondere jeweils unabhängig voneinander H oder (C₁-C₃)Alkyl bedeuten,
R⁷¹ (C₁-C₄)Alkyl, (C₁-C₄)Halogenalkyl, Phenyl oder Benzyl, wobei jeder der letztgenannten 2 Reste unabhängig voneinander unsubstituiert oder durch einen oder mehrere Reste aus der Gruppe Halogen, Cyano, (C₁-C₄)Alkyl, (C₂-C₄)Alkenyl und (C₁-C₄)Haloalkyl substituiert ist, bedeutet, insbesondere (C₁-C₄)Alkyl, (C₁-C₄)Halogenalkyl, Benzyl oder Phenyl, das unsubstituiert oder durch einen oder mehrere Reste aus der Gruppe Halogen, (C₁-C₄)Alkyl und (C₁-C₄)Haloalkyl substituiert ist, bedeutet,
Het¹² einen gesättigten oder teilungesättigten Rest eines Heterocyclus mit 3 bis 6 Ringatomen und mindestens einem N-Atom als Heteroringatom an Position 1 des Rings und gegebenenfalls 1, 2 oder 3 weiteren Heteroringatomen aus der Gruppe N, O und S, wobei der Rest des Heterocyclus am N-Atom in Position 1 des Rings mit dem übrigen Teil des Moleküls der Verbindung der Formel (I) gebunden ist und wobei der Heterocyclus unsubstituiert oder durch einen oder mehrere Reste aus der Gruppe Halogen, (C₁-C₄)Alkyl, (C₁-C₄)Halogenalkyl, (C₁-C₄)Alkoxy, (C₁-C₄)Haloalkoxy, (C₁-C₄)Alkylthio und Oxo substituiert ist, bedeutet.

10. Verbindungen oder deren Salze gemäß einem der Ansprüche 1 bis 9, **dadurch gekennzeichnet, dass** die Verbindung als angereichertes threo-Racemat vorliegt.

11. Verbindungen oder deren Salze gemäß einem der Ansprüche 1 bis 9, **dadurch gekennzeichnet, dass** die Verbindung als angereichertes threo-2-Enantiomer mit der stereochemischen Konfiguration [2R,3R] vorliegt.

12. Verfahren zur Herstellung einer Verbindung der Formel (I), wie sie gemäß einem der Ansprüche 1 bis 11 definiert ist, oder deren Salz, **dadurch gekennzeichnet, dass** man
(a) für die Herstellung von Verbindungen der Formel (I) und deren Salzen, worin L einen Rest der Formel C(=A¹)-B¹, B¹ einen Rest der Formel (B1) bis (B9) und (B11) bis (B14) und Q¹ bis Q⁹ jeweils OH, SH, SM oder OM bedeuten bedeuten und die übrigen Reste wie in der jeweils herzustellenden Verbindung der Formel (I) definiert sind, eine Verbindung der Formel (II), 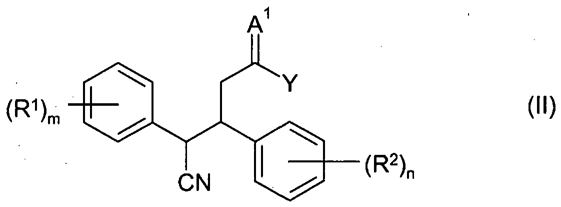 worin A¹, (R¹)ₘ und (R²)ₙ wie in Formel (I) definiert sind und Y eine Abgangsgruppe, vorzugsweise ein Halogenatom, insbesondere ein Chloratom bedeutet,
mit einer Verbindung der Formel H-B¹, worin B¹ wie im Rest L definiert ist und Q¹ bis Q⁹ jeweils wie in der herzustellenden Verbindung der Formel (I) definiert ist, direkt zur Verbindung (I) umsetzt oder zum O- bzw. S-Alkylierungsprodukt als Zwischenprodukt umsetzt und das entstandene Zwischenprodukt der O- bzw. S-Alkylierung in die Verbindung der Formel (I) als C-Alkylierungsprodukt umlagert oder
(b) für die Herstellung von Verbindungen der Formel (I) und deren Salzen, worin L einen Rest der Formel -C(=A¹)-B¹, B¹ einen Rest der Formel (B1) bis (B7) und (B11) bis (B14) und Q¹ bis Q⁹ in der Gruppe B¹ jeweils eine Gruppe der Formel SH oder SM bedeuten und die übrigen Reste wie in der jeweils herzustellenden Verbindung der Formel (I) definiert sind,
eine Verbindung der Formel (I), in der L einen Rest der Formel -C(=A¹)-B¹ und dabei A¹ wie in Formel (I) definiert ist und B¹ analog zum Rest B¹ in Formel (I) definiert ist, mit dem Unterschied, dass Q¹ bis Q⁹ in der Gruppe B¹ jeweils die Gruppe OH oder OM bedeuten, mit einem Halogenierungsmittel zur Verbindung (I), worin Q¹ bis Q⁹ in der Gruppe B¹ jeweils ein Halogenatom bedeuten, mit einem Schwefelreagens umsetzt und die gewünschte Verbindung der Formel (I) gegebenenfalls aus dem Reaktionsgemisch abtrennt oder isoliert oder
(c) für die Herstellung von Verbindungen der Formel (I) und deren Salzen, worin L einen Rest der Formel -C(=A¹)-B¹, B¹ einen Rest der Formel (B1) bis (B7) und (B11) bis (B14) und Q¹ bis Q⁹ in der Gruppe B¹ jeweils eine Gruppe der Formel OR³⁷ bedeuten und die übrigen Reste wie in der jeweils herzustellenden Verbindung der Formel (I) definiert sind,
eine Verbindung der Formel (I), in der L einen Rest der Formel -C(=A¹)-B1 und dabei A¹ wie in Formel (I) definiert ist und B¹ analog zum Rest B¹ in Formel (I) definiert ist, mit dem Unterschied, dass Q¹ bis Q⁹ in der Gruppe B¹ jeweils die Gruppe OH oder OM bedeuten, mit einer Verbindung der Formel Y'-R³⁷, worin Y' eine Abgangsgruppe darstellt und R³⁷ wie in Formel (I) definiert ist, umsetzt und die gewünschte Verbindung der Formel (I) gegebenenfalls aus dem Reaktionsgemisch abtrennt oder isoliert oder
(d) für die Herstellung von Verbindungen der Formel (I) und deren Salzen, worin L einen Rest der Formel -C(=A¹)-B¹, B¹ einen Rest der Formel (B1) bis (B7) und (B11) bis (B14) und Q¹ bis Q⁹ in der Gruppe B¹ jeweils eine Gruppe der Formel SR³⁸ bedeuten und die übrigen Reste wie in der jeweils herzustellenden Verbindung der Formel (I) definiert sind,
eine Verbindung der Formel (I), in der L einen Rest der Formel -C(=A¹)-B¹ und dabei A¹ wie in Formel (I) definiert ist und B¹ analog zum Rest B¹ in Formel (I) definiert ist, mit dem Unterschied, dass Q¹ bis Q⁹ in der Gruppe B¹ jeweils die Gruppe SH oder SM bedeuten, mit einer Verbindung der Formel Y'R³⁸, worin Y' eine Abgangsgruppe darstellt und R³⁸ wie in Formel (I) definiert ist, umsetzt und die gewünschte Verbindung der Formel (I) gegebenenfalls aus dem Reaktionsgemisch abtrennt oder isoliert oder
(e) für die Herstellung von Verbindungen der Formel (I) und deren Salzen, worin L einen Rest der Formel -C(=A¹)-B¹ bedeutet, B¹ einen Rest der Formel (B1) bis (B7) und (B11) bis (B14) bedeutet und Q¹ bis Q⁹ in der Gruppe B¹ jeweils eine Gruppe der Formel SR³⁸ bedeuten und die übrigen Reste wie in der jeweils herzustellenden Verbindung der Formel (I) definiert sind,
eine Verbindung der Formel (I), in der L einen Rest der Formel -C(=A¹)-B¹ und dabei A¹ wie in Formel (I) definiert ist und B¹ analog zum Rest B¹ in Formel (I) definiert ist, mit dem Unterschied, dass Q¹ bis Q⁹ in der Gruppe B¹ jeweils ein Halogenatom bedeuten, mit einer Thioverbindung der Formel H-S-R³⁸ umsetzt und die gewünschte Verbindung der Formel (I) gegebenenfalls aus dem Reaktionsgemisch abtrennt oder isoliert oder
(f) für die Herstellung von Verbindungen der Formel (I) und deren Salzen, worin L einen Rest der Formel -C(=A¹)-B¹ bedeutet und B¹ eine Gruppe der Formel (B10) bedeutet, [= Verbindung (I-B10)]
eine Verbindung der Formel (I), in der L einen Rest der Formel -C(=A¹)-B^{*} bedeutet, A¹ wie in Formel (I) definiert ist und B^{*} einen Rest der Formel -CH₂-CO-R²⁷, worin R²⁷ wie in Formel (B10) definiert ist, bedeutet [= Verbindung (I-B)], 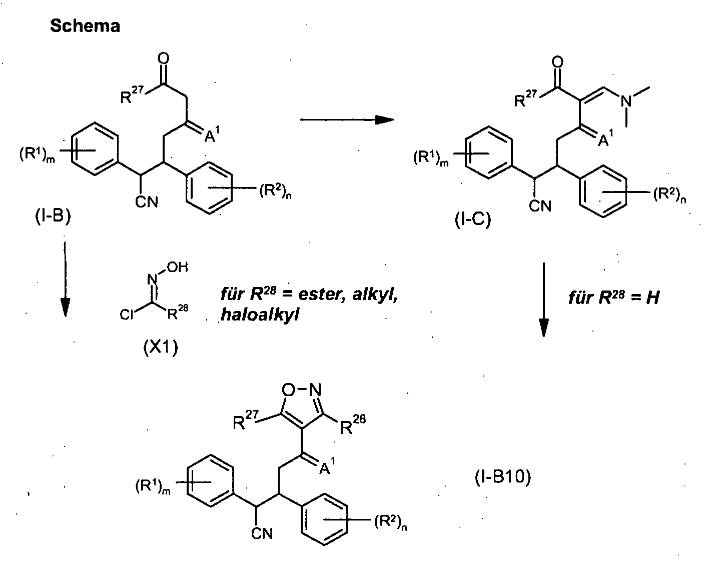 mit N,N-Dimethylformamiddimethylacetal zur Verbindung der Formel (I-C) umsetzt (siehe Schema) und die Verbindung (I-C) unter Ringschluss mit Hydroxylamin oder dessen Salzen zur Verbindung der Formel (I-B10), worin R²⁸ = H ist, umsetzt oder die Verbindung (I-B) mit einer Verbindung der Formel (X1),
HO-N=C(Cl)-R²⁸ (X1)
worin R²⁸ wie in Formel (B10) definiert ist, unter Ringschluss zur Verbindung der Formel (I-B10) umsetzt, wobei R²⁸ in Formel (X1) wie in der herzustellenden Verbindung der Formel (I-B10), ausgenommen R²⁸ = H, definiert ist, oder
(g) für die Herstellung von Verbindungen der Formel (I) und deren Salzen, worin Leinen Rest der Formel -C(=A¹)-B¹ bedeutet und B¹ eine Gruppe der Formel (B10) bedeutet, [= Verbindung (I-B10)]
eine Verbindung der Formel (II), 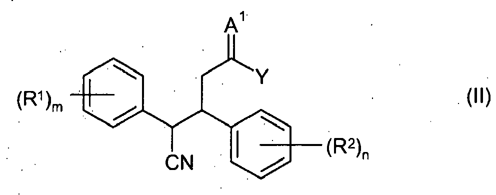 worin A¹, (R¹)ₘ und (R²)ₙ wie in Formel (I) definiert sind und Y eine Abgangsgruppe bedeutet,
mit einer Verbindung der Formel X-(B10), worin (B10) wie in der Gruppe B¹ definiert ist und X ein Abgangsgruppe bedeutet, gegebenenfalls in Gegenwart eines Kupplungsreagenzes zur Verbindung der Formel (I) umsetzt (Kreuzkupplungsreaktion)
oder
mit einer aktivierten Verbindung der Formel X'-(B10), worin.X' ein Metall oder eine Melall-derivatgruppe bedeutet, zur Verbindung der Formel (I) umsetzt,
(h) für die Herstellung von Verbindungen der Formel (I) und deren Salzen, worin L einen Rest der Formel -C(=A²)-B² bedeutet und B² einen Rest der Formel OR³³ oder SR³⁴ bedeutet, eine Verbindung der Formel (I'), 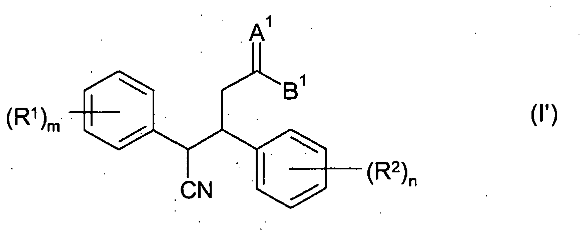 worin (R¹)ₘ und (R²)ₙ wie in Formel (I) definiert sind, A¹ einen Rest O oder S und B¹ einen Rest der Formel (B1), (B2), (B3), (B4), (B5), (B6), (B7), (B8), (B9), (B11), (B12), (B13) oder (B14) wie oben für Verbindungen (I), in denen L einen Rest -C(=A¹)-B¹ bedeutet, definiert ist, wobei Q¹ bis Q⁹ in den Resten B¹ jeweils OH oder OM bedeuten,
mit einer Verbindung der Formel Y'-B², worin B² wie im Rest L definiert ist und Y' eine Abgangsgruppe bedeutet, zur Verbindung (I), in der B² den Rest OR³³, wenn A¹ = O sein soll, oder SR³⁴, wenn A¹ = S sein soll, bedeutet, umsetzt, wobei die Gruppe -C(=A²)- in der Verbindung (I) im Vergleich zur Gruppe -C-B¹ in Verbindung (I') das stabilere Tautomer oder ein strukturell fixiertes Tautomer darstellt, in dem der Rest -C(=A²)- dem Rest =C(-B¹)- tautomer entspricht, oder
(i) für die Herstellung von Verbindungen der Formel (I) und deren Salzen, worin L einen Rest der Formel -C(=A²)-B² bedeutet und B² einen Rest der Formel -NR^{B}R^{C} bedeutet, eine Verbindung der Formel (I'), 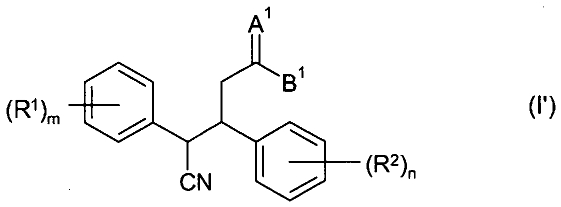 worin (R¹)ₘ und (R²)ₙ wie in Formel (I) definiert sind, A¹ einen Rest der Formel -NR^{B} und B¹ einen Rest der Formel (B1), (B2), (B3), (B4), (B5), (B6), (B7), (B8), (B9), (B11), (B12), (B13) oder (B14) wie oben für Verbindungen (I), in denen L einen Rest -C(=A¹)-B¹ bedeutet, definiert ist, wobei Q¹ bis Q⁹ in den Resten B¹ jeweils OH oder OM bedeuten,
mit einer Verbindung der Formel Y'-R^{C}, worin R^{C} wie im Rest -NR^{B}R^{C} von B² definiert ist und Y' eine Abgangsgruppe bedeutet, zur Verbindung (I), in der B² den Rest -NR^{B}A^{C} bedeutet, umsetzt, wobei die Gruppe -C(=A²)- in der Verbindung (I) im Vergleich zur Gruppe -C-B¹ in Verbindung (I') das stabilere Tautomer oder ein strukturell fixiertes Tautomer darstellt, in dem der Rest -C(=A²)- dem Rest =C(-B¹)-tautomer entspricht.

13. Herbizides oder pflanzenwachstumsregulierendes Mittel, **dadurch gekennzeichnet, dass** es eine oder mehrere Verbindungen der Formel (I) oder deren Salze, wie sie nach einem der Ansprüche 1 bis 11 definiert sind, und im Pflanzenschutz übliche Formulierungshilfsmittel enthält.

14. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen, wie sie nach einem der Ansprüche 1 bis 11 definiert sind, auf die Pflanzen, Pflanzensamen, den Boden, in dem oder auf dem die Pflanzen wachsen, oder die Anbaufläche appliziert.

15. Verfahren nach Anspruch 14, **dadurch gekennzeichnet, dass** die Verbindungen der Formel (I) oder deren Salze zur selektiven Bekämpfung von Schadpflanzen oder zur Wachstumsregulierung in Kulturen von Nutz- oder Zierpflanzen eingesetzt werden.

16. Verfahren nach Anspruch 15, **dadurch gekennzeichnet, dass** die Kulturpflanzen transgene Kulturpflanzen sind.

17. Verwendung der Verbindungen der Formel (I) oder deren Salze gemäß einem Ansprüche 1 bis 11 als Herbizide oder Pflanzenwachstumsregulatoren.

18. Verwendung gemäß Anspruch 17, **dadurch gekennzeichnet, dass** die Verbindungen als selektive Herbizide zur Schadpflanzenkontrolle oder als Pflanzenwachstumsregulatoren in Kulturen von Nutz oder Zierpflanzen eingesetzt werden.

## Claims

1. Compounds of the formula (I) or salts thereof 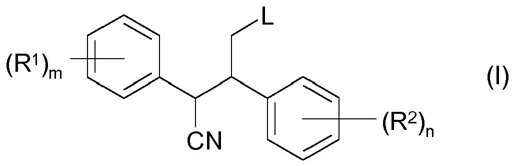 in which
L represents a radical of the formula 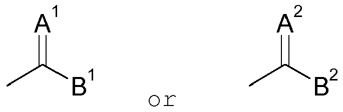
A¹ represents oxygen, sulphur or =N-R^{A},
B¹ represents a radical of the formulae (B1) to (B14), 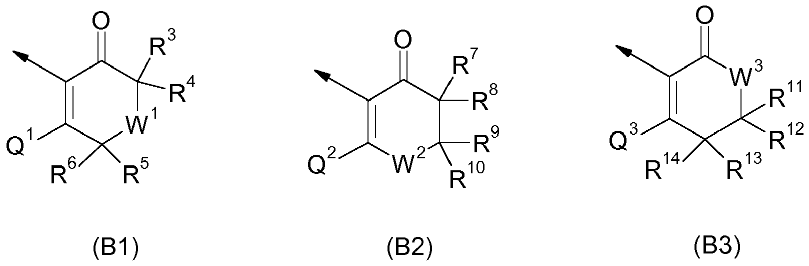 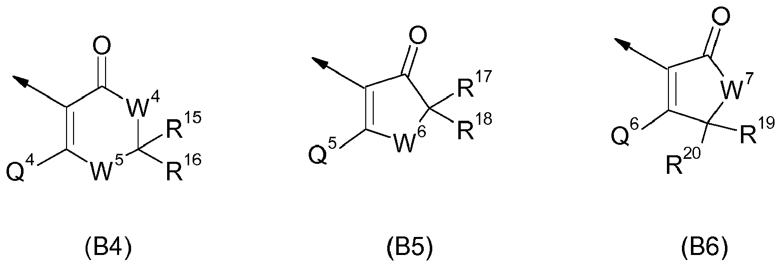 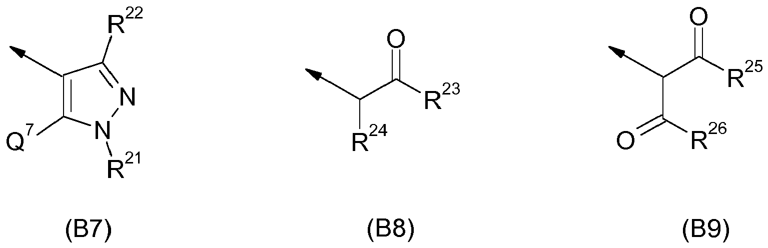 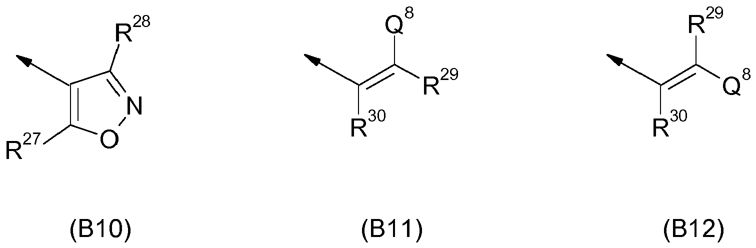 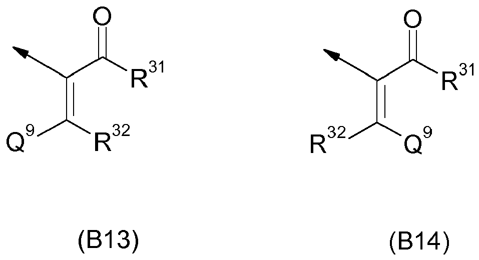
A² represents a radical of the formulae (A1) to (A12), 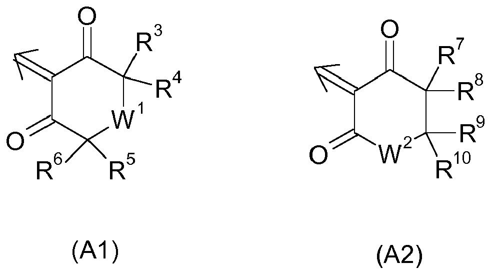 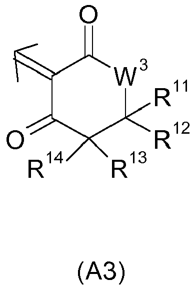 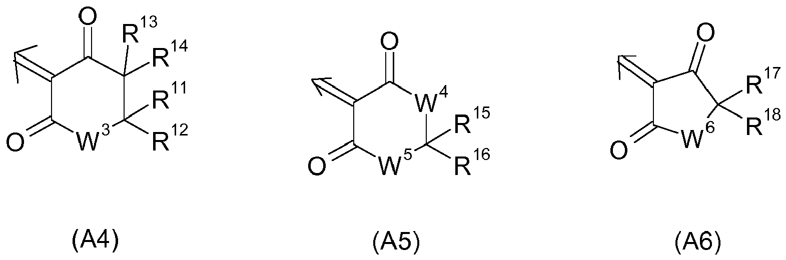 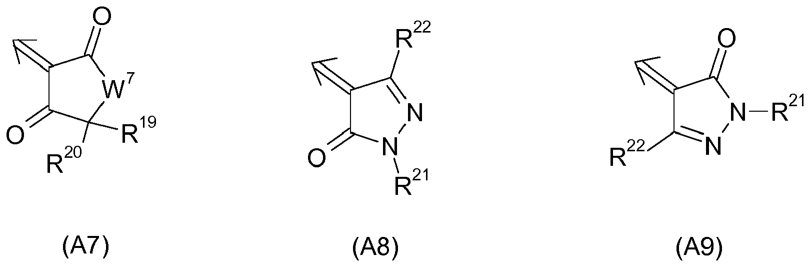 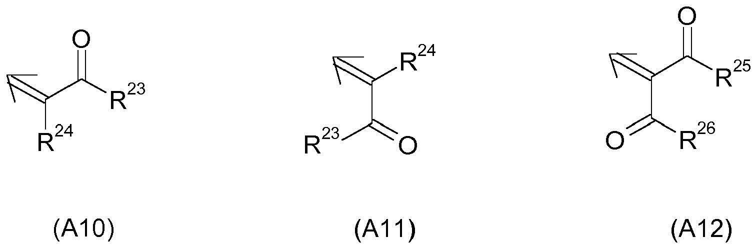
B² represents hydroxy, thio, halogen or a group of the formula OR³³, OM, SR³⁴, SM or -NR^{B}R^{C},
W¹ and W⁶ independently of one another each represent the divalent group oxygen, sulphur or a group of the formula NH, N- [(C₁-C₄)-alkyl], C=O (carbonyl), S=O (sulphinyl), SO₂ (sulphonyl) or CR³⁵R³⁶,
W², W³, W⁴, W⁵ and W⁷ independently of one another each represent the divalent group oxygen, sulphur or a group of the formula NH, N- [(C₁-C₄)-alkyl], C=O (carbonyl), S=O (sulphinyl) or SO₂ (sulphonyl),
Q¹, Q², Q³, Q⁴, Q⁵, Q⁶, Q⁷, Q⁸ and Q⁹ independently of one another each represent hydroxy, thio, halogen or a group of the formula OR³⁷, SR³⁸, SM or OM,
M represents an equivalent of a cation,
R^{A}, R^{B} and R^{C} independently of one another each represent hydrogen, hydroxy, (C₁-C₄)-alkoxy, (C₁-C₄)-alkyl, (C₁-C₄)-haloalkyl, phenyl or benzyl, where each of the two last-mentioned radicals is optionally mono- or polysubstituted by identical or different substituents,
or -NR*R**, where R*, R** independently of one another each represent H, (C₁-C₈)-alkyl, (C₂-C₈)-alkenyl, (C₂-C₈)-alkynyl, (C₁-C₄)-alkoxy-(C₁-C₄)-alkyl, (C₁-C₆)-alkanoyl, [(C₁-C₄)-haloalkyl] carbonyl, [(C₁-C₄)-alkoxy] carbonyl, [(C₁-C₄)-haloalkoxy] carbonyl, (C₃-C₆)-cycloalkyl, (C₃-C₆)-cycloalkyl-(C₁-C₄)-alkyl, phenyl, phenyl-(C₁-C₄)-alkyl, where each of the 4 last-mentioned radicals is unsubstituted in the cycle or substituted by one or more radicals from the group consisting of halogen, (C₁-C₄)-alkyl, (C₁-C₄)-haloalkyl, (C₁-C₄)-alkoxy and (C₁-C₄)-haloalkoxy or, in the case of cycloalkyl, also oxo, or
R* and R** together with the nitrogen atom represent a 3- to 8-membered heterocycle which, in addition to the nitrogen atom, may contain one or two further ring heteroatoms from the group consisting of N, 0 and S and which may be unsubstituted or substituted by one or more radicals from the group consisting of (C₁-C₄)-alkyl, (C₁-C₄)-haloalkyl and oxo, (R¹)ₘ represents m substituents R¹,
where R¹, if m = 1, or each of the substituents R¹, if m is greater than 1, independently of the others represents halogen, cyano, nitro, hydroxy, (C₁-C₈)-alkyl, (C₂-C₆)-alkenyl, (C₂-C₆)-alkynyl, (C₁-C₈)-alkoxy, (C₁-C₈)-alkylthio, (C₁-C₈)-alkylsulphinyl, (C₁-C₈)-alkylsulphonyl, (C₁-C₆)-haloalkyl, (C₁-C₆)-haloalkoxy, (C₁-C₆)-haloalkylthio, (C₁-C₆)-haloalkylsulphinyl, (C₁-C₆)-haloalkylsulphonyl, (C₂-C₆)-haloalkenyl, (C₂-C₆)-haloalkynyl, (C₁-C₆)-alkoxy-(C₁-C₄)-alkyl, (C₁-C₆)-haloalkoxy-(C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formula C(O)OR³⁹, C(O)NR⁴⁰R⁴¹, C(O)-Het¹, NR⁴²R⁴³ or Het²
or where in each case two groups R² located ortho at the ring together are a group of the formula -Z¹-A*-Z² in which
A* represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z¹ represents a direct bond, 0 or S and
Z² represents a direct bond, 0 or S,
where the group -Z¹-A*-Z² together with the carbon atoms, attached to the group, of the phenyl ring form a fused-on 5- or 6-membered ring, and
(R²)ₙ represents n substituents R²,
where R², if n = 1, or each of the substituents R², if n is greater than 1, independently of one another represents halogen, cyano, nitro, hydroxy, (C₁-C₈)-alkyl, (C₂-C₆)-alkenyl, (C₂-C₆)-alkynyl, (C₁-C₈)-alkoxy, (C₁-C₈)-alkylthio, (C₁-C₈)-alkylsulphinyl, (C₁-C₈)-alkylsulphonyl, (C₁-C₆) -haloalkyl, (C₁-C₆) -haloalkoxy, (C₁-C₆)-haloalkylthio, (C₁-C₆)-haloalkylsulphinyl, (C₁-C₆)-haloalkylsulphonyl, (C₂-C₆)-haloalkenyl, (C₂-C₆)-haloalkynyl, (C₁-C₆)-alkoxy-(C₁-C₄)-alkyl, (C₁-C₆)-haloalkoxy-(C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formula C(O)OR⁴⁴, C(O)NR⁴⁵R⁴⁶, C(O)-Het³, NR⁴⁷R⁴⁸ or Het⁴
or
where in each case two groups R² located ortho at the ring together are a group of the formula -Z³-A**-Z⁴ in which
A** represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z³ represents a direct bond, 0 or S and
Z⁴ represents a direct bond, 0 or S,
where the group -Z³-A**-Z⁴ together with the carbon atoms, attached to the group, of the phenyl ring form a fused-on 5- or 6-membered ring,
R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹ and R²⁰ independently of one another each represent hydrogen, halogen, cyano, nitro, hydroxy, (C₁-C₄)-alkyl, (C₁-C₄)-haloalkyl, (C₁-C₄)-alkoxy, (C₁-C₄)-haloalkoxy, (C₁-C₄)-alkoxy-(C₁-C₄)-alkyl, (C₁-C₄)-haloalkoxy-(C₁-C₄)-alkyl, (C₃-C₆)-cycloalkyl, (C₃-C₆)-halocycloalkyl, (C₃-C₆)-cycloalkoxy, (C₂-C₆)-alkenyl, (C₂-C₆)-haloalkenyl, (C₂-C₆)-alkynyl, (C₂-C₆)-haloalkynyl, (C₁-C₄)-alkylthio, (C₁-C₄)-alkylsulphonyl, (C₁-C₄)-alkylsulphinyl, phenyl which is optionally substituted, or a group C(O)OR⁴⁹, C(O)NR⁵⁰R⁵¹, C(O)Het⁵, NR⁵²R⁵³ or Het⁶ or
R³ and R⁵ in the group of the formula (B1) or (A1) together represent a divalent bridge of a straight-chain alkylene or alkenylene group having 2 or 3 carbon atoms, where a CH₂ group in the chain may also be replaced by an oxygen atom and where a CH₂ group or CH group in the chain is optionally substituted by one or more radicals from the group consisting of (C₁-C₄)-alkyl, (C₁-C₄)-haloalkyl, (C₁-C₄)-alkoxy and (C₁-C₄)-haloalkoxy,
R²¹ represents hydrogen, (C₁-C₄)-alkyl, (C₁-C₄)-haloalkyl, (C₃-C₆)-cycloalkyl, (C₃-C₆)-halocycloalkyl or a group C(O)OR⁵⁴, C(O)NR⁵⁵R⁵⁶, C(O)Het⁷, NR⁵⁷R⁵⁸ or Het⁸, R²² represents hydrogen, (C₁-C₄)-alkyl, (C₁-C₄)-haloalkyl, (C₃-C₆)-cycloalkyl, (C₃-C₆)-halocycloalkyl, (C₁-C₄)-alkylthio, (C₁-C₄)-alkylsulphonyl, (C₁-C₄)-alkylsulphinyl or a group of the formula C(O)OR⁵⁹, C(O)NR⁶⁰R⁶¹, C(O)Het⁹, NR⁶²R⁶³ or Het¹⁰,
R²³ and R²⁹ independently of one another each represent hydrogen, (C₁-C₄)-alkyl, (C₁-C₄)-haloalkyl, (C₃-C₆)-cycloalkyl, (C₃-C₆)-halocycloalkyl, (C₁-C₄)-alkoxy or (C₁-C₄)-haloalkoxy,
R²⁴ and R³⁰ independently of one another each represent hydrogen, halogen, cyano, (C₁-C₄)-alkyl, (C₁-C₄)-haloalkyl, (C₃-C₆)-cycloalkyl, (C₃-C₆)-halocycloalkyl, (C₁-C₄)-alkoxy or (C₁-C₄)-haloalkoxy,
R²⁵, R²⁶, R³¹ and R³² independently of one another each represent hydrogen, (C₁-C₄)-alkyl, (C₁-C₄)-haloalkyl, (C₁-C₄)-alkoxy, (C₁-C₄)-haloalkoxy, (C₁-C₄)-alkoxy- (C₁-C₄)-alkyl, (C₁-C₄)-haloalkoxy-(C₁-C₄)-alkyl, (C₃-C₆)-cycloalkyl, (C₃-C₆)-halocycloalkyl, (C₃-C₆)-cycloalkoxy, (C₂-C₆)-alkenyl, (C₂-C₆)-haloalkenyl, (C₂-C₆)-alkynyl, (C₂-C₆) -haloalkynyl, (C₁-C₄)-alkylthio, (C₁-C₄)-alkylsulphonyl, (C₁-C₄)-alkylsulphinyl, NR⁶⁴R⁶⁵ or Het¹¹, R²⁷ represents hydrogen, (C₁-C₆)-alkyl, (C₁-C₄)-haloalkyl, (C₃-C₆)-cycloalkyl or (C₃-C₆)-halocycloalkyl, R²⁸ represents hydrogen, (C₁-C₆)-alkyl, (C₁-C₄)-haloalkyl or a radical of the formula C(O)OR⁶⁶,
R³³, R³⁴, R³⁷ and R³⁸ independently of one another each represent (C₁-C₈)-alkyl, (C₃-C₆)-cycloalkyl, phenyl, where each of the three last-mentioned radicals in each case independently of the others is unsubstituted or substituted by one or more radicals from the group consisting of halogen, cyano, nitro, hydroxy, (C₁-C₄)-alkoxy, (C₁-C₄) -alkylthio and, in the case of a cycloalkyl or phenyl radical as parent radical, also (C₁-C₄)-alkyl and (C₁-C₄)-haloalkyl, or a group of the formula -C(O)R⁶⁷, -C(O)OR⁶⁸, -C(O)NR⁶⁹R⁷⁰, -C(O)Het¹² or -SO₂R⁷¹,
R³⁵ and R³⁶ independently of one another are each defined as R³,
R³⁹, R⁴⁴, R⁴⁹, R⁵⁴, R⁵⁹ and R⁶⁶ independently of one another each represent hydrogen, (C₁-C₄)-alkyl, (C₁-C₄)-haloalkyl, (C₃-C₆)-cycloalkyl, (C₃-C₆)-halocycloalkyl, (C₂-C₄)-alkenyl, (C₂-C₄)-haloalkenyl, (C₂-C₄)-alkynyl or the group M mentioned,
R⁴⁰, R⁴¹, R⁴², R⁴³, R⁴⁵, R⁴⁶, R⁴⁷, R⁴⁸, R⁵⁰, R⁵¹, R⁵², R⁵³, R⁵⁵, R⁵⁶, R⁵⁷, R⁵⁸, R⁶⁰, R⁶¹, R⁶², R⁶³, R⁶⁴, R⁶⁵, R⁶⁹ and R⁷⁰ independently of one another each represent hydrogen, (C₁-C₆)-alkyl, (C₂-C₆)-alkenyl or (C₂-C₆)-alkynyl, where each of the 3 last-mentioned radicals in each case independently of the others is unsubstituted or substituted by one or more radicals from the group consisting of halogen, nitro, cyano and phenyl, which is optionally substituted, or
(C₃-C₆)-cycloalkyl or phenyl, where each of the 2 last-mentioned radicals in each case independently of the other is unsubstituted or substituted by one or more radicals from the group consisting of halogen, nitro, cyano, (C₁-C₄)-alkyl, (C₁-C₄)-haloalkyl, phenyl and benzyl, where each of the 2 last-mentioned radicals is optionally substituted,
R⁶⁷ represents hydrogen, (C₁-C₈)-alkyl or (C₁-C₈)-haloalkyl, where each of the 2 last-mentioned radicals independently of the other is optionally interrupted in the alkyl moiety by oxygen or sulphur, or phenyl or benzyl, where each of the 2 last-mentioned radicals independently of the other is unsubstituted or substituted by one or more radicals from the group consisting of halogen, cyano, (C₁-C₄)-alkyl, (C₂-C₄)-alkenyl, (C₂-C₄)-alkynyl, (C₃-C₆)-cycloalkyl, (C₁-C₄)-haloalkyl and (C₃-C₆)-halocycloalkyl,
R⁶⁸ represents (C₁-C₈)-alkyl, (C₁-C₈)-haloalkyl, (C₃-C₆)-cycloalkyl, (C₃-C₆)-halocycloalkyl, (C₂-C₈)-alkenyl, (C₂-C₈)-haloalkenyl or (C₂-C₄)-alkynyl,
R⁷¹ represents (C₁-C₆)-alkyl, (C₁-C₆)-haloalkyl or phenyl or benzyl, where each of the 2 last-mentioned radicals independently of the other is unsubstituted or substituted by one or more radicals from the group consisting of halogen, cyano, (C₁-C₄)-alkyl, (C₂-C₄)-alkenyl, (C₂-C₄)-alkynyl, (C₃-C₆)-cycloalkyl, (C₁-C₄)-haloalkyl and (C₃-C₆)-halocycloalkyl,
Het¹, Het², Het³, Het⁴, Het⁵, Het⁶, Het⁷, Het⁸, Het⁹, Het¹⁰, Het¹¹ and Het¹² independently of one another are each a saturated or partially unsaturated radical of a heterocycle having 3 to 9 ring atoms and at least one nitrogen atom as ring heteroatom at position 1 of the ring and optionally 1, 2 or 3 further ring heteroatoms from the group consisting of N, 0 and S, where the radical of the heterocycle is attached at the nitrogen atom in position 1 of the ring to the remainder of the molecule of the compound of the formula (I) and where the heterocycle is unsubstituted or substituted by one or more radicals from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo,
and
m, n independently of one another each represent 0, 1, 2, 3, 4 or 5.

2. Compounds or salts thereof according to Claim 1,
**characterized in that**
(R¹)ₘ represents m substituents R¹,
where R¹, if m = 1, or each of the substituents R¹, if m is greater than 1, independently of the others represents halogen, cyano, nitro, hydroxy, (C₁-C₆)-alkyl, (C₂-C₄)-alkenyl, (C₂-C₄)-alkynyl, (C₁-C₆)-alkoxy, (C₁-C₆)-alkylthio, (C₁-C₆)-alkylsulphinyl, (C₁-C₆)-alkylsulphonyl, (C₁-C₄) -haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₂-C₄)-haloalkenyl, (C₂-C₄)-haloalkynyl, (C₁-C₄)-alkoxy-(C₁-C₄)-alkyl, (C₁-C₄)-haloalkoxy-(C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formula C(O)OR³⁹, C(O)NR⁴⁰R⁴¹, C(O)-Het¹, NR⁴²R⁴³ or Het²
or where in each case two groups R¹ located ortho at the ring together are a group of the formula -Z¹-A*-Z² in which
A* represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z¹ represents a direct bond, 0 or S and
Z² represents a direct bond, 0 or S,
where the group -Z¹-A*-Z² together with the carbon atoms, attached to the group, of the phenyl ring form a fused-on 5- or 6-membered ring, and
R³⁹ represents hydrogen, (C₁-C₄)-alkyl, (C₁-C₄)-haloalkyl, (C₃-C₆)-cycloalkyl, (C₃-C₆)-halocycloalkyl, (C₂-C₄)-alkenyl, (C₂-C₄)-haloalkenyl, (C₂-C₄)-alkynyl or the equivalent of a cation,
R⁴⁰, R⁴¹, R⁴², R⁴³ independently of one another each represent hydrogen or (C₁-C₄)-alkyl which is unsubstituted or substituted by one or more radicals from the group consisting of halogen, nitro, cyano and phenyl, or (C₃-C₆)-cycloalkyl or phenyl, where each of the 2 last-mentioned radicals in each case independently of the other is unsubstituted or substituted by one or more radicals from the group consisting of halogen, (C₁-C₄)-alkyl, (C₁-C₄)-haloalkyl, phenyl and benzyl,
Het¹ and Het² independently of one another each represent a saturated or partially unsaturated radical of a heterocycle having 3 to 6 ring atoms and at least one nitrogen atom as ring heteroatoms at position 1 of the ring and optionally 1, 2 or 3 further ring heteroatoms from the group consisting of N, 0 and S, where the radical of the heterocycle is attached at the nitrogen atom in position 1 of the ring to the remainder of the molecule of the compound of the formula (I) and where the heterocycle is unsubstituted or substituted by one or more radicals from the group consisting of halogen, (C₁-C₄)-alkyl, (C₁-C₄)-haloalkyl and oxo, and
m represents 0, 1, 2, 3, 4 or 5.

3. Compounds or salts thereof according to Claim 1 or 2, **characterized in that**
(R²)ₙ represents n substituents R²,
where R², if n = 1, or each of the substituents R², if n is greater than 1, independently of one another represents halogen, cyano, nitro, hydroxy, (C₁-C₆)-alkyl, (C₂-C₄)-alkenyl, (C₂-C₄)-alkynyl, (C₁-C₆)-alkoxy, (C₁-C₆)-alkylthio, (C₁-C₆)-alkylsulphinyl, (C₁-C₆)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₂-C₄)-haloalkenyl, (C₂-C₄)-haloalkynyl, (C₁-C₄)-alkoxy-(C₁-C₄)-alkyl, (C₁-C₄)-haloalkoxy-(C₁-C₄)-alkyl, (C₃-C₆)-cycloalkyl which is optionally substituted by one or more radicals from the group consisting of halogen and (C₁-C₄)-alkyl, (C₃-C₆)-cycloalkoxy which is optionally substituted by one or more radicals from the group consisting of halogen and (C₁-C₄)-alkyl, or a radical of the formula C(O)OR⁴⁴, C(O)NR⁴⁵R⁴⁶, C(O)-Het³, NR⁴⁷R⁴⁸ or Het⁴
or where in each case two groups R² located ortho at the ring together are a group of the formula -Z³-A**-Z⁴ in which
A** represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z³ represents a direct bond, 0 or S and
Z⁴ represents a direct bond, 0 or S,
where the group -Z³-A**-Z⁴ together with the carbon atoms, attached to the group, of the phenyl ring form a fused-on 5- or 6-membered ring,
R⁴⁴ represents hydrogen, (C₁-C₄)-alkyl, (C₁-C₄)-haloalkyl, (C₃-C₆)-cycloalkyl, (C₃-C₆)-halocycloalkyl, (C₂-C₄)-alkenyl, (C₂-C₄)-haloalkenyl, (C₂-C₄)-alkynyl or the equivalent of a cation,
R⁴⁵, R⁴⁶, R⁴⁷ and R⁴⁸ independently of one another each represent hydrogen or (C₁-C₄)-alkyl which is unsubstituted or substituted by one or more radicals from the group consisting of halogen, nitro, cyano and phenyl, or (C₃-C₆)-cycloalkyl or phenyl, where each of the 2 last-mentioned radicals in each case independently of the other is unsubstituted or substituted by one or more radicals from the group consisting of halogen, (C₁-C₄)-alkyl, (C₁-C₄)-haloalkyl, phenyl and benzyl,
Het³ and Het⁴ independently of one another each represent a saturated or partially unsaturated radical of a heterocycle having 3 to 6 ring atoms and at least one nitrogen atom as ring heteroatoms at position 1 of the ring and optionally 1, 2 or 3 further ring heteroatoms from the group consisting of N, 0 and S, where the radical of the heterocycle is attached at the nitrogen atom in position 1 of the ring to the remainder of the molecule of the compound of the formula (I) and where the heterocycle is unsubstituted or substituted by one or more radicals from the group consisting of halogen, (C₁-C₄)-alkyl, (C₁-C₄)-haloalkyl and oxo, and
n represents 0, 1, 2, 3, 4 or 5.

4. Compounds or salts thereof according to any of Claims 1 to 3, **characterized in that**
(R¹)ₘ represents m substituents R¹,
where R¹, if m = 1, or each of the substituents R¹, if m is greater than 1, independently of the others represents halogen, cyano, nitro, hydroxy, (C₁-C₄)-alkyl, (C₁-C₄)-alkoxy, (C₁-C₄)-alkylthio, (C₁-C₄)-alkylsulphinyl, (C₁-C₄)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₁-C₄)-alkoxy-(C₁-C₄)-alkyl, (C₃-C₆)-cycloalkyl, or a radical of the formula C(O)OR³⁹, C(O)NR⁴¹R⁴¹, C(O)-Het¹, NR⁴²R⁴³ or Het²,
or where in each case two groups R¹ located ortho at the ring together are a group of the formula -Z¹-A*-Z² in which
A* represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z¹ represents a direct bond, 0 or S and
Z² represents a direct bond, 0 or S,
where the group -Z¹-A*-Z² together with the carbon atoms, attached to the group, of the phenyl ring form a fused-on 5- or 6-membered ring, and
R³⁹ represents hydrogen, (C₁-C₄)-alkyl or the equivalent of a cation,
R⁴⁰, R⁴¹, R⁴² and R⁴³ independently of one another each represent hydrogen, (C₁-C₄)-alkyl, (C₁-C₄)-haloalkyl, benzyl, (C₃-C₆)-cycloalkyl or phenyl, in particular hydrogen, methyl or ethyl,
Het¹ and Het² independently of one another each represent a morpholino, piperidino or pyrrolidino group,
m represents 0, 1, 2, 3, 4 or 5,
(R²)ₙ represents n substituents R²,
where R², if n = 1, or each of the substituents R², if n is greater than 1, independently of one another represent halogen, cyano, nitro, hydroxy, (C₁-C₄)-alkyl, (C₁-C₄)-alkoxy, (C₁-C₄)-alkylthio, (C₁-C₄)-alkylsulphinyl, (C₁-C₄)-alkylsulphonyl, (C₁-C₄)-haloalkyl, (C₁-C₄)-haloalkoxy, (C₁-C₄)-haloalkylthio, (C₁-C₄)-haloalkylsulphinyl, (C₁-C₄)-haloalkylsulphonyl, (C₁-C₄)-alkoxy-(C₁-C₄)-alkyl, (C₃-C₆)-cycloalkyl, or a radical of the formula C(O)OR⁴⁴, C(O)NR⁴⁵R⁴⁶, C(O)-Het³, NR⁴⁷R⁴⁸ or Het⁴,
or where in each case two groups R² located ortho at the ring together are a group of the formula -Z³-A**-Z⁴ in which
A** represents an alkylene group having 1 to 4 carbon atoms which is optionally substituted by one or more radicals from the group consisting of halogen, (C₁-C₄)-alkyl, (C₁-C₄)-haloalkyl, (C₁-C₄)-alkoxy and (C₁-C₄)-haloalkoxy,
Z³ represents a direct bond, 0 or S and
Z⁴ represents a direct bond, 0 or S,
where the group -Z³-A**-Z⁴ together with the carbon atoms, attached to the group, of the phenyl ring form a fused-on 5- or 6-membered ring,
R⁴⁴ represents hydrogen, (C₁-C₄)-alkyl or the equivalent of a cation,
R⁴⁵, R⁴⁶, R⁴⁷ and R⁴⁸ independently of one another each represent hydrogen, (C₁-C₄)-alkyl, (C₁-C₄)-haloalkyl, benzyl, (C₃-C₆)-cycloalkyl or phenyl, Het³ and Het⁴ independently of one another each represent a morpholino, piperidino or pyrrolidino group and
n represents 0, 1, 2, 3, 4 or 5.

5. Compounds or salts thereof according to any of Claims 1 to 4, **characterized in that**
L represents a radical of the formula 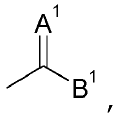
where A¹ and B¹ are as defined in formula (I).

6. Compounds or salts thereof according to Claim 5, **characterized in that**
A¹ represents oxygen, sulphur or =N-R^{A},
B¹ represents a radical of the formulae (B1) to (B14) mentioned,
R^{A} represents hydrogen, hydroxy, (C₁-C₄)-alkoxy, (C₁-C₄)-alkyl, (C₁-C₄)-haloalkyl, phenyl which is unsubstituted or substituted by one or more radicals from the group consisting of halogen, (C₁-C₄)-alkyl, (C₁-C₄)-haloalkyl, (C₁-C₄)-alkoxy, (C₁-C₄) -haloalkoxy, or benzyl which is unsubstituted in the phenyl moiety or substituted by one or more radicals from the group consisting of halogen, (C₁-C₄)-alkyl, (C₁-C₄)-haloalkyl, (C₁-C₄)-alkoxy, (C₁-C₄)-haloalkoxy, or -NR*R**, where R*, R** independently of one another each represent H, (C₁-C₄)-alkyl, (C₂-C₄)-alkenyl, (C₂-C₄)-alkynyl, (C₁-C₄)-alkoxy-(C₁-C₄)-alkyl, (C₁-C₄)-alkanoyl, [(C₁-C₄)-haloalkyl] carbonyl, [(C₁-C₄)-alkoxy]carbonyl, [(C₁-C₄)-haloalkoxy] carbonyl, (C₃-C₆)-cycloalkyl, (C₃-C₆)-cycloalkyl-(C₁-C₄)-alkyl, phenyl, phenyl-(C₁-C₄)alkyl, where each of the 4 last-mentioned radicals is unsubstituted in the cycle or substituted by one or more radicals from the group consisting of halogen, (C₁-C₄)-alkyl, (C₁-C₄)-haloalkyl, (C₁-C₄)-alkoxy or (C₁-C₄)-haloalkoxy or, in the case of cycloalkyl, also oxo, or
R* and R** together with the nitrogen atom represent a 3- to 6-membered heterocycle which, in addition to the nitrogen atom, may contain one or two further ring heteroatoms from the group consisting of N, 0 and S and which may be unsubstituted or substituted by one or more radicals from the group consisting of (C₁-C₄)-alkyl, (C₁-C₄)-haloalkyl and oxo, (R¹)ₘ, (R²)ₙ, R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵, R²⁶, R²⁷, R²⁸, R²⁹, R³⁰, R³¹ and R³² have the meanings mentioned above,
W¹ and W⁶ independently of one another each represent the divalent group oxygen, sulphur or a group of the formula NH, N-[(C₁-C₄)-alkyl], C=O, S=O, SO₂ or CR³⁵R³⁶, W², W³, W⁴, W⁵ and W⁷ independently of one another each represent the divalent group of the formula O, S, NH, N-[(C₁-C₄)-alkyl], C=O, S=O or SO₂,
Q1, Q2, Q3, Q4, Q5, Q6, Q7, Q8 and Q9 independently of one another each represent hydroxy, thio, halogen or a group of the formula OR³⁷, SR³⁸, SM or OM,
M represents the equivalent of a cation,
R³⁵ and R³⁶ independently of one another are each as defined for R³ or preferably independently of one another represent H, (C₁-C₄)-alkyl or (C₁-C₄)-haloalkyl, preferably H or (C₁-C₂)-alkyl, in particular H, methyl or ethyl, and
R³⁷, R³⁸, R⁶⁶, R⁶⁷, R⁶⁸, R⁶⁹, R⁷⁰, R⁷¹, Het¹² and M have the meanings mentioned above.

7. Compounds or salts thereof according to Claim 5 or 6, **characterized in that**
R³⁷ and R³⁸ independently of one another each represent (C₁-C₆)-alkyl, (C₁-C₆) -haloalkyl, (C₁-C₄)-alkoxy-(C₁-C₄)-alkyl, (C₃-C₆)-cycloalkyl which is unsubstituted or substituted by one or more radicals from the group consisting of halogen, (C₁-C₄)-alkyl and (C₁-C₄)-alkoxy, phenyl which is unsubstituted or substituted by one or more radicals from the group consisting of halogen, cyano, hydroxy, nitro, (C₁-C₄)-alkyl, (C₁-C₄)-haloalkyl and (C₁-C₄)-alkoxy, or a group of the formula -C(O)R⁶⁷, -C(O)OR⁶⁸, -C(O)NR⁶⁹R⁷⁰, -C(O)Het¹² or -SO₂R⁷¹,
R⁶⁷ represents hydrogen, (C₁-C₆)-alkyl, (C₁-C₄)-haloalkyl, phenyl or benzyl, where each of the 2 last-mentioned radicals independently of the other is unsubstituted or substituted by one or more radicals from the group consisting of halogen, cyano, (C₁-C₄)-alkyl and (C₁-C₄)-haloalkyl,
R⁶⁸ represents (C₁-C₆)-alkyl or (C₁-C₄)-haloalkyl,
R⁶⁹ and R⁷⁰ independently of one another each represent hydrogen, (C₁-C₄)-alkyl, benzyl, (C₃-C₆)-cycloalkyl or phenyl, where each of the 2 last-mentioned radicals independently of the other is unsubstituted or substituted by one or more radicals from the group consisting of halogen, nitro, cyano, (C₁-C₄)-alkyl and (C₁-C₄)-haloalkyl,
R⁷¹ represents (C₁-C₄)-alkyl, (C₁-C₄)-haloalkyl, phenyl or benzyl, where each of the 2 last-mentioned radicals independently of the other is unsubstituted or substituted by one or more radicals from the group consisting of halogen, cyano, (C₁-C₄)-alkyl, (C₂-C₄)-alkenyl and (C₁-C₄)-haloalkyl,
Het¹² represents a saturated or partially unsaturated radical of a heterocycle having 3 to 6 ring atoms and at least one nitrogen atom as ring heteroatom at position 1 of the ring and optionally 1, 2 or 3 further ring heteroatoms from the group consisting of N, 0 and S, where the radical of the heterocycle at the nitrogen atom in position 1 of the ring is attached to the remainder of the molecule of the compound of the formula (I) and where the heterocycle is unsubstituted or substituted by one or more radicals from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo, and
M represents a metal ion equivalent, an ammonium ion which is optionally substituted by 1 to 4 identical or different radicals from the group consisting of (C₁-C₄)-alkyl, (C₃-C₆)-cycloalkyl, phenyl, (C₃-C₆)-cycloalkyl-(C₁-C₄)-alkyl and phenyl-(C₁-C₄)-alkyl, or a tertiary sulphonium ion which is substituted by 3 identical or different radicals from the group consisting of (C₁-C₄)-alkyl, (C₃-C₆)-cycloalkyl, phenyl, (C₃-C₆)-cycloalkyl-(C₁-C₄)-alkyl and phenyl-(C₁-C₄)-alkyl.

8. Compounds or salts thereof according to any of Claims 1 to 7, **characterized in that**
L represents a radical of the formula 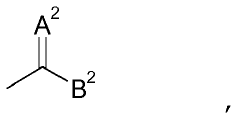 where A² and B² have the meanings mentioned.

9. Compounds or salts thereof according to Claim 8, **characterized in that**
A² represents a radical of the formulae (A1) to (A12) mentioned,
B² represents hydroxy, thio, halogen or a group of the formula OR³³, OM, SR³⁴, SM or -NR^{B}R^{C},
R^{B} and R^{C} independently of one another represent hydrogen, hydroxy, (C₁-C₄)-alkoxy, (C₁-C₄)-alkyl, (C₁-C₄)-haloalkyl, phenyl which is unsubstituted or substituted by one or more radicals from the group consisting of halogen, (C₁-C₄)-alkyl, (C₁-C₄)-haloalkyl, (C₁-C₄)-alkoxy, (C₁-C₄)-haloalkoxy, or benzyl which is unsubstituted in the phenyl moiety or substituted by one or more radicals from the group consisting of halogen, (C₁-C₄)-alkyl, (C₁-C₄)-haloalkyl, (C₁-C₄)-alkoxy, (C₁-C₄)-haloalkoxy, or -NR*R**, where R*, R** independently of one another each represent H, (C₁-C₄)-alkyl, (C₂-C₄)-alkenyl, (C₂-C₄)-alkynyl, (C₁-C₄)-alkoxy-(C₁-C₄)-alkyl, (C₁-C₄)-alkanoyl, [(C₁-C₄)-haloalkyl] carbonyl, [(C₁-C₄)-alkoxy]carbonyl, [(C₁-C₄)-haloalkoxy] carbonyl, (C₃-C₆)-cycloalkyl, (C₃-C₆)-cycloalkyl-(C₁-C₄)-alkyl, phenyl, phenyl-(C₁-C₄)alkyl, where each of the 4 last-mentioned radicals is unsubstituted in the cycle or substituted by one or more radicals from the group consisting of halogen, (C₁-C₄)-alkyl, (C₁-C₄)-haloalkyl, (C₁-C₄)-alkoxy or (C₁-C₄)-haloalkoxy or, in the case of cycloalkyl, also oxo, or
R* and R** together with the nitrogen atom represent a 3- to 6-membered and preferably saturated heterocycle which, in addition to the nitrogen atom, may contain one or two further ring heteroatoms from the group consisting of N, 0 and S and which may be unsubstituted or substituted by one or more radicals from the group consisting of (C₁-C₄)-alkyl, (C₁-C₄)-haloalkyl and oxo,
(R¹)ₘ, (R²)ₙ, R³, R⁴, R⁵, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵ and R²⁶ have the meanings mentioned above,
W¹ and W⁶ independently of one another each represent the divalent group oxygen, sulphur or a group of the formula NH, N-[(C₁-C₄)-alkyl], C=O, S=O, SO₂ or CR³⁵R³⁶, W², W³, W⁴, W⁵ and W⁷ independently of one another each represent the divalent group of the formula O, S, NH, N-[(C₁-C₄)-alkyl], C=O, S=O or SO₂,
R³³ and R³⁴ independently of one another each represent (C₁-C₄)-alkyl, (C₁-C₄)-haloalkyl, (C₁-C₄)-alkoxy-(C₁-C₄)-alkyl, (C₃-C₆)-cycloalkyl which is unsubstituted or substituted by one or more radicals from the group consisting of halogen, (C₁-C₄)-alkyl and (C₁-C₄)-alkoxy, phenyl which is unsubstituted or substituted by one or more radicals from the group consisting of halogen, cyano, hydroxy, nitro, (C₁-C₄)-alkyl, (C₁-C₄)-haloalkyl and (C₁-C₄)-alkoxy, or a group of the formula -C(O)R⁶⁷, -C(O)OR⁶⁸, -C(O)NR⁶⁹R⁷⁰, -C(O)Het¹² or -SO₂R⁷¹,
R³⁵ and R³⁶ independently of one another are each defined as R³,
R⁶⁷ represents hydrogen, (C₁-C₆)-alkyl, (C₁-C₄)-haloalkyl, phenyl or benzyl, where each of the 2 last-mentioned radicals independently of the other is unsubstituted or substituted by one or more radicals from the group consisting of halogen, cyano, (C₁-C₄)-alkyl and (C₁-C₄)-haloalkyl, in particular H or (C₁-C₄)-alkyl,
R⁶⁸ is (C₁-C₆)-alkyl or (C₁-C₄)-haloalkyl, in particular (C₁-C₄)-alkyl,
R⁶⁹ and R⁷⁰ independently of one another each represent hydrogen, (C₁-C₄)-alkyl, benzyl, (C₃-C₆)-cycloalkyl or phenyl, where each of the 2 last-mentioned radicals independently of the other is unsubstituted or substituted by one or more radicals from the group consisting of halogen, nitro, cyano, (C₁-C₄)-alkyl and (C₁-C₄)-haloalkyl, in particular independently of one another each represent H or (C₁-C₃)-alkyl,
R⁷¹ represents (C₁-C₄)-alkyl, (C₁-C₄)-haloalkyl, phenyl or benzyl, where each of the 2 last-mentioned radicals independently of the other is unsubstituted or substituted by one or more radicals from the group consisting of halogen, cyano, (C₁-C₄)-alkyl, (C₂-C₄)-alkenyl and (C₁-C₄)-haloalkyl, in particular (C₁-C₄)-alkyl, (C₁-C₄)-haloalkyl, benzyl or phenyl which is unsubstituted or substituted by one or more radicals from the group consisting of halogen, (C₁-C₄)-alkyl and (C₁-C₄)-haloalkyl,
Het¹² represents a saturated or partially unsaturated radical of a heterocycle having 3 to 6 ring atoms and at least one nitrogen atom as ring heteroatom at position 1 of the ring and optionally 1, 2 or 3 further ring heteroatoms from the group consisting of N, O and S, where the radical of the heterocycle at the nitrogen atom in position 1 of the ring is attached to the remainder of the molecule of the compound of the formula (I) and where the heterocycle is unsubstituted or substituted by one or more radicals from the group consisting of halogen, (C₁-C₄)-alkyl, (C₁-C₄)-haloalkyl, (C₁-C₄)-alkoxy, (C₁-C₄)-haloalkoxy, (C₁-C₄)-alkylthio and oxo.

10. Compounds or salts thereof according to any of Claims 1 to 9, **characterized in that** the compound is present as an enriched threo racemate.

11. Compounds or salts thereof according to any of Claims 1 to 9, **characterized in that** the compound is present as an enriched threo-2 enantiomer having the stereochemical configuration [2R,3R].

12. Process for preparing a compound of the formula (I) as defined in any of Claims 1 to 11 or a salt thereof, **characterized in that**
(a) to prepare compounds of the formula (I) and salts thereof in which L represents a radical of the formula C(=A¹)-B¹, B¹ represents a radical of the formulae (B1) to (B9) and (B11) to (B14) and Q¹ to Q⁹ each represent OH, SH, SM or OM and the other radicals are as defined in the respective compound of the formula (I) to be prepared, a compound of the formula (II) 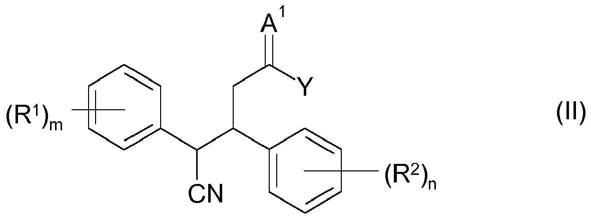 in which A¹, (R¹)ₘ and (R²)ₙ are as defined in formula (I) and Y represents a leaving group, preferably a halogen atom, in particular a chlorine atom,
is reacted directly with a compound of the formula H-B¹ in which B¹ is as defined in the radical L and Q¹ to Q⁹ are each as defined in the compound of the formula (I) to be prepared, to give the compound (I), or to give the 0- or S-alkylation product as intermediate, and the 0- or S-alkylation intermediate formed is rearranged to give the compound of the formula (I) as C-alkylation product, or
(b) to prepare compounds of the formula (I) and salts thereof in which L represents a radical of the formula -C(=A¹)-B¹, B¹ represents a radical of the formulae (B1) to (B7) and (B11) to (B14) and Q¹ to Q⁹ in group B¹ each represent a group of the formula SH or SM and the other radicals are as defined in the respective compound of the formula (I) to be prepared,
a compound of the formula (I) in which L represents a radical of the formula -C(=A¹)-B¹ where A¹ is as defined in formula (I) and B¹ is defined analogously to the radical B¹ in formula (I), with the difference that Q¹ to Q⁹ in group B¹ each represent the group OH or OM, is reacted with a halogenating agent to give the compound (I) in which Q¹ to Q⁹ in group B¹ each represent a halogen atom and reacted with a sulphurizing agent, and the desired compound of the formula (I) is optionally separated from the reaction mixture or isolated or
(c) to prepare compounds of the formula (I) and salts thereof in which L represents a radical of the formula -C(=A¹)-B¹, B¹ represents a radical of the formulae (B1) to (B7) and (B11) to (B14) and Q¹ to Q⁹ in group B¹ each represent a group of the formula OR³⁷ and the other radicals are as defined in the respective compound of the formula (I) to be prepared,
a compound of the formula (I) in which L represents a radical of the formula -C(=A¹)-B¹ where A¹ is as defined in formula (I) and B¹ is defined analogously to the radical B¹ in formula (I), with the difference that Q¹ to Q⁹ in group B¹ each represent the group OH or OM, is reacted with a compound of the formula Y'-R³⁷ in which Y' represents a leaving group and R³⁷ is as defined in formula (I), and the desired compound of the formula (I) is optionally separated from the reaction mixture or isolated or
(d) to prepare compounds of the formula (I) and salts thereof in which L represents a radical of the formula -C(=A¹)-B¹, B¹ represents a radical of the formulae (B1) to (B7) and (B11) to (B14) and Q¹ to Q⁹ in group B¹ each represent a group of the formula SR³⁸ and the other radicals are as defined in the respective compound of the formula (I) to be prepared,
a compound of the formula (I) in which L represents a radical of the formula -C(=A¹)-B¹ where A¹ is as defined in formula (I) and B¹ is defined analogously to the radical B¹ in formula (I), with the difference that Q¹ to Q⁹ in group B¹ each represent the group SH or SM, is reacted with a compound of the formula Y'-R³⁸ in which Y' represents a leaving group and R³⁸ is as defined in formula (I), and the desired compound of the formula (I) is optionally separated from the reaction mixture or isolated or
(e) to prepare compounds of the formula (I) and salts thereof in which L represents a radical of the formula -C(=A¹)-B¹, B¹ represents a radical of the formulae (B1) to (B7) and (B11) to (B14) and Q¹ to Q⁹ in group B¹ each represent a group of the formula SR³⁸ and the other radicals are as defined in the respective compound of the formula (I) to be prepared,
a compound of the formula (I) in which L represents a radical of the formula -C(=A¹)-B¹ where A¹ is as defined in formula (I) and B¹ is defined analogously to the radical B¹ in formula (I), with the difference that Q¹ to Q⁹ in group B¹ each represent a halogen atom, is reacted with a thio compound of the formula H-S-R³⁸, and the desired compound of the formula (I) is optionally separated from the reaction mixture or isolated or
(f) to prepare compounds of the formula (I) and salts thereof in which L represents a radical of the formula -C(=A¹)-B¹ and B¹ represents a group of the formula (B10) [= compound (I-B10)],
a compound of the formula (I) in which L represents a radical of the formula -C(=A¹)-B*, A¹ is as defined in formula (I) and B^{*} is a radical of the formula -CH₂-CO-R²⁷ in which R²⁷ is as defined in formula (B10) [= compound (I-B)] 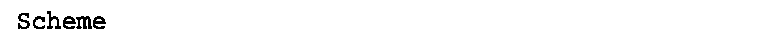 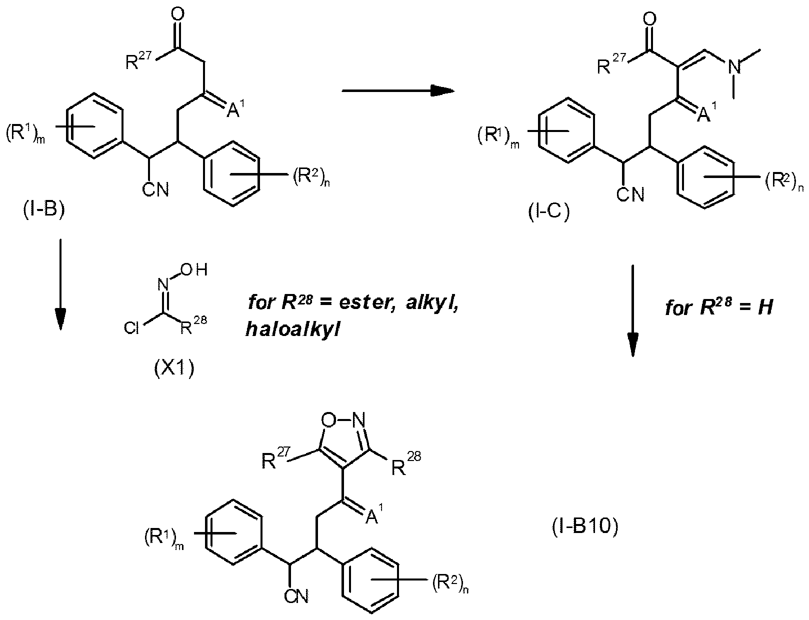 is reacted with N,N-dimethylformamide dimethyl acetal to give the compound of the formula (I-C) (see scheme) and the compound (I-C) is reacted with ring closure with hydroxylamine or salts thereof to give the compound of the formula (I-B10) in which R²⁸ = H or the compound (I-B) is reacted with a compound of the formula (X1)
HO-N=C(Cl)-R²⁸ (X1)
in which R²⁸ is as defined in formula (B10) with ring closure to give the compound of the formula (I-B10) where R²⁸ in formula (X1) is defined as in the compound of the formula (I-B10) to be prepared, except that R²⁸ = H, or
(g) to prepare compounds of the formula (I) and salts thereof in which L represents a radical of the formula -C(=A¹)-B¹ and B¹ represents a group of the formula (B10) [= compound (I-B10)],
a compound of the formula (II) 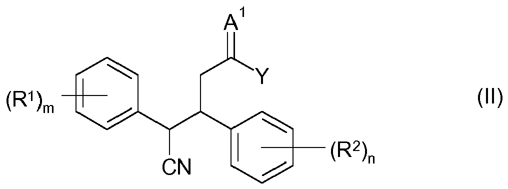 in which A¹, (R¹)ₘ and (R²)ₙ are as defined in formula (I) and Y represents a leaving group,
is reacted with a compound of the formula X-(B10) in which (B10) is defined as in group B¹ and X is a leaving group, optionally in the presence of a coupling agent, to give the compound of the formula (I) (crosscoupling reaction)
or
is reacted with an activated compound of the formula X'-(B10) in which X' represents a metal or a metal derivative group, to give the compound of the formula (I),
(h) to prepare compounds of the formula (I) and salts thereof in which L represents a radical of the formula -C(=A²)-B² and B² represents a radical of the formula OR³³ or SR³⁴, a compound of the formula (I') 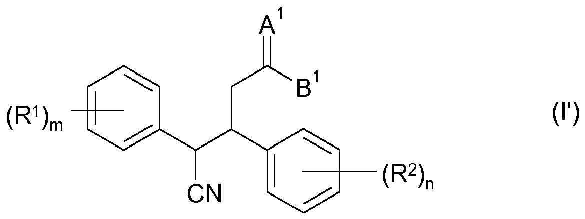 in which (R¹)ₘ and (R²)ₙ are as defined in formula (I), A¹ represents a radical 0 or S and B¹ represents a radical of the formula (B1), (B2), (B3), (B4), (B5), (B6), (B7), (B8), (B9), (B11), (B12), (B13) or (B14) as defined above for compounds (I) in which L represents a radical -C(=A¹)-B¹, where Q¹ to Q⁹ in the radicals B¹ each represent OH or OM,
is reacted with a compound of the formula Y'-B² in which B² is as defined in the radical L and Y' represents a leaving group, to give the compound (I) in which B² represents the radical OR³³ if A¹ = 0 or SR³⁴ if A¹ = S, where the group -C(=A²)- in the compound (I), compared to group -C-B¹ in compound (I'), represents the more stable tautomer or a structurally fixed tautomer in which the radical -C(=A²)- corresponds tautomerically to the radical =C(-B¹)-, or
(i) to prepare compounds of the formula (I) and salts thereof in which L represents a radical of the formula -C(=A²)-B² and B² represents a radical of the formula-NR^{B}R^{C}, a compound of the formula (I') 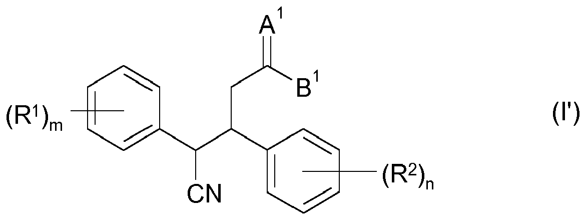 in which (R¹)ₘ and (R²)ₙ are as defined in formula (I), A¹ represents a radical of the formula -NR^{B} and B¹ represents a radical of the formula (B1), (B2), (B3), (B4), (B5), (B6), (B7), (B8), (B9), (B11), (B12), (B13) or (B14) as defined above for compounds (I) in which L represents a radical -C(=A¹)-B¹, where Q¹ to Q⁹ in the radicals B¹ each represent OH or OM,
is reacted with a compound of the formula Y'-R^{C} in which R^{C} is as defined in the radical -NR^{B}R^{C} of B² and Y' represents a leaving group, to give the compound (I) in which B² represents the radical -NR^{B}R^{C}, where the group -C(=A²)- in the compound (I), compared to group-C-B¹ in compound (I'), represents the more stable tautomer or a structurally fixed tautomer in which the radical -C(=A²)- corresponds tautomerically to the radical =C(-B¹)-.

13. Herbicidal or plant growth-regulating composition, **characterized in that** it comprises one or more compounds of the formula (I) or salts thereof as defined in any of Claims 1 to 11 and formulation auxiliaries customary in crop protection.

14. Method for controlling harmful plants or for regulating the growth of plants, **characterized in that** an effective amount of one or more compounds of the formula (I) or salts thereof as defined in any of Claims 1 to 11 is applied onto the plants, plant seeds, the soil in which or on which the plants grow or the area under cultivation.

15. Method according to Claim 14, **characterized in that** the compounds of the formula (I) or salts thereof are employed for the selective control of harmful plants or for regulating the growth in crops of useful plants or ornamental plants.

16. Method according to Claim 15, **characterized in that** the crop plants are transgenic crop plants.

17. Use of the compounds of the formula (I) or the salts thereof according to any of Claims 1 to 11 as herbicides or plant growth regulators.

18. Use according to Claim 17, **characterized in that** the compounds are applied as selective herbicides for controlling harmful plants or as plant growth regulators in crops of useful or ornamental plants.

## Revendications

1. Composés de formule (I) ou leurs sels, 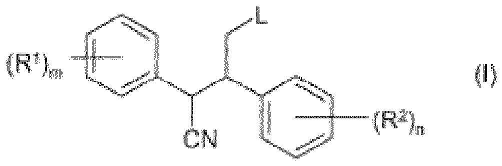 formule dans laquelle
L représente un radical de formule 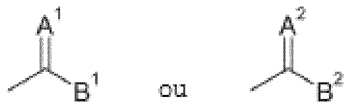
A¹ représente un atome d'oxygène, de soufre ou =N-R^{A},
B¹ représente un radical de formules (B1) à (B14), 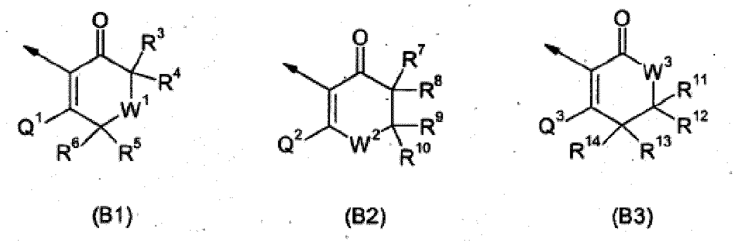 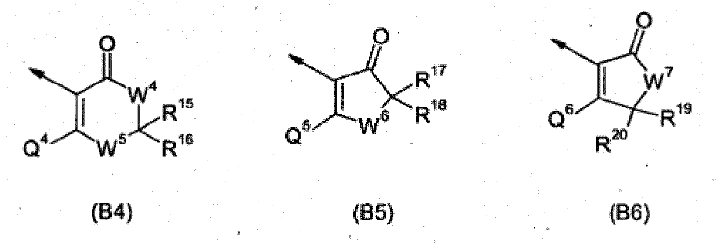 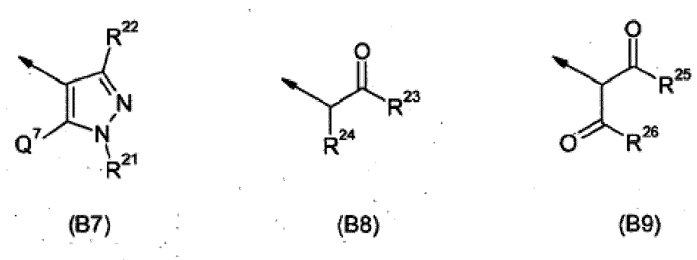 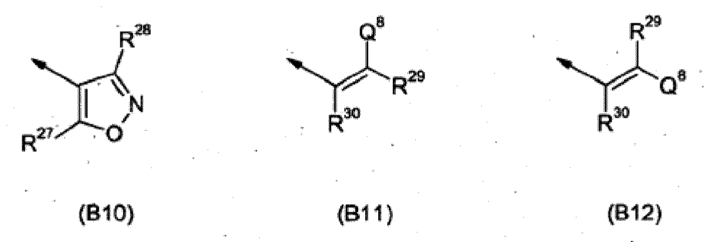 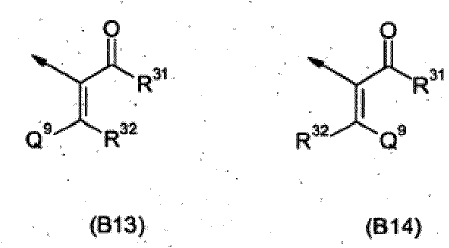
A² représente un radical de formules (A1) à (A12) 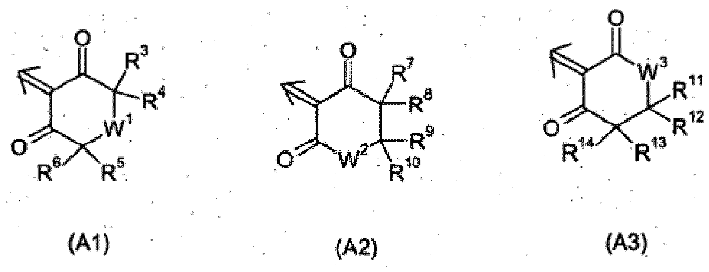 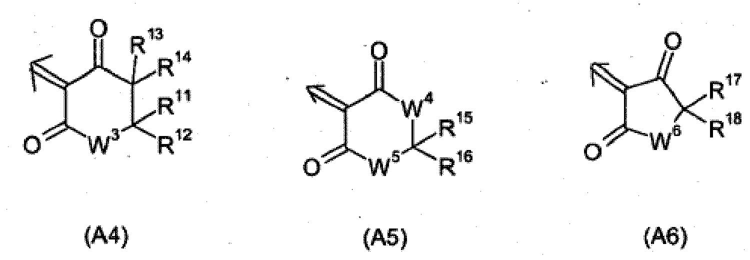 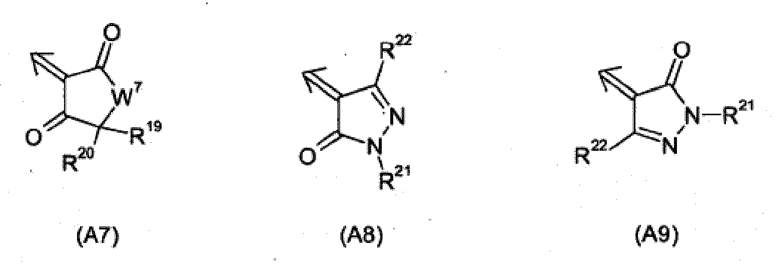 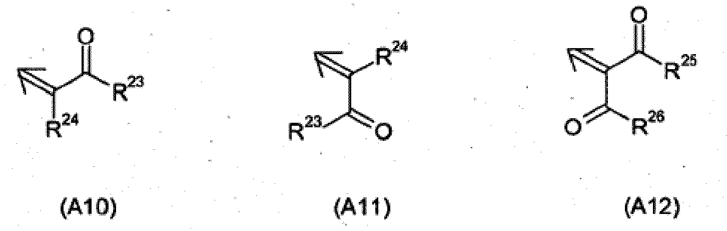
B² représente un groupe hydroxy, thio, halogéno ou un groupe de formule OR³³, OM, SR³⁴, SM ou -NR^{B}R^{C},
W¹ et W⁶ représentent chacun indépendamment l'un de l'autre le groupe divalent oxygène, soufre ou un groupe de formule NH, N-[(alkyle(C₁-C₄)], C=O (carbonyle), S=O (sulfinyle), SO₂ (sulfonyle) ou CR³⁵R³⁶,
W², W³, W⁴, W⁵ et W⁷ représentent chacun indépendamment les uns des autres le groupe divalent oxygène, soufre ou un groupe de formule NH, N-[(alkyle(C₁-C₄)], C=O(carbonyle), S=O (sulfinyle) ou SO₂ (sulfonyle),
Q¹, Q², Q³, Q⁴, Q⁵, Q⁶, Q⁷, Q⁸ et Q⁹ représentent chacun indépendamment les uns des autres le groupe hydroxy, thio, un atome d'halogène ou un groupe de formule OR³⁷, SR³⁸, SM ou OM,
M représente un équivalent d'un cation,
R^{A}, R^{B} et R^{C} représentent chacun indépendamment un atome d'hydrogène, un groupe hydroxy, alcoxy en C₁-C₄, alkyle en C₁-C₄, halogénoalkyle(C₁-C₄), phényle ou benzyle, chacun des deux radicaux nommés de dernier portant éventuellement un ou plusieurs substituants identiques ou différents, ou -NR*R**, R*, R** représentant chacun indépendamment l'un de l'autre H, un groupe alkyle en C₁-C₈, alcényle en C₂-C₈, alcynyle en C₂-C₈, alcoxy(C₁-C₄)-alkyle (C₁-C₄), alcanoyle en C₁-C₆, [halogénoalkyl(C₁-C₄)]-carbonyle, [alcoxy(C₁-C₄)]-carbonyle, [(halogénoalcoxy(C₁-C₄)]-carbonyle, cycloalkyle en C₃-C₆, cycloalkyl (C₃-C₆)-alkyle(C₁-C₄), phényle, phényl-alkyle(C₁-C₄), chacun des 4 radicaux nommés en dernier étant dans le cycle non substitué ou substitué par un ou plusieurs radicaux choisis dans le groupe constitué par halogéno, alkyle en C₁-C₄, halogénoalkyle(C₁-C₄), alcoxy en C₁-C₄ ou halogénoalcoxy(C₁-C₄) ou dans le cas du groupe cycloalkyle également oxo ou
R* et R** représentent ensemble avec l'atome d'azote un hétérocycle à 3 à 8 chaînons, qui peut contenir en plus de l'atome d'azote un ou deux autres hétéroatomes formant le cycle, choisis dans le groupe constitué par N, 0 et S et qui est non substitué ou substitué par un ou plusieurs radicaux choisis dans le groupe constitué par alkyle en C₁-C₄, halogénoalkyle(C₁-C₄) et oxo,
(R¹)ₘ représente m substituants R¹, R¹, lorsque m = 1, ou chacun des substituants R¹, lorsque m est plus grand que 1, représentant chaque fois indépendamment un atome d'halogène, un groupe cyano, nitro, hydroxy, alkyle en C₁-C₈, alcényle en C₂-C₆, alcynyle en C₂-C₆, alcoxy en C₁-C₈, alkyl(C₁-C₈)thio, alkyl(C₁-C₈)sulfinyle, alkyl(C₁-C₈)sulfonyle, halogénoalkyle(C₁-C₆), halogénoalcoxy(C₁-C₆), halogénoalkyl(C₁-C₆)thio, halogénoalkyl(C₁-C₆)sulfinyle, halogénoalkyl(C₁-C₆)-sulfonyle, halogénoalcényle(C₂-C₆), halogéno-alcynyle (C₂-C₆), alcoxy(C₁-C₆)-alkyle(C₁-C₄), halogénoalcoxy(C₁-C₆)-alkyle (C₁-C₄), cycloalkyle en C₃-C₆ qui est éventuellement substitué par un ou plusieurs radicaux choisis dans le groupe constitué par halogéno et alkyle en C₁-C₄, un groupe cycloalcoxy en C₃-C₆ qui est éventuellement substitué par un ou plusieurs radicaux choisis dans le groupe constitué par halogéno et alkyle en C₁-C₄, ou un radical de formule C(O)OR³⁹, C(O)NR⁴⁰R⁴¹, C(O)-Het¹, NR⁴²R⁴³ ou Het²
ou deux groupes R¹ qui se trouvent respectivement en position ortho sur le cycle représentant ensemble un groupe de formule -Z¹-A*-2², dans laquelle
A* représente un groupe alkylène ayant de 1 à 4 atomes de carbone, qui est éventuellement substitué par un ou plusieurs radicaux choisis dans le groupe constitué par halogéno, alkyle en C₁-C₄, halogéno-alkyle(C₁-C₄), alcoxy en C₁-C₄ et halogéno-alcoxy(C₁-C₄),
Z¹ représente une liaison directe, 0 ou S et
Z² représente une liaison directe, O ou S,
le groupe -Z¹-A*-Z² formant conjointement avec les atomes de carbone du cycle phényle qui sont liés au groupe un cycle pentagonal ou hexagonal condensé,
(R²) ₙ représente n substituants R²,
R², lorsque n = 1, ou chacun des substituants R², lorsque n est plus grand que 1, représentant chaque fois indépendamment un atome d'halogène, un groupe cyano, nitro, hydroxy, alkyle en C₁-C₈, alcényle en C₂-C₆, alcynyle en C₂-C₆, alcoxy en C₁-C₈, alkyl(C₁-C₈)thio, alkyl(C₁-C₈)sulfinyle, alkyl(C₁-C₈)sulfonyle, halogénoalkyle (C₁-C₆), halogénoalcoxy(C₁-C₆), halogénoalkyl(C₁-C₆)thio, halogénoalkyl(C₁-C₆)sulfinyle, halogénoalkyl(C₁-C₆)-sulfonyle, halogénoalcényle(C₂-C₆), halogéno-alcynyle (C₂-C₆), alcoxy(C₁-C₆)-alkyle(C₁-C₄), halogénoalcoxy(C₁-C₆)-alkyle(C₁-C₄), cycloalkyle en C₃-C₆ qui est éventuellement substitué par un ou plusieurs radicaux choisis dans le groupe constitué par halogéno et alkyle en C₁-C₄, un groupe cycloalcoxy en C₃-C₆ qui est éventuellement substitué par un ou plusieurs radicaux choisis dans le groupe constitué par halogéno et alkyle en C₁-C₄, ou un radical de formule C(O)OR⁴⁴, C(O)NR⁴⁵R⁴⁶, C(O)-Het³, NR⁴⁷R⁴⁸ ou Het⁴ ou
deux groupes R² qui se trouvent respectivement en position ortho sur le cycle représentant ensemble un groupe de formule -Z³-A**-Z⁴, dans laquelle
A** représente un groupe alkylène ayant de 1 à 4 atomes de carbone, qui est éventuellement substitué par un ou plusieurs radicaux choisis dans le groupe constitué par halogéno, alkyle en C₁-C₄, halogéno-alkyle(C₁-C₄), alcoxy en C₁-C₄ et halogéno-alcoxy(C₁-C₄),
Z³ représente une liaison directe, 0 ou S et
Z⁴ représente une liaison directe, 0 ou S,
le groupe -Z³-A**-Z⁴ formant conjointement avec les atomes de carbone du cycle phényle qui sont liés au groupe un cycle pentagonal ou hexagonal condensé,
R³, R⁴, R⁵, R⁶, R⁷, R⁸, R⁹, R¹⁰, R¹¹, R¹², R¹³, R¹⁴, R¹⁵, R¹⁶,R¹⁷, R¹⁸, R¹⁹ et R²⁰ représentent chacun indépendamment les uns des autres un atome d'hydrogène ou d'halogène, un groupe cyano, nitro, hydroxy, alkyle en C₁-C₄, halogénoalkyle(C₁-C₄), alcoxy en C₁-C₄, halogénoalcoxy(C₁-C₄), alcoxy(C₁-C₄)-alkyle (C₁-C₄), halogénoalcoxy(C₁-C₄)-alkyle(C₁-C₄), cycloalkyle en C₃-C₆, halogéno-cycloalkyle(C₃-C₆), cycloalcoxy en C₃-C₆, alcényle en C₂-C₆, halogénoalcényle(C₂-C₆), alcynyle en C₂-C₆, halogénoalcynyle(C₂-C₆), alkyl(C₁-C₄)thio, alkyl(C₁-C₄)sulfonyle, alkyl(C₁-C₄)sulfinyle, phényle qui est éventuellement substitué, ou un groupe C(O)OR⁴⁹, C(O)NR⁵⁰R⁵¹, C(O)Het⁵, NR⁵²R⁵³ ou Het⁶ ou
R³ et R⁵ dans le groupe de formule (B1) ou (A1) représentent ensemble un pont divalent constitué d'un groupe alkylène ou alcénylène à chaîne droite ayant 2 ou 3 atomes de carbone, un groupe CH₂ dans la chaîne pouvant encore être remplacé par un atome d'oxygène et un groupe CH₂ ou un groupe CH dans la chaîne étant éventuellement substitué par un ou plusieurs radicaux choisis dans le groupe constitué par alkyle en C₁-C₄, halogéno-alkyle(C₁-C₄), alcoxy en C₁-C₄ et halogéno-alcoxy(C₁-C₄),
R²¹ représente un atome d'hydrogène, un groupe alkyle en C₁-C₄, halogénoalkyle(C₁-C₄), cycloalkyle en C₃-C₆, halogénocycloalkyle(C₃-C₆) ou un groupe C(O)OR⁵⁴, C(O)NR⁵⁵R⁵⁶, C(O)Het⁷, NR⁵⁷R⁵⁸ ou Het⁸,
R²² représente un atome d'hydrogène, un groupe alkyle en C₁-C₄, halogénoalkyle(C₁-C₄), cycloalkyle en C₃-C₆, halogénocycloalkyle(C₃-C₆), alkyl(C₁-C₄)thio, alkyl(C₁-C₄)sulfonyle, alkyl(C₁-C₄)sulfinyle ou un groupe de formule C(O)OR⁵⁹, C(O)NR⁶⁰R⁶¹, C(O)Het⁹, NR⁶²R⁶³ ou Het¹⁰,
R²³ et R²⁹ représentent chacun indépendamment l'un de l'autre un atome d'hydrogène, un groupe alkyle en C₁-C₄, halogénoalkyle(C₁-C₄), cycloalkyle en C₃-C₆, halogénocycloalkyle(C₃-C₆), alcoxy en C₁-C₄ ou halogénoalcoxy(C₁-C₄),
R²⁴ et R³⁰ représentent chacun indépendamment l'un de l'autre un atome d'hydrogène ou d'halogène, un groupe cyano, alkyle en C₁-C₄, halogéno-alkyle(C₁-C₄), cycloalkyle en C₃-C₆, halogéno-cycloalkyle(C₃-C₆), alcoxy en C₁-C₄ ou halogéno-alcoxy(C₁-C₄),
R²⁵, R²⁶, R³¹ et R³² représentent chacun indépendamment les uns des autres un atome d'hydrogène, un groupe alkyle en C₁-C₄, halogénoalkyle(C₁-C₄), alcoxy en C₁-C₄, halogénoalcoxy(C₁-C₄), alcoxy(C₁-C₄)-alkyle(C₁-C₄), halogénoalcoxy(C₁-C₄)-alkyle(C₁-C₄), cycloalkyle en C₃-C₆, halogénocycloalkyle(C₃-C₆), cycloalcoxy(C₃-C₆), alcényle en C₂-C₆, halogéno-alcényle(C₂-C₆), alcynyle en C₂-C₆, halogéno-alcynyle(C₂-C₆), alkyl(C₁-C₄)thio, alkyl(C₁-C₄)-sulfonyle, alkyl(C₁-C₄)sulfinyle, NR⁶⁴R⁶⁵ ou Het¹¹,
R²⁷ représente un atome d'hydrogène, un groupe alkyle en C₁-C₆, halogénoalkyle(C₁-C₄), cycloalkyle en C₃-C₆ ou halogénocycloalkyle(C₃-C₆),
R²⁸ représente un atome d'hydrogène, un groupe alkyle en C₁-C₆, halogénoalkyle(C₁-C₄) ou un radical de formule C(O)OR⁶⁶,
R³³, R³⁴, R³⁷ et R³⁸ représentent chacun indépendamment les uns des autres un groupe alkyle en C₁-C₈, cycloalkyle en C₃-C₆, phényle, les trois radicaux nommés en dernier étant chacun indépendamment les uns des autres non substitués ou substitués par un ou plusieurs radicaux choisis dans le groupe constitué par halogéno, cyano, nitro, hydroxy, alcoxy en C₁-C₄, alkyl(C₁-C₄)thio et, dans le cas d'un radical cycloalkyle ou phényle en tant que radical de base, également par alkyle en C₁-C₄ et halogénoalkyle(C₁-C₄), ou un groupe de formule -C(O)R⁶⁷, -C(O)OR⁶⁸, -C(O)NR⁶⁹R⁷⁰, -C(O)Het¹² ou -SO₂R⁷¹,
R³⁵ et R³⁶ sont chacun, indépendamment l'un de l'autre, définis comme R³,
R³⁹, R⁴⁴, R⁴⁹, R⁵⁴, R⁵⁹ et R⁶⁶ représentent chacun indépendamment les uns des autres un atome d'hydrogène, un groupe alkyle en C₁-C₄, halogénoalkyle(C₁-C₄), cycloalkyle en C₃-C₆, halogénocycloalkyle(C₃-C₆), alcényle en C₂-C₄, halogénoalcényle(C₂-C₄), alcynyle en C₂-C₄ ou ledit groupe M,
R⁴⁰, R⁴¹, R⁴², R⁴³, R⁴⁵, R⁴⁶, R⁴⁷, R⁴⁸, R⁵⁰, R⁵¹, R⁵², R⁵³, R⁵⁵,R⁵⁶, R⁵⁷, R⁵⁸, R⁶⁰, R⁶¹, R⁶², R⁶³, R⁶⁴, R⁶⁵, R⁶⁹ et R⁷⁰ représentent chacun indépendamment les uns des autres un atome d'hydrogène, un groupe alkyle en C₁-C₆, alcényle en C₂-C₆ ou alcynyle en C₂-C₆, chacun des 3 radicaux nommés en dernier étant chacun indépendamment non substitué ou substitué par un ou plusieurs radicaux choisis dans le groupe constitué par halogéno, nitro, cyano et phényle qui est éventuellement substitué, ou un groupe cycloalkyle en C₃-C₆ ou phényle, chacun des 2 radicaux nommés en dernier étant chacun indépendamment l'un de l'autre non substitué ou substitué par un ou plusieurs radicaux choisis dans le groupe constitué par halogéno, nitro, cyano, alkyle en C₁-C₄, halogénoalkyle(C₁-C₄), phényle et benzyle, chacun des 2 radicaux nommés en dernier étant éventuellement substitué,
R⁶⁷ représente un atome d'hydrogène, un groupe alkyle en C₁-C₈ ou halogénoalkyle(C₁-C₈), chacun des 2 radicaux nommés en dernier étant indépendamment l'un de l'autre interrompu dans le fragment alkyle par un atome d'oxygène ou de soufre, ou un groupe phényle ou benzyle, chacun des 2 radicaux nommés en dernier étant indépendamment l'un de l'autre non substitué ou substitué par un ou plusieurs radicaux choisis dans le groupe constitué par halogéno, cyano, alkyle en C₁-C₄, alcényle en C₂-C₄, alcynyle en C₂-C₄, cycloalkyle en C₃-C₆, halogénoalkyle(C₁-C₄) et halogénocycloalkyle(C₃-C₆),
R⁶⁸ représente un groupe alkyle en C₁-C₈, halogéno-alkyle(C₁-C₈), cycloalkyle en C₃-C₆, halogéno-cycloalkyle (C₃-C₆), alcényle en C₂-C₈, halogéno-alcényle(C₂-C₈) ou alcynyle en C₂-C₄,
R⁷¹ représente un groupe alkyle en C₁-C₆, halogénoalkyle(C₁-C₆) ou phényle ou benzyle, chacun des 2 radicaux nommés en dernier étant indépendamment l'un de l'autre non substitué ou substitué par un ou plusieurs radicaux choisis dans le groupe constitué par halogéno, cyano, alkyle en C₁-C₄, alcényle en C₂-C₄, alcynyle en C₂-C₄, cycloalkyle en C₃-C₆, halogénoalkyle (C₁-C₄) et halogénocycloalkyle(C₃-C₆),
Het¹, Het², Het³, Het⁴, Het⁵, Het⁶, Het⁷, Het⁸, Het⁹,Het¹⁰, Het¹¹ et Het¹² représentent chacun indépendamment les uns des autres un radical saturé ou partiellement insaturé d'un hétérocycle ayant 3 à 9 atomes formant le cycle et comportant au moins un atome d'azote en tant qu'hétéroatome formant le cycle en position 1 du cycle et éventuellement 1, 2 ou 3 autres hétéroatomes formant le cycle, choisis dans le groupe constitué par N, 0 et S, le radical de l'hétérocycle étant lié au niveau de l'atome d'azote en position 1 du cycle à la partie restante de la molécule du composé de formule (I) et l'hétérocycle étant non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo, et
m, n représentent chacun indépendamment l'un de l'autre 0, 1, 2, 3, 4 ou 5.

2. Composés ou leurs sels selon la revendication 1, **caractérisés en ce que**
(R¹)ₘ représente m substituants R¹,
R¹, lorsque m = 1, ou chacun des substituants R¹, lorsque m est plus grand que 1, représentant chaque fois indépendamment un atome d'halogène, un groupe cyano, nitro, hydroxy, alkyle en C₁-C₆, alcényle en C₂-C₄, alcynyle en C₂-C₄, alcoxy en C₁-C₆, alkyl(C₁-C₆)thio, alkyl(C₁-C₆)sulfinyle, alkyl(C₁-C₆)sulfonyle, halogénoalkyle(C₁-C₄), halogénoalcoxy(C₁-C₄), halogénoalkyl(C₁-C₄)thio, halogénoalkyl(C₁-C₄)sulfinyle, halogénoalkyl(C₁-C₄)-sulfonyle, halogénoalcényle(C₂-C₄), halogéno-alcynyle(C₂-C₄), alcoxy(C₁-C₄)-alkyle(C₁-C₄), halogénoalcoxy(C₁-C₄)-alkyle(C₁-C₄), cycloalkyle en C₃-C₆ qui est éventuellement substitué par un ou plusieurs radicaux choisis dans le groupe constitué par halogéno et alkyle en C₁-C₄, un groupe cycloalcoxy en C₃-C₆ qui est éventuellement substitué par un ou plusieurs radicaux choisis dans le groupe constitué par halogéno et alkyle en C₁-C₄, ou un radical de formule C(O)OR³⁹, C(O)NR⁴⁰R⁴¹, C(O)-Het¹, NR⁴²R⁴³ ou Het²
ou deux groupes R¹ qui se trouvent respectivement en position ortho sur le cycle représentant ensemble un groupe de formule -Z¹-A*-Z², dans laquelle
A* représente un groupe alkylène ayant de 1 à 4 atomes de carbone, qui est éventuellement substitué par un ou plusieurs radicaux choisis dans le groupe constitué par halogéno, alkyle en C₁-C₄, halogéno-alkyle(C₁-C₄), alcoxy en C₁-C₄ et halogéno-alcoxy(C₁-C₄),
Z¹ représente une liaison directe, 0 ou S et
Z² représente une liaison directe, O ou S,
le groupe -Z¹-A*-Z² formant conjointement avec les atomes de carbone du cycle phényle qui sont liés au groupe un cycle pentagonal ou hexagonal condensé,
R³⁹ représente un atome d'hydrogène, un groupe alkyle en C₁-C₄, halogénoalkyle(C₁-C₄), cycloalkyle en C₃-C₆, halogénocycloalkyle(C₃-C₆), alcényle en C₂-C₄, halogénoalcényle(C₂-C₄), alcynyle en C₂-C₄ ou l'équivalent d'un cation,
R⁴⁰, R⁴¹, R⁴², R⁴³ représentent chacun indépendamment les uns des autres un atome d'hydrogène ou un groupe alkyle en C₁-C₄ qui est non substitué ou substitué par un ou plusieurs radicaux choisis dans le groupe constitué par halogéno, nitro, cyano et phényle, ou
un groupe cycloalkyle en C₃-C₆ ou phényle, chacun des 2 radicaux nommés en dernier étant chacun indépendamment l'un de l'autre non substitué ou substitué par un ou plusieurs radicaux choisis dans le groupe constitué par halogéno, alkyle en C₁-C₄, halogénoalkyle(C₁-C₄), phényle et benzyle,
Het¹ et Het² représentent chacun indépendamment l'un de l'autre un radical saturé ou partiellement insaturé d'un hétérocycle ayant 3 à 6 atomes formant le cycle et comportant au moins un atome d'azote en tant qu'hétéroatome formant le cycle en position 1 du cycle et éventuellement 1, 2 ou 3 autres hétéroatomes formant le cycle, choisis dans le groupe constitué par N, 0 et S, le radical de l'hétérocycle étant lié au niveau de l'atome d'azote en position 1 du cycle à la partie restante de la molécule du composé de formule (I) et l'hétérocycle étant non substitué ou substitué par un ou plusieurs radicaux choisis dans le groupe constitué par halogéno, alkyle en C₁-C₄, halogénoalkyle(C₁-C₄) et oxo, et
m représente 0, 1, 2, 3, 4 ou 5.

3. Composés ou leurs sels selon la revendication 1 ou 2, **caractérisés en ce que**
(R²) ₙ représente n substituants R²,
R², lorsque n = 1, ou chacun des substituants R², lorsque n est plus grand que 1, représentant chaque fois indépendamment un atome d'halogène, un groupe cyano, nitro, hydroxy, alkyle en C₁-C₆, alcényle en C₂-C₄, alcynyle en C₂-C₄, alcoxy en C₁-C₆, alkyl(C₁-C₆)thio, alkyl(C₁-C₆)sulfinyle, alkyl(C₁-C₆)sulfonyle, halogénoalkyle(C₁-C₄), halogénoalcoxy(C₁-C₄), halogénoalkyl(C₁-C₄)thio, halogénoalkyl(C₁-C₄)sulfinyle, halogénoalkyl(C₁-C₄)-sulfonyle, halogénoalcényle(C₂-C₄), halogéno-alcynyle(C₂-C₄), alcoxy(C₁-C₄)-alkyle (C₁-C₄), halogénoalcoxy(C₁-C₄)-alkyle(C₁-C₄), cycloalkyle en C₃-C₆ qui est éventuellement substitué par un ou plusieurs radicaux choisis dans le groupe constitué par halogéno et alkyle en C₁-C₄, un groupe cycloalcoxy en C₃-C₆ qui est éventuellement substitué par un ou plusieurs radicaux choisis dans le groupe constitué par halogéno et alkyle en C₁-C₄, ou un radical de formule C(O)OR⁴⁴, C(O)NR⁴⁵R⁴⁶, C(O)-Het³, NR⁴⁷R⁴⁸ ou Het⁴ ou deux groupes R² qui se trouvent respectivement en position ortho sur le cycle représentant ensemble un groupe de formule -Z³-A**-Z⁴, dans laquelle
A** représente un groupe alkylène ayant de 1 à 4 atomes de carbone, qui est éventuellement substitué par un ou plusieurs radicaux choisis dans le groupe constitué par halogéno, alkyle en C₁-C₄, halogéno-alkyle(C₁-C₄), alcoxy en C₁-C₄ et halogéno-alcoxy(C₁-C₄),
Z³ représente une liaison directe, 0 ou S et
Z⁴ représente une liaison directe, 0 ou S,
le groupe -Z³-A**-Z⁴ formant conjointement avec les atomes de carbone du cycle phényle qui sont liés au groupe un cycle pentagonal ou hexagonal condensé,
R⁴⁴ représente un atome d'hydrogène, un groupe alkyle en C₁-C₄, halogénoalkyle(C₁-C₄), cycloalkyle en C₃-C₆, halogénocycloalkyle(C₃-C₆), alcényle en C₂-C₄, halogénoalcényle(C₂-C₄), alcynyle en C₂-C₄ ou l'équivalent d'un cation,
R⁴⁵, R⁴⁶, R⁴⁷ et R⁴⁸ représentent chacun indépendamment les uns des autres un atome d'hydrogène ou un groupe alkyle en C₁-C₄ qui est non substitué ou substitué par un ou plusieurs radicaux choisis dans le groupe constitué par halogéno, nitro, cyano et phényle, ou un groupe cycloalkyle en C₃-C₆ ou phényle, chacun des 2 radicaux nommés en dernier étant chacun indépendamment l'un de l'autre non substitué ou substitué par un ou plusieurs radicaux choisis dans le groupe constitué par halogéno, alkyle en C₁-C₄, halogénoalkyle(C₁-C₄), phényle et benzyle,
Het³ et Het⁴ représentent chacun indépendamment l'un de l'autre un radical saturé ou partiellement insaturé d'un hétérocycle ayant 3 à 6 atomes formant le cycle et comportant au moins un atome d'azote en tant qu'hétéroatome formant le cycle en position 1 du cycle et éventuellement 1, 2 ou 3 autres hétéroatomes formant le cycle, choisis dans le groupe constitué par N, 0 et S, le radical de l'hétérocycle étant lié au niveau de l'atome d'azote en position 1 du cycle à la partie restante de la molécule du composé de formule (I) et l'hétérocycle étant non substitué ou substitué par un ou plusieurs radicaux choisis dans le groupe constitué par halogéno, alkyle en C₁-C₄, halogénoalkyle(C₁-C₄) et oxo, et
n représente 0, 1, 2, 3, 4 ou 5.

4. Composés ou leurs sels selon l'une quelconque des revendications 1 à 3, **caractérisés en ce que**
(R¹)ₘ représente m substituants R¹,
R¹, lorsque m = 1, ou chacun des substituants R¹, lorsque m est plus grand que 1, représentant chaque fois indépendamment un atome d'halogène, un groupe cyano, nitro, hydroxy, alkyle en C₁-C₄, alcoxy en C₁-C₄, alkyl(C₁-C₄)thio, alkyl(C₁-C₄)-sulfinyle, alkyl(C₁-C₄)sulfonyle, halogéno-alkyle(C₁-C₄), halogénoalcoxy(C₁-C₄), halogéno-alkyl(C₁-C₄)thio, halogénoalkyl(C₁-C₄)sulfinyle, halogénoalkyl(C₁-C₄)sulfonyle, alcoxy(C₁-C₄)-alkyle(C₁-C₄), cycloalkyle en C₃-C₆, ou un radical de formule C(O)OR³⁹, C(O)NR⁴⁰R⁴¹ , C(O)-Het¹, NR⁴²R⁴³ ou Het², ou deux groupes R¹ qui se trouvent respectivement en position ortho sur le cycle représentant ensemble un groupe de formule -Z¹-A*-Z², dans laquelle
A* représente un groupe alkylène ayant de 1 à 4 atomes de carbone, qui est éventuellement substitué par un ou plusieurs radicaux choisis dans le groupe constitué par halogéno, alkyle en C₁-C₄, halogéno-alkyle(C₁-C₄), alcoxy en C₁-C₄ et halogéno-alcoxy(C₁-C₄),
Z¹ représente une liaison directe, 0 ou S et
Z² représente une liaison directe, 0 ou S,
le groupe -Z¹-A*-Z² formant conjointement avec les atomes de carbone du cycle phényle qui sont liés au groupe un cycle pentagonal ou hexagonal condensé,
R³⁹ représente un atome d'hydrogène, un groupe alkyle en C₁-C₄ ou l'équivalent d'un cation,
R⁴⁰, R⁴¹, R⁴² et R⁴³ représentent chacun indépendamment les uns des autres un atome d'hydrogène, un groupe alkyle en C₁-C₄, halogénoalkyle(C₁-C₄), benzyle, cycloalkyle en C₃-C₆ ou phényle, en particulier un atome d'hydrogène, le groupe méthyle ou éthyle,
Het¹ et Het² représentent chacun indépendamment l'un de l'autre le groupe morpholino, pipéridino ou pyrrolidino,
m représente 0, 1, 2, 3, 4 ou 5,
(R²)ₙ représente n substituants R²,
R², lorsque n = 1, ou chacun des substituants R², lorsque n est plus grand que 1, représentant chaque fois indépendamment un atome d'halogène, un groupe cyano, nitro, hydroxy, alkyle en C₁-C₄, alcoxy en C₁-C₄, alkyl(C₁-C₄)thio, alkyl(C₁-C₄)-sulfinyle, alkyl(C₁-C₄)sulfonyle, halogéno-alkyle(C₁-C₄), halogénoalcoxy(C₁-C₄), halogéno-alkyl(C₁-C₄)thio, halogénoalkyl(C₁-C₄)sulfinyle, halogénoalkyl(C₁-C₄)sulfonyle, alcoxy(C₁-C₄)-alkyle(C₁-C₄), cycloalkyle en C₃-C₆, ou un radical de formule C(O)OR⁴⁴, C(O)NR⁴⁵R⁴⁶, C(O)-Het³, NR⁴⁷R⁴⁸ ou Het⁴,
ou deux groupes R² qui se trouvent respectivement en position ortho sur le cycle représentant ensemble un groupe de formule -Z³-A**-Z⁴, dans laquelle
A** représente un groupe alkylène ayant de 1 à 4 atomes de carbone, qui est éventuellement substitué par un ou plusieurs radicaux choisis dans le groupe constitué par halogéno, alkyle en C₁-C₄, halogéno-alkyle(C₁-C₄), alcoxy en C₁-C₄ et halogéno-alcoxy(C₁-C₄),
Z³ représente une liaison directe, 0 ou S et
Z⁴ représente une liaison directe, 0 ou S,
le groupe -Z³-A**-Z⁴ formant conjointement avec les atomes de carbone du cycle phényle qui sont liés au groupe un cycle pentagonal ou hexagonal condensé,
R⁴⁴ représente un atome d'hydrogène, un groupe alkyle en C₁-C₄ ou l'équivalent d'un cation,
R⁴⁵, R⁴⁶, R⁴⁷, R⁴⁸ représentent chacun indépendamment les uns des autres un atome d'hydrogène ou un groupe alkyle en C₁-C₄, halogénoalkyle(C₁-C₄), benzyle, cycloalkyle en C₃-C₆ ou phényle,
Het³ et Het⁴ représentent chacun indépendamment l'un de l'autre le groupe morpholino, pipéridino ou pyrrolidino et
n représente 0, 1, 2, 3, 4 ou 5.

5. Composés ou leurs sels selon l'une quelconque des revendications 1 à 4, **caractérisés en ce que**
L représente un radical de formule 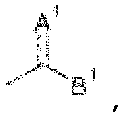 dans laquelle A¹ et B¹ sont tels que définis dans la formule (I).

6. Composés ou leurs sels selon la revendication 5, **caractérisés en ce que**
A¹ représente un atome d'oxygène, de soufre ou =N-R^{A},
B¹ représente un radical de formules (B1) à (B14),
R^{A} représente un atome d'hydrogène, un groupe hydroxy, alcoxy en C₁-C₄, alkyle en C₁-C₄, halogénoalkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), ou un groupe benzyle qui dans le fragment phényle est non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), ou un groupe -NR*R**, R*, R** représentant chacun indépendamment l'un de l'autre H, un groupe alkyle en C₁-C₄, alcényle en C₂-C₄, alcynyle en C₂-C₄, alcoxy(C₁-C₄)-alkyle (C₁-C₄), alcanoyle en C₁-C₄, [halogénoalkyl(C₁-C₄)]-carbonyle, [alcoxy(C₁-C₄)]-carbonyle, [halogénoalcoxy(C₁-C₄)]-carbonyle, cycloalkyle en C₃-C₆, cycloalkyl(C₃-C₆)-alkyle(C₁-C₄), phényle, phényl-alkyle(C₁-C₄), chacun des 4 radicaux nommés en dernier étant dans le cycle non substitué ou substitué par un ou plusieurs radicaux choisis dans le groupe constitué par halogéno, alkyle en C₁-C₄, halogénoalkyle(C₁-C₄), alcoxy en C₁-C₄ ou halogénoalcoxy(C₁-C₄) ou, dans le cas du radical cycloalkyle également oxo, ou
R* et R** représentent ensemble avec l'atome d'azote un hétérocycle à 3 à 6 chaînons, qui en plus de l'atome d'azote peut contenir un ou deux autres hétéroatomes formant le cycle, choisis dans le groupe constitué par N, 0 et S et qui est non substitué ou substitué par un ou plusieurs radicaux choisis dans le groupe constitué par alkyle en C₁-C₄, halogénoalkyle(C₁-C₄) et oxo,
(R¹)ₘ, (R²)ₙ, R³, R⁴, R⁵, R⁶, R⁷, R⁸, R⁹, R¹⁰, R¹¹, R¹², R¹³,R¹⁴, R¹⁵, R¹⁶, R¹⁷, R¹⁸, R¹⁹, R²⁰, R²¹, R²², R²³, R²⁴,R²⁵, R²⁶, R²⁷, R²⁸, R²⁹, R³⁰, R³¹ et R³² ont les significations indiquées plus haut,
W¹ et W⁶ représentent chacun indépendamment l'un de l'autre le groupe divalent oxygène, soufre ou un groupe de formule NH, N-[alkyle(C₁-C₄)], C=O, S=O, SO₂ ou CR³⁵R³⁶,
W², W³, W⁴, W⁵ et W⁷ représentent chacun indépendamment les uns des autres le groupe divalent de formule 0, S, NH, N-[alkyle(C₁-C₄)], C=O, S=O ou SO₂,
Q¹, Q², Q³, Q⁴, Q⁵, Q⁶, Q⁷, Q⁸ et Q⁹ représentent chacun indépendamment les uns des autres le groupe hydroxy, thio, un atome d'halogène ou un groupe de formule OR³⁷, SR³⁸, SM ou OM,
M représente l'équivalent d'un cation,
R³⁵ et R³⁶ sont chacun indépendamment l'un de l'autre définis comme R³ ou de préférence représentent indépendamment l'un de l'autre H, un groupe alkyle en C₁-C₄ ou halogénoalkyle(C₁-C₄), de préférence H ou un groupe alkyle en C₁-C₂, en particulier méthyle ou éthyle et
R³⁷, R³⁸, R⁶⁶, R⁶⁷, R⁶⁸, R⁶⁹, R⁷⁰, R⁷¹, Het¹² et M ont les significations indiquées plus haut.

7. Composés ou leurs sels selon la revendication 5 ou 6, **caractérisés en ce que**
R³⁷ et R³⁸ représentent chacun indépendamment les uns des autres un groupe alkyle en C₁-C₆, halogénoalkyle(C₁-C₆), alcoxy(C₁-C₄)-alkyle(C₁-C₄), cycloalkyle en C₃-C₆ qui est non substitué ou substitué par un ou plusieurs radicaux choisis dans le groupe constitué par halogéno, alkyle en C₁-C₄ et alcoxy en C₁-C₄, phényle qui est non substitué ou substitué par un ou plusieurs radicaux choisis dans le groupe constitué par halogéno, cyano, hydroxy, nitro, alkyle en C₁-C₄, halogénoalkyle(C₁-C₄) et alcoxy en C₁-C₄, ou un groupe de formule -C(O)R⁶⁷, -C(O)OR⁶⁸, -C(O)NR⁶⁹R⁷⁰, -C(O)Het¹² ou -SO₂R⁷¹,
R⁶⁷ représente un atome d'hydrogène, un groupe alkyle en C₁-C₆, halogénoalkyle(C₁-C₄), phényle ou benzyle, chacun des 2 radicaux nommés en dernier étant indépendamment l'un de l'autre non substitué ou substitué par un ou plusieurs radicaux choisis dans le groupe constitué par halogéno, cyano, alkyle en C₁-C₄ et halogénoalkyle(C₁-C₄),
R⁶⁸ représente un groupe alkyle en C₁-C₆ ou halogéno-alkyle(C₁-C₄),
R⁶⁹ et R⁷⁰ représentent chacun indépendamment l'un de l'autre un atome d'hydrogène, un groupe alkyle en C₁-C₄, benzyle, cycloalkyle en C₃-C₆ ou phényle, chacun des 2 radicaux nommés en dernier étant indépendamment l'un de l'autre non substitué ou substitué par un ou plusieurs radicaux choisis dans le groupe constitué par halogéno, nitro, cyano, alkyle en C₁-C₄, halogénoalkyle(C₁-C₄),
R⁷¹ représente un groupe alkyle en C₁-C₄, halogénoalkyle(C₁-C₄), phényle ou benzyle, chacun des 2 radicaux nommés en dernier étant indépendamment l'un de l'autre non substitué ou substitué par un ou plusieurs radicaux choisis dans le groupe constitué par halogéno, cyano, alkyle en C₁-C₄, alcényle en C₂-C₄ et halogénoalkyle(C₁-C₄),
Het¹² représente un radical saturé ou partiellement insaturé d'un hétérocycle ayant 3 à 6 atomes formant le cycle et comportant au moins un atome d'azote en tant qu'hétéroatome formant le cycle en position 1 du cycle et éventuellement 1, 2 ou 3 autres hétéroatomes formant le cycle, choisis dans le groupe constitué par N, 0 et S, le radical de l'hétérocycle étant lié au niveau de l'atome d'azote en position 1 du cycle à la partie restante de la molécule du composé de formule (I) et l'hétérocycle étant non substitué ou substitué par un ou plusieurs radicaux choisis dans le groupe constitué par halogéno, alkyle en C₁-C₄, halogénoalkyle(C₁-C₄), alcoxy en C₁-C₄, halogéno-alcoxy(C₁-C₄), alkyl(C₁-C₄)thio et oxo,
M représente un équivalent d'un ion métallique, un ion ammonium qui est éventuellement substitué par 1 à 4 radicaux identiques ou différents, choisis parmi les radicaux choisis dans le groupe constitué par alkyle en C₁-C₄, cycloalkyle en C₃-C₆, phényle, cycloalkyl(C₃-C₆)-alkyle(C₁-C₄) et phényl-alkyle(C₁-C₄), ou un ion sulfonium tertiaire qui est substitué par 3 radicaux identiques ou différents, choisis dans le groupe constitué par alkyle en C₁-C₄, cycloalkyle en C₃-C₆, phényle, cycloalkyl(C₃-C₆)-alkyle(C₁-C₄) et phényl-alkyle(C₁-C₄).

8. Composés ou leurs sels selon l'une quelconque des revendications 1 à 7, **caractérisés en ce que** sont préférés également des composés (I) dans lesquels
L représente un radical de formule 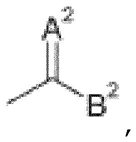 dans laquelle A² et B² ont les significations indiquées plus haut.

9. Composés ou leurs sels selon la revendication 8, **caractérisés en ce que**
A² représente un radical desdites formules (A1 à (A12),
B² représente un groupe hydroxy, thio, un atome d'halogène ou un groupe de formule OR³³, OM, SR³⁴, SM ou -NR^{B}R^{c},
R^{B} et R^{c} représentent indépendamment l'un de l'autre un atome d'hydrogène, un groupe hydroxy, alcoxy en C₁-C₄, alkyle en C₁-C₄, halogénoalkyle(C₁-C₄), phényle qui est non substitué ou substitué par un ou plusieurs radicaux choisis dans le groupe constitué par halogéno, alkyle en C₁-C₄, halogénoalkyle(C₁-C₄), alcoxy en C₁-C₄, halogéno-alcoxy(C₁-C₄), ou benzyle qui dans le fragment phényle est non substitué ou substitué par un ou plusieurs radicaux choisis dans le groupe constitué par halogéno, alkyle en C₁-C₄, halogénoalkyle(C₁-C₄), alcoxy en C₁-C₄, halogéno-alcoxy(C₁-C₄), ou un groupe -NR*R**, R*, R** représentant chacun indépendamment l'un de l'autre H, un groupe alkyle en C₁-C₄, alcényle en C₂-C₄, alcynyle en C₂-C₄, alcoxy(C₁-C₄)-alkyle(C₁-C₄), alcanoyle en C₁-C₄, [halogénoalkyl(C₁-C₄)]-carbonyle, [alcoxy(C₁-C₄)]-carbonyle, [(halogéno-alcoxy(C₁-C₄)]-carbonyle, cycloalkyle en C₃-C₆, cycloalkyl(C₃-C₆)-alkyle(C₁-C₄), phényle, phényl-alkyle(C₁-C₄), chacun des 4 radicaux nommés en dernier étant dans le cycle non substitué ou substitué par un ou plusieurs radicaux choisis dans le groupe constitué par halogéno, alkyle en C₁-C₄, halogénoalkyle(C₁-C₄), alcoxy en C₁-C₄ ou halogénoalcoxy(C₁-C₄) ou dans le cas du groupe cycloalkyle également oxo ou R* et R** représentent ensemble avec l'atome d'azote un hétérocycle à 3 à 6 chaînons et de préférence saturé, qui peut contenir en plus de l'atome d'azote un ou deux autres hétéroatomes formant le cycle, choisis dans le groupe constitué par N, 0 et S et qui est non substitué ou substitué par un ou plusieurs radicaux choisis dans le groupe constitué par alkyle en C₁-C₄, halogénoalkyle(C₁-C₄) et oxo,
(R¹)ₘ, (R²)ₙ, R³, R⁴, R⁵, R⁶, R⁷, R⁸, R⁹, R¹⁰, R¹¹, R¹², R¹³,R¹⁴, R¹⁵, R¹⁶, R¹⁷, R¹⁸, R¹⁹, R²⁰, R²¹, R²², R²³, R²⁴, R²⁵et R²⁶ ont les significations indiquées plus haut,
W¹ et W⁶ représentent chacun indépendamment l'un de l'autre le groupe divalent oxygène, soufre ou un groupe de formule NH, N-[alkyle(C₁-C₄)], C=O, S=O, SO₂ ou CR³⁵R³⁶,
W², W³, W⁴, W⁵ et W⁷ représentent chacun indépendamment les uns des autres le groupe divalent de formule 0, S, NH, N-[alkyle(C₁-C₄)], C=O, S=O ou SO₂,
R³³ et R³⁴ représentent chacun indépendamment l'un de l'autre un groupe alkyle en C₁-C₄, halogéno-alkyle(C₁-C₄), alcoxy(C₁-C₄)-alkyle(C₁-C₄), cycloalkyle en C₃-C₆ qui est non substitué ou substitué par un ou plusieurs radicaux choisis dans le groupe constitué par halogéno, alkyle en C₁-C₄ et alcoxy en C₁-C₄, un groupe phényle qui est non substitué ou substitué par un ou plusieurs radicaux choisis dans le groupe constitué par halogéno, cyano, hydroxy, nitro, alkyle en C₁-C₄, halogénoalkyle(C₁-C₄) et alcoxy en C₁-C₄, ou un groupe de formule -C(O)R⁶⁷, -C(O)OR⁶⁸, -C(O)NR⁶⁹R⁷⁰, -C(O)Het¹² ou -SO₂R⁷¹,
R³⁵ et R³⁶ sont chacun, indépendamment l'un de l'autre, définis comme R³,
R⁶⁷ représente un atome d'hydrogène, un groupe alkyle en C₁-C₆, halogénoalkyle(C₁-C₄), phényle ou benzyle, chacun des 2 radicaux nommés en dernier étant indépendamment l'un de l'autre non substitué ou substitué par un ou plusieurs radicaux choisis dans le groupe constitué par halogéno, cyano, alkyle en C₁-C₄ et halogénoalkyle(C₁-C₄), en particulier représente H ou un groupe alkyle en C₁-C₄,
R⁶⁸ représente un groupe alkyle en C₁-C₆ ou halogéno-alkyle(C₁-C₄) , en particulier alkyle en C₁-C₄,
R⁶⁹ et R⁷⁰ représentent chacun indépendamment l'un de l'autre un atome d'hydrogène, un groupe alkyle en C₁-C₄, benzyle, cycloalkyle en C₃-C₆ ou phényle, chacun des 2 radicaux nommés en dernier étant indépendamment l'un de l'autre non substitué ou substitué par un ou plusieurs radicaux choisis dans le groupe constitué par halogéno, nitro, cyano, alkyle en C₁-C₄, halogénoalkyle(C₁-C₄), ou en particulier représentent chacun indépendamment l'un de l'autre H ou un groupe alkyle en C₁-C₃,
R⁷¹ représente un groupe alkyle en C₁-C₄, halogénoalkyle(C₁-C₄), phényle ou benzyle, chacun des 2 radicaux nommés en dernier étant indépendamment l'un de l'autre non substitué ou substitué par un ou plusieurs radicaux choisis dans le groupe constitué par halogéno, cyano, alkyle en C₁-C₄, alcényle en C₂-C₄ et halogénoalkyle(C₁-C₄), en particulier représente un groupe alkyle en C₁-C₄, halogénoalkyle(C₁-C₄), benzyle ou phényle qui est non substitué ou substitué par un ou plusieurs radicaux choisis dans le groupe constitué par halogéno, alkyle en C₁-C₄ et halogénoalkyle en C₁-C₄,
Het¹² représente un radical saturé ou partiellement insaturé d'un hétérocycle ayant 3 à 6 atomes formant le cycle et comportant au moins un atome d'azote en tant qu'hétéroatome formant le cycle en position 1 du cycle et éventuellement 1, 2 ou 3 autres hétéroatomes formant le cycle, choisis dans le groupe constitué par N, 0 et S, le radical de l'hétérocycle étant lié au niveau de l'atome d'azote en position 1 du cycle à la partie restante de la molécule du composé de formule (I) et l'hétérocycle étant non substitué ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄), alkyl(C₁-C₄)thio et oxo.

10. Composés ou leurs sels selon l'une quelconque des revendications 1 à 9, **caractérisés en ce que** le composé se trouve sous forme de thréo-racémate enrichi.

11. Composés ou leurs sels selon l'une quelconque des revendications 1 à 9, **caractérisés en ce que** le composé se trouve sous forme d'énantiomère 2-thréo enrichi ayant la configuration stéréochimique [2R,3R].

12. Procédé pour la préparation d'un composé de formule (I), tel qu'il est défini dans l'une quelconque des revendications 1 à 11, ou d'un sel d'un tel composé, **caractérisé en ce que**
(a) pour la préparation de composés de formule (I) et de leurs sels, dans lesquels L représente un radical de formule C(=A¹)-B¹, B¹ représente un radical de formule (B1) à (B9) et (B11) à (B14) et Q¹ à Q⁹ représentent chacun OH, SH, SM ou OM et les autres radicaux sont tels que définis dans le composé de formule (I) respectif à préparer, on fait réagir un composé de formule (II), 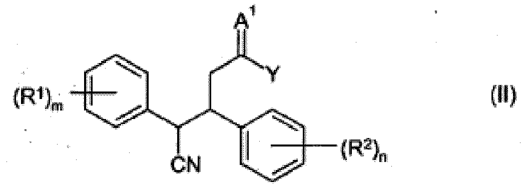 dans laquelle A¹, (R¹)ₘ et (R²)ₙ sont tels que définis dans la formule (I) et Y représente un groupe partant, de préférence un atome d'halogène, en particulier un atome de chlore,
avec un composé de formule H-B¹, dans laquelle B¹ est tel que défini dans le radical L et Q¹ à Q⁹ sont définis chacun comme dans le composé de formule (I) à préparer, pour obtenir directement le composé (I) ou le produit d'O- ou respectivement de S-alkylation en tant que produit intermédiaire et le produit intermédiaire de l'O- ou respectivement de la S-alkylation résultant est transposé en le composé de formule (I) en tant que produit de C-alkylation ou
(b) pour la préparation de composés de formule (I) et de leurs sels, dans lesquels L représente un radical de formule -C(=A¹)-B¹, B¹ représente un radical de formule (B1) à (B7) et (B11) à (B14) et Q¹ à Q⁹ dans le groupe B¹ représentent chacun un groupe de formule SH ou SM et les autres radicaux sont tels que définis dans le composé de formule (I) respectif à préparer,
on fait réagir avec un réactif soufré un composé de formule (I), dans lequel L représente un radical de formule -C(=A¹)-B¹ et dans cette formule A¹ est tel que défini dans la formule (I) et B¹ est défini comme pour le radical B¹ dans la formule (I), mis à part que Q¹ à Q⁹ dans le groupe B¹ représentent chacun le groupe OH ou OM, avec un agent d'halogénation, pour obtenir le composé (I) dans lequel Q¹ à Q⁹ dans le groupe B¹ représentent chacun un atome d'halogène, et éventuellement on sépare ou isole du mélange réactionnel le composé de formule (I) recherché ou
(c) pour la préparation de composés de formule (I) et de leurs sels, dans lesquels L représente un radical de formule -C(=A¹)-B¹, B¹ représente un radical de formule (B1) à (B7) et (B11) à (B14) et Q¹ à Q⁹ dans le groupe B¹ représentent chacun un groupe de formule OR³⁷ et les autres radicaux sont tels que définis dans le composé de formule (I) respectif à préparer,
on fait réagir un composé de formule (I), dans lequel L représente un radical de formule -C(=A¹)-B¹ et dans cette formule A¹ est tel que défini dans la formule (I) et B¹ est défini comme pour le radical B¹ dans la formule (I), mis à part que Q¹ à Q⁹ dans le groupe B¹ représentent chacun le groupe OH ou OM, avec un composé de formule Y'-R³⁷, dans laquelle Y' représente un groupe partant et R³⁷ est tel que défini dans la formule (I), et éventuellement on sépare ou isole du mélange réactionnel le composé de formule (I) recherché ou
(d) pour la préparation de composés de formule (I) et de leurs sels, dans lesquels L représente un radical de formule -C(=A¹)-B¹, B¹ représente un radical de formule (B1) à (B7) et (B11) à (B14) et Q¹ à Q⁹ dans le groupe B¹ représentent chacun un groupe de formule SR³⁸ et les autres radicaux sont tels que définis dans le composé de formule (I) respectif à préparer,
on fait réagir un composé de formule (I), dans lequel L représente un radical de formule -C(=A¹)-B¹ et dans cette formule A¹ est tel que défini dans la formule (I) et B¹ est défini comme pour le radical B¹ dans la formule (I), mis à part que Q¹ à Q⁹ dans le groupe B¹ représentent chacun le groupe SH ou SM, avec un composé de formule Y'R³⁸, dans laquelle Y' représente un groupe partant et R³⁸ est tel que défini dans la formule (I), et éventuellement on sépare ou isole du mélange réactionnel le composé de formule (I) recherché ou
(e) pour la préparation de composés de formule (I) et de leurs sels, dans lesquels L représente un radical de formule -C(=A¹)-B¹, B¹ représente un radical de formule (B1) à (B7) et (B11) à (B14) et Q¹ à Q⁹ dans le groupe B¹ représentent chacun un groupe de formule SR³⁸ et les autres radicaux sont tels que définis dans le composé de formule (I) respectif à préparer,
on fait réagir un composé de formule (I), dans lequel L représente un radical de formule -C(=A¹)-B¹ et dans cette formule A¹ est tel que défini dans la formule (I) et B¹ est défini comme pour le radical B¹ dans la formule (I), mis à part que Q¹ à Q⁹ dans le groupe B¹ représentent chacun un atome d'halogène, avec un composé thio de formule H-S-R³⁸ et éventuellement on sépare ou isole du mélange réactionnel le composé de formule (I) recherché ou
(f) pour la préparation de composés de formule (I) et de leurs sels, dans lesquels L représente un radical de formule -C(=A¹)-B¹ et B¹ représente un groupe de formule (B10) [= composé (I-B10)],
on fait réagir un composé de formule (I), dans lequel L représente un radical de formule -C(=A¹)-B*, A¹ est tel que défini dans la formule (I) et B* représente un radical de formule -CH₂-CO-R²⁷, dans laquelle R²⁷ est tel que défini dans la formule (B10) [= composé (I-B)], 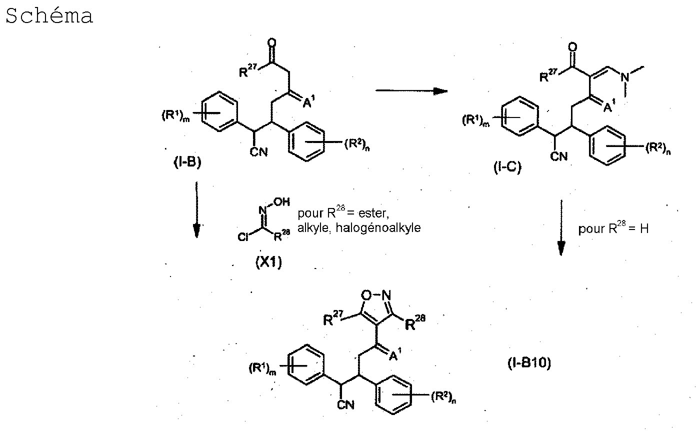 avec du N,N-diméthylformamide-diméthylacétal pour obtenir le composé de formule (I-C) (voir schéma) et on fait réagir le composé (I-C) avec de l'hydroxylamine ou ses sels, avec cyclisation, pour aboutir au composé de formule (I-B10) dans lequel R²⁸ = H, ou on fait réagir le composé (I-B) avec un composé de formule (X1),
HO-N=C(Cl)-R²⁸ (X1)
dans laquelle R²⁸ est tel que défini dans la formule (B10), avec cyclisation, pour aboutir au composé de formule (I-B10), R²⁸ dans la formule (XI) étant tel que défini dans le composé de formule (I-B10) à préparer, hormis R²⁸ = H, ou
(g) pour la préparation de composés de formule (I) et de leurs sels, dans lesquels L représente un radical de formule -C(=A¹)-B¹ et B¹ représente un groupe de formule (B10) [= composé (I-B10)]
on fait réagir un composé de formule (II), 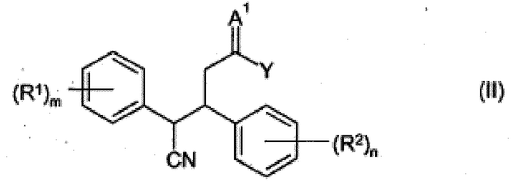 dans laquelle A¹, (R¹)ₘ et (R²)ₙ sont tels que définis dans la formule (I) et Y représente un groupe partant,
avec un composé de formule X-(B10), dans laquelle (B10) est tel que défini dans le groupe B¹ et X représente un groupe partant, éventuellement in présence d'un réactif de couplage, pour aboutir au composé de formule (I) (réaction de couplage croisé)
ou
avec un composé activé de formule X'-(B10), dans laquelle X' représente un métal ou un groupe dérivé de métal, pour aboutir au composé de formule (I),
(h) pour la préparation de composés de formule (I) et de leurs sels, dans lesquels L représente un radical de formule -C(=A²)-B² et B² représente un radical de formule OR³³ ou SR³⁴, on fait réagir un composé de formule (I'), 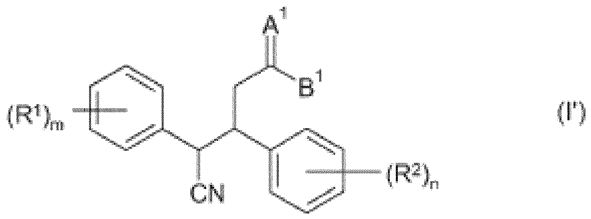 dans laquelle (R¹)ₘ et (R²)ₙ sont tels que définis dans la formule (I), A¹ représente un radical 0 ou S et B¹ représente un radical de formule (B1), (B2), (B3), (B4), (B5), (B6), (B7), (B8), (B9), (B11), (B12), (B13) ou (B14) comme défini plus haut pour les composés (I) dans lesquels L représente un radical -C(=A¹)-B¹, Q¹ à Q⁹ dans les radicaux B¹ représentant chacun OH ou OM,
avec un composé de formule Y'-B², dans laquelle B² est tel que défini dans le radical L et Y' représente un groupe partant, pour aboutir au composé (I) dans lequel B² représente le radical OR³³, lorsque A¹ doit être un atome d'oxygène, ou SR³⁴, lorsque A¹ doit être un atome de soufre, le groupe -C(=A²)- dans le composé (I) représentant le tautomère le plus stable en comparaison du groupe -C-B¹ dans le composé (I') ou un tautomère structuralement fixé, dans lequel le radical -C(=A²)-est en correspondance tautomérique avec le radical =C(-B¹)-, ou
(i) pour la préparation de composés de formule (I) et de leurs sels, dans lesquels L représente un radical de formule -C(=A²)-B² et B² représente un radical de formule -NR^{B}R^{C}, on fait réagir un composé de formule (I'), 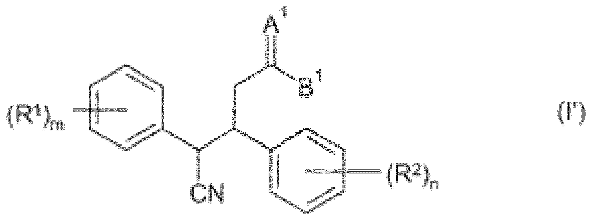 dans laquelle (R¹)ₘ et (R²)ₙ sont tels que définis dans la formule (I), A¹ représente un radical de formule -NR^{B} et B¹ représente un radical de formule (B1), (B2), (B3), (B4), (B5), (B6), (B7), (B8), (B9), (B11), (B12), (B13) ou (B14) comme défini plus haut pour les composés (I) dans lesquels L représente un radical -C(=A¹)-B¹, Q¹ à Q⁹ dans les radicaux B¹ représentant chacun OH ou OM,
avec un composé de formule Y'-R^{C}, dans laquelle R^{C} est tel que défini dans le radical -NR^{B}R^{C} de B² et Y' représente un groupe partant, pour aboutir au composé (I) dans lequel B² représente le radical -NR^{B}R^{C}, le groupe -C(=A²)- dans le composé (I) représentant le tautomère le plus stable en comparaison du groupe -C-B¹ dans le composé (I') ou un tautomère structuralement fixé, dans lequel le radical -C(=A²)- est en correspondance tautomérique avec le radical =C(-B¹)-.

13. Composition herbicide ou régulatrice de croissance végétale, **caractérisée en ce qu'**elle contient un ou plusieurs composés de formule (I) ou sels de tels composés, tels qu'ils sont définis selon l'une quelconque des revendications 1 à 11, et contient des adjuvants de formulation usuels dans la protection des plantes.

14. Procédé pour la lutte contre des plantes nuisibles ou pour la régulation de la croissance de plantes, **caractérisé en ce qu'**on applique sur les plantes, semences de plantes, le sol dans lequel ou sur lequel poussent les plantes, ou l'aire de culture une quantité efficace d'un ou de plusieurs composés de formule (I) ou sels de tels composés, tels qu'ils sont définis selon l'une quelconque des revendications 1 à 11.

15. Procédé selon la revendication 14, **caractérisé en ce qu'**on utilise les composés de formule (I) ou leurs sels pour la lutte sélective contre des pantes nuisibles ou pour la régulation de la croissance dans des cultures de plantes utiles ou ornementales.

16. Procédé selon la revendication 15, **caractérisé en ce que** les plantes cultivées sont des plantes cultivées transgéniques.

17. Utilisation des composés de formule (I) ou de leurs sels selon l'une quelconque des revendications 1 à 11, en tant qu'herbicides ou régulateurs de croissance végétale.

18. Utilisation selon la revendication 17, **caractérisée en ce qu'**on utilise les composés en tant qu'herbicides sélectifs pour la lutte contre des plantes nuisibles ou en tant que régulateurs de la croissance végétale dans des cultures de plantes utiles ou ornementales.
